# EUROPEAN PATENT SPECIFICATION

(11) **EP 4 587 429 B1**
(45) Date of publication and mention of the grant of the patent: **18.02.2026**
(21) Application number: 24782711.6
(22) Date of filing: 12.09.2024
(51) Int. Cl.: C07D 401/04, C07D 401/14, C07D 405/14, C07D 409/14, A61P 25/00, A61K 31/45

(54) **SUBSTITUTED OXOISOINDOLINYL PIPERIDINE-2,6-DIONE COMPOUNDS**
SUBSTITUIERTE OXOISOINDOLINYLPIPERIDIN-2,6-DIONVERBINDUNGEN
COMPOSÉS OXOISOINDOLINYL PIPÉRIDINE-2,6-DIONE SUBSTITUÉS

(30) Priority: 13.09.2023 US 202363582266 P
(43) Date of publication of application: 23.07.2025
(73) Proprietor: Bristol-Myers Squibb Company, Princeton, NJ 08543 (US)
(72) Inventor: FERRANTE, Daniel, Princeton, New Jersey 08543 (US); LU, Zhonghui, Princeton, New Jersey 08543 (US); PARK, Peter Kinam, Princeton, New Jersey 08543 (US); TOKARSKI, John S., Princeton, New Jersey 08543 (US); YADAV, Vipin, Princeton, New Jersey 08543 (US); YANG, Michael G., Princeton, New Jersey 08543 (US); ZHU, Jinyi, Princeton, New Jersey 08543 (US); KEMPSON, James, Princeton, New Jersey 08543 (US); DEGNAN, Andrew P., Princeton, New Jersey 08543 (US); BURRELL, Richard Charles, Princeton, New Jersey 08543 (US)
(74) Representative: Mewburn Ellis LLP
(86) International application number: PCT/US2024/046299
(87) International publication number: WO 2025/059245

(56) References cited:
- WO-A1-2022/152822
- WO-A1-2023/069720

## Description

The present invention generally relates to substituted oxoisoindolinyl piperidine-2,6-dione compounds useful for decreasing the levels of the Cyclin E1 protein. Provided herein are substituted oxoisoindolinyl piperidine-2,6-dione compounds, compositions comprising such compounds, and methods of their use. The invention further pertains to pharmaceutical compositions comprising at least one compound according to the invention that are useful for the treatment of proliferative disorders, such as cancer.

### BACKGROUND OF THE INVENTION

Cyclin E1 is a member of the E-type cyclin family encoded by the CCNE1 gene. Cyclin E1 regulates various cellular processes, including cell cycle progression, DNA replication, histone biosynthesis, and transcriptional regulation. During the G1 phase of the cell cycle, Cyclin E1 binds and activates Cyclin-dependent kinases (CDKs), particularly CDK2, to form an active Cyclin E1/CDK2 complex. This complex phosphorylates RB1 and other RB family proteins, leading to the activation of E2F family transcriptional factor proteins and subsequent progression of the cell cycle (Asghar et al., Nat. Rev. Drug. Dis. 14:130-146 (2015); Otto & Sicinski, Nat. Rev. Can. 17:93-115 (2017)).

Dysregulation of the cell cycle is a hallmark of cancer. Cyclin E1 is frequently overexpressed and amplified in cancers and is associated with cell cycle dysregulation (Witkiewicz et al., Trends in Cancer Vol 8(9) 711-725 (2022)). Amplification of the CCNE1 gene has been linked to poor clinical outcomes in various cancers, including gynecologic cancers (breast, ovarian, endometrial), gastrointestinal cancers (e.g. gastric, bladder), and other cancers of either solid or hematological origins (Keyomarsi et al., NEJM347(20):1566-1575 (2002), Macheret et al., Nature 555:112-116 (2018), Etemadmoghadam et al., PLos One 5(11):e15498 pages 1-14 (2010); Au-Yeung et al., Clin. Can. Res. 23(7):1862-1874 (2017)). Depletion of Cyclin E1 protein is a potential therapeutic strategy for the treatment of such cancers.

Cyclin E1 overexpression has been implicated in resistance to several anti-cancer agents such as CDK4/6 inhibitors, endocrine therapies, Her2-targeting agents, PARP inhibitors and chemotherapeutic agents, suggesting that depletion of Cyclin E1 protein could help overcome or prevent resistance to such agents, either as a single agent or in combination with other therapies (Turner et al. J. Clin. Oncol. 37(14):1169-1178 (2019); Herrera-Abreu et al., Cancer Res. 76(8):2301-2313 (2016); Scaltriti et al., PNAS 108(9):3761-3766 (2011); Freeman-Cook et al., Cancer Cell 39, 1-18 (2021)).

There remains a need for therapies that can target Cyclin E1 directly to decrease the levels of Cyclin E1 protein in cancers. There remains a need for therapies that can selectively decrease the levels of Cyclin E1 protein in cancer cells while minimizing the degradation of GSPT1 (translation termination factor G1 to S phase transition proteins 1) in the normal tissues to maximize therapeutic index and facilitate efficacious responses in the clinic. Reduction of GSPT1 protein in normal human tissues is reportedly associated with dose-limiting and on-target toxicities (Uy et al., Blood 13 November 2019, http://doi.org/10.1182/blood-2019-123966). WO2022152822 relates to methods to predict the responsiveness of cancer patients to GSPT1 negative modulators. WO2023069720 relates to compounds that mediate the degradation of cyclin-dependent kinase 2 (CDK2).

There remains a need for therapies that can decrease the levels of Cyclin E protein.

There remains a need for therapies that can decrease the levels of Cyclin E protein and minimize degradation of GSPT1.

The present invention fills the foregoing need by providing compounds that are useful to decrease the level of Cyclin E and are selective against degradation of GSPT1.

### SUMMARY OF THE INVENTION

The present invention is defined in the appended claims.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention provides substituted oxoisoindolinyl piperidine-2,6-dione compounds of Formula (I), including stereoisomers, tautomers, and salts thereof, which are useful to decrease the levels of the Cyclin E1. The claimed compounds and compositions may be for use in the methods of treatment mentioned herein.

The present invention also provides pharmaceutical compositions comprising a compound of Formula (I), stereoisomers, tautomers, or pharmaceutically acceptable salts thereof; and a pharmaceutically acceptable carrier.

The present invention also provides a method of treating a disease or disorder by decreasing the levels of the Cyclin E1, the method comprising administering to a patient a compound of Formula (I), stereoisomers, tautomers, or pharmaceutically acceptable salts thereof.

The present invention also provides processes and intermediates for making the compounds of Formula (I), stereoisomers, tautomers, or salts thereof.

The present invention also provides the use of the compounds of Formula (I), or stereoisomers, tautomers, or pharmaceutically acceptable salts thereof, for the manufacture of a medicament to decrease Cyclin E protein levels, for the treatment of certain diseases, including cancer.

The compounds of Formula (I) and compositions comprising the compounds of Formula (I) may be used in treating, preventing, or curing various proliferative disorders, such as cancer. Pharmaceutical compositions comprising these compounds are useful in treating, preventing, or slowing the progression of diseases or disorders in a variety of therapeutic areas, such as cancer.

These and other features of the invention will be set forth in expanded form as the disclosure continues.

### BRIEF DESCRIPTION OF THE DRAWINGS

The invention is illustrated by reference to the accompanying drawings described below.
FIG. 1 shows the dose response of Example 35 on the degradation of Cyclin E1 (FIG. 1A), the inhibition of RB1 phosphorylation (FIG. 1B), and the degradation of GSPT1 (FIG. 1C) in HCC1569 cells.
FIG. 2 shows the antitumor activity of Example 35 dosed orally, once daily, for 28 days at 10 mpk, 30 mpk, and 100 mpk against HCC1569 xenografts in mice.

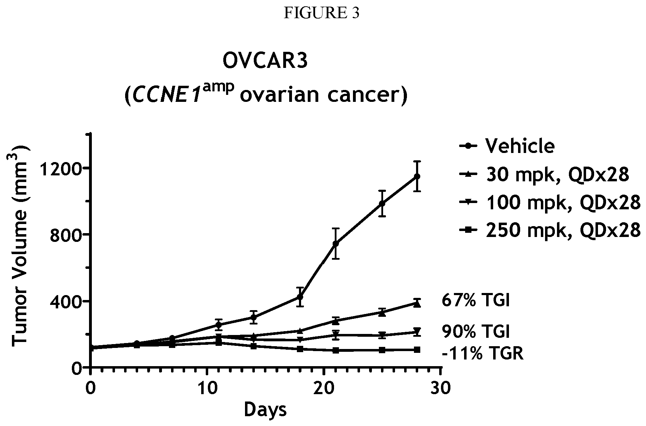

FIG. 3 shows the antitumor activity of Example 35 dosed orally, once daily, for 28 days at 30 mpk, 100 mpk, and 250 mpk against OVCAR3 xenografts in mice.

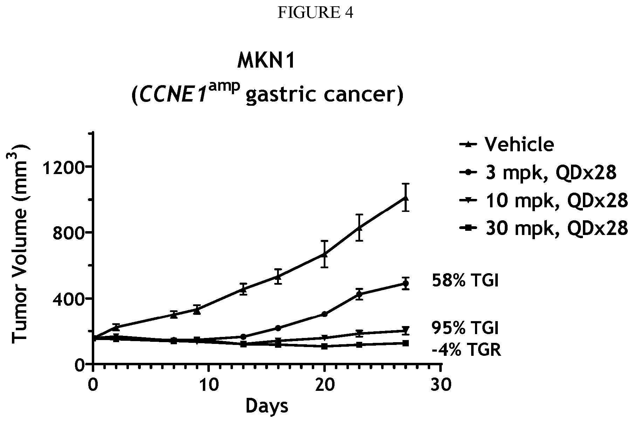

FIG. 4 shows the antitumor activity of Example 35 dosed orally, once daily, for 28 days at 3 mpk, 10 mpk, and 30 mpk against MKN1 xenografts in mice.

### DETAILED DESCRIPTION

Applicants have found substituted oxoisoindolinyl piperidine-2,6-dione compounds that decrease the levels of Cyclin E1 protein. The substituted oxoisoindolinyl piperidine-2,6-dione compounds are believed to facilitate the interaction of Cyclin E1 protein with the corresponding E3 ubiquitin ligase complex (Cullin4-Cereblon, CUL4-CRBN), with concomitant degradation of the Cyclin E1 protein. These compounds are useful for the treatment of certain diseases, including cancer. The compounds are provided to be useful as pharmaceuticals with desirable stability, bioavailability, therapeutic index, and toxicity values that are important to their drugability.

The first aspect of the present invention provides at least one compound of Formula (I): 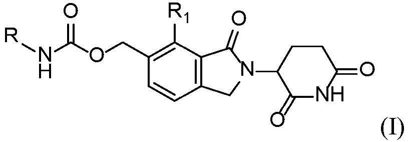 or stereoisomers, tautomers, or salts thereof, wherein:
R is 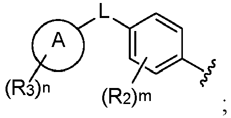
L is -O-, -CH₂-, -C(CH₃)₂-, -CF₂-, -NH-, -N(CH₃)-, or 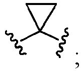
Ring A is phenyl, naphthalenyl, or benzo[b]thiophenyl;
R₁ is -OCH₃, -OCH₂CH₃, -OCH(CH₃)₂, -OCH₂CH(CH₃)₂, -OCHF₂, -OCH₂C(CH₃)₂OH, -OCH₂CH₂OCH₃, -OCH₂C(O)OCH₃, -OCH₂C(O)NH₂, -OCH₂C(O)N(CH₃)₂, -OCH₂C(O)N(CH₃)(CH₂CH₃), -OCH₂CH₂CH₂(phenyl), -OCH₂C(O)(morpholinyl), -O(C₄₋₆ cycloalkyl), -O(hydroxycyclohexyl), -O(oxetanyl), -O(tetrahydrofuranyl), -O(tetrahydropyranyl), -O(oxepanyl), -O(phenyl), -O(pyridinyl), or -O(acetylpiperidinyl);
R₂ is F, Cl, -CH₃, or -OCH₃;
each R₃ is independently F, Cl, Br, -CH₃, -CD₃, -CHF₂, -CF₃, -OCH₃, or cyclopropyl; m is zero or 1; and
n is zero, 1, 2, or 3.

The second aspect of the present invention provides at least one compound of Formula (I): 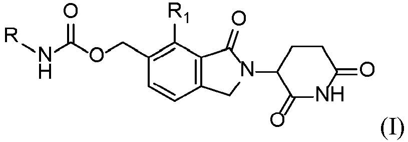 or stereoisomers, tautomers, or salts thereof, wherein:
R is 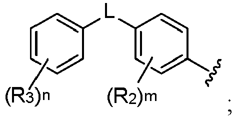
L is -O-, -CH₂-, -C(CH₃)₂-, -CF₂-, -NH-, -N(CH₃)-, or 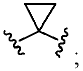
R₁ is -OCH₃, -OCH₂CH₃, -OCH(CH₃)₂, -OCHF₂, or -O(phenyl);
R₂ is F, Cl, -CH₃, or -OCH₃;
each R₃ is independently F, Cl, Br, -CH₃, or -OCH₃;
m is zero or 1; and
n is zero, 1, 2, or 3.

One embodiment provides a compound of Formula (I), or stereoisomers, tautomers, or pharmaceutically acceptable salts thereof.

One embodiment provides a compound of Formula (I), or stereoisomers or tautomers thereof.

One embodiment provides a salt of the compound of Formula (I), or stereoisomers or tautomers thereof.

One embodiment provides a pharmaceutically acceptable salt of the compound of Formula (I), or stereoisomers or tautomers thereof.

One embodiment provides a compound of Formula (I) or stereoisomers, tautomers, or salts thereof, wherein Ring A is phenyl.

One embodiment provides a compound of Formula (I) or stereoisomers, tautomers, or salts thereof, wherein Ring A is naphthalenyl or benzo[b]thiophenyl.

One embodiment provides a compound of Formula (I) or stereoisomers, tautomers, or salts thereof, wherein R is:

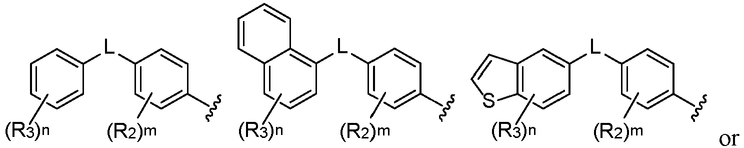

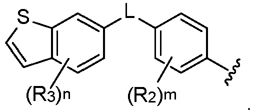

One embodiment provides a compound of Formula (I) or stereoisomers, tautomers, or salts thereof, wherein R is:

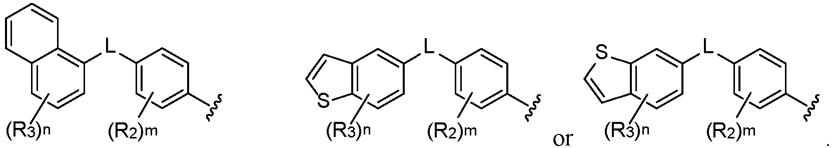

One embodiment provides a compound of Formula (I) or stereoisomers, tautomers, or salts thereof, wherein L is -O-, -CH₂-, -C(CH₃)₂-, -CF₂-, -NH-, or -N(CH₃)-.

One embodiment provides a compound of Formula (I), or stereoisomers or tautomers, thereof, wherein L is -O-, -CH₂-, or -C(CH₃)₂-.

One embodiment provides a compound of Formula (I), or stereoisomers or tautomers, thereof, wherein L is -O- or -CH₂-.

One embodiment provides a compound of Formula (I), or stereoisomers or tautomers, thereof, wherein L is -0-.

One embodiment provides a compound of Formula (I), or stereoisomers or tautomers, thereof, wherein L is -CH₂-, -C(CH₃)₂-, -CF₂-, or

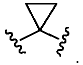

One embodiment provides a compound of Formula (I), or stereoisomers or tautomers, thereof, wherein L is -CH₂-, -C(CH₃)₂-, or

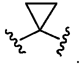

One embodiment provides a compound of Formula (I), or stereoisomers or tautomers, thereof, wherein L is -CH₂- or -C(CH₃)₂-.

One embodiment provides a compound of Formula (I), or stereoisomers or tautomers, thereof, wherein L is -CH₂-.

One embodiment provides a compound of Formula (I), or stereoisomers or tautomers, thereof, wherein L is -C(CH₃)₂-.

One embodiment provides a compound of Formula (I), or stereoisomers or tautomers, thereof, wherein L is -CF₂-.

One embodiment provides a compound of Formula (I), or stereoisomers or tautomers, thereof, wherein L is

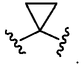

One embodiment provides a compound of Formula (I) or stereoisomers, tautomers, or salts thereof, wherein L is -NH- or -N(CH₃)-.

One embodiment provides a compound of Formula (I) or stereoisomers, tautomers, or salts thereof, wherein L is -NH-.

One embodiment provides a compound of Formula (I) or stereoisomers, tautomers, or salts thereof, wherein L is -N(CH₃)-.

One embodiment provides a compound of Formula (I) or stereoisomers, tautomers, or salts thereof, wherein each R₃ is independently F, Cl, Br, -CH₃, -CD₃, -CHF₂, -CF₃, or -OCH₃.

One embodiment provides a compound of Formula (I) or stereoisomers, tautomers, or salts thereof, wherein each R₃ is independently F, Cl, Br, -CH₃, or -CD₃.

One embodiment provides a compound of Formula (I) or stereoisomers, tautomers, or salts thereof, wherein each R₃ is independently F, Cl, Br, or -CH₃.

One embodiment provides a compound of Formula (I) or stereoisomers, tautomers, or salts thereof, wherein each R₃ is independently F, Cl, or -CH₃.

One embodiment provides a compound of Formula (I) or stereoisomers, tautomers, or salts thereof, wherein each R₃ is independently F or Cl.

One embodiment provides a compound of Formula (I) or stereoisomers, tautomers, or salts thereof, wherein each R₃ is F.

One embodiment provides a compound of Formula (I) or stereoisomers, tautomers, or salts thereof, wherein each R₃ is independently F, Cl, -CH₃, -CD₃, or -OCH₃.

One embodiment provides a compound of Formula (I) or stereoisomers, tautomers, or salts thereof, wherein each R₃ is independently F, Cl, -CH₃, or -OCH₃.

One embodiment provides a compound of Formula (I) or stereoisomers, tautomers, or salts thereof, wherein each R₃ is independently F, -CH₃, or -OCH₃.

One embodiment provides a compound of Formula (I) or stereoisomers, tautomers, or salts thereof, wherein each R₃ is independently F, -CH₃, -CD₃, or -OCH₃.

One embodiment provides a compound of Formula (I) or stereoisomers, tautomers, or salts thereof, wherein each R₃ is F.

One embodiment provides a compound of Formula (I) or stereoisomers, tautomers, or salts thereof, wherein m is zero.

One embodiment provides a compound of Formula (I) or stereoisomers, tautomers, or salts thereof, wherein m is 1.

One embodiment provides a compound of Formula (I) or stereoisomers, tautomers, or salts thereof, wherein n is zero.

One embodiment provides a compound of Formula (I) or stereoisomers, tautomers, or salts thereof, wherein n is 1, 2, or 3.

One embodiment provides a compound of Formula (I) or stereoisomers, tautomers, or salts thereof, wherein n is 1 or 2.

One embodiment provides a compound of Formula (I) or stereoisomers, tautomers, or salts thereof, wherein n is 2 or 3.

One embodiment provides a compound of Formula (I) or stereoisomers, tautomers, or salts thereof, wherein n is 1.

One embodiment provides a compound of Formula (I) or stereoisomers, tautomers, or salts thereof, wherein n is 2.

One embodiment provides a compound of Formula (I) or stereoisomers, tautomers, or salts thereof, wherein n is 3.

One embodiment provides a compound of Formula (I) or stereoisomers, tautomers, or salts thereof, wherein m is zero and n is zero, 1, or 2.

One embodiment provides a compound of Formula (I) or stereoisomers, tautomers, or salts thereof, wherein m is zero and n is 1, 2, or 3.

One embodiment provides a compound of Formula (I) or stereoisomers, tautomers, or salts thereof, wherein m is zero and n is zero or 2.

One embodiment provides a compound of Formula (I) or stereoisomers, tautomers, or salts thereof, wherein m is zero and n is zero.

One embodiment provides a compound of Formula (I) or stereoisomers, tautomers, or salts thereof, wherein m is zero and n is 1.

One embodiment provides a compound of Formula (I) or stereoisomers, tautomers, or salts thereof, wherein m is zero and n is 2.

One embodiment provides a compound of Formula (I) or stereoisomers, tautomers, or salts thereof, wherein m is zero and n is 3.

One embodiment provides a compound of Formula (I) or stereoisomers, tautomers, or salts thereof, wherein m is 1 and n is zero, 1, or 2.

One embodiment provides a compound of Formula (I) or stereoisomers, tautomers, or salts thereof, wherein m is 1 and n is 1, 2, or 3.

One embodiment provides a compound of Formula (I) or stereoisomers, tautomers, or salts thereof, wherein m is 1 and n is zero or 2.

One embodiment provides a compound of Formula (I) or stereoisomers, tautomers, or salts thereof, wherein m is 1 and n is zero.

One embodiment provides a compound of Formula (I) or stereoisomers, tautomers, or salts thereof, wherein m is 1 and n is 1.

One embodiment provides a compound of Formula (I) or stereoisomers, tautomers, or salts thereof, wherein m is 1 and n is 2.

One embodiment provides a compound of Formula (I) or stereoisomers, tautomers, or salts thereof, wherein m is 1 and n is 3.

One embodiment provides a compound of Formula (I) or stereoisomers, tautomers, or salts thereof, wherein m is 1; and R₂ is F, -CH₃, or -OCH₃.

One embodiment provides a compound of Formula (I) or stereoisomers, tautomers, or salts thereof, wherein m is 1; and R₂ is Cl, -CH₃, or -OCH₃.

One embodiment provides a compound of Formula (I) or stereoisomers, tautomers, or salts thereof, wherein m is 1; and R₂ is F, Cl, or -OCH₃.

One embodiment provides a compound of Formula (I) or stereoisomers, tautomers, or salts thereof, wherein m is 1; and R₂ is F, Cl, or -CH₃.

One embodiment provides a compound of Formula (I) or stereoisomers, tautomers, or salts thereof, wherein m is 1; and R₂ is F.

One embodiment provides a compound of Formula (I) or stereoisomers, tautomers, or salts thereof, wherein m is 1; and R₂ is Cl.

One embodiment provides a compound of Formula (I) or stereoisomers, tautomers, or salts thereof, wherein m is 1; and R₂ is F or Cl.

One embodiment provides a compound of Formula (I) or stereoisomers, tautomers, or salts thereof, wherein m is 1; and R₂ is -CH₃.

One embodiment provides a compound of Formula (I) or stereoisomers, tautomers, or salts thereof, wherein m is 1; and R₂ is -OCH₃.

One embodiment provides a compound of Formula (I) or stereoisomers, tautomers, or salts thereof, wherein m is 1; and R₂ is -CH₃ or -OCH₃.

One embodiment provides a compound of Formula (I) or stereoisomers, tautomers, or salts thereof, wherein m is 1; and R₂ is F or -OCH₃.

One embodiment provides a compound of Formula (I) or stereoisomers, tautomers, or salts thereof, wherein n is 1; and R₃ is F, Cl, -CH₃, or -OCH₃. Included in this embodiment are compounds in which R₃ is F, Cl, or -CH₃.

One embodiment provides a compound of Formula (I) or stereoisomers, tautomers, or salts thereof, wherein n is 2; and each R₃ is independently F, Cl, -CH₃, or -OCH₃. Included in this embodiment are compounds in which each R₃ is independently F, Cl, or -CH₃.

One embodiment provides a compound of Formula (I) or stereoisomers, tautomers, or salts thereof, wherein n is 3; and each R₃ is independently F, Cl, -CH₃, or -OCH₃. Included in this embodiment are compounds in which each R₃ is independently F, Cl, or -CH₃.

One embodiment provides a compound of Formula (I) or stereoisomers, tautomers, or salts thereof, wherein m is zero; n is 1; and R₃ is F, Cl, -CH₃, or -OCH₃. Included in this embodiment are compounds in which R₃ is F, Cl, or -CH₃.

One embodiment provides a compound of Formula (I) or stereoisomers, tautomers, or salts thereof, wherein m is zero; n is 2; and each R₃ is independently F, Cl, -CH₃, or -OCH₃. Included in this embodiment are compounds in which each R₃ is independently F, Cl, or -CH₃.

One embodiment provides a compound of Formula (I) or stereoisomers, tautomers, or salts thereof, wherein m is zero; n is 3; and each R₃ is independently F, Cl, -CH₃, or -OCH₃.

One embodiment provides a compound of Formula (I) or stereoisomers, tautomers, or salts thereof, wherein m is 1; n is 1; and R₃ is F, Cl, -CH₃, or -OCH₃. Included in this embodiment are compounds in which R₃ is F, Cl, or -CH₃.

One embodiment provides a compound of Formula (I) or stereoisomers, tautomers, or salts thereof, wherein m is 1; n is 2; and each R₃ is independently F, Cl, -CH₃, or -OCH₃. Included in this embodiment are compounds in which each R₃ is independently F, Cl, or -CH₃.

One embodiment provides a compound of Formula (I) or stereoisomers, tautomers, or salts thereof, wherein m is 1; n is 3; and each R₃ is independently F, Cl, -CH₃, or -OCH₃. Included in this embodiment are compounds in which each R₃ is independently F, Cl, or -CH₃.

One embodiment provides a compound of Formula (I) or stereoisomers, tautomers, or salts thereof, wherein R₁ is -OCH₃, -OCH₂CH₃, -OCH(CH₃)₂, -OCH₂CH(CH₃)₂, -OCHF₂, -OCH₂C(CH₃)₂OH, -OCH₂CH₂OCH₃, -OCH₂C(O)OCH₃, -OCH₂C(O)NH₂, -OCH₂C(O)N(CH₃)₂, or -OCH₂C(O)N(CH₃)(CH₂CH₃).

One embodiment provides a compound of Formula (I) or stereoisomers, tautomers, or salts thereof, wherein R₁ is -OCH₃, -OCH₂CH₃, -OCH(CH₃)₂, -OCH₂CH(CH₃)₂, -OCHF₂, -OCH₂C(CH₃)₂OH, -OCH₂CH₂OCH₃, or -OCH₂C(O)OCH₃.

One embodiment provides a compound of Formula (I) or stereoisomers, tautomers, or salts thereof, wherein R₁ is -OCH₃, -OCH₂CH₃, -OCH(CH₃)₂, or -OCH₂CH(CH₃)₂.

One embodiment provides a compound of Formula (I) or stereoisomers, tautomers, or salts thereof, wherein R₁ is -OCH₃, -OCH₂CH₃, or -OCH(CH₃)₂.

One embodiment provides a compound of Formula (I) or stereoisomers, tautomers, or salts thereof, wherein R₁ is -OCH₃ or -OCH₂CH₃.

One embodiment provides a compound of Formula (I) or stereoisomers, tautomers, or salts thereof, wherein R₁ is -OCH₃.

One embodiment provides a compound of Formula (I) or stereoisomers, tautomers, or salts thereof, wherein R₁ is -OCH₂CH₃.

One embodiment provides a compound of Formula (I) or stereoisomers, tautomers, or salts thereof, wherein R₁ is -OCH₂CH₂CH₂(phenyl), -OCH₂C(O)(morpholinyl), -O(C₄₋₆ cycloalkyl), -O(hydroxycyclohexyl), -O(oxetanyl), -O(tetrahydrofuranyl), -O(tetrahydropyranyl), -O(oxepanyl), -O(phenyl), -O(pyridinyl), or -O(acetylpiperidinyl).

One embodiment provides a compound of Formula (I) or stereoisomers, tautomers, or salts thereof, wherein R₁ is -O(C₄₋₆ cycloalkyl) or -O(hydroxycyclohexyl). Included in this embodiment are compounds wherein R₁ is -O(cyclobutyl) or -O(cyclopentyl). Also included in this embodiment are compounds in which R₁ is -O(cyclohexyl) or -O(hydroxycyclohexyl).

One embodiment provides a compound of Formula (I) or stereoisomers, tautomers, or salts thereof, wherein R₁ is -OCH₃, -OCH₂CH₃, or -OCH(CH₃)₂; m is zero; n is 1; and R₃ is F, Cl, -CH₃, or -OCH₃. Included in this embodiment are compounds in which R₃ is F, Cl, or -CH₃. Also included in this embodiment are compounds in which R₁ is -OCH₃; and R₃ is F, Cl, or -CH₃.

One embodiment provides a compound of Formula (I) or stereoisomers, tautomers, or salts thereof, wherein R₁ is -OCH₃, -OCH₂CH₃, or -OCH(CH₃)₂; m is zero; n is 2; and each R₃ is independently F, Cl, -CH₃, or -OCH₃. Included in this embodiment are compounds in which each R₃ is independently F, Cl, or -CH₃. Also included in this embodiment are compounds in which R₁ is -OCH₃; and each R₃ is independently F, Cl, or -CH₃.

One embodiment provides a compound of Formula (I) or stereoisomers, tautomers, or salts thereof, wherein R₁ is -OCH₃, -OCH₂CH₃, or -OCH(CH₃)₂; m is zero; n is 3; and each R₃ is independently F, Cl, -CH₃, or -OCH₃. Included in this embodiment are compounds in which each R₃ is independently F, Cl, or -CH₃. Also included in this embodiment are compounds in which R₁ is -OCH₃; and each R₃ is independently F, Cl, or -CH₃.

One embodiment provides a compound of Formula (I) or stereoisomers, tautomers, or salts thereof, wherein R₁ is -OCH₃, -OCH₂CH₃, or -OCH(CH₃)₂; m is 1; n is 1; and R₃ is F, Cl, -CH₃, or -OCH₃. Included in this embodiment are compounds in which R₃ is F, Cl, or -CH₃. Also included in this embodiment are compounds in which R₁ is -OCH₃; and R₃ is F, Cl, or -CH₃.

One embodiment provides a compound of Formula (I) or stereoisomers, tautomers, or salts thereof, wherein R₁ is -OCH₃, -OCH₂CH₃, or -OCH(CH₃)₂; m is 1; n is 2; and each R₃ is independently F, Cl, -CH₃, or -OCH₃. Included in this embodiment are compounds in which each R₃ is independently F, Cl, or -CH₃. Also included in this embodiment are compounds in which R₁ is -OCH₃; and each R₃ is independently F, Cl, or -CH₃.

One embodiment provides a compound of Formula (I) or stereoisomers, tautomers, or salts thereof, wherein R₁ is -OCH₃, -OCH₂CH₃, or -OCH(CH₃)₂; m is 1; n is 3; and each R₃ is independently F, Cl, -CH₃, or -OCH₃. Included in this embodiment are compounds in which each R₃ is independently F, Cl, or -CH₃. Also included in this embodiment are compounds in which R₁ is -OCH₃; and each R₃ is independently F, Cl, or -CH₃.

One embodiment provides a compound of Formula (I) or stereoisomers, tautomers, or salts thereof, wherein the compound has the structure: 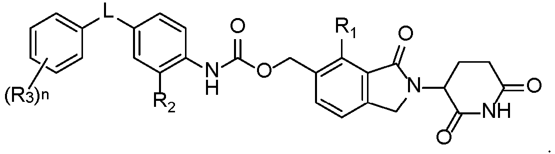 Included in this embodiment are compounds in which n is zero, 1, or 2. Also included in this embodiment are compounds in which n is 1. Additionally, included in this embodiment are compounds in which n is 2. Furthermore, included in this embodiment are compounds in which L is -O- or -CH₂-.

One embodiment provides a compound of Formula (I), or stereoisomers or tautomers thereof, wherein the compound has the structure: 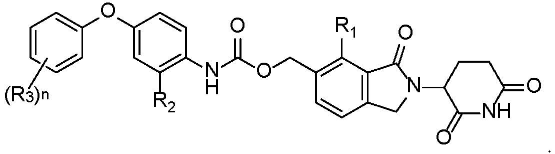 Included in this embodiment are compounds in which n is zero, 1, or 2. Also included in this embodiment are compounds in which n is 1. Additionally, included in this embodiment are compounds in which n is 2.

One embodiment provides a compound of Formula (I) ), or stereoisomers or tautomers thereof, wherein the compound has the structure: 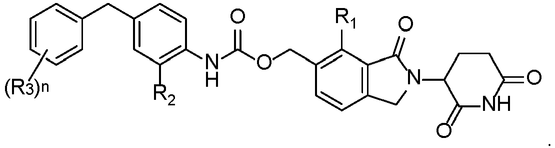 Included in this embodiment are compounds in which n is zero, 1, or 2. Also included in this embodiment are compounds in which n is 1. Additionally, included in this embodiment are compounds in which n is 2.

One embodiment provides a compound of Formula (I) or stereoisomers, tautomers, or salts thereof, wherein the compound has the structure: 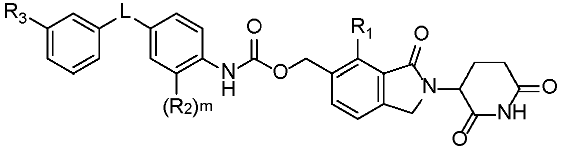 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 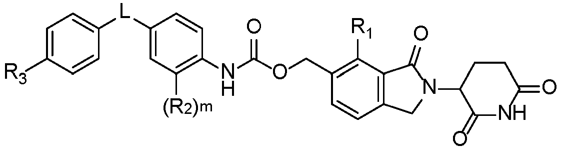 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 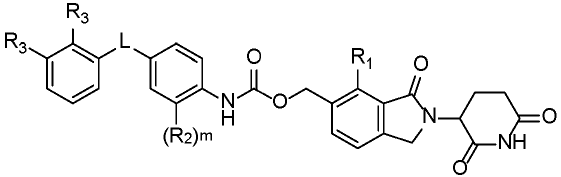 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 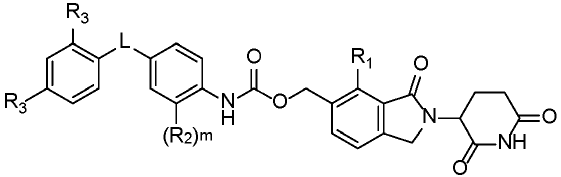 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 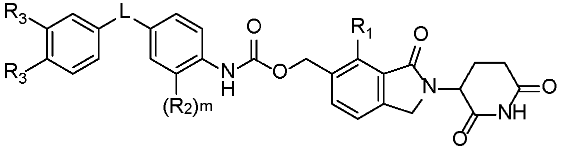 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -O-.

One embodiment provides a compound of Formula (I) or stereoisomers, tautomers, or salts thereof, wherein the compound has the structure: 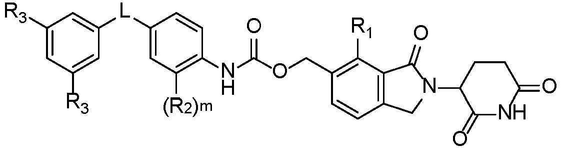 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -O-.

One embodiment provides a compound of Formula (I) or stereoisomers, tautomers, or salts thereof, wherein the compound has the structure: 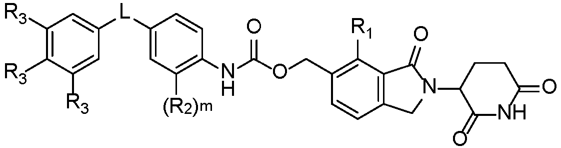 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 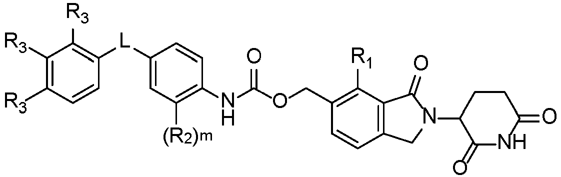 and wherein m is zero or 1. Included in this embodiment are compounds in which m is zero. Also included in this embodiment are compounds in which m is 1. Additionally, included in this embodiment are compounds in which L is -O- or -CH₂-. Furthermore, included in this embodiment are compounds in which L is -0-.

One embodiment provides a compound of Formula (I) or stereoisomers, tautomers, or salts thereof, wherein the compound has the structure: 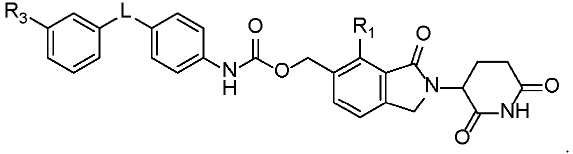 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 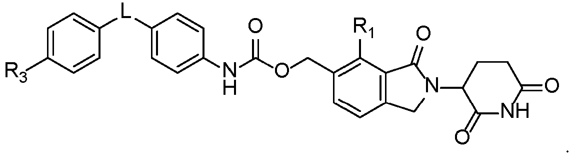 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 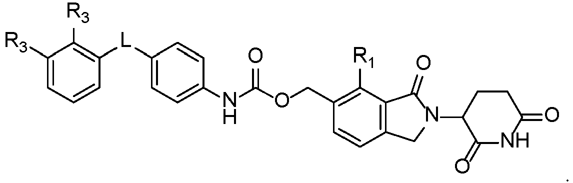 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 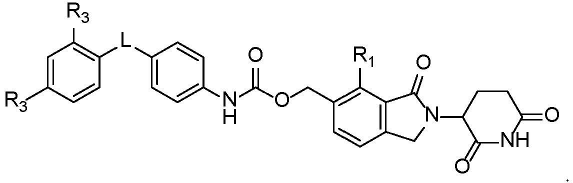 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 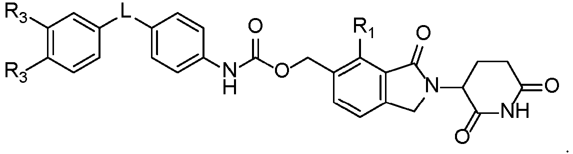 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 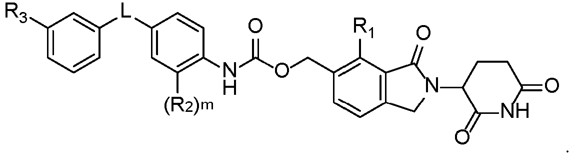 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 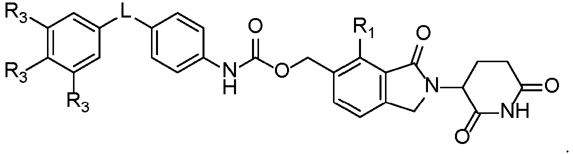 Included in this embodiment are compounds in which L is -O- or -CH₂-. Also included in this embodiment are compounds in which L is -0-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ is -OCH₃; and R₃ is F, Cl, or -CH₃.

One embodiment provides a compound of Formula (I) or stereoisomers, tautomers, or salts thereof, wherein the compound has the structure: 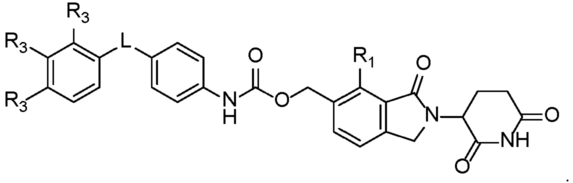 Included in this embodiment are compounds in which L is -O- or -CH₂-. Also included in this embodiment are compounds in which L is -O-. Additionally, included in this embodiment are compounds in which R₁ is -OCH₃ or -OCH₂CH₃; and R₃ is F, Cl, -CH₃, or -OCH₃. Also included in this embodiment are compounds in which L is -O-; R₁ -OCH₃; and R₃ is F, Cl, or -CH₃.

One embodiment provides a compound of Formula (I) or stereoisomers, tautomers, or salts thereof, wherein n is 2; and each R₃ is independently F, Cl, or -CH₃.

One embodiment provides a compound of Formula (I) or tautomers or salts thereof, wherein the compound has the structure:

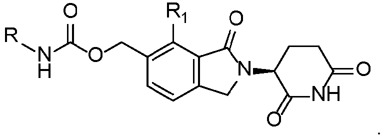

One embodiment provides a compound of Formula (I) or tautomers or salts thereof, wherein the compound has the structure:

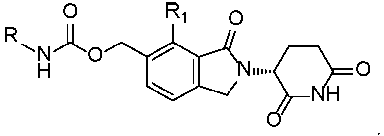

One embodiment provides a compound having the structure: 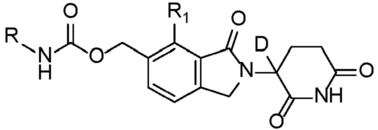 or stereoisomers, tautomers, or salts thereof.

One embodiment provides a compound having the structure: 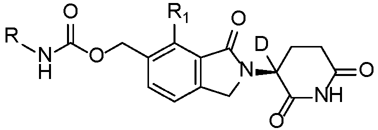 or tautomers or salts thereof.

One embodiment provides a compound having the structure: 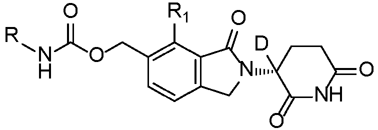 or tautomers or pharmaceutically acceptable salts thereof.

One embodiment provides a compound having the structure: 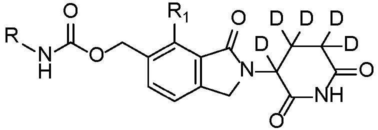 or stereoisomers, tautomers, or salts thereof.

One embodiment provides a compound having the structure: 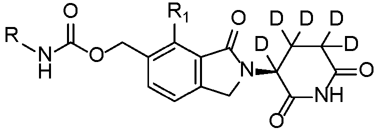 or stereoisomers, tautomers, or salts thereof.

One embodiment provides a compound having the structure: 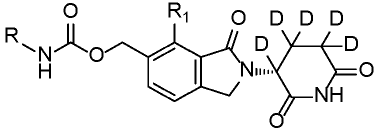 or stereoisomers, tautomers, or salts thereof.

One embodiment provides a compound of Formula (I), or stereoisomers, tautomers, or salts thereof, wherein said compound is: [2-(2,6-dioxopiperidin-3-yl)-4-methoxy-3-oxo-2,3-dihydro-1H-isoindol-5-yl]methyl N-[4-(3,4-difluorophenoxy)phenyl]carbamate (1); [2-(2,6-dioxopiperidin-3-yl)-4-methoxy-3-oxo-2,3-dihydro-1H-isoindol-5-yl]methyl N-(4-phenoxyphenyl)carbamate (2); [2-(2,6-dioxopiperidin-3-yl)-4-methoxy-3-oxo-2,3-dihydro-1H-isoindol-5-yl]methyl N-[4-(3-chlorophenoxy)phenyl]carbamate (3); [2-(2,6-dioxopiperidin-3-yl)-4-methoxy-3-oxo-2,3-dihydro-1H-isoindol-5-yl]methyl N-[4-(3-fluorophenoxy)phenyl]carbamate (4); [2-(2,6-dioxopiperidin-3-yl)-4-methoxy-3-oxo-2,3-dihydro-1H-isoindol-5-yl]methyl N-[4-(2,4-difluorophenoxy)phenyl]carbamate (5); [2-(2,6-dioxopiperidin-3-yl)-4-methoxy-3-oxo-2,3-dihydro-1H-isoindol-5-yl]methyl N-{4-[(3-fluorophenyl)methyl]phenyl}carbamate (6); [2-(2,6-dioxopiperidin-3-yl)-4-methoxy-3-oxo-2,3-dihydro-1H-isoindol-5-yl]methyl N-[4-(1-phenylcyclopropyl)phenyl]carbamate (7); [2-(2,6-dioxopiperidin-3-yl)-4-methoxy-3-oxo-2,3-dihydro-1H-isoindol-5-yl]methyl N-{4-[(3-fluorophenyl)(methyl)amino]phenyl}carbamate (8); [2-(2,6-dioxopiperidin-3-yl)-4-methoxy-3-oxo-2,3-dihydro-1H-isoindol-5-yl]methyl N-{4-[(3-fluorophenyl)amino]phenyl}carbamate (9); [2-(2,6-dioxopiperidin-3-yl)-4-methoxy-3-oxo-2,3-dihydro-1H-isoindol-5-yl]methyl N-[4-(3-bromophenoxy)phenyl]carbamate (10); [2-(2,6-dioxopiperidin-3-yl)-4-methoxy-3-oxo-2,3-dihydro-1H-isoindol-5-yl]methyl N-[4-(3-methylphenoxy)phenyl]carbamate (11); [2-(2,6-dioxopiperidin-3-yl)-4-methoxy-3-oxo-2,3-dihydro-1H-isoindol-5-yl]methyl N-[4-(3,5-dimethylphenoxy)phenyl]carbamate (12); [2-(2,6-dioxopiperidin-3-yl)-4-methoxy-3-oxo-2,3-dihydro-1H-isoindol-5-yl]methyl N-[4-(3,5-difluorophenoxy)phenyl]carbamate (13); [2-(2,6-dioxopiperidin-3-yl)-4-methoxy-3-oxo-2,3-dihydro-1H-isoindol-5-yl]methyl N-[4-(3-chloro-4-fluorophenoxy)phenyl]carbamate (14); [2-(2,6-dioxopiperidin-3-yl)-4-methoxy-3-oxo-2,3-dihydro-1H-isoindol-5-yl]methyl N-[2-fluoro-4-(3,4,5-trifluorophenoxy)phenyl]carbamate (15); [2-(2,6-dioxopiperidin-3-yl)-4-methoxy-3-oxo-2,3-dihydro-1H-isoindol-5-yl]methyl N-[2-fluoro-4-(3-fluorophenoxy)phenyl]carbamate (16); [2-(2,6-dioxopiperidin-3-yl)-4-methoxy-3-oxo-2,3-dihydro-1H-isoindol-5-yl]methyl N-{ 4-[(3,4-difluorophenyl)methyl] phenyl}carbamate (17); [2-(2,6-dioxopiperidin-3-yl)-4-methoxy-3-oxo-2,3-dihydro-1H-isoindol-5-yl]methyl N-{4-[(3,4-difluorophenyl)methyl]-2-methylphenyl}carbamate (18); [2-(2,6-dioxopiperidin-3-yl)-4-methoxy-3-oxo-2,3-dihydro-1H-isoindol-5-yl]methyl N-{4-[(3,4,5-trifluorophenyl)methyl]phenyl}carbamate (19); [2-(2,6-dioxopiperidin-3-yl)-4-methoxy-3-oxo-2,3-dihydro-1H-isoindol-5-yl]methyl N-{2-fluoro-4-[(3-fluorophenyl) methyl]phenyl}carbamate (20); [2-(2,6-dioxopiperidin-3-yl)-4-methoxy-3-oxo-2,3-dihydro-1H-isoindol-5-yl]methyl N-{4-[(3-chloro-4-fluorophenyl)methyl]phenyl } carbamate (21); [2-(2,6-dioxopiperidin-3-yl)-4-methoxy-3-oxo-2,3-dihydro-1H-isoindol-5-yl]methyl N-[4-(2,3-difluorophenoxy)-2-fluorophenyl]carbamate (22); [2-(2,6-dioxopiperidin-3-yl)-4-methoxy-3-oxo-2,3-dihydro-1H-isoindol-5-yl]methyl N-[4-(3,4-difluorophenoxy)-2-fluorophenyl] carbamate (23); [2-(2,6-dioxopiperidin-3-yl)-4-methoxy-3-oxo-2,3-dihydro-1H-isoindol-5-yl]methyl N-[4-(3,5-difluorophenoxy)-2-fluorophenyl]carbamate (24); [2-(2,6-dioxopiperidin-3-yl)-4-methoxy-3-oxo-2,3-dihydro-1H-isoindol-5-yl]methyl N-[2-chloro-4-(3,4,5-trifluorophenoxy)phenyl]carbamate (25); [2-(2,6-dioxopiperidin-3-yl)-4-methoxy-3-oxo-2,3-dihydro-1H-isoindol-5-yl]methyl N-[4-(3-chlorophenoxy)-2-fluorophenyl]carbamate (26); [2-(2,6-dioxopiperidin-3-yl)-4-methoxy-3-oxo-2,3-dihydro-1H-isoindol-5-yl]methyl N-[2-chloro-4-(3-fluorophenoxy)phenyl]carbamate (27); [2-(2,6-dioxopiperidin-3-yl)-4-methoxy-3-oxo-2,3-dihydro-1H-isoindol-5-yl]methyl N-[2-fluoro-4-(4-fluorophenoxy) phenyl]carbamate (28); [2-(2,6-dioxopiperidin-3-yl)-4-methoxy-3-oxo-2,3-dihydro-1H-isoindol-5-yl]methyl N-{ 4-[difluoro(phenyl)methyl]phenyl } carbamate (29); [2-(2,6-dioxopiperidin-3-yl)-4-methoxy-3-oxo-2,3-dihydro-1H-isoindol-5-yl]methyl N-[4-(2,4-difluorophenoxy)-2-fluorophenyl]carbamate (30); [2-(2,6-dioxopiperidin-3-yl)-4-methoxy-3-oxo-2,3-dihydro-1H-isoindol-5-yl]methyl N-[4-(2-methoxyphenoxy)phenyl]carbamate (31); [2-(2,6-dioxopiperidin-3-yl)-4-methoxy-3-oxo-2,3-dihydro-1H-isoindol-5-yl]methyl N-[4-(3-methoxyphenoxy)phenyl]carbamate (32); [2-(2,6-dioxopiperidin-3-yl)-4-methoxy-3-oxo-2,3-dihydro-1H-isoindol-5-yl]methyl N-[4-(4,5-difluoro-2-methoxyphenoxy)-2-methylphenyl] carbamate (33); [2-(2,6-dioxopiperidin-3-yl)-4-methoxy-3-oxo-2,3-dihydro-1H-isoindol-5-yl]methyl N-[2-methoxy-4-(2,4,5-trifluorophenoxy)phenyl]carbamate (34); {2-[(3S)-2,6-dioxopiperidin-3-yl]-4-methoxy-3-oxo-2,3-dihydro-1H-isoindol-5-yl}methyl N-[4-(3,4-difluorophenoxy)phenyl]carbamate (35); {2-[(3R)-2,6-dioxopiperidin-3-yl]-4-methoxy-3-oxo-2,3-dihydro-1H-isoindol-5-yl}methyl N-[4-(3,4-difluorophenoxy)phenyl]carbamate (36); [2-(2,6-dioxopiperidin-3-yl)-4-methoxy-3-oxo-2,3-dihydro-1H-isoindol-5-yl]methyl N-[4-(4-fluorophenoxy)-2-methoxyphenyl]carbamate (37); [2-(2,6-dioxopiperidin-3-yl)-4-methoxy-3-oxo-2,3-dihydro-1H-isoindol-5-yl]methyl N-[4-(3,4-difluorophenoxy)-2-methylphenyl]carbamate (38); [2-(2,6-dioxopiperidin-3-yl)-4-methoxy-3-oxo-2,3-dihydro-1H-isoindol-5-yl]methyl N-[4-(3-fluorophenoxy)-2-methylphenyl]carbamate (39); [2-(2,6-dioxopiperidin-3-yl)-4-methoxy-3-oxo-2,3-dihydro-1H-isoindol-5-yl]methyl N-[2-methyl-4-(3,4,5-trifluorophenoxy)phenyl]carbamate (40); [2-(2,6-dioxopiperidin-3-yl)-4-methoxy-3-oxo-2,3-dihydro-1H-isoindol-5-yl]methyl N-[4-(2-phenylpropan-2-yl)phenyl]carbamate (41); [2-(2,6-dioxopiperidin-3-yl)-4-methoxy-3-oxo-2,3-dihydro-1H-isoindol-5-yl]methyl N-[4-(2,4-difluorophenoxy)-2-methylphenyl]carbamate (42); [2-(2,6-dioxopiperidin-3-yl)-4-methoxy-3-oxo-2,3-dihydro-1H-isoindol-5-yl]methyl N-[2-fluoro-4-(2,4,5-trifluorophenoxy) phenyl]carbamate (43); [2-(2,6-dioxopiperidin-3-yl)-4-methoxy-3-oxo-2,3-dihydro-1H-isoindol-5-yl]methyl N-[4-(2,4,5-trifluorophenoxy)phenyl]carbamate (44); [2-(2,6-dioxopiperidin-3-yl)-4-methoxy-3-oxo-2,3-dihydro-1H-isoindol-5-yl]methyl N-[2-methyl-4-(2,4,5-trifluorophenoxy)phenyl]carbamate (45); [2-(2,6-dioxopiperidin-3-yl)-4-methoxy-3-oxo-2,3-dihydro-1H-isoindol-5-yl]methyl N-[2-chloro-4-(3,4-difluorophenoxy)phenyl] carbamate (46); [2-(2,6-dioxopiperidin-3-yl)-4-methoxy-3-oxo-2,3-dihydro-1H-isoindol-5-yl]methyl N-[4-(3,4-difluorophenoxy)-2-methoxyphenyl]carbamate (47); [2-(2,6-dioxopiperidin-3-yl)-4-methoxy-3-oxo-2,3-dihydro-1H-isoindol-5-yl]methyl N-[4-(3-fluorophenoxy)-2-methoxyphenyl]carbamate (48); [2-(2,6-dioxopiperidin-3-yl)-4-methoxy-3-oxo-2,3-dihydro-1H-isoindol-5-yl]methyl N-[4-(2,4-difluorophenoxy)-2-methoxyphenyl] carbamate (49); [2-(2,6-dioxopiperidin-3-yl)-4-methoxy-3-oxo-2,3-dihydro-1H-isoindol-5-yl]methyl N-[4-(4-fluoro-2-methoxyphenoxy)-2-methoxyphenyl]carbamate (50); [2-(2,6-dioxopiperidin-3-yl)-4-methoxy-3-oxo-2,3-dihydro-1H-isoindol-5-yl]methyl N-[4-(4-fluorophenoxy)-2-methylphenyl]carbamate (51); [2-(2,6-dioxopiperidin-3-yl)-4-ethoxy-3-oxo-2,3-dihydro-1H-isoindol-5-yl]methyl N-(4-phenoxyphenyl)carbamate (52); [2-(2,6-dioxopiperidin-3-yl)-4-ethoxy-3-oxo-2,3-dihydro-1H-isoindol-5-yl]methyl N-[4-(3,4-difluorophenoxy)phenyl]carbamate (53); [2-(2,6-dioxopiperidin-3-yl)-4-ethoxy-3-oxo-2,3-dihydro-1H-isoindol-5-yl]methyl N-[4-(3-chlorophenoxy)phenyl]carbamate (54); [2-(2,6-dioxopiperidin-3-yl)-4-ethoxy-3-oxo-2,3-dihydro-1H-isoindol-5-yl]methyl N-[4-(3-fluorophenoxy)phenyl]carbamate (55); [2-(2,6-dioxopiperidin-3-yl)-3-oxo-4-(propan-2-yloxy)-2,3-dihydro-1H-isoindol-5-yl]methyl N-[4-(3,4-difluorophenoxy)phenyl]carbamate (56); [2-(2,6-dioxopiperidin-3-yl)-3-oxo-4-(propan-2-yloxy)-2,3-dihydro-1H-isoindol-5-yl]methyl N-(4-phenoxyphenyl)carbamate (57); [2-(2,6-dioxopiperidin-3-yl)-3-oxo-4-(propan-2-yloxy)-2,3-dihydro-1H-isoindol-5-yl]methyl N-[4-(3-chlorophenoxy)phenyl] carbamate (58); [2-(2,6-dioxopiperidin-3-yl)-3-oxo-4-(propan-2-yloxy)-2,3-dihydro-1H-isoindol-5-yl]methyl N-[4-(4-fluorophenoxy)phenyl]carbamate (59); [4-(difluoromethoxy)-2-(2,6-dioxopiperidin-3-yl)-3-oxo-2,3-dihydro-1H-isoindol-5-yl]methyl N-(4-phenoxyphenyl) carbamate (60); [4-(difluoromethoxy)-2-(2,6-dioxopiperidin-3-yl)-3-oxo-2,3-dihydro-1H-isoindol-5-yl]methyl N-[4-(3,4-difluorophenoxy)phenyl]carbamate (61); [2-(2,6-dioxopiperidin-3-yl)-3-oxo-4-phenoxy-2,3-dihydro-1H-isoindol-5-yl]methyl N-(4-phenoxyphenyl)carbamate (62); [2-(2,6-dioxopiperidin-3-yl)-3-oxo-4-phenoxy-2,3-dihydro-1H-isoindol-5-yl]methyl N-[4-(3,4-difluorophenoxy)phenyl]carbamate (63); [2-(2,6-dioxopiperidin-3-yl)-3-oxo-4-phenoxy-2,3-dihydro-1H-isoindol-5-yl]methyl N-[4-(3-fluorophenoxy)phenyl]carbamate (64); [2-(2,6-dioxopiperidin-3-yl)-3-oxo-4-phenoxy-2,3-dihydro-1H-isoindol-5-yl]methyl N-[4-(2-fluorophenoxy)phenyl]carbamate (65); [2-(2,6-dioxopiperidin-3-yl)-3-oxo-4-phenoxy-2,3-dihydro-1H-isoindol-5-yl]methyl N-[4-(2-chlorophenoxy)phenyl]carbamate (66); [2-(2,6-dioxopiperidin-3-yl)-3-oxo-4-phenoxy-2,3-dihydro-1H-isoindol-5-yl]methyl N-[4-(2,4-difluorophenoxy)phenyl]carbamate (67); [2-(2,6-dioxopiperidin-3-yl)-3-oxo-4-phenoxy-2,3-dihydro-1H-isoindol-5-yl]methyl N-[4-(2,3-difluorophenoxy)phenyl]carbamate (68); [2-(2,6-dioxopiperidin-3-yl)-3-oxo-4-phenoxy-2,3-dihydro-1H-isoindol-5-yl]methyl N-[4-(3,4-difluorophenoxy)-2-fluorophenyl]carbamate (69); [2-(2,6-dioxopiperidin-3-yl)-3-oxo-4-phenoxy-2,3-dihydro-1H-isoindol-5-yl]methyl N-[4-(2-chloro-4-fluorophenoxy)phenyl]carbamate (70); [2-(2,6-dioxopiperidin-3-yl)-4-methoxy-3-oxo-2,3-dihydro-1H-isoindol-5-yl]methyl N-[4-(4-chloro-3-fluorophenoxy) phenyl]carbamate (71); [2-(2,6-dioxopiperidin-3-yl)-4-methoxy-3-oxo-2,3-dihydro-1H-isoindol-5-yl]methyl N-[2-methoxy-4-(3,4,5-trifluorophenoxy)phenyl]carbamate (72); [2-(2,6-dioxopiperidin-3-yl)-4-methoxy-3-oxo-2,3-dihydro-1H-isoindol-5-yl]methyl N-[4-(4,5-difluoro-2-methylphenoxy)-2-methoxyphenyl]carbamate (73); [2-(2,6-dioxopiperidin-3-yl)-4-methoxy-3-oxo-2,3-dihydro-1H-isoindol-5-yl]methyl N-[4-(4,5-difluoro-2-methylphenoxy)-2-methylphenyl]carbamate (74); [2-(2,6-dioxopiperidin-3-yl)-4-methoxy-3-oxo-2,3-dihydro-1H-isoindol-5-yl]methyl N-[4-(4-fluoro-2-methylphenoxy)-2-methoxyphenyl]carbamate (75); [2-(2,6-dioxopiperidin-3-yl)-4-methoxy-3-oxo-2,3-dihydro-1H-isoindol-5-yl]methyl N-[4-(4-fluoro-2-methylphenoxy)-2-methylphenyl]carbamate (76); [2-(2,6-dioxopiperidin-3-yl)-4-methoxy-3-oxo-2,3-dihydro-1H-isoindol-5-yl]methyl N-[4-(3,4,5-trifluorophenoxy)phenyl]carbamate (77); [2-(2,6-dioxopiperidin-3-yl)-4-methoxy-3-oxo-2,3-dihydro-1H-isoindol-5-yl]methyl N-[4-(4,5-difluoro-2-methylphenoxy)phenyl] carbamate (78); [2-(2,6-dioxopiperidin-3-yl)-4-methoxy-3-oxo-2,3-dihydro-1H-isoindol-5-yl]methyl N-[4-(2-fluorophenoxy)phenyl]carbamate (79); [2-(2,6-dioxopiperidin-3-yl)-4-methoxy-3-oxo-2,3-dihydro-1H-isoindol-5-yl]methyl N-[4-(2-chlorophenoxy)phenyl] carbamate (80); [2-(2,6-dioxopiperidin-3-yl)-4-methoxy-3-oxo-2,3-dihydro-1H-isoindol-5-yl]methyl N-[4-(4-fluoro-2-methylphenoxy)phenyl]carbamate (81); [2-(2,6-dioxopiperidin-3-yl)-4-methoxy-3-oxo-2,3-dihydro-1H-isoindol-5-yl]methyl N-[4-(4-fluoro-3-methylphenoxy)-2-methylphenyl]carbamate (82); [2-(2,6-dioxopiperidin-3-yl)-4-methoxy-3-oxo-2,3-dihydro-1H-isoindol-5-yl]methyl N-[4-(3-chloro-4-fluorophenoxy)-2-methylphenyl] carbamate (83); [2-(2,6-dioxopiperidin-3-yl)-4-methoxy-3-oxo-2,3-dihydro-1H-isoindol-5-yl]methyl N-[4-(4-fluorophenoxy)phenyl]carbamate (84); [2-(2,6-dioxopiperidin-3-yl)-4-methoxy-3-oxo-2,3-dihydro-1H-isoindol-5-yl]methyl N-{4-[2-(difluoromethyl)phenoxy] phenyl}carbamate (85); [2-(2,6-dioxopiperidin-3-yl)-4-methoxy-3-oxo-2,3-dihydro-1H-isoindol-5-yl]methyl N-{4-[2-(difluoromethyl)phenoxy]-2-fluorophenyl}carbamate (86); [2-(2,6-dioxopiperidin-3-yl)-4-methoxy-3-oxo-2,3-dihydro-1H-isoindol-5-yl]methyl N-[4-(2-cyclopropylphenoxy)phenyl]carbamate (87); [2-(2,6-dioxopiperidin-3-yl)-4-methoxy-3-oxo-2,3-dihydro-1H-isoindol-5-yl]methyl N-{4-[2-(difluoromethyl)phenoxy]-2-methoxyphenyl} carbamate (88); [2-(2,6-dioxopiperidin-3-yl)-4-methoxy-3-oxo-2,3-dihydro-1H-isoindol-5-yl]methyl N-{4-[2-(difluoromethyl)-4-fluorophenoxy]phenyl}carbamate (89); [2-(2,6-dioxopiperidin-3-yl)-4-methoxy-3-oxo-2,3-dihydro-1H-isoindol-5-yl]methyl N-{2-chloro-4-[2-(trifluoromethyl)phenoxy]phenyl}carbamate (90); [2-(2,6-dioxopiperidin-3-yl)-4-methoxy-3-oxo-2,3-dihydro-1H-isoindol-5-yl]methyl N-[4-(4-chloro-3-fluorophenoxy)-2-fluorophenyl]carbamate (91); [2-(2,6-dioxopiperidin-3-yl)-4-methoxy-3-oxo-2,3-dihydro-1H-isoindol-5-yl]methyl N-{4-[2-(²H3)methylphenoxy]phenyl}carbamate (92); [2-(2,6-dioxopiperidin-3-yl)-4-methoxy-3-oxo-2,3-dihydro-1H-isoindol-5-yl]methyl N-(2-methoxy-4-phenoxyphenyl)carbamate (93); [2-(2,6-dioxopiperidin-3-yl)-4-methoxy-3-oxo-2,3-dihydro-1H-isoindol-5-yl]methyl N-[4-(4,5-difluoro-2-methylphenoxy)-2-fluorophenyl] carbamate (94); [2-(2,6-dioxopiperidin-3-yl)-4-methoxy-3-oxo-2,3-dihydro-1H-isoindol-5-yl]methyl N-[2-fluoro-4-(4-fluoro-2-methylphenoxy)phenyl]carbamate (95); [2-(2,6-dioxopiperidin-3-yl)-4-methoxy-3-oxo-2,3-dihydro-1H-isoindol-5-yl]methyl N-[4-(4-fluoro-3-methylphenoxy)phenyl]carbamate (96); [2-(2,6-dioxopiperidin-3-yl)-4-methoxy-3-oxo-2,3-dihydro-1H-isoindol-5-yl]methyl N-[4-(4-fluoro-3-methylphenoxy)-2-methoxyphenyl] carbamate (97); [2-(2,6-dioxopiperidin-3-yl)-4-methoxy-3-oxo-2,3-dihydro-1H-isoindol-5-yl]methyl N-[4-(3-chloro-4-fluorophenoxy)-2-fluorophenyl]carbamate (98); [2-(2,6-dioxopiperidin-3-yl)-4-methoxy-3-oxo-2,3-dihydro-1H-isoindol-5-yl]methyl N-[2-fluoro-4-(4-fluoro-3-methylphenoxy)phenyl]carbamate (99); [2-(2,6-dioxopiperidin-3-yl)-4-methoxy-3-oxo-2,3-dihydro-1H-isoindol-5-yl]methyl N-[4-(3-chloro-4-fluorophenoxy)-2-methoxyphenyl]carbamate (100); [2-(2,6-dioxopiperidin-3-yl)-4-methoxy-3-oxo-2,3-dihydro-1H-isoindol-5-yl]methyl N-[4-(2-methylphenoxy)phenyl]carbamate (101); [2-(2,6-dioxopiperidin-3-yl)-4-methoxy-3-oxo-2,3-dihydro-1H-isoindol-5-yl]methyl N-[4-(2-chloro-4-fluorophenoxy)phenyl]carbamate (102); [2-(2,6-dioxopiperidin-3-yl)-4-methoxy-3-oxo-2,3-dihydro-1H-isoindol-5-yl]methyl N-[4-(2-chloro-4-fluorophenoxy)-2-methylphenyl] carbamate (103); [2-(2,6-dioxopiperidin-3-yl)-4-methoxy-3-oxo-2,3-dihydro-1H-isoindol-5-yl]methyl N-[4-(2-chloro-4-fluorophenoxy)-2-methoxyphenyl]carbamate (104); [2-(2,6-dioxopiperidin-3-yl)-4-methoxy-3-oxo-2,3-dihydro-1H-isoindol-5-yl]methyl N-[4-(2-chloro-4-fluorophenoxy)-2-fluorophenyl]carbamate (105); [2-(2,6-dioxopiperidin-3-yl)-4-methoxy-3-oxo-2,3-dihydro-1H-isoindol-5-yl]methyl N-[4-(2-chloro-5-fluorophenoxy)phenyl] carbamate (106); [2-(2,6-dioxopiperidin-3-yl)-4-methoxy-3-oxo-2,3-dihydro-1H-isoindol-5-yl]methyl N-[4-(2-chlorophenoxy)-2-fluorophenyl]carbamate (107); [2-(2,6-dioxopiperidin-3-yl)-4-methoxy-3-oxo-2,3-dihydro-1H-isoindol-5-yl]methyl N-[4-(2-chloro-4,5-difluorophenoxy)-2-methoxyphenyl]carbamate (108); [2-(2,6-dioxopiperidin-3-yl)-4-methoxy-3-oxo-2,3-dihydro-1H-isoindol-5-yl]methyl N-[4-(2-chloro-4,5-difluorophenoxy)-2-fluorophenyl]carbamate (109); [2-(2,6-dioxopiperidin-3-yl)-4-methoxy-3-oxo-2,3-dihydro-1H-isoindol-5-yl]methyl N-[4-(2,3-difluorophenoxy)phenyl]carbamate (110); [2-(2,6-dioxopiperidin-3-yl)-4-methoxy-3-oxo-2,3-dihydro-1H-isoindol-5-yl]methyl N-[4-(2-chloro-3-fluorophenoxy)phenyl]carbamate (111); [2-(2,6-dioxopiperidin-3-yl)-4-methoxy-3-oxo-2,3-dihydro-1H-isoindol-5-yl]methyl N-[4-(2,6-difluorophenoxy)phenyl]carbamate (112); [2-(2,6-dioxopiperidin-3-yl)-4-methoxy-3-oxo-2,3-dihydro-1H-isoindol-5-yl]methyl N-[4-(naphthalen-1-yloxy)phenyl]carbamate (113); [2-(2,6-dioxopiperidin-3-yl)-4-methoxy-3-oxo-2,3-dihydro-1H-isoindol-5-yl]methyl N-[4-(1-benzothiophen-5-yloxy)phenyl]carbamate (114); [2-(2,6-dioxopiperidin-3-yl)-4-methoxy-3-oxo-2,3-dihydro-1H-isoindol-5-yl]methyl N-[4-(1-benzothiophen-6-yloxy)phenyl]carbamate (115); [2-(2,6-dioxopiperidin-3-yl)-4-methoxy-3-oxo-2,3-dihydro-1H-isoindol-5-yl]methyl N-[4-(2,3-dichlorophenoxy)-2-fluorophenyl]carbamate (116); [2-(2,6-dioxopiperidin-3-yl)-4-methoxy-3-oxo-2,3-dihydro-1H-isoindol-5-yl]methyl N-[4-(3,4-dichlorophenoxy)-2-fluorophenyl]carbamate (117); [2-(2,6-dioxopiperidin-3-yl)-4-methoxy-3-oxo-2,3-dihydro-1H-isoindol-5-yl]methyl N-[4-(3,5-dichlorophenoxy)-2-fluorophenyl]carbamate (118); [2-(2,6-dioxopiperidin-3-yl)-3-oxo-4-(propan-2-yloxy)-2,3-dihydro-1H-isoindol-5-yl]methyl N-[4-(3,4-difluorophenoxy)-2-fluorophenyl]carbamate (119); [2-(2,6-dioxopiperidin-3-yl)-3-oxo-4-(propan-2-yloxy)-2,3-dihydro-1H-isoindol-5-yl]methyl N-[4-(2,3-difluorophenoxy)phenyl]carbamate (120); [2-(2,6-dioxopiperidin-3-yl)-3-oxo-4-(propan-2-yloxy)-2,3-dihydro-1H-isoindol-5-yl]methyl N-[4-(2-fluorophenoxy)phenyl]carbamate (121); [2-(2,6-dioxopiperidin-3-yl)-3-oxo-4-(propan-2-yloxy)-2,3-dihydro-1H-isoindol-5-yl]methyl N-[4-(2-chlorophenoxy)phenyl]carbamate (122); [2-(2,6-dioxopiperidin-3-yl)-3-oxo-4-(propan-2-yloxy)-2,3-dihydro-1H-isoindol-5-yl]methyl N-[4-(3-fluorophenoxy)phenyl]carbamate (123); [2-(2,6-dioxopiperidin-3-yl)-3-oxo-4-(propan-2-yloxy)-2,3-dihydro-1H-isoindol-5-yl]methyl N-[4-(2-chloro-4-fluorophenoxy)phenyl]carbamate (124); {2-[(3S)-2,6-dioxopiperidin-3-yl]-3-oxo-4-(propan-2-yloxy)-2,3-dihydro-1H-isoindol-5-yl}methyl N-[4-(3,4-difluorophenoxy)phenyl]carbamate (125); [4-cyclobutoxy-2-(2,6-dioxopiperidin-3-yl)-3-oxo-2,3-dihydro-1H-isoindol-5-yl] methyl N-[4-(3,4-difluorophenoxy)phenyl]carbamate (126); [4-cyclobutoxy-2-(2,6-dioxopiperidin-3-yl)-3-oxo-2,3-dihydro-1H-isoindol-5-yl]methyl N-[4-(2-chlorophenoxy) phenyl]carbamate (127); [4-cyclobutoxy-2-(2,6-dioxopiperidin-3-yl)-3-oxo-2,3-dihydro-1H-isoindol-5-yl]methyl N-[4-(2-chloro-4-fluorophenoxy)phenyl]carbamate (128); [2-(2,6-dioxopiperidin-3-yl)-4-(oxetan-3-yloxy)-3-oxo-2,3-dihydro-1H-isoindol-5-yl]methyl N-[4-(3,4-difluorophenoxy)phenyl]carbamate (129); [2-(2,6-dioxopiperidin-3-yl)-4-(oxan-4-yloxy)-3-oxo-2,3-dihydro-1H-isoindol-5-yl]methyl N-[4-(3,4-difluorophenoxy)phenyl] carbamate (130); [2-(2,6-dioxopiperidin-3-yl)-4-(oxan-4-yloxy)-3-oxo-2,3-dihydro-1H-isoindol-5-yl]methyl N-[4-(2-chlorophenoxy)phenyl]carbamate (131); [2-(2,6-dioxopiperidin-3-yl)-4-(oxan-4-yloxy)-3-oxo-2,3-dihydro-1H-isoindol-5-yl]methyl N-[4-(3,4-difluorophenoxy)-2-fluorophenyl]carbamate (132); [2-(2,6-dioxopiperidin-3-yl)-4-(oxan-4-yloxy)-3-oxo-2,3-dihydro-1H-isoindol-5-yl]methyl N-[4-(4-fluorophenoxy)phenyl] carbamate (133); [2-(2,6-dioxopiperidin-3-yl)-4-(oxan-4-yloxy)-3-oxo-2,3-dihydro-1H-isoindol-5-yl]methyl N-(4-phenoxyphenyl)carbamate (134); [2-(2,6-dioxopiperidin-3-yl)-4-(oxan-4-yloxy)-3-oxo-2,3-dihydro-1H-isoindol-5-yl]methyl N-[2-fluoro-4-(4-fluorophenoxy) phenyl]carbamate (135); [2-(2,6-dioxopiperidin-3-yl)-4-(oxan-4-yloxy)-3-oxo-2,3-dihydro-1H-isoindol-5-yl]methyl N-[4-(2,4-difluorophenoxy)-2-fluorophenyl]carbamate (136); [4-(cyclohexyloxy)-2-(2,6-dioxopiperidin-3-yl)-3-oxo-2,3-dihydro-1H-isoindol-5-yl]methyl N-[4-(3,4-difluorophenoxy)phenyl]carbamate (137); [2-(2,6-dioxopiperidin-3-yl)-4-(2-methylpropoxy)-3-oxo-2,3-dihydro-1H-isoindol-5-yl]methyl N-[4-(3,4-difluorophenoxy) phenyl]carbamate (138); [2-(2,6-dioxopiperidin-3-yl)-4-(oxan-4-yloxy)-3-oxo-2,3-dihydro-1H-isoindol-5-yl]methyl N-[4-(2,4-difluorophenoxy)phenyl]carbamate (139); [2-(2,6-dioxopiperidin-3-yl)-4-(oxan-4-yloxy)-3-oxo-2,3-dihydro-1H-isoindol-5-yl]methyl N-[4-(3,5-difluorophenoxy)-2-fluorophenyl]carbamate (140); [2-(2,6-dioxopiperidin-3-yl)-4-[(3R)-oxan-3-yloxy]-3-oxo-2,3-dihydro-1H-isoindol-5-yl]methyl N-[4-(3,4-difluorophenoxy) phenyl]carbamate (141); [2-(2,6-dioxopiperidin-3-yl)-4-[(3R)-oxan-3-yloxy]-3-oxo-2,3-dihydro-1H-isoindol-5-yl]methyl N-(4-phenoxyphenyl)carbamate (142); [2-(2,6-dioxopiperidin-3-yl)-4-[(3R)-oxan-3-yloxy]-3-oxo-2,3-dihydro-1H-isoindol-5-yl]methyl N-[4-(3-fluorophenoxy)phenyl]carbamate (143); [2-(2,6-dioxopiperidin-3-yl)-4-[(3R)-oxan-3-yloxy]-3-oxo-2,3-dihydro-1H-isoindol-5-yl]methyl N-[4-(4-fluorophenoxy)phenyl]carbamate (144); [2-(2,6-dioxopiperidin-3-yl)-4-[(3R)-oxan-3-yloxy]-3-oxo-2,3-dihydro-1H-isoindol-5-yl]methyl N-[4-(2,4-difluorophenoxy)phenyl]carbamate (145); [2-(2,6-dioxopiperidin-3-yl)-4-[(3R)-oxan-3-yloxy]-3-oxo-2,3-dihydro-1H-isoindol-5-yl]methyl N-[4-(3,4-difluorophenoxy)-2-fluorophenyl]carbamate (146); [2-(2,6-dioxopiperidin-3-yl)-4-[(3R)-oxan-3-yloxy]-3-oxo-2,3-dihydro-1H-isoindol-5-yl]methyl N-[2-fluoro-4-(4-fluorophenoxy) phenyl]carbamate (147); [2-(2,6-dioxopiperidin-3-yl)-4-[(3R)-oxan-3-yloxy]-3-oxo-2,3-dihydro-1H-isoindol-5-yl]methyl N-[4-(2,4-difluorophenoxy)-2-fluorophenyl]carbamate (148); [2-(2,6-dioxopiperidin-3-yl)-4-[(3R)-oxan-3-yloxy]-3-oxo-2,3-dihydro-1H-isoindol-5-yl]methyl N-[4-(3,5-difluorophenoxy)-2-fluorophenyl]carbamate (149); [2-(2,6-dioxopiperidin-3-yl)-4-[(3R)-oxan-3-yloxy]-3-oxo-2,3-dihydro-1H-isoindol-5-yl]methyl N-[2-fluoro-4-(3,4,5-trifluorophenoxy)phenyl]carbamate (150); [4-(cyclopentyloxy)-2-(2,6-dioxopiperidin-3-yl)-3-oxo-2,3-dihydro-1H-isoindol-5-yl]methyl N-[4-(3,4-difluorophenoxy) phenyl]carbamate (151); [2-(2,6-dioxopiperidin-3-yl)-3-oxo-4-[(3S)-oxolan-3-yloxy]-2,3-dihydro-1H-isoindol-5-yl]methyl N-[4-(3,4-difluorophenoxy)phenyl]carbamate (152); [2-(2,6-dioxopiperidin-3-yl)-3-oxo-4-[(3R)-oxolan-3-yloxy]-2,3-dihydro-1H-isoindol-5-yl] methyl N-[4-(3,4-difluorophenoxy)phenyl]carbamate (153); [2-(2,6-dioxopiperidin-3-yl)-3-oxo-4-[(3R)-oxolan-3-yloxy]-2,3-dihydro-1H-isoindol-5-yl]methyl N-[4-(3,4-difluorophenoxy)-2-fluorophenyl]carbamate (154); [2-(2,6-dioxopiperidin-3-yl)-3-oxo-4-[(3R)-oxolan-3-yloxy]-2,3-dihydro-1H-isoindol-5-yl]methyl N-[4-(2-chloro-4-fluorophenoxy)phenyl]carbamate (155); [2-(2,6-dioxopiperidin-3-yl)-3-oxo-4-[(3R)-oxolan-3-yloxy]-2,3-dihydro-1H-isoindol-5-yl]methyl N-[4-(2,3-difluorophenoxy)phenyl]carbamate (156); [2-(2,6-dioxopiperidin-3-yl)-3-oxo-4-[(3R)-oxolan-3-yloxy]-2,3-dihydro-1H-isoindol-5-yl]methyl N-[4-(2,4-difluorophenoxy)phenyl]carbamate (157); [2-(2,6-dioxopiperidin-3-yl)-3-oxo-4-[(3R)-oxolan-3-yloxy]-2,3-dihydro-1H-isoindol-5-yl]methyl N-[4-(3-chlorophenoxy)phenyl]carbamate (158); [2-(2,6-dioxopiperidin-3-yl)-4-(2-hydroxy-2-methylpropoxy)-3-oxo-2,3-dihydro-1H-isoindol-5-yl]methyl N-[4-(3,4-difluorophenoxy) phenyl]carbamate (159); [2-(2,6-dioxopiperidin-3-yl)-4-(2-hydroxy-2-methylpropoxy)-3-oxo-2,3-dihydro-1H-isoindol-5-yl]methyl N-(4-phenoxyphenyl)carbamate (160); [2-(2,6-dioxopiperidin-3-yl)-4-(2-hydroxy-2-methylpropoxy)-3-oxo-2,3-dihydro-1H-isoindol-5-yl] methyl N-[4-(3,4-difluorophenoxy)-2-fluorophenyl]carbamate (161); [2-(2,6-dioxopiperidin-3-yl)-4-(2-hydroxy-2-methylpropoxy)-3-oxo-2,3-dihydro-1H-isoindol-5-yl]methyl N-[2-fluoro-4-(4-fluorophenoxy)phenyl]carbamate (162); [2-(2,6-dioxopiperidin-3-yl)-4-(2-hydroxy-2-methylpropoxy)-3-oxo-2,3-dihydro-1H-isoindol-5-yl]methyl N-[4-(2,4-difluorophenoxy)-2-fluorophenyl]carbamate (163); [2-(2,6-dioxopiperidin-3-yl)-4-(2-hydroxy-2-methylpropoxy)-3-oxo-2,3-dihydro-1H-isoindol-5-yl]methyl N-[4-(3,5-difluorophenoxy)-2-fluorophenyl]carbamate (164); [2-(2,6-dioxopiperidin-3-yl)-4-(2-hydroxy-2-methylpropoxy)-3-oxo-2,3-dihydro-1H-isoindol-5-yl]methyl N-[2-fluoro-4-(3,4,5-trifluorophenoxy)phenyl]carbamate (165); [2-(2,6-dioxopiperidin-3-yl)-3-oxo-4-{[(1r,4r)-4-hydroxycyclohexyl]oxy}-2,3-dihydro-1H-isoindol-5-yl]methyl N-[4-(3,4-difluorophenoxy)phenyl]carbamate (166); [2-(2,6-dioxopiperidin-3-yl)-3-oxo-4-{[(1s,4s)-4-hydroxycyclohexyl]oxy}-2,3-dihydro-1H-isoindol-5-yl]methyl N-[4-(3,4-difluorophenoxy) phenyl]carbamate (167); [2-(2,6-dioxopiperidin-3-yl)-4-(2-methoxyethoxy)-3-oxo-2,3-dihydro-1H-isoindol-5-yl]methyl N-[4-(3,4-difluorophenoxy)phenyl]carbamate (168); [2-(2,6-dioxopiperidin-3-yl)-4-(2-methoxyethoxy)-3-oxo-2,3-dihydro-1H-isoindol-5-yl]methyl N-[4-(3,4-difluorophenoxy)-2-fluorophenyl]carbamate (169); [2-(2,6-dioxopiperidin-3-yl)-4-(oxepan-4-yloxy)-3-oxo-2,3-dihydro-1H-isoindol-5-yl]methyl N-[4-(3,4-difluorophenoxy) phenyl]carbamate (170); [2-(2,6-dioxopiperidin-3-yl)-3-oxo-4-(pyridin-3-yloxy)-2,3-dihydro-1H-isoindol-5-yl]methyl N-(4-phenoxyphenyl)carbamate (171); [2-(2,6-dioxopiperidin-3-yl)-3-oxo-4-(pyridin-3-yloxy)-2,3-dihydro-1H-isoindol-5-yl]methyl N-[4-(3,4-difluorophenoxy)phenyl]carbamate (172); {2-[(3S)-2,6-dioxopiperidin-3-yl]-3-oxo-4-(3-phenylpropoxy)-2,3-dihydro-1H-isoindol-5-yl}methyl N-[4-(3,4-difluorophenoxy)phenyl] carbamate (173); methyl 2-({5-[({[4-(3,4-difluorophenoxy)phenyl]carbamoyl}oxy)methyl]-2-(2,6-dioxopiperidin-3-yl)-3-oxo-2,3-dihydro-1H-isoindol-4-yl}oxy)acetate (174); {4-[(dimethylcarbamoyl)methoxy]-2-(2,6-dioxopiperidin-3-yl)-3-oxo-2,3-dihydro-1H-isoindol-5-yl}methyl N-[4-(3,4-difluorophenoxy)phenyl]carbamate (175); [4-(carbamoylmethoxy)-2-(2,6-dioxopiperidin-3-yl)-3-oxo-2,3-dihydro-1H-isoindol-5-yl]methyl N-[4-(3,4-difluorophenoxy)phenyl]carbamate (176); [2-(2,6-dioxopiperidin-3-yl)-4-{[ethyl(methyl) carbamoyl]methoxy}-3-oxo-2,3-dihydro-1H-isoindol-5-yl]methyl N-[4-(3,4-difluorophenoxy)phenyl]carbamate (177); [2-(2,6-dioxopiperidin-3-yl)-4-[2-(morpholin-4-yl)-2-oxoethoxy]-3-oxo-2,3-dihydro-1H-isoindol-5-yl]methyl N-[4-(3,4-difluorophenoxy) phenyl]carbamate (178); {4-[(1-acetylpiperidin-4-yl)oxy]-2-(2,6-dioxopiperidin-3-yl)-3-oxo-2,3-dihydro-1H-isoindol-5-yl}methyl N-[4-(3,4-difluorophenoxy)phenyl]carbamate (179); or {2-[(3S)-2,6-dioxo(3,4,4,5,5-²H₅)piperidin-3-yl]-4-methoxy-3-oxo-2,3-dihydro-1H-isoindol-5-yl}methyl N-[4-(3,4-difluorophenoxy)phenyl]carbamate (180).

The compounds of Formula (I) or stereoisomers, tautomers, or salts thereof, are useful to decrease the level of Cyclin E1 protein.

This invention encompasses all combinations of the aspects and/or embodiments of the invention noted herein. It is understood that any and all embodiments of the present invention may be taken in conjunction with any other embodiment or embodiments to describe additional embodiments. It is also to be understood that each individual element of the embodiments is meant to be combined with any and all other elements from any embodiment to describe an additional embodiment.

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. Embodiments identified herein as exemplary or preferred are intended to be illustrative and not limiting.

Unless specifically stated otherwise herein, references made in the singular may also include the plural. For example, "a" and "an" may refer to either one, or one or more.

As used herein, the phrase "compounds and/or salts thereof" refers to at least one compound, at least one salt of the compounds, or a combination thereof. For example, compounds of Formula (I) and/or salts thereof includes a compound of Formula (I); two compounds of Formula (I); a salt of a compound of Formula (I); a compound of Formula (I) and one or more salts of the compound of Formula (I); and two or more salts of a compound of Formula (I).

Unless otherwise indicated, any atom with unsatisfied valences is assumed to have hydrogen atoms sufficient to satisfy the valences.

The definitions set forth herein take precedence over definitions set forth in any patent, patent application, and/or patent application publication.

Listed below are definitions of various terms used to describe the present invention. These definitions apply to the terms as they are used throughout the specification (unless they are otherwise limited in specific instances) either individually or as part of a larger group.

Throughout the specification, groups and substituents thereof may be chosen by one skilled in the field to provide stable moieties and compounds.

In accordance with a convention used in the art, 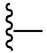 is used in structural formulas herein to depict the bond that is the point of attachment of the moiety or substituent to the core or backbone structure.

The term "amino" refers to the group -NH₂.

The term "oxo" refers to the group =O.

The compounds of the present invention include all isotopes of atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

As used herein, the term "tautomer" refers to each of two or more isomers of a compound that exist together in equilibrium, and are readily interchanged by migration of an atom or group within the molecule. For example, one skilled in the art would readily understand that a 1,2,3-triazole exists in two tautomeric forms as defined above:

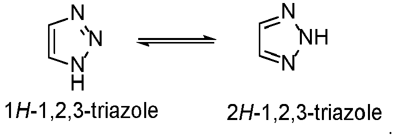

Thus, this disclosure is intended to cover all possible tautomers even when a structure depicts only one of them. For example, the compounds of Formula (I) can exist in tautomer forms:

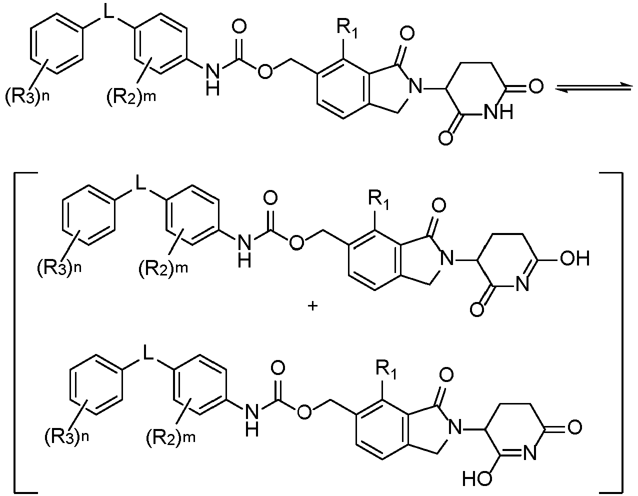

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compounds of Formula (I) can form salts which are also within the scope of this invention. Unless otherwise indicated, reference to an inventive compound is understood to include reference to one or more salts thereof. The term "salt(s)" denotes acidic salt(s) formed with inorganic and/or organic acids. In addition, the term "salt(s) may include zwitterions (inner salts), *e.g.,* when a compound of Formula (I) contains both a basic moiety, such as an amine or a pyridine or imidazole ring, and an acidic moiety, such as a carboxylic acid. Pharmaceutically acceptable (*i.e.,* non-toxic, physiologically acceptable) salts are preferred, such as, for example, acceptable metal and amine salts in which the cation does not contribute significantly to the toxicity or biological activity of the salt. However, other salts may be useful, *e.g.,* in isolation or purification steps which may be employed during preparation, and thus, are contemplated within the scope of the invention. Salts of the compounds of the formula (I) may be formed, for example, by reacting a compound of the Formula (I) with an amount of acid or base, such as an equivalent amount, in a medium such as one in which the salt precipitates or in an aqueous medium followed by lyophilization.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, maleates (formed with maleic acid), 2-hydroxyethanesulfonates, lactates, methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The compounds of Formula (I) can be provided as amorphous solids or crystalline solids. Lyophilization can be employed to provide the compounds of Formula (I) as a solid.

It should further be understood that solvates (e.g., hydrates) of the compounds of Formula (I) are also within the scope of the present invention. The term "solvate" means a physical association of a compound of Formula (I) with one or more solvent molecules, whether organic or inorganic. This physical association includes hydrogen bonding. In certain instances, the solvate will be capable of isolation, for example when one or more solvent molecules are incorporated in the crystal lattice of the crystalline solid. "Solvate" encompasses both solution-phase and isolable solvates. Exemplary solvates include hydrates, ethanolates, methanolates, isopropanolates, acetonitrile solvates, and ethyl acetate solvates. Methods of solvation are known in the art.

Various forms of prodrugs are well known in the art and are described in Rautio, J. et al., Nature Review Drug Discovery, 17, 559-587 (2018).

In addition, compounds of Formula (I), subsequent to their preparation, can be isolated and purified to obtain a composition containing an amount by weight equal to or greater than 99% of a compound of Formula (I) ("substantially pure"), which is then used or formulated as described herein. Such "substantially pure" compounds of Formula (I) are also contemplated herein as part of the present invention.

"Stable compound" and "stable structure" are meant to indicate a compound that is sufficiently robust to survive isolation to a useful degree of purity from a reaction mixture, and formulation into an efficacious therapeutic agent. The present invention is intended to embody stable compounds.

The term "Cyclin E1 degrader" refers to an agent capable of reducing the level of the Cyclin E1 protein by degradation and/or inactivation and/or inhibition and/or reducing the expression levels of the Cyclin E1 protein, or a combination thereof.

As used herein, "Cyclin E1" protein is encoded by the CCNE1 gene. Cyclin E1 is also known as CCNE, cyclin E, E1-type cyclin, G1/S-specific cyclin E1 or pCCNE1. "Cyclin E1" protein includes all human isoforms encoded by CCNE1 gene as listed below-
**Isoform** 1 **(UniPort P24864-1)** 
MPRERRERDAKERDTMKEDGGAEFSARSRKRKANVTVFLQDPDEEMAKIDRTA
RDQCGSQPWDNNAVCADPCSLIPTPDKEDDDRVYPNSTCKPRIIAPSRGSPLPVLS
WANREEVWKIMLNKEKTYLRDQHFLEQHPLLQPKMRAILLDWLMEVCEVYKL
HRETFYLAQDFFDRYMATQENVVKTLLQLIGISSLFIAAKLEEIYPPKLHQFAYVT
DGACSGDEILTMELMIMKALKWRLSPLTIVSWLNVYMQVAYLNDLHEVLLPQY
PQQIFIQIAELLDLCVLDVDCLEFPYGILAASALYHFSSSELMQKVSGYQWCDIEN
CVKWMVPFAMVIRETGSSKLKHFRGVADEDAHNIQTHRDSLDLLDKARAKKAM
LSEQNRASPLPSGLLTPPQSGKKQSSGPEMA (SEQ ID NO: 1)
**Isoform 2 (UniProt P24864-2)** 
MPRERRERDAKERDTMKEDGGAEFSARSRKRKANVTVFLQDPDEEMAKIDRTA
RDQCGSQPWDNNAVCADPCSLIPTPDKEDDDRVYPNSTCKPRIIAPSRGSPLPVLS
WANREEVWKIMLNKEKTYLRDQHFLEQHPLLQPKMRAILLDWLMFIAAKLEEIY
PPKLHQFAYVTDGACSGDEILTMELMIMKALKWRLSPLTIVSWLNVYMQVAYL
NDLHEVLLPQYPQQIFIQIAELLDLCVLDVDCLEFPYGILAASALYHFSSSELMQK
VSGYQWCDIENCVKWMVPFAMVIRETGSSKLKHFRGVADEDAHNIQTHRDSLD
LLDKARAKKAMLSEQNRASPLPSGLLTPPQSGKKQSSGPEMA (SEQ ID NO: 2)
**Isoform 3 (UniProt P24864-3)** 
MKEDGGAEFSARSRKRKANVTVFLQDPDEEMAKIDRTARDQCGSQPWDNNAV
CADPCSLIPTPDKEDDDRVYPNSTCKPRIIAPSRGSPLPVLSWANREEVWKIMLNK
EKTYLRDQHFLEQHPLLQPKMRAILLDWLMEVCEVYKLHRETFYLAQDFFDRY
MATQENVVKTLLQLIGISSLFIAAKLEEIYPPKLHQFAYVTDGACSGDEILTMELM
IMKALKWRLSPLTIVSWLNVYMQVAYLNDLHEVLLPQYPQQIFIQIAELLDLCVL
DVDCLEFPYGILAASALYHFSSSELMQKVSGYQWCDIENCVKWMVPFAMVIRET
GSSKLKHFRGVADEDAHNIQTHRDSLDLLDKARAKKAMLSEQNRASPLPSGLLT
PPQSGKKQSSGPEMA (SEQ ID NO: 3)
**Isoform 4 (UniProt C9J2U0)** 
MKEDGGAEFSARSRKRKANVTVFLQDPDEEMAKIDRTARDQCGSQPWD
NNAVCADPCSLIPTPDKEDDDRVYPNSTCKPRIIAPSRGSPLPVLSWANREEVWKI
MLNKEKTYLRDQHFLEQHPLLQPKMRAILLDWLMEVCEVYKLHRETFYLAQDF
FDRYMATQENVVKTLLQLIGISSLFIAAKLEEIYPPKLHQFAYVTDGACSGDEILT
MELMIMKLLDLCVLDVDCLEFPYGILAASALYHFSSSELMQKVSGYQWCDIENC
VKWMVPFAMVIRETGSSKLKHFRGVADEDAHNIQTHRDSLDLLDKARAKKAML
SEQNRASPLPSGLLTPPQSGKKQSSGPEMA (SEQ ID NO: 4)
**Isoform 5 (UniProt I3L413)** 
DDRVYPNSTCKPRIIAPSRGSPLPVLSWANREEVWKIMLNKEKTYLRDQH
FLEQHPLLQPKMRAILLDWLMEVCEVYKLHRETFYLAQDFFDRYMATQENVVK
TLLQLIGISSLFIAAKLEEIYPPKLHQFAYVTDGACSGDEILTMELMIMKLLDLCVL
DVDCLEFPYGILAASALYHFSSSELMQKVSGYQWCDIENCVKWMVPFAMVIRET
GSSKLKHFRGVADEDAHNIQTHRDSLDLLDKARAKKAMLSEQNRASPLPSGLLT
PPQSGKKQSSGPEMA (SEQ ID NO: 5)
**Isoform 6 (UniProt I3L1Q9)** 
MPRERRERDAKERDTMKEDGGAEFSARSRKRKANVTVDPDEEMAKIDRT
ARDQCGSQPWDNNAVCADPCSLIPTPDKEDDDRVYPNSTCKPRIIAPSRGSPLPVL
SWANREEVWKIMLNKEKTYLRDQHFLEQHPLLQPKMRAILLDWLMEVCEVYKL
HRETFYLAQDFFDRYMATQENVVKTLLQLIGISSLFIAAKLEEIYPPKLHQFAYVT
D (SEQ ID NO: 6)
**Isoform 7 (UniProt I3L1Q9)** 
MPRERRERDAKERDTMKEDGGAEFSARSRKRKANVTVDPDEEMAKIDRT
ARDQCGSQPWDNNAVCADPCSLIPTPDKEDDDRVYPNSTCKPRIIAPSRGSPLPVL
SWANREEVWKILNKEKTYLRDQHFLEQHPLLQPKMRAILLDWLMEVCEVYKLH
RETFYLAQDFFDRYMATQENVVKTLLQLIGISSLFIAAKLEEIYPPKLHQFAYVTD
(SEQ ID NO: 7)
**Isoform 8 (UniProt A0A0G3DHS8)** 
MPRERRERDAKERDTMKEDGGAEFSARSRKRKANVTVFLQDPDEEMAKI
DRTARDQCGSQPWDNNAVCADPCSLIPTPDKEDDDRVYPNSTCKPRIIAPSRGSP
LPVLSWANREEVWKIMLNKEKTYLRDQHFLEQHPLLQPKMRAILLDWLMEVCE
VYKLHRETFYLAQDFFDRYMATQENVVKTLLQLIGISSLFIAAKLEEIYPPKLHQF
AYVTDGACSGDEILTMELMIMKLLDLCVLDVDCLEFPYGILAASALYHFSSSELM
QKVSGYQWCDIENCVKWMVPFAMVIRETGSSKLKHFRGVADEDAHNIQTHRDS
LDLLDKARAKKAMLSEQNRASPLPSGLLTPPQSGKKQSSGPEMA (SEQ ID NO: 8)
**Isoform 9 (UniProt V5W5X2)**

MPRERRERDAKERDTMKEDGGAEFSARSRKRKANVTVFLQDPDEEMAKI
DRTARDQCGSQPWDNNAVCADPCSLIPTPDKEDDDRVYPNSTCKPRIIAPSRGSP
LPVLSWANREEVWKIMLNKEKTYLRDQHFLEQHPLLQPKMRAILLDWLMEEIYP
PKLHQFAYVTDGACSGDEILTMELMIMKALKWRLSPLTIVSWLNVYMQVAYLN
DLHEVLLPQYPQQIFIQIAELLDLCVLDVDCLEFPYGILAASALYHFSSSELMQKV
SGYQWCDIENCVKWMVPFAMVIRETGSSKLKHFRGVADEDAHNIQTHRDSLDL
LDKARAKKAMLSEQNRASPLPSGLLTPPQSGKKQSSGPEMA (SEQ ID NO: 9)

As used herein, the term "contacting" refers to the bringing together of indicated moieties in an *in vitro* system or an *in vivo* system. For example, "contacting" Cyclin E1 protein with a compound of Formula (I) includes the administration of a compound of the present invention to an individual or patient, such as a human, having Cyclin E1 protein as, for example, introducing a compound of Formula (I) into a sample containing a cellular or purified preparation containing Cyclin E1 protein.

The terms "treat," "treating," and "treatment," as used herein, refer to any type of intervention or process performed on, or administering an active agent to, the subject with the objective of reversing, alleviating, ameliorating, inhibiting, or slowing down or preventing the progression, development, severity or recurrence of a symptom, complication, condition or biochemical indicia associated with a disease. In contrast, "prophylaxis" or "prevention" refers to administration to a subject who does not have a disease to prevent the disease from occurring. "Treat," "treating," and "treatment" does not encompass prophylaxis or prevention.

"Therapeutically effective amount" is intended to include an amount of a compound of the present invention alone or an amount of the combination of compounds claimed or an amount of a compound of the present invention in combination with other active ingredients effective to decrease the level of the Cyclin E1 protein in the cells, or effective to treat proliferative disorders, such as cancer.

As used herein, the term "cell" is meant to refer to a cell that is *in vitro, ex vivo* or *in vivo.* In some embodiments, an *ex vivo* cell can be part of a tissue sample excised from an organism such as a mammal. In some embodiments, an *in vitro* cell can be a cell in a cell culture. In some embodiments, an *in vivo* cell is a cell living in an organism such as a mammal.

The term "patient" includes human subjects.

The phrase "pharmaceutically acceptable carrier" as used herein means a pharmaceutically acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation, including, *i.e.,* adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms; and not injurious to the patient.

The term "pharmaceutical composition" means a composition comprising a compound of the invention in combination with at least one additional pharmaceutically acceptable carrier.

### UTILITY

The compounds of Formula (I) are useful for the treatment of cancer.

In one embodiment, a method is provided for the treatment of cancer in a patient comprising administering to said patient a therapeutically effective amount of a compound according to Formula (I), or a stereoisomer, a tautomer, or a pharmaceutically acceptable salt thereof.

One aspect provides a method of treating a disease or disorder by decreasing the level of the Cyclin E1 protein, the method comprising administering to a patient a therapeutically effective amount of an agent to decrease the Cyclin E1 protein level. Suitable agents include small molecules and heterobifunctional molecules such as proteolysis targeting chimeras (PROTAC) molecule. In one embodiment, the disease or disorder is cancer. In another embodiment, the agent is a small compound having a molecular weight of 1000 or less. In a further embodiment, the agent is a compound of Formula (I), a stereoisomer, a tautomer, or a pharmaceutically acceptable salt thereof. In an additional embodiment, the agent is a proteolysis targeting chimera molecule. Examples of small molecules include molecular glues (Sasso et al., Biochemistry 2023, 62, 601-623).

In one embodiment, a method is provided for the treatment of a disease or disorder in a patient comprising administering to said patient a therapeutically effective amount of an agent to decrease the Cyclin E1 protein level, wherein: the Cyclin E1 protein is the amino acid sequence encoded by SEQ ID NOs: 1, 2, 3, 4, 5, 6, 7, 8, or 9. In one embodiment, the disease or disorder is cancer. In another embodiment, the agent is a small compound having a molecular weight of 1000 or less. In a further embodiment, the agent is a compound of Formula (I), a stereoisomer, a tautomer, or a pharmaceutically acceptable salt thereof. In an additional embodiment, the agent is a proteolysis targeting chimera molecule.

In Embodiment 1, a method is provided for the treatment of disease or disorder in a patient comprising administering to said patient a therapeutically effective amount of an agent to decrease the Cyclin E1 protein level, wherein the Cyclin E1 protein level is decreased by at least 30%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 2, a method is provided for the treatment of disease or disorder in a patient comprising administering to said patient a therapeutically effective amount of an agent to decrease the Cyclin E1 protein level, wherein the Cyclin E1 protein level is decreased by at least 40%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 3, a method is provided for the treatment of disease or disorder in a patient comprising administering to said patient a therapeutically effective amount of an agent to decrease the Cyclin E1 protein level, wherein the Cyclin E1 protein level is decreased by at least 50%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 4, a method is provided for the treatment of disease or disorder in a patient comprising administering to said patient a therapeutically effective amount of an agent to decrease the Cyclin E1 protein level, wherein the Cyclin E1 protein level is decreased by at least 60%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 5, a method is provided for the treatment of disease or disorder in a patient comprising administering to said patient a therapeutically effective amount of an agent to decrease the Cyclin E1 protein level, wherein the Cyclin E1 protein level is decreased by at least 70%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 6, a method is provided for the treatment of disease or disorder in a patient comprising administering to said patient a therapeutically effective amount of an agent to decrease the Cyclin E1 protein level, wherein the Cyclin E1 protein level is decreased by at least 80%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 7, a method is provided for the treatment of disease or disorder in a patient comprising administering to said patient a therapeutically effective amount of an agent to decrease the Cyclin E1 protein level, wherein the Cyclin E1 protein level is decreased by at least 90%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 8, a method is provided for the treatment of disease or disorder in a patient comprising administering to said patient a therapeutically effective amount of an agent to decrease the Cyclin E1 protein level, wherein the Cyclin E1 protein level is decreased by at least 95%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 9, a method is provided for the treatment of disease or disorder in a patient comprising administering to said patient a therapeutically effective amount of an agent to decrease the Cyclin E1 protein level, wherein the Cyclin E1 protein level is decreased by at least 98%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiment 10, a method is provided for the treatment of disease or disorder in a patient comprising administering to said patient a therapeutically effective amount of an agent to decrease the Cyclin E1 protein level, wherein the Cyclin E1 protein level is decreased by at least 99%. Included in this embodiment is a method where the disease or disorder is cancer. Also included in this embodiment is a method wherein the agent is the compound of Formula (I), a stereoisomer, a tautomer, or a pharmaceutically acceptable salt thereof.

In Embodiments 1 to 10, the decrease in the protein levels of the Cyclin E1 protein can be measured using the Cyclin E1 Cellular Degradation assay described hereinbelow.

Types of cancers that may be treated with the compound of Formula (I) include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, anal cancer, familiar adenomatous polyposis carcinoma and hereditary non-polyposis colorectal cancer, esophageal carcinoma, nasopharyngeal carcinoma, labial carcinoma, larynx carcinoma, hypopharynx carcinoma, tongue carcinoma, salivary gland carcinoma, thymic carcinoma, esophagogastric cancer, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, mesothelioma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer has amplification, overexpression, or detectable expression of the CCNE1 gene.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is melanoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is lung cancer, including small cell lung cancer (SCLC) and non-small cell lung cancer (NSCLC).

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is mesothelioma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is breast cancer, including ductal carcinoma, invasive ductal carcinoma metastatic breast cancer, triple-negative breast cancer, human epidermal growth factor receptor 2 (HER2)-positive breast cancer, estrogen receptor (ER)-positive breast cancer, hormone receptor-positive breast cancer, and hormone receptor-negative breast cancer.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is prostate cancer, including adenocarcinoma of the prostate and castration-resistant prostate cancer.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is pancreatic cancer, including pancreatic adenocarcinoma, exocrine pancreatic cancer and neuroendocrine pancreatic cancer.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is kidney cancer, including renal cell carcinoma, clear cell renal cell carcinoma, and non-clear cell renal cell carcinomas, papillary renal cell carcinoma, Wilms tumor, and renal sarcoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is gastric cancer, including gastric carcinoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is kidney cancer, including renal carcinoma and kidney parenchymal carcinoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is liver cancer, including hepatocellular carcinoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is ovarian cancer, including ovarian carcinoma.

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is lymphoma, including Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia, and diffuse large B-cell lymphoma (DLBCL).

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is leukemia, including acute lymphatic leukemia (ALL), chronic lymphatic leukemia (CLL), acute myeloid leukemia (AML), chronic myeloid leukemia (CML), adult T-cell leukemia lymphoma, and diffuse large B-cell lymphoma (DLBCL).

In one embodiment, a method is provided for the treatment of cancer, in a patient comprising administering to said patient a therapeutically effective amount of a compound according to Formula (I), or a stereoisomer, a tautomer, or a pharmaceutically acceptable salt thereof, wherein said cancer is multiple myeloma.

The compounds for Formula (I) and pharmaceutical compositions comprising at least one compound of Formula (I) are useful in treating or preventing any diseases or conditions that are associated with the activity of Cyclin E1 proteins. Any method of administration may be used to deliver the compound or pharmaceutical composition to the patient. In certain embodiments, the compound of Formula (I) or pharmaceutical composition comprising at least compound of Formula (I) is administered orally. In other embodiments, the Formula (I) or pharmaceutical composition comprising at least compound of Formula (I) is administered parenterally.

In one embodiment, the present invention provides a combined preparation of a compound of Formula (I), and/or a pharmaceutically acceptable salt thereof; and additional therapeutic agent(s) for simultaneous, separate or sequential use in the treatment and/or prophylaxis of multiple diseases or disorders associated with the activity of Cyclin E1 protein. The combined preparation can be used to decrease the protein level, to decrease the protein activity level, and/or to inhibit the expression level of Cyclin E1 proteins.

In one aspect, the compound(s) of Formula (I) are sequentially administered prior to administration of the immuno-oncology agent. In another aspect, compound(s) of Formula (I) are administered concurrently with the immuno-oncology agent. In yet another aspect, compound(s) of Formula (I) are sequentially administered after administration of the immuno-oncology agent.

In another aspect, compounds of Formula (I) may be co-formulated with an immuno-oncology agent.

Immuno-oncology agents include, for example, a small molecule drug, antibody, or other biologic or small molecule. Examples of biologic immuno-oncology agents include, but are not limited to, cancer vaccines, antibodies, and cytokines. In one aspect, the antibody is a monoclonal antibody. In another aspect, the monoclonal antibody is humanized or human.

In one aspect, the immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DRS, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α 1β2, FAS, FASL, RELT, DR6, TROY, NGFR.

In one aspect, T cell responses can be stimulated by a combination of a compound of Formula (I) and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of Formula (I) for the treatment of cancer include antagonists of inhibitory receptors on NK cells or agonists of activating receptors on NK cells. For example, compounds of Formula (I) can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

In another aspect, compounds of Formula (I) can be used with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

In one aspect, the immuno-oncology agent is a CTLA-4 antagonist, such as an antagonistic CTLA-4 antibody. Suitable CTLA-4 antibodies include, for example, YERVOY (ipilimumab) or tremelimumab.

In another aspect, the immuno-oncology agent is a PD-1 antagonist, such as an antagonistic PD-1 antibody. Suitable PD-1 antibodies include, for example, OPDIVO (nivolumab), KEYTRUDA (pembrolizumab), MEDI-0680 (AMP-514; WO2012/145493), LIBTAYO (cemiplimab), JEMPERLI (dostarlimab), and ZYNYZ (retifanlimab) The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgG1, called AMP-224.

In another aspect, the immuno-oncology agent is a PD-L1 antagonist, such as an antagonistic PD-L1 antibody. Suitable PD-L1 antibodies include, for example, MPDL3280A (RG7446; WO2010/077634), durvalumab (MEDI4736), BMS-936559 (WO207/005874), MSB0010718C (WO2013/79174), TECENTRIQ (atezolizumab), and BAVENCIO (avelumab).

In another aspect, the immuno-oncology agent is a LAG-3 antagonist, such as an antagonistic LAG-3 antibody. Suitable LAG3 antibodies include, for example, BMS-986016 (WO10/19570, WO14/08218), or IMP-731 or IMP-321 (WO08/132601, WO09/44273).

In another aspect, the immuno-oncology agent is a CD137 (4-1BB) agonist, such as an agonistic CD137 antibody. Suitable CD137 antibodies include, for example, urelumab and PF-05082566 (WO12/32433).

In another aspect, the immuno-oncology agent is a GITR agonist, such as an agonistic GITR antibody. Suitable GITR antibodies include, for example, BMS-986153, BMS-986156, TRX-518 (WO06/105021, WO09/009116) and MK-4166 (WO 11/028683).

In another aspect, the immuno-oncology agent is an IDO antagonist. Suitable IDO antagonists include, for example, INCB-024360 (WO206/122150, WO07/75598, WO08/36653, WO08/36642), indoximod, or NLG-919 (WO09/73620, WO09/1156652, WO11/56652, WO12/142237).

In another aspect, the immuno-oncology agent is an OX40 agonist, such as an agonistic OX40 antibody. Suitable OX40 antibodies include, for example, MEDI-6383 or MEDI-6469.

In another aspect, the immuno-oncology agent is an OX40L antagonist, such as an antagonistic OX40 antibody. Suitable OX40L antagonists include, for example, RG-7888 (WO06/029879).

In another aspect, the immuno-oncology agent is a CD40 agonist, such as an agonistic CD40 antibody. In yet another embodiment, the immuno-oncology agent is a CD40 antagonist, such as an antagonistic CD40 antibody. Suitable CD40 antibodies include, for example, lucatumumab or dacetuzumab.

In another aspect, the immuno-oncology agent is a CD27 agonist, such as an agonistic CD27 antibody. Suitable CD27 antibodies include, for example, varlilumab.

In another aspect, the immuno-oncology agent is MGA271 (to B7H3) (WO11/109400).

In another aspect, the immuno-oncology agent is an anti-TIGIT agent. Suitable anti-TIGIT agents include antibodies such as an BMS-986207, tiragolumab, or MK-7684.

In another aspect, the immuno-oncology agent is a KRAS G12C inhibitor. Suitable KRAS G12C inhibitors include LUMAKRAS (sotorasib) or KRAZATI (adagrasib).

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (e.g., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

One or more additional pharmaceutical agents or treatment methods such as, for example, chemotherapeutics or other anti-cancer agents, immune enhancers, immunosuppressants, radiation, anti-tumor vaccines, cytokine therapy (e.g., IL-2 and GM-CSF), and/or tyrosine kinase inhibitors can be optionally used in combination with the compounds of Formula (I) for treatment of Cyclin E1 protein associated diseases, disorders or conditions. The agents can be combined with the present compounds in a single dosage form, or the agents can be administered simultaneously or sequentially as separate dosage forms.

Suitable chemotherapeutic or other anti-cancer agents include, for example, alkylating agents (including, without limitation, nitrogen mustards, ethylenimine derivatives, alkyl sulfonates, nitrosoureas and triazenes) such as uracil mustard, chlormethine, cyclophosphamide (CYTOXAN^{®}), ifosfamide, melphalan, chlorambucil, pipobroman, triethylene-melamine, triethylenethiophosphoramine, busulfan, carmustine, lomustine, streptozocin, dacarbazine, and temozolomide.

Suitable chemotherapeutic or other anti-cancer agents include, for example, antimetabolites (including, without limitation, folic acid antagonists, pyrimidine analogs, purine analogs and adenosine deaminase inhibitors) such as methotrexate, 5-fluorouracil, floxuridine, cytarabine, 6-mercaptopurine, 6-thioguanine, fludarabine phosphate, pentostatine, and gemcitabine.

Suitable chemotherapeutic or other anti-cancer agents further include, for example, certain natural products and their derivatives (for example, vinca alkaloids, antitumor antibiotics, enzymes, lymphokines and epipodophyllotoxins) such as vinblastine, vincristine, vindesine, vinorelbine (Vavelbene^{®}), bleomycin, dactinomycin, daunorubicin, doxorubicin, epirubicin, idarubicin, ara-C, paclitaxel (Taxol), mithramycin, deoxyco-formycin, mitomycin-C, L-asparaginase, interferons (especially IFN-alpha), etoposide, and teniposide.

Also suitable are cytotoxic agents such as epidophyllotoxin; an antineoplastic enzyme inhibitor; a topoisomerase inhibitor such as irinotecan (Camptosar^{®}, CPT-11); procarbazine; mitoxantrone; platinum coordination complexes such as cisplatin and carboplatin; biological response modifiers; growth inhibitors; tegafur; capecitabine; and haematopoietic growth factors.

Other anti-cancer agents include antihormonal or endocrine therapeutic agents. Suitable endocrine therapeutic agents include, but are not limited to, aromatase inhibitors such as letrozole, anastrozole, and exemestane; selective estrogen receptor modulators (SERMs) such as tamoxifen, raloxifene, and droloxifene; and selective estrogen receptor degraders (SERDs) such as fulvestrant.

Other anti-cancer agents include antibody therapeutics such as pertuzumab, antibodies to costimulatory molecules such as CTLA-4, 4-1BB and PD-1, or antibodies to cytokines (IL-10 or TGF-β).

Other anti-cancer agents also include those that block immune cell migration such as antagonists to chemokine receptors, including CCR2 and CCR4.

Other anti-cancer agents also include those that augment the immune system such as adjuvants or adoptive T cell transfer.

Anti-cancer vaccines include dendritic cells, synthetic peptides, DNA vaccines and recombinant viruses.

The pharmaceutical composition of the invention may optionally include at least one signal transduction modulator (STM). A "signal transduction modulator" is an agent that selectively modulates one or more vital steps in signaling pathways, in the normal function of cancer cells, thereby leading to growth arrest and/or apoptosis. Suitable STMs include, but are not limited to: (i) bcr/abl kinase inhibitors such as, for example, STI 571 (GLEEVEC^{®}); (ii) epidermal growth factor (EGF) receptor inhibitors such as, for example, kinase inhibitors (IRESSA^{®}, SSI-774) and antibodies (Imclone: C225 [Goldstein et al., Clin. Cancer Res., 1:1311-1318 (1995)], and Abgenix: ABX-EGF); (iii) her-2/neu receptor inhibitors such as, for example, trastuzumab (Herceptin^{®}) and farnesyl transferase inhibitors (FTI) such as, for example, L-744,832 (Kohl et al., Nat. Med., 1(8):792-797 (1995)); (iv) inhibitors of Akt family kinases or the Akt pathway, such as, for example, rapamycin (see, for example, Sekulic et al., Cancer Res., 60:3504-3513 (200)); (v) cell cycle kinase inhibitors such as, for example, palbociclib (Ibrance^{®}), ribociclib (Kisqali^{®}), and abemaciclib (Verzenio^{®}) (see, for example, Jhaveri et al., ExpertRev. Anticancer Ther., 21(10):1105-1124 (2021)); and (vi) phosphatidyl inositol kinase inhibitors such as, for example, LY294002 (see, for example, Vlahos et al., J. Biol. Chem., 269:5241-5248 (1994)). Alternatively, at least one STM and at least one compound of Formula (I) may be in separate pharmaceutical compositions. In a specific embodiment of the present invention, at least one compound of Formula (I) and at least one STM may be administered to the patient concurrently or sequentially. In other words, at least one compound of Formula (I) may be administered first, at least one STM may be administered first, or at least one compound of Formula (I) and at least one STM may be administered at the same time. Additionally, when more than one compound of Formula (I) and/or STM is used, the compounds may be administered in any order.

In a specific embodiment of the present invention, at least one compound of Formula (I) and at least one chemotherapeutic agent are administered to the patient concurrently or sequentially. In other words, at least one compound of Formula (I) may be administered first, at least one chemotherapeutic agent may be administered first, or at least one compound of Formula (I) and the at least one STM may be administered at the same time. Additionally, when more than one compound of Formula (I) and/or chemotherapeutic agent is used, the compounds may be administered in any order. Similarly, any STM may also be administered at any point in comparison to the administration of the compound of Formula (I).

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (e.g., surgery or radiation treatment). Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

### PHARMACEUTICAL COMPOSITIONS

The invention also provides pharmaceutical compositions which comprise a therapeutically effective amount of one or more of the compounds of Formula (I), formulated together with one or more pharmaceutically acceptable carriers (additives) and/or diluents, and optionally, one or more additional therapeutic agents described above.

The compounds of Formula (I) may be administered by any suitable route, preferably in the form of a pharmaceutical composition adapted to such a route, and in a dose effective for the treatment intended. The compounds and compositions of the compound of Formula (I) can be administered for any of the uses described herein by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques *(e.g.,* as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

For oral administration, the pharmaceutical composition may be in the form of, for example, a tablet, capsule, liquid capsule, suspension, or liquid. The pharmaceutical composition is preferably made in the form of a dosage unit containing a particular amount of the active ingredient. For example, the pharmaceutical composition may be provided as a tablet or capsule comprising an amount of active ingredient in the range of from about 0.1 to 1000 mg, preferably from about 0.25 to 250 mg, and more preferably from about 0.5 to 100 mg. A suitable daily dose for a human or other mammal may vary widely depending on the condition of the patient and other factors, but can be determined using routine methods.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the invention can contain at least one agent selected from sweetening agents, flavoring agents, coloring agents, demulcents, antioxidants, and preserving agents.

A tablet can, for example, be prepared by admixing at least one compound of Formula (I) and/or at least one pharmaceutically acceptable salt thereof with at least one non-toxic pharmaceutically acceptable excipient suitable for the manufacture of tablets. Exemplary excipients include, but are not limited to, for example, inert diluents, such as, for example, calcium carbonate, sodium carbonate, lactose, calcium phosphate, and sodium phosphate; granulating and disintegrating agents, such as, for example, microcrystalline cellulose, sodium crosscarmellose, corn starch, and alginic acid; binding agents, such as, for example, starch, gelatin, polyvinyl-pyrrolidone, and acacia; and lubricating agents, such as, for example, magnesium stearate, stearic acid, and talc. Additionally, a tablet can either be uncoated, or coated by known techniques to either mask the bad taste of an unpleasant tasting drug, or delay disintegration and absorption of the active ingredient in the gastrointestinal tract thereby sustaining the effects of the active ingredient for a longer period. Exemplary water soluble taste masking materials, include, but are not limited to, hydroxypropyl-methylcellulose and hydroxypropylcellulose. Exemplary time delay materials, include, but are not limited to, ethyl cellulose and cellulose acetate butyrate.

Hard gelatin capsules can, for example, be prepared by mixing at least one compound of Formula (I) and/or at least one salt thereof with at least one inert solid diluent, such as, for example, calcium carbonate; calcium phosphate; and kaolin.

Soft gelatin capsules can, for example, be prepared by mixing at least one compound of Formula (I) and/or at least one pharmaceutically acceptable salt thereof with at least one water soluble carrier, such as, for example, polyethylene glycol; and at least one oil medium, such as, for example, peanut oil, liquid paraffin, and olive oil.

An aqueous suspension can be prepared, for example, by admixing at least one compound of Formula (I) and/or at least one pharmaceutically acceptable salt thereof with at least one excipient suitable for the manufacture of an aqueous suspension. Exemplary excipients suitable for the manufacture of an aqueous suspension, include, but are not limited to, for example, suspending agents, such as, for example, sodium carboxymethylcellulose, methylcellulose, hydroxypropylmethyl-cellulose, sodium alginate, alginic acid, polyvinyl-pyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example heptadecaethylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) and/or at least one pharmaceutically acceptable salt thereof in either a vegetable oil, such as, for example, arachis oil; olive oil; sesame oil; and coconut oil; or in mineral oil, such as, for example, liquid paraffin. An oily suspension can also contain at least one thickening agent, such as, for example, beeswax; hard paraffin; and cetyl alcohol. In order to provide a palatable oily suspension, at least one of the sweetening agents already described hereinabove, and/or at least one flavoring agent can be added to the oily suspension. An oily suspension can further contain at least one preservative, including, but not limited to, for example, an anti-oxidant, such as, for example, butylated hydroxyanisol, and alpha-tocopherol.

Dispersible powders and granules can, for example, be prepared by admixing at least one compound of Formula (I) and/or at least one pharmaceutically acceptable salt thereof with at least one dispersing and/or wetting agent; at least one suspending agent; and/or at least one preservative. Suitable dispersing agents, wetting agents, and suspending agents are as already described above. Exemplary preservatives include, but are not limited to, for example, anti-oxidants, e.g., ascorbic acid. In addition, dispersible powders and granules can also contain at least one excipient, including, but not limited to, for example, sweetening agents; flavoring agents; and coloring agents.

An emulsion of at least one compound of Formula (I) and/or at least one pharmaceutically acceptable salt thereof can, for example, be prepared as an oil-in-water emulsion. The oily phase of the emulsions comprising compounds of Formula (I) may be constituted from known ingredients in a known manner. The oil phase can be provided by, but is not limited to, for example, a vegetable oil, such as, for example, olive oil and arachis oil; a mineral oil, such as, for example, liquid paraffin; and mixtures thereof. While the phase may comprise merely an emulsifier, it may comprise a mixture of at least one emulsifier with a fat or an oil or with both a fat and an oil. Suitable emulsifying agents include, but are not limited to, for example, naturally-occurring phosphatides, e.g., soybean lecithin; esters or partial esters derived from fatty acids and hexitol anhydrides, such as, for example, sorbitan monooleate; and condensation products of partial esters with ethylene oxide, such as, for example, polyoxyethylene sorbitan monooleate. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. An emulsion can also contain a sweetening agent, a flavoring agent, a preservative, and/or an antioxidant. Emulsifiers and emulsion stabilizers suitable for use in the formulation of the present invention include Tween 60, Span 80, cetostearyl alcohol, myristyl alcohol, glyceryl monostearate, sodium lauryl sulfate, glyceryl distearate alone or with a wax, or other materials well known in the art.

The compounds of Formula (I) and/or at least one pharmaceutically acceptable salt thereof can, for example, also be delivered intravenously, subcutaneously, and/or intramuscularly via any pharmaceutically acceptable and suitable injectable form. Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e. Captisol), cosolvent solubilization (i.e. propylene glycol) or micellar solubilization (i.e. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

A sterile injectable oil-in-water microemulsion can, for example, be prepared by 1) dissolving at least one compound of Formula (I) in an oily phase, such as, for example, a mixture of soybean oil and lecithin; 2) combining the Formula (I) containing oil phase with a water and glycerol mixture; and 3) processing the combination to form a microemulsion.

A sterile aqueous or oleaginous suspension can be prepared in accordance with methods already known in the art. For example, a sterile aqueous solution or suspension can be prepared with a non-toxic parenterally-acceptable diluent or solvent, such as, for example, 1,3-butane diol; and a sterile oleaginous suspension can be prepared with a sterile non-toxic acceptable solvent or suspending medium, such as, for example, sterile fixed oils, e.g., synthetic mono- or diglycerides; and fatty acids, such as, for example, oleic acid.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Allen, L. V. Jr. et al. Remington: The Science and Practice of Pharmacy (2 Volumes), 22nd Edition (2012), Pharmaceutical Press.

Pharmaceutically acceptable carriers, adjuvants, and vehicles that may be used in the pharmaceutical compositions of this invention include, but are not limited to, ion exchangers, alumina, aluminum stearate, lecithin, self-emulsifying drug delivery systems (SEDDS) such as d-alpha-tocopherol polyethyleneglycol 1000 succinate, surfactants used in pharmaceutical dosage forms such as Tweens, polyethoxylated castor oil such as CREMOPHOR surfactant (BASF), or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as alpha-, beta-, and gamma-cyclodextrin, or chemically modified derivatives such as hydroxyalkylcyclodextrins, including 2- and 3-hydroxypropyl-cyclodextrins, or other solubilized derivatives may also be advantageously used to enhance delivery of compounds of the formulae described herein.

The pharmaceutically active compounds of this invention can be processed in accordance with conventional methods of pharmacy to produce medicinal agents for administration to patients, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

For therapeutic purposes, the active compounds of this invention are ordinarily combined with one or more adjuvants appropriate to the indicated route of administration. If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

The amounts of compounds that are administered and the dosage regimen for treating a disease condition with the compounds and/or compositions of this invention depends on a variety of factors, including the age, weight, sex, the medical condition of the subject, the type of disease, the severity of the disease, the route and frequency of administration, and the particular compound employed. Thus, the dosage regimen may vary widely, but can be determined routinely using standard methods. A daily dose of about 0.001 to 100 mg/kg body weight, preferably between about 0.0025 and about 50 mg/kg body weight and most preferably between about 0.005 to 10 mg/kg body weight, may be appropriate. The daily dose can be administered in one to four doses per day. Other dosing schedules include one dose per week and one dose per two day cycle.

Pharmaceutical compositions of this invention comprise at least one compound of Formula (I) and/or at least one pharmaceutically acceptable salt thereof, and optionally an additional agent selected from any pharmaceutically acceptable carrier, adjuvant, and vehicle. Alternate compositions of this invention comprise a compound of the Formula (I) described herein, and a pharmaceutically acceptable carrier, adjuvant, or vehicle.

The present invention also includes pharmaceutical kits useful, for example, in the treatment or prevention of Cyclin E1 protein-associated diseases or disorders, and other diseases referred to herein which include one or more containers containing a pharmaceutical composition comprising a therapeutically effective amount of a compound of Formula (I). Such kits can further include, if desired, one or more of various conventional pharmaceutical kit components, such as, for example, containers with one or more pharmaceutically acceptable carriers, additional containers, as will be readily apparent to those skilled in the art. Instructions, either as inserts or as labels, indicating quantities of the components to be administered, guidelines for administration, and/or guidelines for mixing the components, can also be included in the kit.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.001 to about 5000 mg per day, preferably between about 0.01 to about 1000 mg per day, and most preferably between about 0.1 to about 250 mg per day. Intravenously, the most preferred doses will range from about 0.01 to about 10 mg/kg/minute during a constant rate infusion. Compounds of Formula (I) may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, e.g., oral tablets, capsules, elixirs, and syrups, and consistent with conventional pharmaceutical practices.

Dosage forms (pharmaceutical compositions) suitable for administration may contain from about 1 milligram to about 200 milligrams of active ingredient per dosage unit. In these pharmaceutical compositions the active ingredient will ordinarily be present in an amount of about 0.1-95% by weight based on the total weight of the composition.

A typical capsule for oral administration contains at least one of the compounds of Formula (I) (250 mg), lactose (75 mg), and magnesium stearate (15 mg). The mixture is passed through a 60 mesh sieve and packed into a No. 1 gelatin capsule.

A typical injectable preparation is produced by aseptically placing at least one of the compounds of Formula (I) (250 mg) into a vial, aseptically freeze-drying and sealing. For use, the contents of the vial are mixed with 2 mL of physiological saline, to produce an injectable preparation.

The present invention includes within its scope pharmaceutical compositions comprising, as an active ingredient, a therapeutically effective amount of at least one of the compounds of Formula (I), alone or in combination with a pharmaceutical carrier. Optionally, compounds of Formula (I) can be used alone, in combination with other compounds of Formula (I), or in combination with one or more other therapeutic agent(s), *e.g.,* an anticancer agent or other pharmaceutically active material.

Regardless of the route of administration selected, the compounds of Formula (I), which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the therapeutic response for a particular patient, composition, and mode of administration, without being toxic to the patient.

The selected dosage level will depend upon a variety of factors including the activity of the particular compound of Formula (I) employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of Formula (I) employed in the pharmaceutical composition at levels lower than that required in order to achieve the therapeutic effect and gradually increase the dosage until the effect is achieved.

In general, a suitable daily dose of a compound of Formula (I) will be that amount of the compound which is the lowest dose effective to produce a therapeutic effect. Such an effective dose will generally depend upon the factors described above. Generally, oral, intravenous, intracerebroventricular and subcutaneous doses of the compounds of Formula (I) for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of Formula (I) to be administered alone, it is preferable to administer the compound as a pharmaceutical formulation (composition).

The above other therapeutic agents, when employed in combination with the compounds of Formula (I), may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds.

### METHODS OF PREPARATION

The compounds of the present invention can be prepared in a number of ways well known to one skilled in the art of organic synthesis. The compounds of the present invention can be synthesized using the methods described below, together with synthetic methods know in the art of synthetic organic chemistry, or variations thereon as appreciated by those skilled in the art. Preferred methods include, but are not limited to, those described below.

The compounds of this invention may be prepared using the reactions and techniques described in this section. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and work up procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art and alternate methods must then be used. This will sometimes require a judgment to modify the order of the synthetic steps or to select one particular process scheme over another in order to obtain a desired compound of the invention. It will also be recognized that another major consideration in the planning of any synthetic route in this field is the judicious choice of the protecting group used for protection of the reactive functional groups present the compounds described in this invention. An authoritative account describing the many alternatives to the trained practitioner is Wuts, P. G.; Greene, T. W. Greene's Protective Groups in Organic Synthesis, 4th ed.; Wiley-Interscience: Hoboken, N.J., 2007.

### EXAMPLES

The following examples illustrate the particular embodiments of the present invention and do not limit the scope of the present invention. Chemical abbreviations and symbols as well as scientific abbreviations and symbols have their usual and customary meanings unless otherwise specified. Additional abbreviations employed in the Examples and elsewhere in this application are defined above. Common intermediates are generally useful for the preparation of more than one Example. Compounds of the Examples are identified by the example and step in which they were prepared (e.g., "1-A" denotes the Example 1, step A), or by the example only where the compound is the title compound of the example (for example, "1" denotes the title compound of Example 1). In some instances, alternate preparations of intermediates or examples are described. Frequently chemists skilled in the art of synthesis may devise alternative preparations which may be desirable based on one or more considerations such as shorter reaction time, less expensive starting materials, ease of operation or isolation, improved yield, amenable to catalysis, avoidance of toxic reagents, accessibility of specialized instrumentation, and decreased number of linear steps, etc. The intent of describing alternative preparations is to further enable the preparation of the examples of this invention. In some instances, some functional groups in the outlined examples and claims may be replaced by well-known bioisosteric replacements known in the art, for example, replacement of a carboxylic acid group with a tetrazole or a phosphate moiety.

### ABBREVIATIONS

- AIBN: azobisiosbutyronitrile
- Boc: tert-butoxycarbonyl
- BPR: back pressure regulator
- cataCXium^{®} A Pd G3: mesylate[(di(1-adamantyl)-n-butylphosphine)-2-(2'-amino-1,1'-biphenyl)]palladium(II)
- CH₂Cl₂: dichloromethane
- DCE: 1,2-dichloroethane
- DCM: dichloromethane
- DIEA: N,N-diisopropylethylamine
- DMA: N,N-dimethylacetamide
- DMF: N,N-dimethylformamide
- DMSO: dimethyl sulfoxide
- EtOAc: ethyl acetate
- EtOH: ethanol
- GCMS: gas chromatography mass spectrometry
- h: hour(s)
- HATU: hexafluorophosphate azabenzotriazole tetramethyl uronium
- HCl: hydrochloric acid
- Hex: hexanes
- HFIP: 1,1,1,3,3,3-hexafluoroisopropanol
- HPLC: high performance liquid chromatography
- LCMS: liquid chromatography mass spectrometry
- MeOH: methanol
- MgSO₄: magnesium sulfate
- min: minute(s)
- mL: milliliter(s)
- Na₂SO₄: sodium sulfate
- NBS: N-bromosuccinimide
- NMP: N-methylpyrrolidinone
- Pd(dppf)Cl₂: [1,1'-bis(diphenylphosphino)ferrocene]dichloropalladium(II)
- Pd(PPh₃)₄: tetrakis(triphenylphosphine)palladium(0)
- RT: retention time
- SFC: supercritical fluid chromatography
- SiO₂: silica gel
- TBS: tert-butyldimethylsilyl
- TEA: triethylamine
- TFA: trifluoroacetic acid
- THF: tetrahydrofuran
- VCD: vibrational circular dichroism
- XPhos Pd G2: second generation XPhos pre-catalyst

### ANALYTICAL HPLC CONDITIONS

Method A: Column: Acquity UPLC BEH C18, 3.0 mm x 50 mm, 1.7 µm particles; Mobile Phase A: acetonitrile/water (2:98) with 5 mM ammonium formate (pH 3.3); Mobile Phase: B: acetonitrile/water (98:2) with 5 mM ammonium formate (pH 3.3); Temperature: 25 °C; Gradient: 5-98%B (0.0-1.5 min), 98%B (1.5-2.0 min); Flow: 0.7 mL/min; Detection: UV (220 nm) and MS (DUIS-ESI+APCI).

Method B: Column: Acquity UPLC BEH C18, 2.1 mm x 50 mm, 1.7 µm particles; Mobile Phase A: water with 0.1% TFA; Mobile Phase: B: acetonitrile with 0.1% TFA; Temperature: 25 °C; Gradient: 5%B (0.0-0.5 min), 5-98%B (0.5-2.5 min), 98%B (2.5-3.0 min); Flow: 0.7 mL/min; Detection: UV (220 nm) and MS (DUIS-ESI+APCI).

Method C: Column: XBridge C18, 2.1 mm x 50 mm, 1.7 µm particles; Mobile Phase A: acetonitrile/water (5:95) with 10 mM ammonium acetate; Mobile Phase B: acetonitrile/water (95:5) with 10 mM ammonium acetate; Temperature: 50 °C; Gradient: 0-100%B (0.0-3.0 min), 100%B (3.0-3.5 min); Flow: 1.0 mL/min; Detection: UV (220 nm) and MS (ESI⁺).

Method D: Column: XBridge C18, 2.1 mm x 50 mm, 1.7 µm particles; Mobile Phase A: acetonitrile/water (5:95) with 0.05% TFA; Mobile Phase B: acetonitrile/water (95:5) with 0.05% TFA; Temperature: 50 °C; Gradient: 0-100%B (0.0-3.0 min), 100%B (3.0-3.5 min); Flow: 1.0 mL/min; Detection: UV (220 nm) and MS (ESI⁺).

Method E: Column: Acquity UPLC BEH C18, 2.1 mm x 50 mm, 1.7 µm particles; Mobile Phase A: acetonitrile/water (5:95) with 0.05% TFA; Mobile Phase B: acetonitrile/water (95:5) with 0.05% TFA; Temperature: 50 °C; Gradient: 0-100%B (0.0-1.0 min), 100%B (1.0-1.5 min); Flow: 1.0 mL/min; Detection: UV (220 nm) and MS (DUIS-ESI+APCI).

Method F: Column: Acquity UPLC BEH C18, 2.1 mm x 50 mm, 1.7 µm particles; Mobile Phase A: acetonitrile/water (5:95) with 10 mM ammonium acetate; Mobile Phase B: acetonitrile/water (95:5) with 10 mM ammonium acetate; Temperature: 50 °C; Gradient: 0-100%B (0.0-1.0 min), 100%B (1.0-1.5 min); Flow: 1.0 mL/min; Detection: UV (220 nm) and MS (DUIS-ESI+APCI).

Method G: Column: CHIRALPAK AS, 0.46 cm x 25 cm, 5 µm particles; Mobile Phase: carbon dioxide/MeOH (70:30); Temperature: 40 °C; BPR Pressure: 140 bar; Flow: 3.0 mL/min; Detection: UV (200-400 nm).

Method H: Column: Acquity UPLC BEH C18, 2.1 mm x 50 mm, 1.7 µm particles; Mobile Phase A: water with 0.1% TFA; Mobile Phase B: acetonitrile with 0.1% TFA; Temperature: 25 °C; Gradient: 20-98%B (0.0-1.5 min), 98%B (1.5-2.0 min); Flow: 0.7 mL/min; Detection: UV (254 nm) and MS (DUIS-ESI+APCI).

Method I: Column: Acquity UPLC BEH C18, 2.1 mm x 50 mm, 1.7 µm particles; Mobile Phase A: water with 0.1% TFA; Mobile Phase B: acetonitrile with 0.1% TFA; Temperature: 25 °C; Gradient: 60-98%B (0.0-1.0 min), 98%B (1.0-2.0 min); Flow: 0.7 mL/min; Detection: UV (220 nm) and MS (DUIS-ESI+APCI).

Method J: Column: Kinetex XB-C18, 3 mm x 75 mm, 2.6 µm particles; Mobile Phase A: water with 0.1% TFA; Mobile Phase B: acetonitrile with 0.1% TFA; Temperature: 25 °C; Gradient: 5-95%B (0.0-2.5 min), 95%B (2.5-4.0 min); Flow: 1.0 mL/min; Detection: UV (220 nm) and MS (DUIS-ESI+APCI).

Method K: Kinetex XB-C18, 3 mm x 75 mm, 2.6 µm particles; Mobile Phase A: acetonitrile/water (2:98) with 5 mM ammonium formate; Mobile Phase B: acetonitrile/water (98:2) with 5 mM ammonium formate; Temperature: 25 °C; Gradient: 20-100%B (0.0-4.0 min), 100%B (4.0-4.6 min); Flow: 1.0 mL/min; Detection: UV (220 nm) and MS (DUIS-ESI+APCI).

Method L: Column: Acquity UPLC BEH C18, 3.0 mm x 50 mm, 1.7 µm particles; Mobile Phase A: water with 0.1% TFA; Mobile Phase: B: acetonitrile with 0.1% TFA; Temperature: 25 °C; Gradient: 20-98%B (0.0-1.5 min), 98%B (1.5-2.0 min); Flow: 0.7 mL/min; Detection: UV (220 nm) and MS (DUIS-ESI+APCI).

Method M: Column: Acquity UPLC BEH C18, 3.0 mm x 50 mm, 1.7 µm particles; Mobile Phase A: acetonitrile/water (2:98) with 5 mM ammonium formate (pH 3.3); Mobile Phase: B: acetonitrile/water (98:2) with 5 mM ammonium formate (pH 3.3); Temperature: 25 °C; Gradient: 20-98%B (0.0-1.5 min), 98%B (1.5-2.0 min); Flow: 0.7 mL/min; Detection: UV (220 nm) and MS (DUIS-ESI+APCI).

Method N: Column: Acquity UPLC BEH C18, 2.1 mm x 50 mm, 1.7 µm particles; Mobile Phase A: water with 0.1% TFA; Mobile Phase B: acetonitrile with 0.1% TFA; Temperature: 25 °C; Gradient: 5-98%B (0.0-1.5 min), 98%B (1.5-2.0 min); Flow: 0.7 mL/min; Detection: UV (220 nm) and MS (DUIS-ESI+APCI).

Method O: Column: Cellulose-4, 0.46 cm x 25 cm, 5 µm particles; Mobile Phase: carbon dioxide/MeOH (50:50); Temperature: 40 °C; BPR Pressure: 140 bar; Flow: 3.0 mL/min; Detection: UV (200-400 nm).

Method P: Kinetex XB-C18, 3 mm x 75 mm, 2.6 µm particles; Mobile Phase A: acetonitrile/water (2:98) with 5 mM ammonium formate; Mobile Phase B: acetonitrile/water (98:2) with 5 mM ammonium formate; Temperature: 25 °C; Gradient: 40-100%B (0.0-3.5 min), 100%B (3.5-4.5 min); Flow: 1.0 mL/min; Detection: UV (220 nm) and MS (DUIS-ESI+APCI).

Method Q: Kinetex XB-C18, 3 mm x 75 mm, 2.6 µm particles; Mobile Phase A: acetonitrile/water (2:98) with 5 mM ammonium formate; Mobile Phase B: acetonitrile/water (98:2) with 5 mM ammonium formate; Temperature: 25 °C; Gradient: 20-100%B (0.0-4.0 min), 100%B (4.0-4.06 min); Flow: 1.0 mL/min; Detection: UV (220 nm) and MS (DUIS-ESI+APCI).

Method R: Kinetex XB-C18, 3 mm x 75 mm, 2.6 µm particles; Mobile Phase A: acetonitrile/water (2:98) with 5 mM ammonium formate; Mobile Phase B: acetonitrile/water (98:2) with 5 mM ammonium formate; Temperature: 25 °C; Gradient: 80-100%B (0.0-4.0 min), 100%B (4.0-4.06 min); Flow: 1.0 mL/min; Detection: UV (220 nm) and MS (DUIS-ESI+APCI).

Method S: Column: Kinetex XB-C18, 3 mm x 75 mm, 2.6 µm particles; Mobile Phase A: water with 0.1% TFA; Mobile Phase B: acetonitrile with 0.1% TFA; Temperature: 25 °C; Gradient: 5-95%B (0.0-2.5 min), 95%B (2.5-4.5 min); Flow: 1.0 mL/min; Detection: UV (220 nm) and MS (DUIS-ESI+APCI).

Method T: Column: (R,R) Whelk-O 1, 0.46 cm x 25 cm, 5 µm particles; Mobile Phase A: carbon dioxide; Mobile Phase B: methanol with 0.1% ammonium hydroxide; Temperature: 40 °C; BPR Pressure: 140 bar; Gradient: 35-70%B (0.0-6.0 min), 70%B (6.0-9.0 min); Flow: 3.0 mL/min; Detection: UV (200 nm).

### EXAMPLE 1

### [2-(2,6-dioxopiperidin-3-yl)-4-methoxy-3-oxo-2,3-dihydro-1H-isoindol-5-yl]methyl N-[4-(3,4-difluorophenoxy)phenyl]carbamate

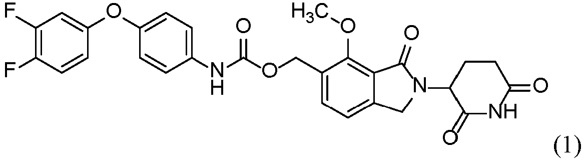

### Intermediate 1A: methyl 3-bromo-2-methoxy-6-methylbenzoate

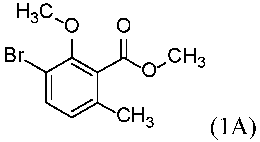

To a solution of 3-bromo-2-methoxybenzoic acid (13 g, 56.3 mmol) in HFIP (250 mL, 56.3 mmol) was added potassium carbonate (15.55 g, 113 mmol), silver carbonate (31.0 g, 113 mmol), pentamethylcyclopentadienyliridium(III) chloride dimer (4.48 g, 5.63 mmol), and potassium methyltrifluoroborate (13.72 g, 113 mmol) at room temperature. The resulting mixture was stirred at 100 °C for 4 h. The reaction mixture was then passed through a sintered funnel, and the filtrate was concentrated under reduced pressure. The crude residue was diluted with ethyl acetate (100 mL) and aqueous HCl (1.5 N). The layers were separated, and the organic phase was washed with brine (90 mL), dried (Na₂SO₄), filtered, and concentrated to afford crude 3-bromo-2-methoxy-6-methylbenzoic acid (12 g).

To a solution of crude 3-bromo-2-methoxy-6-methylbenzoic acid (12 g) in DMF (70 mL) were added cesium carbonate (15.95 g, 49.0 mmol) and methyl iodide (3.06 mL, 49.0 mmol) under nitrogen atmosphere. The resulting mixture was stirred at room temperature for 12 h. The reaction was then quenched with cold water (100 mL). The reaction mixture was extracted with ethyl acetate (3 x 90 mL). The organic layer was washed with brine (100 mL), dried (Na₂SO₄), filtered, and concentrated. The crude material was purified by flash chromatography (SiO₂, 10% ethyl acetate/petroleum ether) to afford methyl 3-bromo-2-methoxy-6-methylbenzoate (12 g, 82% yield over two steps). ¹H NMR (400 MHz, CDCl₃): δ 7.49 (d, *J* = 8.0 Hz, 1H) , 6.88 (d, *J =* 8.2 Hz, 1H), 3.96 (s, 3H), 3.90 (s, 3H), 2.29 (s, 3H).

### Intermediate 1B: 3-(6-bromo-7-methoxy-1-oxoisoindolin-2-yl)piperidine-2,6-dione

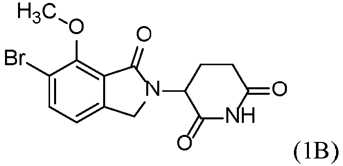

To a solution of methyl 3-bromo-2-methoxy-6-methylbenzoate (12 g, 46.3 mmol) in DCE (100 mL) was added NBS (9.89 g, 55.6 mmol) and AIBN (0.761 g, 4.63 mmol) under nitrogen atmosphere. The resulting mixture was stirred at 80 °C for 90 min. The reaction mixture was then concentrated. The residue was taken up in petroleum ether, filtered, and concentrated to afford crude methyl 3-bromo-6-(bromomethyl)-2-methoxybenzoate (15 g).

To a solution of crude methyl 3-bromo-6-(bromomethyl)-2-methoxybenzoate (15 g) and 3-aminopiperidine-2,6-dione HCl (10.23 g, 62.1 mmol) in DMF (100 mL) was added DIEA (16.28 mL, 93 mmol) under nitrogen atmosphere. The resulting mixture was stirred at 100 °C for 2 h. The reaction mixture was then concentrated under reduced pressure to remove excess solvent. Cold water (200 mL) was added, resulting in the formation of a precipitate. The mixture was filtered through a Buchner funnel and dried under vacuum to afford 3-(6-bromo-7-methoxy-1-oxoisoindolin-2-yl)piperidine-2,6-dione (10 g). LCMS (Method A): retention time 1.12 min, [M+H]⁺ 353.0, 355.0.

### Intermediate 1C: 3-(6-(hydroxymethyl)-7-methoxy-1-oxoisoindolin-2-yl)piperidine-2,6-dione

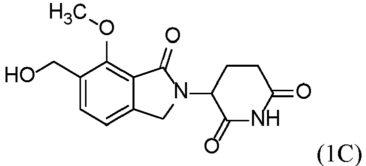

A solution of 3-(6-bromo-7-methoxy-1-oxoisoindolin-2-yl)piperidine-2,6-dione (2.0 g, 5.66 mmol) in 1,4-dioxane (20 mL) and DMA (5 mL) was purged with nitrogen for 5 min, and then Pd(PPh₃)₄ (0.654 g, 0.566 mmol) and (tributylstannyl)methanol (3.64 g, 11.3 mmol) were added. The resulting mixture was stirred at 120 °C for 12 h. The reaction mixture was then concentrated, followed by addition of ethyl acetate, which resulted in the formation of a precipitate. The solid was filtered and dried under vacuum. The crude material was purified by reversed phase chromatography (30% acetonitrile in water with 0.1% TFA) to afford 3-(6-(hydroxymethyl)-7-methoxy-1-oxoisoindolin-2-yl)piperidine-2,6-dione (1.0 g, 58% yield). LCMS (Method B): retention time 1.21 min, [M+H]⁺ 305.0.

### Example 1:

A mixture of 3-(6-(hydroxymethyl)-7-methoxy-1-oxoisoindolin-2-yl)piperidine-2,6-dione (25 mg, 0.082 mmol), 4-(3,4-difluorophenoxy)benzoic acid (26.7 mg, 0.107 mmol), diphenyl phosphorazidate (29.4 mg, 0.107 mmol), and TEA (0.029 mL, 0.205 mmol) in 1,4-dioxane (1 mL) was heated at 110 °C for 5 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 46-66%B (0.0-20.0 min), 66-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-4-methoxy-3-oxo-2,3-dihydro-1H-isoindol-5-yl]methyl N-[4-(3,4-difluorophenoxy)phenyl]carbamate (15.5 mg, 34% yield). LCMS (Method C): retention time 2.00 min, [M+H]⁺ 552.1; ¹H NMR (500 MHz, DMSO-d₆) δ 10.97 (br s, 1H), 9.76 (br s, 1H), 7.66 (d, J=7.8 Hz, 1H), 7.48 (br d, J=8.9 Hz, 2H), 7.44-7.36 (m, 1H), 7.33 (d, J=7.7 Hz, 1H), 7.11-7.04 (m, 1H), 7.01 (d, J=8.9 Hz, 2H), 6.80-6.73 (m, 1H), 5.21 (s, 2H), 5.06 (dd, J=13.3, 5.1 Hz, 1H), 4.43 (d, J=17.3 Hz, 1H), 4.30 (d, J=17.6 Hz, 1H), 4.03 (s, 3H), 2.97-2.81 (m, 1H), 2.67-2.56 (m, 1H), 2.45-2.31 (m, 1H), 2.08-1.93 (m, 1H).

### INTERMEDIATE I-1

### 4-(4,5-difluoro-2-methoxyphenoxy)-2-methylbenzoic acid

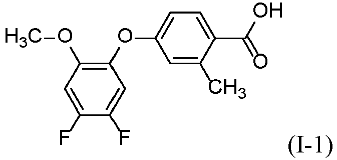

To a solution of tert-butyl 4-bromo-2-methylbenzoate (150 mg, 0.553 mmol) and 4,5-difluoro-2-methoxyphenol (97 mg, 0.609 mmol) in 1,4 dioxane (3 mL) were added cesium carbonate (360 mg, 1.11 mmol), N,N-dimethylglycine (17.1 mg, 0.166 mmol), and copper(I) bromide (15.9 mg, 0.111 mmol). The resulting mixture was stirred for 12 h at 120 °C under nitrogen atmosphere. The reaction mixture was then diluted with EtOAc and filtered through a pad of Celite. The filtrate was concentrated, and the residue was treated with 50% TFA in DCM and stirred at room temperature for 2 h. The reaction mixture was then diluted with water, treated with saturated aqueous sodium bicarbonate, and washed with EtOAc. The aqueous layer was collected, acidified with 1 N HCl, and extracted with EtOAc (3x). The combined organic extracts were washed with brine, dried (Na₂SO₄), filtered, and concentrated to afford 4-(4,5-difluoro-2-methoxyphenoxy)-2-methylbenzoic acid (90 mg) as a tan solid. LCMS (Method E): retention time 1.12 min, [M+H]⁺ 295.0.

### INTERMEDIATE I-2

### 2-fluoro-4-(3-fluorobenzyl)benzoic acid

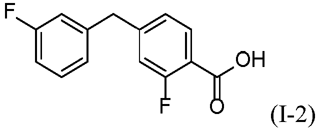

To a mixture of methyl 4-bromo-2-fluorobenzoate (100 mg, 0.429 mmol), trifluoro(3-fluorobenzyl)borate (114 mg, 0.644 mmol), and aqueous tribasic potassium phosphate (2.0 M, 429 µL, 0.858 mmol) in 1,4-dioxane (2 mL) was added Pd(dppf)Cl₂•CH₂Cl₂ complex (17.5 mg, 0.021 mmol). The resulting mixture was purged with nitrogen and heated at 110 °C for 6 h. The reaction mixture was then filtered through a pad of Celite, rinsed with EtOAc, washed with diluted brine, dried (MgSO₄), filtered, and concentrated. The residue was dissolved in a mixture of THF (1 mL), MeOH (1 mL), and water (0.5 mL), then lithium hydroxide (103 mg, 4.29 mmol) was added. The mixture was stirred at room temperature for 2 h. The reaction mixture was then diluted with water and washed with EtOAc (2x). The aqueous layer was separated, acidified with 1N HCl, and extracted with EtOAc. The organic phase was separated, dried (Na₂SO₄), filtered, and concentrated to give 2-fluoro-4-(3-fluorobenzyl)benzoic acid (94 mg) as a tan powder. LCMS (Method F): retention time 0.75 min, [M-H]⁻ 246.9.

### INTERMEDIATE I-3

### 4-((3-fluorophenyl)(methyl)amino)benzoic acid

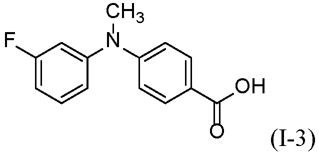

To a solution of tert-butyl 4-bromobenzoate (370 mg, 1.44 mmol) and 3-fluoro-N-methylaniline (150 mg, 1.20 mmol) in 1,4-dioxane (5 mL) were added cesium carbonate (781 mg, 2.40 mmol) and XPhos Pd G2 (56.6 mg, 0.072 mmol) at room temperature. The reaction vessel was purged with nitrogen and heated at 95 °C overnight. The reaction mixture was then filtered through a pad of Celite and rinsed with EtOAc. The filtrate was concentrated, and the residue was purified via silica gel flash chromatography to afford tert-butyl 4-((3-fluorophenyl)(methyl)amino)benzoate. The ester was subsequently dissolved in 1: 1 DCM/TFA (3 mL) and stirred at room temperature for 1 h. Volatiles were then removed under a stream of nitrogen. The crude material was dissolved in 1: 1 DCM/TEA (2 mL) and purified by flash chromatography (SiO₂, 0-10% MeOH/DCM) to afford 4-((3-fluorophenyl) (methyl)amino)benzoic acid (217 mg, 74% yield) as a white solid. LCMS (Method E): retention time 0.94 min, [M+H]⁺ 245.9.

### INTERMEDIATE I-4

### 4-(1-phenylcyclopropyl)benzoic acid

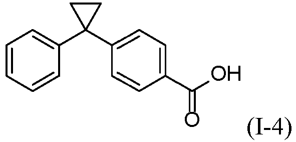

To a mixture of potassium trifluoro(1-phenylcyclopropyl)borate (125 mg, 0.558 mmol) and methyl 4-bromobenzoate (240 mg, 1.116 mmol) in toluene (12 mL) and water (1.6 mL) were added cesium carbonate (545 mg, 1.67 mmol) and cataCXium^{®} A Pd G3 (24.4 mg, 0.033 mmol). The resulting mixture was degassed by bubbling nitrogen for 10 min and stirred overnight at 95 °C. The reaction mixture was then cooled to room temperature, filtered through a pad of Celite, and rinsed with EtOAc. The filtrate was concentrated, and the residue was purified via silica gel flash chromatography to afford methyl 4-(1-phenylcyclopropyl)benzoate as a colorless oil. To the oil was added a mixture of MeOH (1 mL), THF (3 mL), and water (1 mL), followed by lithium hydroxide monohydrate (134 mg, 5.58 mmol). The resulting mixture was stirred at 60 °C for 1 h. The reaction mixture was then cooled to room temperature, acidified with 1 N HCl, and extracted with EtOAc (3x). The combined organic layers were dried (Na₂SO₄), filtered, and concentrated. The resulting solid was dried under vacuum to give 4-(1-phenylcyclopropyl)benzoic acid (105 mg, 77% yield) as a light tan solid. LCMS (Method E): retention time 1.04 min, [M+H]⁺ 239.0.

### INTERMEDIATE I-5

### 4-(difluoro(phenyl)methyl)benzoic acid

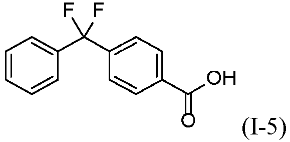

Methyl 4-benzoylbenzoate (200 mg, 0.832 mmol) and boron trifluoride acetic acid complex (876 µL, 2.08 mmol) were combined in dry DCM under nitrogen atmosphere. Ethane-1,2-dithiol (140 µL, 1.67 mmol) was added via syringe, and the resulting solution was stirred overnight at room temperature. The reaction mixture was partitioned between DCM and water. The organic layer was concentrated and purified by silica gel flash chromatography to afford methyl 4-(2-phenyl-1,3-dithiolan-2-yl)benzoate as a clear oil. In a plastic vial, the oil was dissolved in DCM (3 mL), then hydrogen fluoride pyridine (70% HF, 1 mL) and Selectfluor (369 mg, 1.04 mmol) were added. The resulting mixture was stirred at room temperature for 1 h. The reaction mixture was then diluted with DCM and washed with water, saturated aqueous sodium bicarbonate, and brine. The organic phase was dried (Na₂SO₄), filtered, and concentrated. The residue was dissolved in a mixture of MeOH (1 mL) and THF (2 mL), and treated with a solution of lithium hydroxide monohydrate (100 mg, 4.16 mmol) in water (1 mL). The resulting mixture was stirred overnight at room temperature. The reaction mixture was then diluted with water and washed with EtOAc. The aqueous layer was acidified by addition of excess 1N HCl and extracted with EtOAc (3x). The combined organic extracts were washed with brine, dried (Na₂SO₄), filtered, and concentrated to afford 4-(difluoro(phenyl)methyl)benzoic acid (115 mg, 56% yield) as a tan solid. LCMS (Method F): retention time 0.82 min, [M-H]⁻ 247.2.

### INTERMEDIATE I-6

### 2-methoxy-4-(2,4,5-trifluorophenoxy)benzoic acid

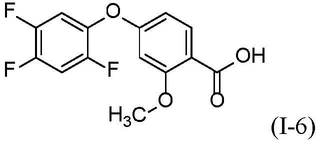

To a solution of tert-butyl 4-bromo-2-methoxybenzoate (129 mg, 0.450 mmol) and 2,4,5-trifluorophenol (80 mg, 0.540 mmol) in 1,4 dioxane (3 mL) were added cesium carbonate (293 mg, 0.900 mmol), N,N-dimethylglycine (13.9 mg, 0.135 mmol), and copper(I) bromide (12.9 mg, 0.090 mmol). The resulting mixture was stirred for 12 h at 110 °C under nitrogen atmosphere. The reaction mixture was filtered and solvent was removed from the filtrate under a stream of air. The residue was dissolved in DCM (1 mL) and treated with TFA (4 mL). The reaction mixture was stirred at room temperature for 2 h, then diluted with water, and extracted with EtOAc. The organic layer was collected and concentrated to give 2-methoxy-4-(2,4,5-trifluorophenoxy)benzoic acid. LCMS (Method E): retention time 0.96 min, [M+H]⁺ 298.8.

### EXAMPLES 2-34

The compounds in Table 1 were prepared according to the general procedures described for Example 1, replacing 4-(3,4-difluorophenoxy)benzoic acid with the appropriate acid (which was either commercially available, or synthesized as described for Intermediate I-1, I-2, I-3, I-4, I-5, or I-6, or in a similar manner):

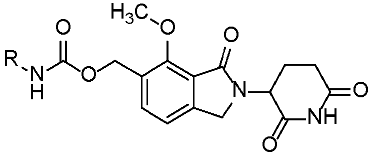

**TABLE 1**

| Ex. No. | R | LCMS [M+H]⁺ | RT (min) | HPLC Method |
|---|---|---|---|---|
| 2 | 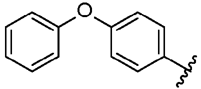 | 516.1 | 0.92 | E |
| 3 | 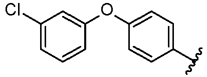 | 550.1 | 2.16 | D |
| 4 | 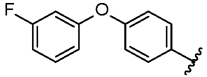 | 533.8 | 1.99 | C |
| 5 | 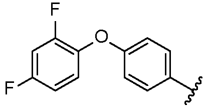 | 552.0 | 1.94 | D |
| 6 | 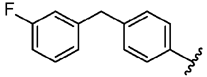 | 532.0 | 1.99 | C |
| 7 | 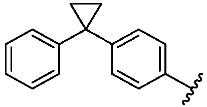 | 540.0 | 2.11 | D |
| 8 | 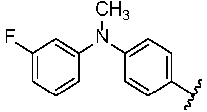 | 547.2 | 2.12 | C |
| 9 | 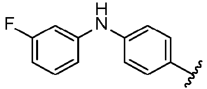 | 533.0 | 1.91 | D |
| 10 | 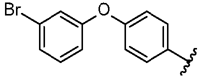 | 594.0 | 2.06 | C |
| 11 | 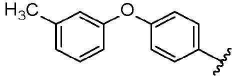 | 530.1 | 2.15 | C |
| 12 | 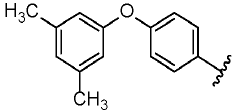 | 544.0 | 2.06 | C |
| 13 | 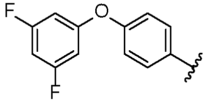 | 552.0 | 1.95 | C |
| 14 | 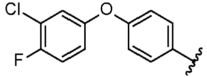 | 567.9 | 2.02 | C |
| 15 | 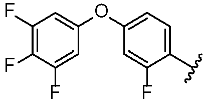 | 588.1 | 1.99 | C |
| 16 | 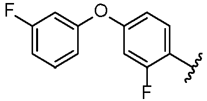 | 552.0 | 1.93 | C |
| 17 | 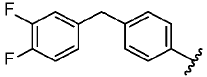 | 549.9 | 1.99 | D |
| 18 | 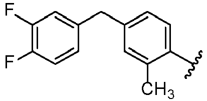 | 564.2 | 2.14 | C |
| 19 | 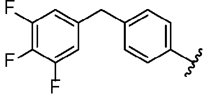 | 568.2 | 2.17 | D |
| 20 | 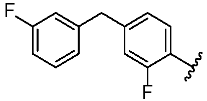 | 550.2 | 2.13 | C |
| 21 | 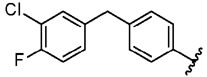 | 566.0 | 1.98 | C |
| 22 | 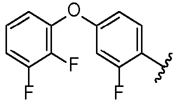 | 570.3 | 1.93 | D |
| 23 | 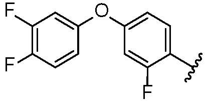 | 570.1 | 1.96 | C |
| 24 | 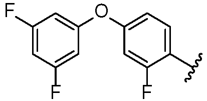 | 570.0 | 2.00 | C |
| 25 | 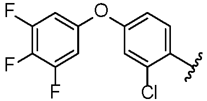 | 604.0 | 2.19 | C |
| 26 | 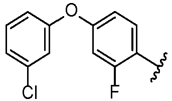 | 568.0 | 2.07 | C |
| 27 | 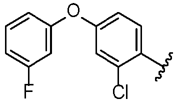 | 568.1 | 1.99 | D |
| 28 | 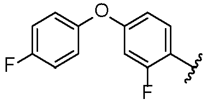 | 552.3 | 1.86 | D |
| 29 | 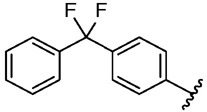 | 550.1 | 1.91 | C |
| 30 | 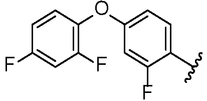 | 570.1 | 1.86 | C |
| 31 | 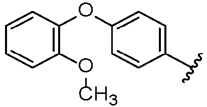 | 546.0 | 1.74 | C |
| 32 | 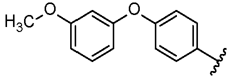 | 546.2 | 1.83 | C |
| 33 | 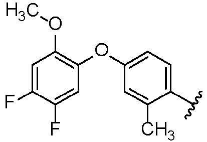 | 596.0 | 1.87 | D |
| 34 | 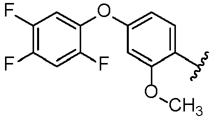 | 599.9 | 1.96 | C |

### EXAMPLES 35 AND 36

### {2-[(3S)-2,6-dioxopiperidin-3-yl]-4-methoxy-3-oxo-2,3-dihydro-1H-isoindol-5-yl}methyl N-[4-(3,4-difluorophenoxy)phenyl]carbamate

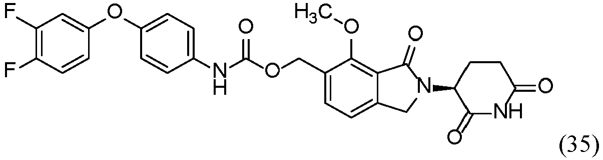

### {2-[(3R)-2,6-dioxopiperidin-3-yl]-4-methoxy-3-oxo-2,3-dihydro-1H-isoindol-5-yl}methyl N-[4-(3,4-difluorophenoxy)phenyl]carbamate

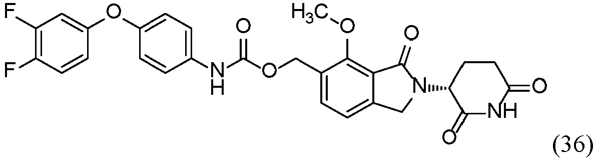

Racemic [2-(2,6-dioxopiperidin-3-yl)-4-methoxy-3-oxo-2,3-dihydro-1H-isoindol-5-yl]methyl N-[4-(3,4-difluorophenoxy)phenyl]carbamate (700 mg) was dissolved in MeOH/DCM (1:1) and resolved by SFC chiral separation (Column: Cellulose-4, 3 x 25 cm, 5 µm particles; Mobile Phase: carbon dioxide/MeOH (48:52); Temperature: 35 °C; BPR Pressure: 100 bar; Flow: 200 mL/min).

Fractions containing the first peak were combined and lyophilized to dryness to afford {2-[(3S)-2,6-dioxopiperidin-3-yl]-4-methoxy-3-oxo-2,3-dihydro-1H-isoindol-5-yl} methyl N-[4-(3,4-difluorophenoxy)phenyl]carbamate (270 mg). The absolute configuration was assigned as (S) by comparing the experimental VCD spectrum with the predicted VCD spectra for the (R) and (S) isomers. SFC (Method G): retention time: 5.50 min (>99% ee); LCMS (Method E): retention time 1.05 min, [M+H]⁺ 552.2; ¹H NMR (400 MHz, METHANOL-d₄) δ 7.71 (d, J=7.7 Hz, 1H), 7.47 (br d, J=8.8 Hz, 1H), 7.31 (d, J=7.7 Hz, 1H), 7.27-7.15 (m, 1H), 7.01-6.92 (m, 2H), 6.86 (ddd, *J=*11.8, 6.7, 2.9 Hz, 1H), 6.77-6.69 (m, 1H), 5.30 (s, 2H), 5.12 (dd, J=13.3, 5.2 Hz, 1H), 4.52-4.39 (m, 2H), 4.10 (s, 3H), 2.98-2.85 (m, 1H), 2.84-2.72 (m, 1H), 2.48 (qd, J=13.2, 4.6 Hz, 1H), 2.23-2.11 (m, 1H).

Fractions containing the second peak were combined and lyophilized to dryness to afford {2-[(3R)-2,6-dioxopiperidin-3-yl]-4-methoxy-3-oxo-2,3-dihydro-1H-isoindol-5-yl} methyl N-[4-(3,4-difluorophenoxy)phenyl]carbamate (260 mg). The absolute configuration was assigned as (R) by comparing the experimental VCD spectrum with the predicted VCD spectra for the (R) and (S) isomers. SFC (Method G): retention time: 4.19 min (>99% ee); LCMS (Method E): retention time 1.05 min, [M+H]⁺ 552.2; ¹H NMR (400 MHz, METHANOL-d₄) δ 7.70 (d, J=7.7 Hz, 1H), 7.46 (br d, J=8.7 Hz, 2H), 7.30 (d, J=7.7 Hz, 1H), 7.26-7.14 (m, 1H), 7.01-6.93 (m, 2H), 6.86 (ddd, *J*=11.7, 6.7, 2.9 Hz, 1H), 6.78-6.68 (m, 1H), 5.29 (s, 2H), 5.12 (dd, J=13.4, 5.2 Hz, 1H), 4.53-4.37 (m, 2H), 4.10 (s, 3H), 2.98-2.84 (m, 1H), 2.83-2.72 (m, 1H), 2.48 (qd, J=13.2, 4.7 Hz, 1H), 2.24-2.10 (m, 1H).

### EXAMPLE 37

### [2-(2,6-dioxopiperidin-3-yl)-4-methoxy-3-oxo-2,3-dihydro-1H-isoindol-5-ylmethyl N-[4-(4-fluorophenoxy)-2-methoxyphenyl]carbamate

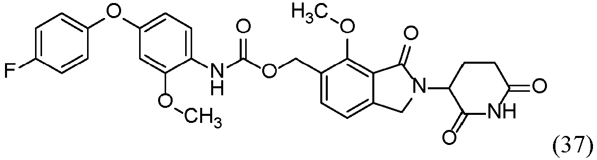

To a solution of 4-nitrophenyl carbonochloridate (42.8 mg, 0.212 mmol) in THF (5 mL) was added 4-(4-fluorophenoxy)-2-methoxyaniline (45 mg, 0.193 mmol). The resulting mixture was stirred for 5 min, then pyridine (46.8 µL, 0.579 mmol) was added dropwise. The reaction mixture was stirred at room temperature for 3 h. The solvent was removed under a stream of air, and the residue was redissolved in DMF (2 mL), followed by addition of 3-(6-(hydroxymethyl)-7-methoxy-1-oxoisoindolin-2-yl)piperidine-2,6-dione (41.1 mg, 0.135 mmol) and triethylamine (81 µL, 0.579 mmol). The resulting mixture was stirred at 80 °C for 3 h. The reaction mixture was then diluted with water and extracted with EtOAc. The organic layer was collected and concentrated. The crude material was purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 30-70%B (0.0-20.0 min), 70-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-4-methoxy-3-oxo-2,3-dihydro-1H-isoindol-5-yl]methyl N-[4-(4-fluorophenoxy)-2-methoxyphenyl]carbamate (10.6 mg, 28% yield) as an off-white solid. LCMS (Method D): retention time 1.88 min, [M+H]⁺ 563.9; ¹H NMR (500 MHz, DMSO-d₆) δ 10.98 (s, 1H), 8.59 (s, 1H), 7.67 (br d, J=8.1 Hz, 1H), 7.56-7.44 (m, 1H), 7.33 (d, J=7.8 Hz, 1H), 7.25-7.15 (m, 2H), 7.08-6.99 (m, 2H), 6.74 (d, J=2.6 Hz, 1H), 6.47 (dd, J=8.7, 2.5 Hz, 1H), 5.18 (s, 2H), 5.07 (dd, J=13.4, 5.2 Hz, 1H), 4.43 (d, J=17.4 Hz, 1H), 4.30 (d, J=17.5 Hz, 1H), 4.01 (s, 3H), 3.74 (s, 3H), 2.97-2.82 (m, 1H), 2.66-2.57 (m, 1H), 2.46-2.31 (m, 1H), 2.07-1.94 (m, 1H).

### EXAMPLES 38-51

The compounds in Table 2 were prepared according to the general procedures described for Example 37, replacing 4-(4-fluorophenoxy)-2-methoxyaniline with the appropriate aniline:

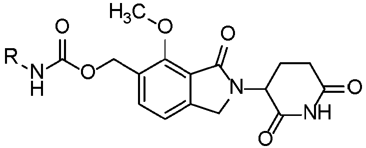

**TABLE 2**

| Ex. No. | R | LCMS [M+H]⁺ | RT (min) | HPLC Method |
|---|---|---|---|---|
| 38 | 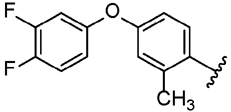 | 565.9 | 2.00 | D |
| 39 | 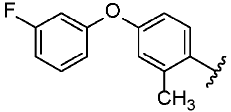 | 547.7 | 1.98 | D |
| 40 | 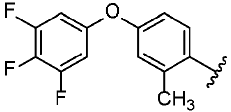 | 584.2 | 2.22 | C |
| 41 | 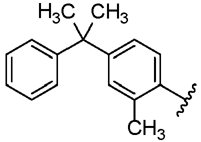 | 542.2 | 2.28 | C |
| 42 | 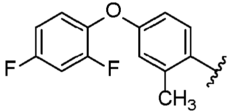 | 566.1 | 2.01 | D |
| 43 | 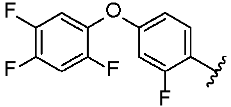 | 587.7 | 2.16 | C |
| 44 | 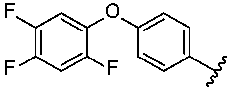 | 570.1 | 1.87 | D |
| 45 | 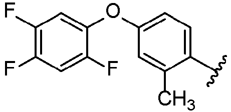 | 584.0 | 2.04 | D |
| 46 | 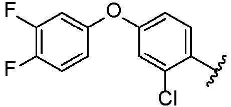 | 586.0 | 2.26 | C |
| 47 | 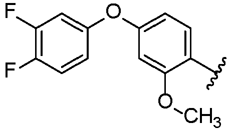 | 582.1 | 1.95 | C |
| 48 | 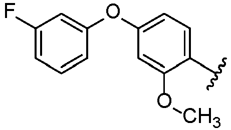 | 564.1 | 1.91 | C |
| 49 | 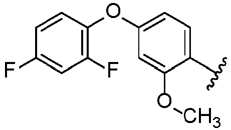 | 582.1 | 1.91 | C |
| 50 | 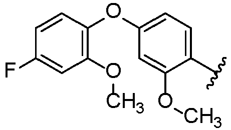 | 594.1 | 1.83 | C |
| 51 | 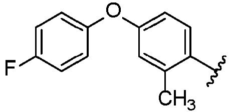 | 548.1 | 1.92 | C |

### EXAMPLE 52

### [2-(2,6-dioxopiperidin-3-yl)-4-ethoxy-3-oxo-2,3-dihydro-1H-isoindol-5-yl]methyl N-(4-phenoxyphenyl)carbamate

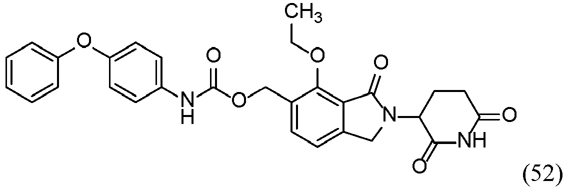

### Intermediate 52A: methyl 3-bromo-2-ethoxy-6-methylbenzoate

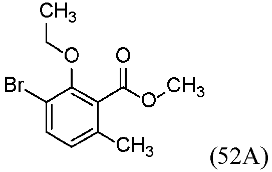

A mixture of methyl 3-bromo-2-hydroxy-6-methylbenzoate (800 mg, 3.26 mmol), iodoethane (611 mg, 3.92 mmol), and potassium carbonate (1.12 g, 8.16 mmol) in acetone (6 mL) was stirred at 60 °C for 2 h. The reaction mixture was cooled to room temperature and filtered through a pad of Celite, which was rinsed with DCM (10 mL). The filtrate was concentrated and the resulting residue was purified by flash chromatography (SiO₂, 0-20% EtOAc/Hex) to afford methyl 3-bromo-2-ethoxy-6-methylbenzoate (700 mg, 79% yield). LCMS (Method E): retention time 1.05 min, [M+H]⁺ 272.9, 274.8; ¹H NMR (400 MHz, CHLOROFORM-d) δ 7.47 (d, *J* = 8.2 Hz, 1H), 6.84 (dd, *J =* 8.2, 0.7 Hz, 1H), 4.14-4.05 (m, 2H), 3.93 (s, 3H), 2.26 (s, 3H), 1.39 (t, *J= 7.0* Hz, 3H).

### Intermediate 52B: methyl 3-bromo-6-(bromomethyl)-2-ethoxybenzoate

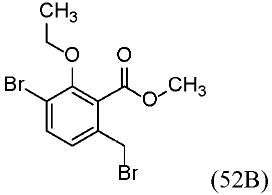

A mixture of methyl 3-bromo-2-ethoxy-6-methylbenzoate (700 mg, 2.56 mmol), NBS (547 mg, 3.08 mmol) and AIBN (21.0 mg, 0.128 mmol) in 1,2-dichloroethane (8 mL) was stirred at 60 °C for 4 h. The reaction mixture was cooled to room temperature and concentrated. The residue was purified by flash chromatography (SiO₂, 0-10% EtOAc/Hex) to afford methyl 3-bromo-6-(bromomethyl)-2-ethoxybenzoate (550 mg, 61% yield). LCMS (Method E): retention time 1.06 min, [M+H]⁺ 350.7, 352.7, 354.7; ¹H NMR (400 MHz, CHLOROFORM-d) δ 7.58 (d, *J* = 8.2 Hz, 1H), 7.06 (d, *J* = 8.3 Hz, 1H), 4.47 (s, 2H), 4.12 (q, *J =* 7.0 Hz, 2H), 3.98 (s, 3H), 1.40 (t, *J =* 7.0 Hz, 3H).

### Intermediate 52C: 3-(6-bromo-7-ethoxy-1-oxoisoindolin-2-yl)piperidine-2,6-dione

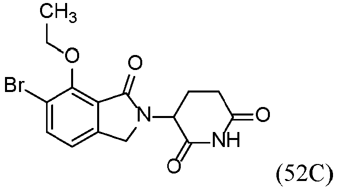

A mixture of methyl 3-bromo-6-(bromomethyl)-2-ethoxybenzoate (400 mg, 1.14 mmol), 3-aminopiperidine-2,6-dione hydrochloride (206 mg, 1.25 mmol), and DIEA (0.50 mL, 2.84 mmol) in acetonitrile (8 mL) was stirred at 80 °C for 15 h. The reaction mixture was cooled to room temperature and concentrated. The residue was purified by flash chromatography (SiO₂, 0-100% EtOAc/Hex) to give 3-(6-bromo-7-ethoxy-1-oxoisoindolin-2-yl)piperidine-2,6-dione (350 mg, 84% yield). LCMS (Method E): retention time 0.82 min, [M+H]⁺ 366.9, 368.8; ¹H NMR (400 MHz, DMSO-d₆) δ 10.99 (s, 1H), 7.85 (d, *J =* 8.0 Hz, 1H), 7.27 (d, *J* = 8.0 Hz, 1H), 5.06 (dd, *J=* 13.3, 5.1 Hz, 1H), 4.57-4.29 (m, 2H), 4.28-4.18 (m, 2H), 3.57 (s, 2H), 2.84 (s, 1H), 2.67-2.56 (m, 1H), 2.43-2.29 (m, 1H), 2.06-1.92 (m, 1H), 1.35 (t, *J =* 7.0 Hz, 3H).

### Intermediate 52D: 3-(7-ethoxy-6-(hydroxymethyl)-1-oxoisoindolin-2-yl)piperidine-2,6-dione

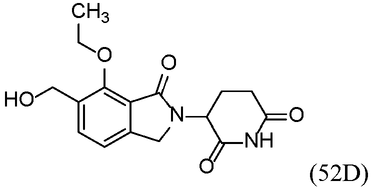

A mixture of 3-(6-bromo-7-ethoxy-1-oxoisoindolin-2-yl)piperidine-2,6-dione (280 mg, 0.763 mmol), (tributylstannyl)methanol (294 mg, 0.915 mmol), and Pd(PPh₃)₄ (44.1 mg, 0.038 mmol) in 1,4-dioxane (2 mL) was stirred at 100 °C in a sealed tube for 15 h under nitrogen atmosphere. The reaction mixture was cooled to room temperature and concentrated. The residue was purified by flash chromatography (SiO₂, 0-10% MeOH/DCM) to afford 3-(7-ethoxy-6-(hydroxymethyl)-1-oxoisoindolin-2-yl)piperidine-2,6-dione (100 mg, 41% yield). LCMS (Method E): retention time 0.73 min, [M+H]⁺ 319.0; ¹H NMR (400 MHz, DMSO-d₆) δ 10.97 (s, 1H), 7.65 (d, *J* = 7.7 Hz, 1H), 7.28 (d, *J =* 7.7 Hz, 1H), 5.14 (t, *J* = 5.6 Hz, 1H), 5.05 (dd, *J =* 13.3, 5.2 Hz, 1H), 4.59 (d, *J* = 5.6 Hz, 2H), 4.47-4.27 (m, 2H), 4.28-4.11 (m, 2H), 2.96-2.83 (m, 1H), 2.62 (br d, *J* = 2.4 Hz, 1H), 2.42-2.30 (m, 1H), 2.02-1.89 (m, 1H), 1.30 (t, *J* = 7.0 Hz, 3H).

### Example 52:

A mixture of 3-(7-ethoxy-6-(hydroxymethyl)-1-oxoisoindolin-2-yl)piperidine-2,6-dione (25 mg, 0.079 mmol), 4-phenoxybenzoic acid (25.2 mg, 0.118 mmol), diphenyl phosphorazidate (32.4 mg, 0.118 mmol), and TEA (0.033 mL, 0.236 mmol) in 1,4-dioxane (1 mL) was heated at 105 °C for 15 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford [2-(2,6-dioxopiperidin-3-yl)-4-ethoxy-3-oxo-2,3-dihydro-1H-isoindol-5-yl]methyl N-(4-phenoxyphenyl)carbamate (3.4 mg, 8% yield). LCMS (Method C): retention time 2.01 min, [M+1]⁺ 530.1; ¹H NMR (500 MHz, DMSO-d₆) δ 9.70 (br s, 1H), 7.66 (d, *J* = 7.6 Hz, 1H), 7.46 (br d, *J* = 8.6 Hz, 2H), 7.40-7.29 (m, 3H), 7.08 (t, *J* = 7.3 Hz, 1H), 6.95 (br dd, *J* = 17.8, 8.3 Hz, 4H), 5.21 (s, 2H), 5.03 (dd, *J* = 13.4, 5.0 Hz, 1H), 4.56-4.15 (m, 4H), 2.92-2.77 (m, 1H), 2.62 (br s, 1H), 2.43-2.31 (m, 1H), 2.06-1.96 (m, 1H), 1.32 (t, *J* = 7.0 Hz, 3H).

### EXAMPLES 53-55

The compounds in Table 3 were prepared according to the general procedures described for Example 51, replacing 4-phenoxybenzoic acid with the appropriate acid:

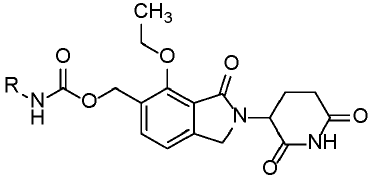

**TABLE 3**

| Ex. No. | R | LCMS [M+H]⁺ | RT (min) | HPLC Method |
|---|---|---|---|---|
| 53 | 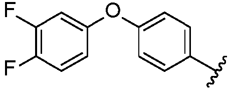 | 566.1 | 2.12 | C |
| 54 | 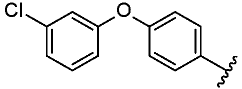 | 564.2 | 2.16 | C |
| 55 | 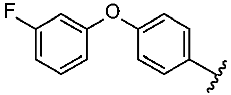 | 548.2 | 2.05 | C |

### INTERMEDIATE I-7

### 4-nitrophenyl (4-(3,4-difluorophenoxy)phenyl)carbamate

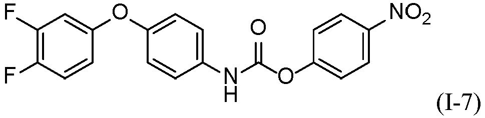

To a cooled (0 °C), well stirred solution of 4-(3,4-difluorophenoxy)aniline (5 g, 22.6 mmol) in 1,4-dioxane (40 mL) was added pyridine (3.66 mL, 45.2 mmol) followed by 4-nitrophenyl carbonochloridate (5.01 g, 24.9 mmol). The resulting mixture was allowed to stir at room temperature for 2 h. The reaction mixture was then quenched with water (50 mL) and extracted with EtOAc (2 x 200 mL). The combined organic layers were dried (Na₂SO₄), filtered, and concentrated. The residue was triturated with diethyl ether (3 x 20 mL) to obtain crude 4-nitrophenyl (4-(3,4-difluorophenoxy)phenyl)carbamate (10.5 g) as a pale white solid, which was used in subsequent steps without further purification. ¹H NMR (400 MHz, DMSO-d₆) δ 10.51 (s, 1H), 8.31-8.38 (m, 2H), 7.56-8.14 (m, 4H), 7.43-7.54 (m, 2H), 7.11-7.14 (m, 1H), 7.08-7.10 (m, 1H), 6.80-6.83 (m, 1H).

### EXAMPLE 56

### [2-(2,6-dioxopiperidin-3-yl)-3-oxo-4-(propan-2-yloxy)-2,3-dihydro-1H-isoindol-5-yl]methyl N-[4-(3,4-difluorophenoxy)phenyl]carbamate

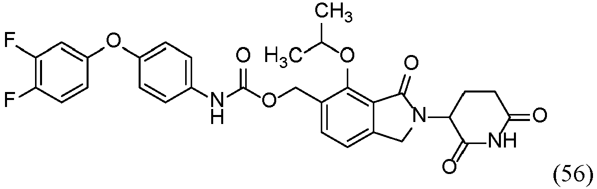

### Intermediate 56A: ethyl 3-bromo-2-hydroxy-6-methylbenzoate

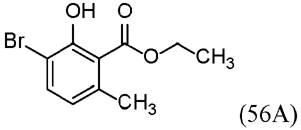

To a cooled (0 °C), stirred solution of ethyl 2-hydroxy-6-methylbenzoate (15 g, 83 mmol) and diisopropylamine (1.19 mL, 8.32 mmol) in DCM (250 mL) was added a solution of NBS (14.8 g, 83 mmol) in DCM (750 mL) over a period of 1 h. The resulting mixture was stirred at ambient temperature for 2 h. The reaction mixture was diluted with water (500 mL) and extracted with DCM (2 x 500 mL). The combined organic layers were washed with brine (500 mL), dried (Na₂SO₄), filtered, and concentrated. The crude product was purified by reversed phase chromatography to afford ethyl 3-bromo-2-hydroxy-6-methylbenzoate (18 g, 63.0 mmol, 76% yield) as a pale brown solid. LCMS (Method H): retention time 1.61 min, [M+H]⁺ 259.0, 261.0.

### Intermediate 56B: ethyl 3-bromo-2-isopropoxy-6-methylbenzoate

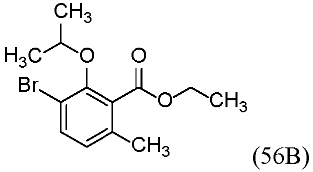

To a stirred solution of ethyl 3-bromo-2-hydroxy-6-methylbenzoate (5 g, 19.3 mmol) and cesium carbonate (12.58 g, 38.6 mmol) in DMF (50 mL) was added 2-iodopropane (4.92 g, 28.9 mmol). The resulting mixture was stirred at 100 °C for 16 h. The reaction mixture was diluted with water (200 mL) and extracted with DCM (2 x 200 mL). The combined organic layers were washed with water (200 mL), brine (200 mL), dried (Na₂SO₄), filtered, and concentrated. The crude product was purified by flash chromatography (SiO₂, 0-10% EtOAc/petroleum ether) to afford ethyl 3-bromo-2-isopropoxy-6-methylbenzoate (4 g, 12.4 mmol, 64% yield) as a colorless liquid. LCMS (Method I): retention time 1.05 min, [M+H]⁺ 301.0, 303.2.

### Intermediate 56C: tert-butyl 5-amino-4-(6-bromo-7-isopropoxy-1-oxoisoindolin-2-yl)-5-oxopentanoate

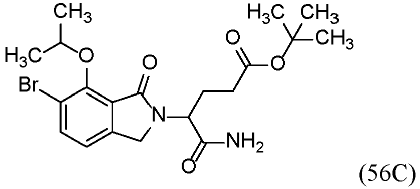

To a stirred solution of ethyl 3-bromo-2-isopropoxy-6-methylbenzoate (7 g, 23.2 mmol) in 1,2-dichloroethane (50 mL) was added NBS (4.55 g, 25.6 mmol) and AIBN (1.145 g, 6.97 mmol). The resulting mixture was stirred at 80 °C for 6 h. The reaction mixture was diluted with water and extracted with DCM (2 x 150 mL). The combined organic layers were dried (Na₂SO₄), filtered, and concentrated. The residue was purified by flash chromatography (SiO₂, 10% EtOAc/petroleum ether) to afford ethyl 3-bromo-6-(bromomethyl)-2-isopropoxybenzoate (7.5 g, ~58% purity by HPLC) as a light brown solid.

To a stirred solution of ethyl 3-bromo-6-(bromomethyl)-2-isopropoxybenzoate (6.5 g) in DMF (70 mL) were added DIEA (9.0 mL, 51.3 mmol) and tert-butyl (S)-4,5-diamino-5-oxopentanoate (4.15 g, 20.5 mmol). The resulting mixture was stirred at 80 °C for 16 h. The reaction was quenched with the addition of water. The reaction mixture was extracted with EtOAc (3 x 100 mL). The combined organic layers were dried (Na₂SO₄), filtered, and concentrated. The crude product was purified by flash chromatography (SiO₂, 0-70% EtOAc/petroleum ether) to afford tert-butyl 5-amino-4-(6-bromo-7-isopropoxy-1-oxoisoindolin-2-yl)-5-oxopentanoate (7.1 g, ~60% purity by HPLC) as a light brown, gummy solid. The enantiomeric excess of this material and subsequent intermediates were not determined. LCMS (Method H): retention time 1.44 min; [M+H]⁺ 455.2, 457.2.

### Intermediate 56D: 3-(6-bromo-7-isopropoxy-1-oxoisoindolin-2-yl)piperidine-2,6-dione

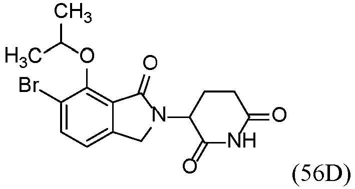

To a solution of tert-butyl 5-amino-4-(6-bromo-7-isopropoxy-1-oxoisoindolin-2-yl)-5-oxopentanoate (3 g) in acetonitrile (15 mL) was added TFA (2.54 mL, 32.9 mmol). The resulting mixture was heated in a microwave reactor at 120 °C for 1 h. The reaction mixture was then concentrated. The reaction was quenched with the addition of saturated aqueous sodium bicarbonate. The mixture was diluted with water and extracted with EtOAc (2 x 100 mL). The combined organic layers were dried (Na₂SO₄), filtered, and concentrated. The crude product was purified by flash chromatography (SiO₂, 60% EtOAc/petroleum ether) to afford 3-(6-bromo-7-isopropoxy-1-oxoisoindolin2-yl)piperidine-2,6-dione (1.4 g, ~81% purity by HPLC) as an off-white solid. LCMS (Method H): retention time 1.27 min; [M+H]⁺ 381.0, 383.0.

### Intermediate 56E: 3-(6-(hydroxymethyl)-7-isopropoxy-1-oxoisoindolin-2-yl)piperidine-2,6-dione

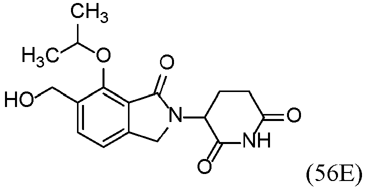

A solution of 3-(6-bromo-7-isopropoxy-1-oxoisoindolin-2-yl)piperidine-2,6-dione (1.2 g, 3.15 mmol) and (tributylstannyl)methanol (1.01 g, 3.15 mmol) in 1,4-dioxane (10 mL) was purged with nitrogen for 5 min, then Pd(PPh₃)₄ (0.364 g, 0.315 mmol) was added. The resulting mixture was purged with nitrogen for another 2 min, then it was stirred at 100 °C for 16 h. The reaction was quenched with the addition of water. The reaction mixture was extracted with EtOAc. The combined organic layers were dried (Na₂SO₄), filtered, and concentrated. The crude product was purified by flash chromatography (SiO₂, 70-80% EtOAc/petroleum ether) to afford 3-(6-(hydroxymethyl)-7-isopropoxy-1-oxoisoindolin-2-yl)piperidine-2,6-dione (0.43 g, 40% yield) as an off-white solid. LCMS (Method J): retention time 1.64 min, [M+H]⁺ 333.1; ¹H NMR (400 MHz, DMSO-d₆): δ 7.67 (d, *J* = 7.6 Hz, 1H), 7.26 (d, *J* = 7.6 Hz, 1H), 5.08-5.03 (m, 2H), 4.83-4.87 (m, 1H), 4.57 (d, *J* = 6.4 Hz, 2H), 4.39 (d, *J =* 17.2 Hz, 1H), 4.27 (d, *J* = 17.2 Hz, 1H), 2.89-2.84 (m, 1H), 2.68-2.59 (m, 1H), 2.41-2.36 (m, 1H), 2.02-2.00 (m, 1H), 1.25-1.20 (m, 6H).

### Example 56:

A mixture of 3-(6-(hydroxymethyl)-7-isopropoxy-1-oxoisoindolin-2-yl)piperidine-2,6-dione (50 mg, 0.150 mmol) and DIEA (0.079 mL, 0.451 mmol) in DMF (2 mL) was stirred for 10 minutes at room temperature under nitrogen atmosphere. Then 4-nitrophenyl (4-(3,4-difluorophenoxy)phenyl)carbamate (87 mg, 0.226 mmol) was added, and the resulting mixture was stirred at room temperature for 6 h. The reaction mixture was concentrated and purified via preparatory HPLC (Column: XBridge C18, 19 mm x 200 mm, 5 µm particles; Mobile Phase A: water with 5 mM ammonium formate, Mobile Phase B: acetonitrile; Temperature: 25 °C; Gradient: 40-60%B (0.0-20.0 min); Flow: 25 mL/min). Fractions containing the product were combined and lyophilized to dryness to afford [2-(2,6-dioxopiperidin-3-yl)-3-oxo-4-(propan-2-yloxy)-2,3-dihydro-1H-isoindol-5-yl]methyl N-[4-(3,4-difluorophenoxy)phenyl]carbamate (12 mg, 14% yield) as an off-white solid. LCMS (Method K): retention time 3.03 min, [M-H]⁻ 578.0; ¹H NMR (400 MHz, DMSO-d₆): δ 9.77 (s, 1H), 8.36 (s, 1H), 7.66 (d, *J* = 7.6 Hz, 1H), 7.50 (d, *J* = 8.8 Hz, 1H), 7.42 (q, *J =* 8.0 Hz, 1H), 7.39 (d, *J =* 9.2 Hz, 1H), 7.12-7.09 (m, 1H), 7.07-7.02 (m, 2H), 6.78-6.76 (m, 1H), 5.23 (s, 2H), 5.09-5.05 (m, 1H), 4.96-4.93 (m, 1H), 4.43 (d, *J =* 17.6 Hz, 1H), 4.31 (d, *J* = 17.6 Hz, 1H), 2.89-2.87 (m, 1H), 2.61-2.56 (m, 1H), 2.49-2.40 (m, 1H), 2.08-2.04 (m, 1H), 1.29-1.24 (m, 6H).

### EXAMPLES 57-59

The compounds in Table 4 were prepared according to the general procedures described for Example 56, replacing 4-nitrophenyl (4-(3,4-difluorophenoxy)phenyl) carbamate with the appropriate 4-nitrophenyl carbamate (prepared in a manner similar to Intermediate I-7):

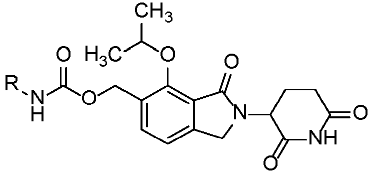

**TABLE 4**

| Ex. No. | R | LCMS [M+H]⁺ | RT (min) | HPLC Method |
|---|---|---|---|---|
| 57 | 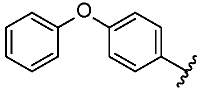 | 544.2 | 2.91 | K |
| 58 | 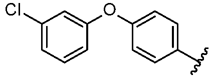 | 578.0 | 3.19 | K |
| 59 | 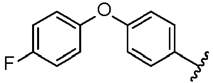 | 562.0 | 2.94 | K |

### EXAMPLE 60

### [4-(difluoromethoxy)-2-(2,6-dioxopiperidin-3-yl)-3-oxo-2,3-dihydro-1H-isoindol-5-yl] methyl N-(4-phenoxyphenyl)carbamate

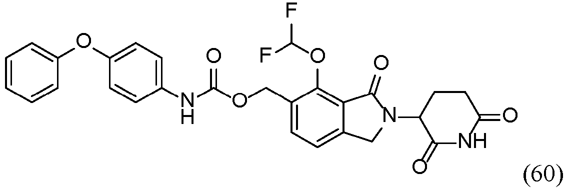

### Intermediate 60A: ethyl 3-bromo-2-(difluoromethoxy)-6-methylbenzoate

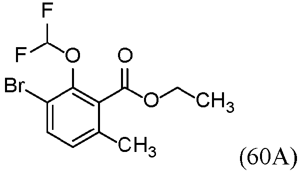

To a cooled (-20 °C), well-stirred solution of ethyl 3-bromo-2-hydroxy-6-methylbenzoate (5 g, 19.3 mmol) in acetonitrile (50 mL) was added a cold solution of potassium hydroxide (10.83 g, 193 mmol) in water (30 mL), followed by dropwise addition of diethyl(bromodifluoromethyl)phosphonate (10.3 mL, 57.9 mmol) while maintaining the temperature below -20 °C. At the end of the addition, the reaction medium was warmed to room temperature and stirred for 16 h. The reaction mixture was extracted with EtOAc (2 x 100 mL), and the combined organic layers were washed with brine (100 mL), dried (Na₂SO₄), and concentrated. The crude product was purified by reversed phase chromatography to afford ethyl 3-bromo-2-(difluoromethoxy)-6-methylbenzoate (3 g, ~76% purity by GC). GCMS: retention time 5.89 min, [M]⁺ 308.0, 310.0.

### Intermediate 60B: 3-(6-bromo-7-(difluoromethoxy)-1-oxoisoindolin-2-yl)piperidine-2,6-dione

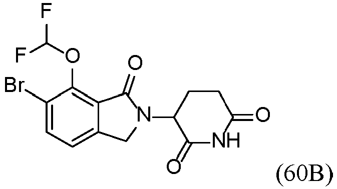

To a well-stirred solution of ethyl 3-bromo-2-(difluoromethoxy)-6-methylbenzoate (3.4 g, 7.37 mmol) in DCE (30 mL) were added AIBN (0.363 g, 2.21 mmol) and NBS (1.71 g, 9.58 mmol). The resulting mixture was stirred at 90 °C for 3 h. The reaction mixture was cooled to room temperature and filtered through a bed of silica gel, which was rinsed with 20% EtOAc/petroleum ether. The filtrate was concentrated and the crude residue was purified by flash chromatography (SiO₂, 100 g column, 0-100% EtOAc/petroleum ether) to afford ethyl 3-bromo-6-(bromomethyl)-2-(difluoromethoxy)benzoate as a pale yellow oil. GCMS: retention time 6.73 min, [M]⁺ 387.9.

To a well-stirred solution of ethyl 3-bromo-6-(bromomethyl)-2-(difluoromethoxy) benzoate (4.85 g, 8.58 mmol) in DMF (25 mL) was added 3-aminopiperidine-2,6-dione (2.20 g, 17.2 mmol), followed by slow addition of DIEA (4.50 mL, 25.7 mmol). The resulting mixture was stirred at 90 °C for 16 h. The solvent was then removed and the crude product was purified by reversed phase chromatography to afford 3-(6-bromo-7-(difluoromethoxy)-1-oxoisoindolin-2-yl)piperidine-2,6-dione (2.09 g, 60.0% yield) as a dark brown solid. LCMS (Method L): retention time 1.06 min, [M+H]⁺ 389.0, 391.0.

### Example 60:

To a well-stirred solution of 3-(7-(difluoromethoxy)-6-(hydroxymethyl)-1-oxoisoindolin-2-yl)piperidine-2,6-dione (117 mg, 0.325 mmol) in DMF (2 mL) were added 4-nitrophenyl (4- phenoxyphenyl)carbamate (228 mg, 0.649 mmol) and DIEA (0.284 mL, 1.62 mmol). After 6 h, the reaction mixture was concentrated. The crude material was purified via reversed phase preparative HPLC to afford [4-(difluoromethoxy)-2-(2,6-dioxopiperidin-3-yl)-3-oxo-2,3-dihydro-1H-isoindol-5-yl]methyl N-(4-phenoxyphenyl) carbamate (34 mg, 16% yield) as a white solid. LCMS (Method K): retention time 2.84 min, [M+NH₄]⁺ 569.0. ¹H NMR (400 MHz, DMSO-d₆) δ 10.01 (s, 1H), 9.80 (s, 1H), 7.81 (d, *J =* 8.0 Hz, 1H), 7.59 (d, *J* = 8.0 Hz, 1H), 7.48 (d, *J* = 8.8 Hz, 2H), 7.37-7.33 (m, 3H), 7.11-7.06 (m, 1H), 6.98-6.93 (m, 4H), 5.28 (s, 2H), 5.13-5.08 (m, 1H), 4.51 (d, *J =* 18.0 Hz, 1H), 4.40 (d, *J* = 18.0 Hz, 1H), 2.90 (m, 1H), 2.67-2.63 (m, 1H).

### EXAMPLE 61

### [4-(difluoromethoxy)-2-(2,6-dioxopiperidin-3-yl)-3-oxo-2,3-dihydro-1H-isoindol-5-yl] methyl N-[4-(3,4-difluorophenoxy)phenyl]carbamate

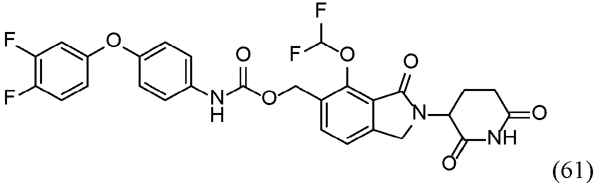

To a well-stirred solution of 3-(7-(difluoromethoxy)-6-(hydroxymethyl)-1-oxoisoindolin-2-yl)piperidine-2,6-dione (117 mg, 0.325 mmol) and DIEA (0.284 mL, 1.624 mmol) in DMF (2 mL) was added 4-nitrophenyl (4-(3,4-difluorophenoxy)phenyl)carbamate (251 mg, 0.649 mmol) in DMF dropwise over 3 min. The resulting mixture was stirred at room temperature for 16 h. The reaction mixture was concentrated and the crude product was purified via preparative HPLC (Column: SunFire C18, 19 mm x 150 mm, 5 µm particles; Mobile Phase A: water with 5 mM ammonium formate, Mobile Phase B: acetonitrile; Temperature: 25 °C; Gradient: 60-90%B (0.0-12.0 min); Flow: 12 mL/min). Fractions containing the product were combined and lyophilized to dryness to afford [4-(difluoromethoxy)-2-(2,6-dioxopiperidin-3-yl)-3-oxo-2,3-dihydro-1H-isoindol-5-yl]methyl N-[4-(3,4-difluorophenoxy)phenyl]carbamate (20 mg, 10% yield) as a white solid. LCMS (Method K): retention time 2.96 min, [M+H]⁺ 588.0; ¹H NMR (400 MHz, DMSO-d₆) δ 11.01 (s, 1H), 9.84 (s, 1H), 7.81 (d, *J* = 8.0 Hz, 1H), 7.59 (d, *J =* 7.6 Hz, 1H), 7.50 (d, *J =* 8.8 Hz, 2H), 7.44 (d, *J =* 9.2 Hz, 1H), 7.36 (t, *J* = 75.2 Hz, 1H), 7.13-7.07 (m, 1H), 7.02 (d, *J =* 2.0 Hz, 2H), 6.80-6.76 (m, 1H), 5.28 (s, 2H), 5.13-5.08 (m, 1H), 4.51 (d, *J =* 18.0 Hz, 1H), 4.40 (d, *J =* 17.6 Hz, 1H), 2.90 (m, 1H), 2.61 (m, 1H), 2.41 (m, 1H), 2.08 (m, 1H).

### EXAMPLE 62

### [2-(2,6-dioxopiperidin-3-yl)-3-oxo-4-phenoxy-2,3-dihydro-1H-isoindol-5-yl]methyl N-(4-phenoxyphenyl)carbamate

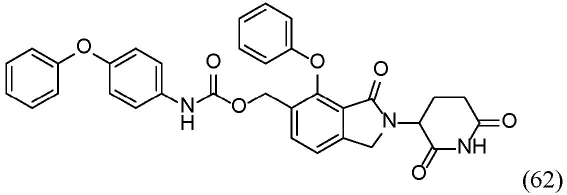

### Intermediate 62A: methyl 3-bromo-2-fluorobenzoate

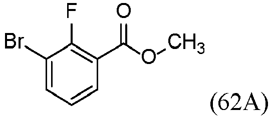

To a cooled (0 °C) solution of 3-bromo-2-fluorobenzoic acid (25 g, 114 mmol) in DMF (80 mL) were added cesium carbonate (74.4 g, 228 mmol) and iodomethane (24.30 g, 171 mmol). The resulting mixture was stirred at room temperature for 12 h. The reaction was quenched with the addition of cold water (100 mL). The reaction mixture was extracted with ethyl acetate (3 x 90 mL). The combined organic layers were washed with brine (90 mL), dried (Na₂SO₄), filtered, and concentrated. The residue was purified by flash chromatography (SiO₂, 10% EtOAc/petroleum ether) to afford methyl 3-bromo-2-fluorobenzoate (25 g, 94% yield) as a colorless oil. ¹H NMR (400 MHz, CDCl₃) δ 7.92-7.88 (m, 1H), 7.84-7.78 (m, 1H), 7.14-7.10 (m, 1H), 3.97 (s, 3H).

### Intermediate 62B: methyl 3-bromo-2-phenoxybenzoate

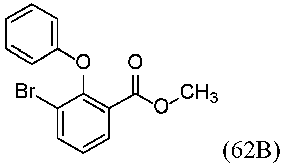

To a solution of methyl 3-bromo-2-fluorobenzoate (25 g, 107 mmol) in NMP (100 mL) were added phenol (12.12 g, 129 mmol) and potassium carbonate (26.7 g, 193 mmol) under a nitrogen atmosphere. The resulting mixture was stirred at 130 °C for 12 h. The reaction as quenched with the addition of cold water (150 mL). The reaction mixture was extracted with ethyl acetate (3 x 90 mL). The combined organic layers were washed with brine (90 mL), dried (Na₂SO₄), filtered, and concentrated. The crude product was purified by flash chromatography (SiO₂, 3% EtOAc/petroleum ether) to afford methyl 3-bromo-2-phenoxybenzoate (16 g, 49% yield) as a yellow oil. ¹H NMR (400 MHz, CDCl₃) δ 7.93-7.90 (m, 1H), 7.86-7.84 (m, 1H), 7.32-7.28 (m, 2H), 7.24-7.20 (m, 1H), 7.06-7.02 (m, 1H), 6.83-6.79 (m, 2H), 3.71 (s, 3H).

### Intermediate 62C: methyl 3-bromo-6-methyl-2-phenoxybenzoate

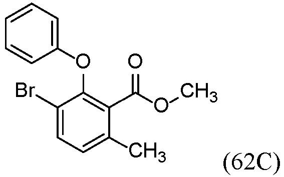

To a solution of methyl 3-bromo-2-phenoxybenzoate (15 g, 48.8 mmol) in MeOH (50 mL) and THF (50 mL) was added a solution of lithium hydroxide (3.51 g, 147 mmol) in water (50 mL). The resulting mixture was stirred at 80 °C for 5 h. The reaction mixture was concentrated, diluted with water (100 mL), and washed with EtOAc (1 x 150 mL). The aqueous phase was acidified with excess 1.5 N HCl (60 mL) and extracted with ethyl acetate (3 x 100 mL). The combined organic layers were washed with brine (100 mL), dried (Na₂SO₄), filtered, and concentrated to afford 3-bromo-2-phenoxybenzoic acid. LCMS (Method M): retention time 1.26 min, [M-H]⁻ 291.0, 293.0.

To a solution of 3-bromo-2-phenoxybenzoic acid (5 g, 17.1 mmol) in HFIP (50 mL) were added potassium carbonate (4.72 g, 34.1 mmol), silver carbonate (9.41 g, 34.1 mmol), potassium methyltrifluoroborate (4.16 g, 34.1 mmol) and pentamethylcyclopentadienyliridium(III) chloride dimer (1.36 g, 1.71 mmol). The resulting mixture was stirred at 100 °C for 12 h. The reaction mixture was then passed through a sintered funnel and the filtrate was concentrated. The residue was diluted with EtOAc (150 mL) and acidified with 1.5 N HCl. The organic layer was washed with brine (50 mL), dried (Na₂SO₄), filtered, and concentrated to afford 3-bromo-6-methyl-2-phenoxybenzoic acid.

To a cooled (0 °C) solution of 3-bromo-6-methyl-2-phenoxybenzoic acid (9 g, 29.3 mmol) in DMF (50 mL) were added potassium carbonate (4.05 g, 29.3 mmol) and methyl iodide (1.83 mL, 29.3 mmol) under nitrogen atmosphere. The resulting mixture was stirred at room temperature for 12 h. The reaction was quenched with the addition of cold water (100 mL). The reaction mixture was extracted with EtOAc (3 x 100 mL). The combined organic layers were washed with brine (70 mL), dried (Na₂SO₄), filtered, and concentrated. The crude residue was purified by flash chromatography (SiO₂, 10% EtOAc/petroleum ether) to afford methyl 3-bromo-6-methyl-2-phenoxybenzoate (9.0 g, 80% yield) as a colorless oil. LCMS (Method K) retention time 3.23 min, [M]⁺ 320.0, 322.0; ¹H NMR (400 MHz, CDCl₃) δ 7.56 (d, *J =* 8.4 Hz, 1H), 7.30-7.24 (m, 2H), 7.04-6.98 (m, 2H), 6.79-6.83 (m, 2H), 3.68 (s, 3H), 2.33 (s, 3H).

### Intermediate 62D: tert-butyl 5-amino-4-(6-bromo-1-oxo-7-phenoxyisoindolin-2-yl)-5-oxopentanoate

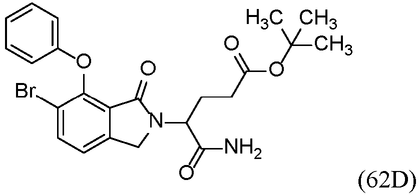

To a solution of methyl 3-bromo-6-methyl-2-phenoxybenzoate (8 g, 24.9 mmol) in DCE (70 mL) were added NBS (5.32 g, 29.9 mmol) and AIBN (0.409 g, 2.491 mmol) under nitrogen atmosphere. The resulting mixture was stirred at 85 °C for 2 h. The reaction mixture was then passed through a sintered funnel, rinsed with petroleum ether (100 mL), and the filtrate was concentrated to afford methyl 3-bromo-6-(bromomethyl)-2-phenoxybenzoate.

To a solution of tert-butyl (S)-4,5-diamino-5-oxopentanoate hydrochloride (7.61 g, 31.9 mmol) in DMF (50 mL) were added DIEA (11.1 mL, 63.7 mmol) and methyl 3-bromo-6-(bromomethyl)-2-phenoxybenzoate (8.5 g, 21.3 mmol) under nitrogen atmosphere. The resulting mixture was stirred at 90 °C for 12 h. The reaction was quenched with the addition of cold water (100 mL). The reaction mixture was extracted with ethyl acetate (3 x 100 mL). The combined organic layers were washed with brine (100 mL), dried (Na₂SO₄), filtered, and concentrated. The crude product was purified by flash chromatography (SiO₂, 70% EtOAc/petroleum ether) to afford tert-butyl 5-amino-4-(6-bromo-1-oxo-7-phenoxyisoindolin-2-yl)-5-oxopentanoate (4.0 g, 27% yield) as a light brown solid. The enantiomeric excess of this material and subsequent intermediates were not determined. LCMS (Method M): retention time 1.41 min, [M+Na]⁺ 511.0, 513.0.

### Intermediate 62E: tert-butyl 5-amino-5-oxo-4-(1-oxo-7-phenoxy-6-vinylisoindolin-2-yl) pentanoate

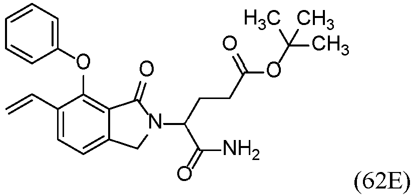

To a solution of tert-butyl 5-amino-4-(6-bromo-1-oxo-7-phenoxyisoindolin-2-yl)-5-oxopentanoate (4.0 g, 8.17 mmol) in 1,4-dioxane (50 mL) were added vinyl tributyltin (3.89 g, 12.26 mmol) and PdCl₂(dppf)₂ (0.598 g, 0.817 mmol) under nitrogen atmosphere. The resulting mixture was purged with nitrogen for 10 min and stirred at 100 °C for 12 h. The reaction mixture was then passed through a Celite pad, which was rinsed with EtOAc (100 ml). The filtrate was concentrated and the crude product was purified by flash chromatography (SiO₂, 70% EtOAc/petroleum ether) to afford tert-butyl 5-amino-5-oxo-4-(1-oxo-7-phenoxy-6-vinylisoindolin-2-yl)pentanoate (3.0 g, 61% yield) as a light brown solid. LCMS (Method N): retention time 1.48 min, [M+H]⁺ 437.2.

### Intermediate 62F: tert-butyl 5-amino-4-(6-(hydroxymethyl)-1-oxo-7-phenoxyisoindolin-2-yl)-5-oxopentanoate

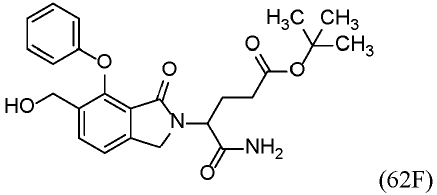

A cooled (-78 °C) solution of tert-butyl 5-amino-5-oxo-4-(1-oxo-7-phenoxy-6-vinylisoindolin-2-yl)pentanoate (3 g, 6.87 mmol) in DCM (20 mL) and tert-butanol (2 mL) was purged with ozone for 40 min while stirring. The reaction was quenched with the addition of dimethyl sulfide (0.508 mL, 6.87 mmol) at the same temperature. The resulting mixture was warmed to room temperature and distilled to afford tert-butyl 5-amino-4-(6-formyl-1-oxo-7-phenoxyisoindolin-2-yl)-5-oxopentanoate.

To a cooled (0 °C) solution of tert-butyl 5-amino-4-(6-formyl-1-oxo-7-phenoxyisoindolin-2-yl)-5-oxopentanoate (5.0 g, 5.93 mmol) in THF (60 mL) was added sodium triacetoxyborohydride (2.51 g, 11.9 mmol). The resulting mixture was stirred at room temperature for 12 h. The reaction was quenched with the addition of cold water (50 mL). The reaction mixture was extracted with EtOAc (3 x 100 mL). The combined organic layers were washed with brine (100 mL), dried (Na₂SO₄), filtered, and concentrated. The crude product was purified by reversed phase chromatography (80 g RediSep Silver column, 40% acetonitrile/water with 0.1% ammonium formate). The fractions were lyophilized to dryness to afford tert-butyl 5-amino-4-(6-(hydroxymethyl)-1-oxo-7-phenoxyisoindolin-2-yl)-5-oxopentanoate (1.2 g, 44% yield) as an off-white solid. LCMS (Method M): retention time 1.07 min, [M+H]⁺ 441.2.

### Intermediate 62G: 3-(6-(hydroxymethyl)-1-oxo-7-phenoxyisoindolin-2-yl)piperidine-2,6-dione

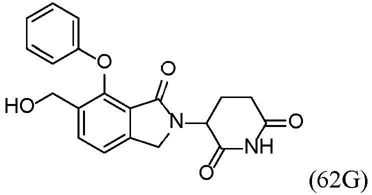

To a solution of tert-butyl 5-amino-4-(6-(hydroxymethyl)-1-oxo-7-phenoxyisoindolin-2-yl)-5-oxopentanoate (1.1 g, 2.50 mmol) in 1,4-dioxane (10 mL) was added methanesulfonic acid (0.480 g, 4.99 mmol) under nitrogen atmosphere. The resulting mixture was stirred at 85 °C for 4 h. The reaction mixture was concentrated and the crude product was purified by reversed phase chromatography (40 g RediSep Gold column, 40% acetonitrile/water with 0.1% TFA). The fractions were lyophilized to dryness to afford 3-(6-(hydroxymethyl)-1-oxo-7-phenoxyisoindolin-2-yl)piperidine-2,6-dione (0.2 g, 16% yield) as a light yellow solid. LCMS (Method N): retention time 0.93 min, [M+H]⁺ 367.2.

### Example 62:

To a solution of 3-(6-(hydroxymethyl)-1-oxo-7-phenoxyisoindolin-2-yl)piperidine-2,6-dione (80 mg, 0.159 mmol) in THF (5 mL) was added potassium carbonate (66.1 mg, 0.478 mmol). The resulting mixture was stirred for 10 min, then 4-nitrophenyl (4-(phenoxy) phenyl)carbamate (123 mg, 0.319 mmol) in THF (5 mL) was added dropwise over 15 min. The reaction mixture was stirred at room temperature for 12 h. The mixture was then concentrated and purified via reversed phase chromatography (80 g RediSep column, 75% acetonitrile/water with 0.1% TFA). Fractions containing the product were combined and lyophilized to dryness to afford [2-(2,6-dioxopiperidin-3-yl)-3-oxo-4-phenoxy-2,3-dihydro-1H-isoindol-5-yl]methyl N-(4-phenoxyphenyl)carbamate (35 mg, 32% yield). LCMS (Method K): retention time 2.94 min, [M+H]⁺ 578.2; ¹H NMR (400 MHz, DMSO-d₆): δ 10.95 (s, 1H), 9.73 (s, 1H), 7.84 (d, *J =* 7.6 Hz, 1H), 7.57 (d, *J =* 7.6 Hz, 1H), 7.41 (d, *J =* 8.6 Hz, 2H), 7.29-7.25 (m, 2H), 7.09 (t, *J =* 7.2 Hz, 1H), 6.98-6.92 (m, 5H), 6.79 (d, *J =* 0.8 Hz, 2H), 4.95 (dd, *J* = 4.8, 13.2 Hz, 1H), 4.48 (d, *J* = 17.6 Hz, 1H), 4.48 (d, *J* = 17.6 Hz, 1H), 2.83-2.67 (m, 1H), 2.51-2.50 (m, 1H), 2.36-2.30 (m, 1H), 1.96-1.93 (m, 1H).

### EXAMPLE 63

### [2-(2,6-dioxopiperidin-3-yl)-3-oxo-4-phenoxy-2,3-dihydro-1H-isoindol-5-yl]methyl N-[4-(3,4-difluorophenoxy)phenyl]carbamate

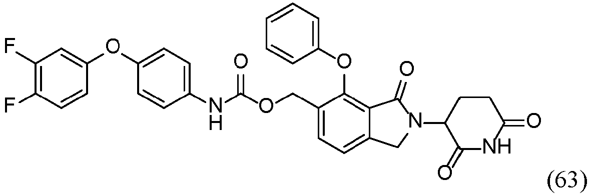

To a solution of 3-(6-(hydroxymethyl)-1-oxo-7-phenoxyisoindolin-2-yl)piperidine-2,6-dione (80 mg, 0.159 mmol) in THF (5 mL) was added potassium carbonate (66.1 mg, 0.478 mmol). The resulting mixture was stirred for 10 min, then 4-nitrophenyl (4-(3,4-difluorophenoxy)phenyl)carbamate (123 mg, 0.319 mmol) in THF (5 mL) was added dropwise over 15 min. The reaction mixture was stirred at room temperature for 12 h. The mixture was then concentrated and purified via reversed phase chromatography (80 g RediSep column, 75% acetonitrile/water with 0.1% TFA). The product was further purified via preparatory HPLC (Column: SunFire C8, 19 mm x 250 mm, 5 µm particles; Mobile Phase A: water, Mobile Phase B: acetonitrile; Temperature: 25 °C; Eluent: 95%B (0.0-13.0 min); Flow: 15 mL/min). Fractions containing the product were combined and lyophilized to dryness to afford [2-(2,6-dioxopiperidin-3-yl)-3-oxo-4-phenoxy-2,3-dihydro-1H-isoindol-5-yl]methyl N-[4-(3,4-difluorophenoxy)phenyl]carbamate (36 mg, 31% yield) as an off-white solid. LCMS (Method K): retention time 3.16 min, [M+H]⁺ 614.2; ¹H NMR (400 MHz, DMSO-d₆): δ 10.96 (s, 1H), 9.76 (s, 1H), 7.85 (d, *J* = 7.6 Hz, 1H), 7.57 (d, *J* = 7.6 Hz, 1H), 7.47-7.40 (m, 3H), 7.29-7.25 (m, 2H), 7.10 (m, 1H), 7.02-6.99 (m, 3H), 6.80-6.78 (m, 3H), 5.13 (s, 2H), 4.95 (dd, *J* = 5.2, 13.4 Hz, 1H), 4.49 (d, *J* = 17.6 Hz, 1H), 4.36 (d, *J* = 17.6 Hz, 1H), 2.87-2.79 (m, 1H), 2.59-2.50 (m, 1H), 2.50-2.32 (m, 1H), 1.98-1.93 (m, 1H).

### EXAMPLE 64

### [2-(2,6-dioxopiperidin-3-yl)-3-oxo-4-phenoxy-2,3-dihydro-1H-isoindol-5-yl]methyl N-[4-(3-fluorophenoxy)phenyl]carbamate

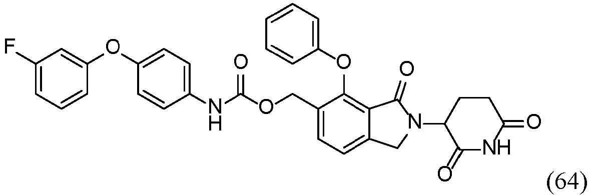

A mixture of 3-(6-(hydroxymethyl)-1-oxo-7-phenoxyisoindolin-2-yl)piperidine-2,6-dione (30 mg, 0.082 mmol), 4-(3-fluorophenoxy)benzoic acid (15.95 mg, 0.069 mmol), diphenyl phosphoryl azide (25.8 mg, 0.094 mmol), and DIEA (0.027 mL, 0.156 mmol) in 1,4-dioxane (1 mL) was heated at 105 °C for 15 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 41-71%B (0.0-20.0 min), 71-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-3-oxo-4-phenoxy-2,3-dihydro-1H-isoindol-5-yl]methyl N-[4-(3-fluorophenoxy)phenyl]carbamate (22 mg, 57% yield). LCMS (Method C): retention time 2.07 min, [M+H]⁺ 596.1; ¹H NMR (500 MHz, DMSO-d₆) δ 9.75 (br s, 1H), 7.84 (d, J=7.9 Hz, 1H), 7.57 (br d, J=7.7 Hz, 1H), 7.45 (br d, J=7.6 Hz, 2H), 7.37 (q, J=7.9 Hz, 1H), 7.30-7.22 (m, 2H), 7.07-6.96 (m, 3H), 6.92-6.83 (m, 1H), 6.80-6.68 (m, 4H), 5.12 (s, 2H), 4.93 (dd, J=13.3, 5.0 Hz, 1H), 4.58-4.29 (m, 2H), 2.84-2.74 (m, 1H), 2.59 (br d, J=1.8 Hz, 1H), 2.39-2.26 (m, 1H), 2.01-1.92 (m, 1H).

### EXAMPLES 65-66

The compounds in Table 5 were prepared according to the general procedures described for Example 64, replacing 4-(3-fluorophenoxy)benzoic acid with the appropriate acid:

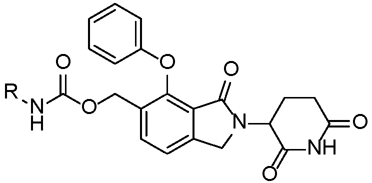

**TABLE 5**

| Ex. No. | R | LCMS [M+H]⁺ | RT (min) | HPLC Method |
|---|---|---|---|---|
| 65 | 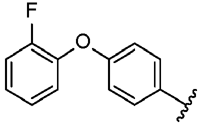 | 596.0 | 2.08 | C |
| 66 | 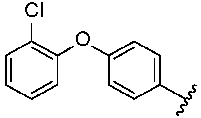 | 612.1 | 1.83 | D |

### EXAMPLE 67

### [2-(2,6-dioxopiperidin-3-yl)-3-oxo-4-phenoxy-2,3-dihydro-1H-isoindol-5-yl]methyl N-[4-(2,4-difluorophenoxy)phenyl]carbamate

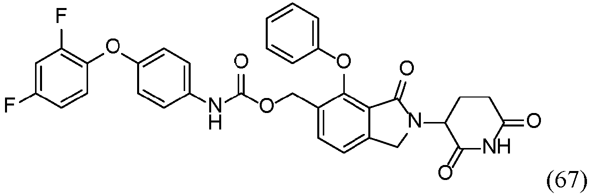

Phosgene (15% in toluene, 64.8 mg, 0.098 mmol) was added to 3-(6-(hydroxymethyl)-1-oxo-7-phenoxyisoindolin-2-yl)piperidine-2,6-dione (30 mg, 0.082 mmol) in THF (1 mL) and DCM (1 mL). The resulting mixture was stirred at room temperature for 1 h and then concentrated in vacuo. The residue was dissolved in THF (1 mL), then 4-(2,4-difluorophenoxy)aniline (18.1 mg, 0.082 mmol) was added, followed by TEA (0.029 mL, 0.205 mmol). The reaction mixture was stirred at room temperature for 1 h and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 50-80%B (0.0-20.0 min), 80-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-3-oxo-4-phenoxy-2,3-dihydro-1H-isoindol-5-yl]methyl N-[4-(2,4-difluorophenoxy)phenyl]carbamate (2.5 mg, 5% yield). LCMS (Method D): retention time 2.02 min, [M+H]⁺ 614.1; ¹H NMR (500 MHz, DMSO-d₆) δ 9.69 (br s, 1H), 7.82 (d, J=7.8 Hz, 1H), 7.56 (d, J=7.8 Hz, 1H), 7.45-7.33 (m, 3H), 7.26 (t, J=8.0 Hz, 2H), 7.21-7.14 (m, 1H), 7.10-7.03 (m, 1H), 7.01-6.96 (m, 1H), 6.90 (d, J=9.0 Hz, 2H), 6.77 (d, J=7.9 Hz, 2H), 5.10 (s, 2H), 4.92 (dd, J=13.3, 5.1 Hz, 1H), 4.54-4.29 (m, 2H), 2.86-2.75 (m, 1H), 2.65-2.55 (m, 1H), 2.38-2.24 (m, 1H), 2.01-1.91 (m, 1H).

### EXAMPLES 68-70

The compounds in Table 6 were prepared according to the general procedures described for Example 67, replacing 4-(2,4-difluorophenoxy)aniline with the appropriate aniline:

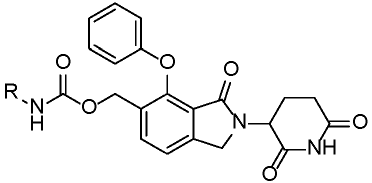

**TABLE 6**

| Ex. No. | R | LCMS [M+H]⁺ | RT (min) | HPLC Method |
|---|---|---|---|---|
| 68 | 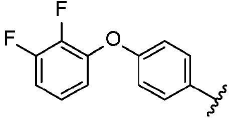 | 614.1 | 2.15 | C |
| 69 | 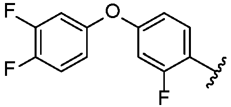 | 631.8 | 2.20 | D |
| 70 | 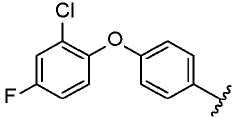 | 630.0 | 2.18 | D |

### EXAMPLES 71-92

The compounds in Table 7 were prepared according to the general procedures described for Example 1, replacing 4-(3,4-difluorophenoxy)benzoic acid with the appropriate acid (which was either commercially available, or synthesized as described for Intermediate I-1, I-2, I-3, I-4, I-5, or I-6, or in a similar manner):

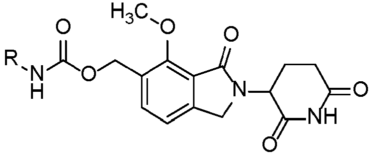

**TABLE 7**

| Ex. No. | R | LCMS [M+H]⁺ | RT (min) | HPLC Method |
|---|---|---|---|---|
| 71 | 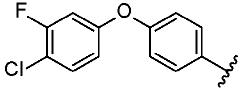 | 568.0 | 2.08 | C |
| 72 | 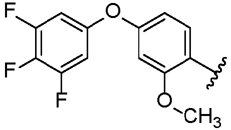 | 600.2 | 2.31 | C |
| 73 | 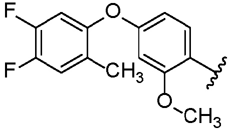 | 596.2 | 2.4 | C |
| 74 | 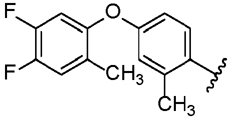 | 580.1 | 2.01 | C |
| 75 | 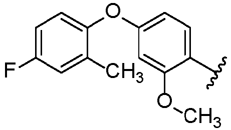 | 578.0 | 2.09 | C |
| 76 | 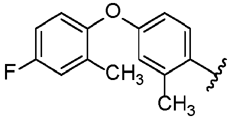 | 562.1 | 1.96 | D |
| 77 | 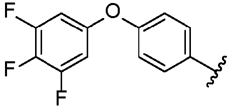 | 570.2 | 2 | C |
| 78 | 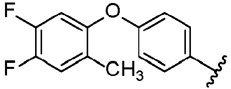 | 565.9 | 2.01 | C |
| 79 | 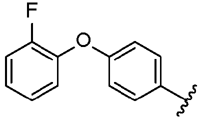 | 534.0 | 1.81 | D |
| 80 | 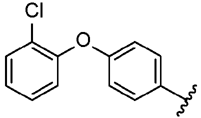 | 550.2 | 1.97 | C |
| 81 | 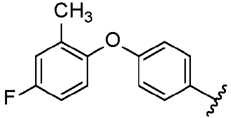 | 548.2 | 1.96 | D |
| 82 | 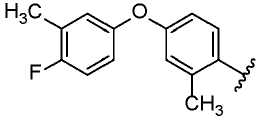 | 562.5 | 2.08 | C |
| 83 | 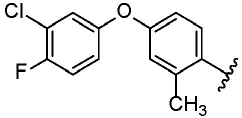 | 581.9 | 2.08 | C |
| 84 | 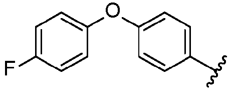 | 534.0 | 1.95 | D |
| 85 | 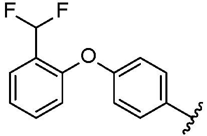 | 566.2 | 1.92 | C |
| 86 | 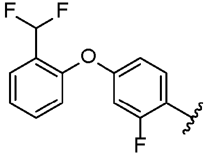 | 584.2 | 1.88 | C |
| 87 | 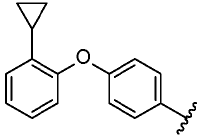 | 556.2 | 2.09 | C |
| 88 | 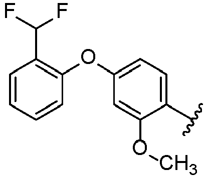 | 596.2 | 1.93 | D |
| 89 | 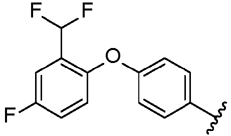 | 584.1 | 1.97 | C |
| 90 | 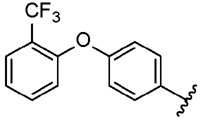 | 618.0 | 2.11 | C |
| 91 | 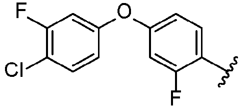 | 586.0 | 2.08 | C |
| 92 | 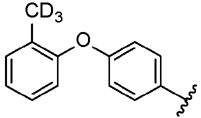 | 533.1 | 1.97 | C |

### EXAMPLES 93-112

The compounds in Table 8 were prepared according to the general procedures described for Example 37, replacing 4-(4-fluorophenoxy)-2-methoxyaniline with the appropriate aniline:

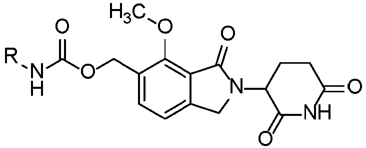

**TABLE 8**

| Ex No. | R | LCMS [M+H]⁺ | RT (min) | HPLC Method |
|---|---|---|---|---|
| 93 | 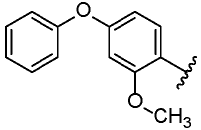 | 546.2 | 1.89 | C |
| 94 | 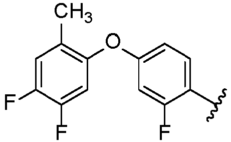 | 584.3 | 2.07 | C |
| 95 | 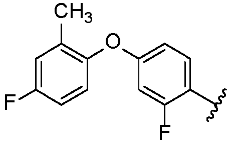 | 566.1 | 2.26 | C |
| 96 | 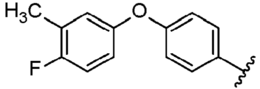 | 547.9 | 2.07 | D |
| 97 | 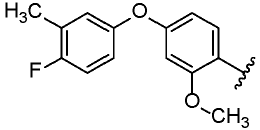 | 578.1 | 2.37 | C |
| 98 | 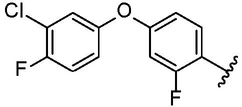 | 586.0 | 2.36 | C |
| 99 | 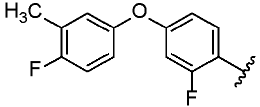 | 566.0 | 2.07 | D |
| 100 | 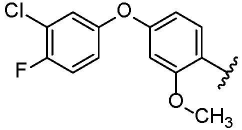 | 598.2 | 2.07 | D |
| 101 | 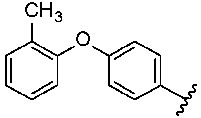 | 529.9 | 2.08 | D |

| Ex No. | R | LCMS [M+H]⁺ | RT (min) | HPLC Method |
|---|---|---|---|---|
| 102 | 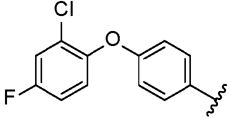 | 568.0 | 2.10 | D |
| 103 | 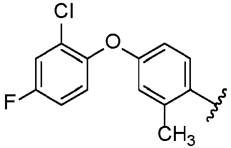 | 582.0 | 2.09 | D |
| 104 | 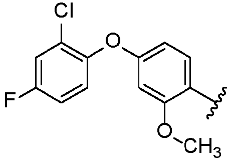 | 598.4 | 2.10 | C |
| 105 | 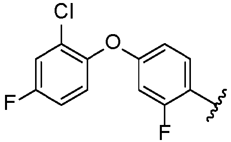 | 586.2 | 2.05 | C |
| 106 | 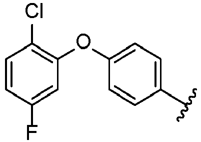 | 568.3 | 2.04 | D |
| 107 | 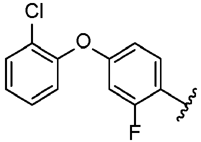 | 567.9 | 1.99 | C |
| 108 | 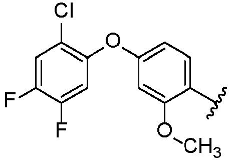 | 615.9 | 2.13 | C |

| Ex No. | R | LCMS [M+H]⁺ | RT (min) | HPLC Method |
|---|---|---|---|---|
| 109 | 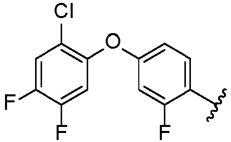 | 604.0 | 2.21 | D |
| 110 | 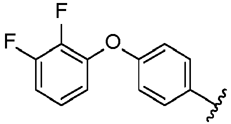 | 552.2 | 1.86 | D |
| 111 | 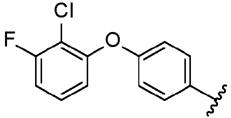 | 568.3 | 2.05 | D |
| 112 | 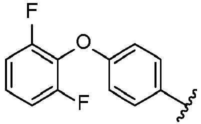 | 552.0 | 1.87 | D |

### INTERMEDIATE I-8

### 4-(naphthalen-1-yloxy)aniline

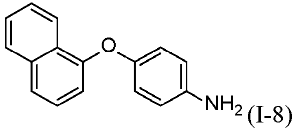

### Intermediate I-8A: 1-(4-nitrophenoxy)naphthalene

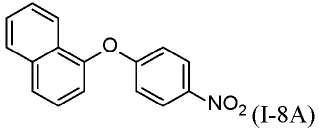

To a 20 mL vial, containing a well-stirred solution of naphthalen-1-ol (1.0 g, 6.94 mmol) and 1-fluoro-4-nitrobenzene (1.077 g, 7.63 mmol) in N,N-dimethylformamide (10 mL), was added cesium carbonate (5.65 g, 17.34 mmol) under nitrogen. The reaction mixture was stirred at 120 °C for 12 h. The reaction mixture was filtered through a pad of Celite, rinsing with EtOAc (300 mL). The combined filtrate was concentrated under reduced pressure. The resulting residue was partitioned between water (50 mL) and EtOAc. The aqueous layer was extracted with EtOAc (2 x 100 mL). The combined organics were dried over Na₂SO₄, filtered, and concentrated. The resulting residue was purified by silica gel (100-230 mesh) chromatography with petroleum ether/EtOAc to afford 1-(4-nitrophenoxy) naphthalene (1.2 g, 65% yield) as a brown solid. LCMS (Method K): retention time 0.75 min, [M+H]⁺ 265.0.

### Intermediate I-8B:

To a 100 mL round bottom flask, containing a well-stirred solution of 1-(4-nitrophenoxy)naphthalene (500 mg, 1.885 mmol) in anhydrous DMF (10 mL), was added hypodiboric acid (676 mg, 7.54 mmol) and 4,4'-bipyridine (118 mg, 0.754 mmol) at ambient temperature under nitrogen. The reaction mixture was stirred at ambient temperature for 1 h. The reaction mixture was quenched with ice water (100 mL) and filtered through a pad of Celite, rinsing with EtOAc (200 mL). The combined filtrate was concentrated under reduced pressure and purified by flash silica-gel (230-400 mesh) chromatography with 0-100% petroleum ether/EtOAc to afford 4-(naphthalen-1-yloxy)aniline (240 mg, 52% yield) as a thick brown liquid. LCMS (Method K): retention time 2.93 min, [M+H]⁺ 236.0; ¹H NMR (400 MHz, DMSO-d₆) δ 8.23 (dd, J=2.0, 8.2 Hz, 1H), 7.94 (dd, J=2.8, 6.8 Hz, 1H), 7.53-7.58 (m, 3H), 7.38 (t, J=8.0 Hz, 1H), 6.82-6.85 (m, 2H), 6.68 (dd, *J =*0.4, 7.6 Hz, 1H), 6.64 (d, J=3.6 Hz, 2H), 5.00 (s, 2H).

### EXAMPLE 113

### [2-(2,6-dioxopiperidin-3-yl)-4-methoxy-3-oxo-2,3-dihydro-1H-isoindol-5-yl]methyl N-[4-(naphthalen-1-yloxy)phenyl]carbamate

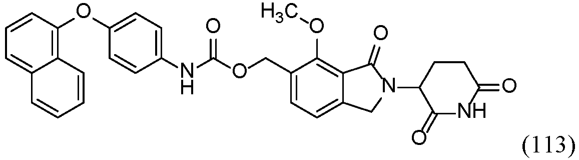

A suspension of 3-(6-(hydroxymethyl)-7-methoxy-1-oxoisoindolin-2-yl)piperidine-2,6-dione (15 mg, 0.049 mmol) in THF (1 mL) was warmed to 50 °C and stirred vigorously for 15 min to give a cloudy mixture. The mixture was cooled to room temperature and treated with phosgene (20% in toluene; 0.039 mL, 0.074 mmol). After 15 min, the reaction mixture was warmed to 45 °C and stirred vigorously at that temperature for 90 min. The reaction mixture was concentrated. The resulting residue was suspended in THF (1 mL) and treated with 4-(naphthalen-1-yloxy)aniline (11.60 mg, 0.049 mmol), followed by DIEA (0.019 mL, 0.108 mmol). The reaction mixture was stirred at room temperature for 10 min, warmed to 45 °C, stirred for 15 min, and then allowed to cool to room temperature. The reaction mixture was concentrated, dissolved in DMF, treated with acetic acid (0.014 mL, 0.246 mmol), filtered, and purified by preparative HPLC (Column: XBridge C18, 19 mm x 200 mm, 5 µm particles; Mobile Phase A: acetonitrile/water (5:95) with 0.05% TFA, Mobile Phase B: acetonitrile/water (95:5) with 0.05% TFA; Temperature: 25 °C; Gradient: 35-75%B (0.0-20.0 min), 75-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-4-methoxy-3-oxo-2,3-dihydro-1H-isoindol-5-yl]methyl N-[4-(naphthalen-1-yloxy)phenyl]carbamate (7.2 mg, 26% yield). LCMS (Method C): retention time 2.14 min, [M+H]⁺ 566.2; ¹H NMR (500 MHz, DMSO-d₆) δ 10.99 (s, 1H), 9.74 (br s, 1H), 8.13 (br d, J=8.0 Hz, 1H), 7.97 (br d, J=8.1 Hz, 1H), 7.68 (br d, J=7.8 Hz, 2H), 7.61-7.52 (m, 2H), 7.49 (br d, J=7.7 Hz, 2H), 7.44 (br t, J=7.9 Hz, 1H), 7.34 (br d, J=7.6 Hz, 1H), 7.05-7.00 (m, 2H), 6.87 (d, J=7.6 Hz, 1H), 5.22 (s, 2H), 5.07 (br dd, J=13.2, 5.1 Hz, 1H), 4.47-4.38 (m, 1H), 4.35-4.26 (m, 1H), 4.04 (s, 3H), 2.95-2.85 (m, 1H), 2.62 (br d, J=17.9 Hz, 1H), 2.45-2.32 (m, 1H), 2.08-1.94 (m, 1H).

### INTERMEDIATE I-9

### 4-(2,3-dichlorophenoxy)-2-fluoroaniline

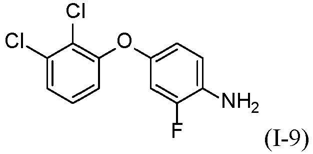

To a stirred solution of 4-bromo-2-fluoroaniline (10 g, 52.6 mmol) and 2,3-dichlorophenol (8.58 g, 52.6 mmol) in 1,4-dioxane (100 mL) was added cesium carbonate (34.3 g, 105 mmol). The resulting mixture was purged with nitrogen for 5 min. To this was added N,N-dimethylglycine (1.628 g, 15.79 mmol) and copper(I) bromide (2.265 g, 15.79 mmol). The mixture was warmed to 80 °C and held at that temperature for 16 h. The reaction mixture was cooled to room temperature and filtered through Celite. The filtrate was quenched with water (100 mL) and extracted with ethyl acetate (2 x 150mL). The combined organic layers were washed with brine, dried over Na₂SO₄, filtered, and concentrated. The residue was purified using silica gel chromatography, eluting with petroleum ether/EtOAc to afford 4-(2,3-dichlorophenoxy)-2-fluoroaniline (1.77 g, 12% yield). LCMS (Method K): retention time 3.21 min, [M+H]⁺ 274.0. ¹H NMR (400 MHz, DMSO-d₆) δ 7.35 (dd, J=1.2, 8.2 Hz, 1H), 7.28 (t, J=8.4 Hz, 1H), 6.90 (dd, J=2.8, 12.0 Hz, 1H), 6.78-6.83 (m, 2H), 6.65-6.68 (m, 1H), 5.08 (s, 1H).

### EXAMPLES 114-118

The compounds in Table 9 were prepared according to the general procedures described for Example 113, replacing 4-(naphthalen-1-yloxy)aniline with the appropriate aniline (synthesized as described for Intermediate I-8 or I-9, or in a similar manner):

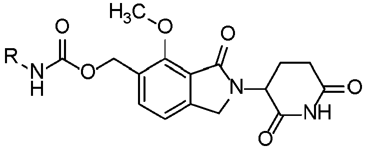

**TABLE 9**

| Ex. No. | R | LCMS [M+H]⁺ | RT (min) | HPLC Method |
|---|---|---|---|---|
| 114 | 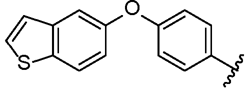 | 572.2 | 2.07 | C |
| 115 | 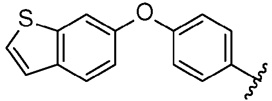 | 572.0 | 2.06 | D |
| 116 | 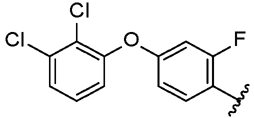 | 602.2 | 2.19 | C |
| 117 | 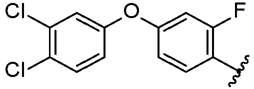 | 601.8 | 2.23 | D |
| 118 | 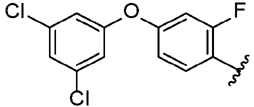 | 601.9 | 2.30 | D |

### EXAMPLE 119

### [2-(2,6-dioxopiperidin-3-yl)-3-oxo-4-(propan-2-yloxy)-2,3-dihydro-1H-isoindol-5-yl]methyl N-[4-(3,4-difluorophenoxy)-2-fluorophenyl]carbamate

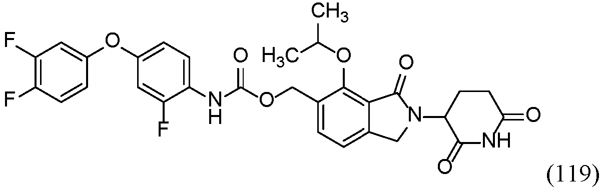

Phosgene (15% in toluene, 71.4 mg, 0.108 mmol) was added to 3-(6-(hydroxymethyl)-7-isopropoxy-1-oxoisoindolin-2-yl)piperidine-2,6-dione (30 mg, 0.090 mmol) in THF (1 mL) and DCM (1 mL). The resulting mixture was stirred at room temperature for 1 h and then concentrated in vacuo. The residue was dissolved in THF (1 mL), then 4-(3,4-difluorophenoxy)-2-fluoroaniline (21.59 mg, 0.090 mmol) was added, followed by TEA (0.031 mL, 0.226 mmol). The reaction mixture was stirred at room temperature for 1 h and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 34-74%B (0.0-20.0 min), 74-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-3-oxo-4-(propan-2-yloxy)-2,3-dihydro-1H-isoindol-5-yl]methyl N-[4-(3,4-difluorophenoxy)-2-fluorophenyl] carbamate (4.1 mg, 8% yield). LCMS (Method D): retention time 2.20 min, [M+H]⁺ 598.2; ¹H NMR (500 MHz, DMSO-d₆) δ 9.37 (br s, 1H), 7.65 (br d, J=7.6 Hz, 1H), 7.61-7.52 (m, 1H), 7.52-7.38 (m, 1H), 7.33-7.17 (m, 2H), 7.01 (dd, *J =11.4,* 2.5 Hz, 1H), 6.94-6.79 (m, 2H), 5.21 (s, 2H), 5.05 (br dd, *J=*13.0*,* 4.9 Hz, 1H), 4.93 (quin, J=6.0 Hz, 1H), 4.50-4.21 (m, 2H), 2.97-2.83 (m, 1H), 2.65-2.58 (m, 1H), 2.41-2.30 (m, 1H), 2.09-1.93 (m, 1H), 1.24 (br dd, J=17.3, 6.0 Hz, 6H).

### EXAMPLES 120-124

The compounds in Table 10 were prepared according to the general procedures described for Example 119, replacing 4-(3,4-difluorophenoxy)-2-fluoroaniline with the appropriate aniline:

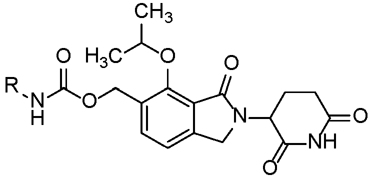

**TABLE 10**

| Ex. No. | R | LCMS [M+H]⁺ | RT (min) | HPLC Method |
|---|---|---|---|---|
| 120 | 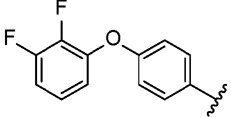 | 580.2 | 2.15 | D |
| 121 | 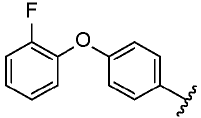 | 562.2 | 2.03 | D |
| 122 | 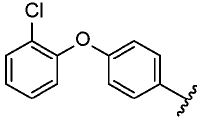 | 578.2 | 2.14 | D |
| 123 | 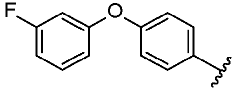 | 561.9 | 2.10 | C |
| 124 | 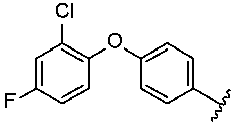 | 596.2 | 2.18 | D |

### EXAMPLE 125

### {2-[(3S)-2,6-dioxopiperidin-3-yl]-3-oxo-4-(propan-2-yloxy)-2,3-dihydro-1H-isoindol-5-yl} methyl N-[4-(3,4-difluorophenoxy)phenyl]carbamate

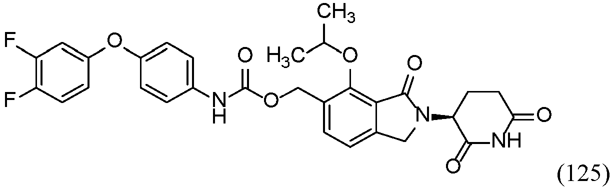

Phosgene (20% in toluene, 446 mg, 0.903 mmol) was added to 3-(6-(hydroxymethyl)-7-isopropoxy-1-oxoisoindolin-2-yl)piperidine-2,6-dione (200 mg, 0.602 mmol) (Intermediate 56E, derived from tert-butyl (S)-4,5-diamino-5-oxopentanoate building block) in THF (6 mL). The resulting mixture was stirred at room temperature for 1 h and then concentrated in vacuo. The residue was dissolved in THF (6 mL), then 4-(3,4-difluorophenoxy)aniline (146 mg, 0.662 mmol) was added, followed by DIEA (0.231 mL, 1.324 mmol). The reaction mixture was stirred at room temperature for 1 h and purified via flash chromatography on silica gel to afford [2-(2,6-dioxopiperidin-3-yl)-3-oxo-4-(propan-2-yloxy)-2,3-dihydro-1H-isoindol-5-yl]methyl N-[4-(3,4-difluorophenoxy)phenyl]carbamate as a ~82:18 mixture of (S)- and (R)- enantiomers (determined by SFC). The material was dissolved in MeOH/DCM (1:1) and resolved by SFC chiral separation (Column: Cellulose-4, 5 x 25 cm, 5 µm particles; Mobile Phase: carbon dioxide/MeOH (40:60); Temperature: 35 °C; BPR Pressure: 120 bar; Flow: 250 mL/min). Fractions containing the major peak (peak 1) were combined and lyophilized to dryness to afford {2-[(3S)-2,6-dioxopiperidin-3-yl]-4-methoxy-3-oxo-2,3-dihydro-1H-isoindol-5-yl} methyl N-[4-(3,4-difluorophenoxy)phenyl]carbamate (160 mg, 45% yield). SFC (Method O): retention time: 4.31 min (>99% ee); LCMS (Method E): retention time 1.05 min, [M+H]⁺ 580.1; ¹H NMR (400 MHz, METHANOL-d₄) δ 7.69 (d, J=7.7 Hz, 1H), 7.46 (br d, J=8.7 Hz, 2H), 7.32-7.14 (m, 2H), 7.01-6.92 (m, 2H), 6.86 (ddd, J=11.8, 6.6, 2.9 Hz, 1H), 6.72 (dtd, J=9.0, 3.3, 1.8 Hz, 1H), 5.30 (s, 2H), 5.12-4.95 (m, 2H), 4.54-4.35 (m, 2H), 2.96-2.73 (m, 2H), 2.47 (dd, J=13.0, 4.9 Hz, 1H), 2.15 (dtd, J=12.8, 5.3, 2.4 Hz, 1H), 1.32 (d, J=6.1 Hz, 6H).

### EXAMPLES 126-131

The compounds in Table 11 were prepared according to the general procedures described for Example 52, replacing iodoethane in the first step with the appropriate alkyl halide, and replacing 4-phenoxybenzoic acid in the final step with the appropriate benzoic acid:

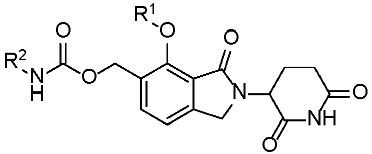

**TABLE 11**

| Ex. No. | R¹ | R² | LCMS [M+H]⁺ | RT (min) | HPLC Method |
|---|---|---|---|---|---|
| 126 | 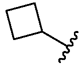 | 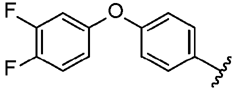 | 592.1 | 2.14 | D |
| 127 | 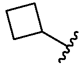 | 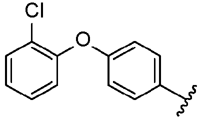 | 590.1 | 2.13 | C |
| 128 | 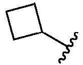 | 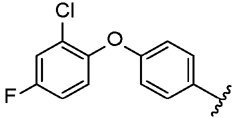 | 608.1 | 2.19 | D |
| 129 | 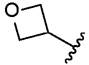 | 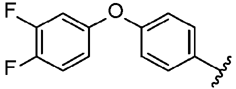 | 594.0 | 1.96 | D |
| 130 | 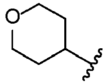 | 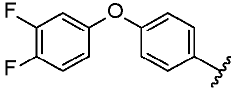 | 622.2 | 1.01 | E |
| 131 | 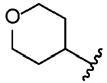 | 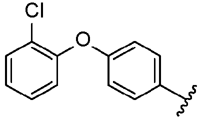 | 620.0 | 2.01 | C |

### EXAMPLE 132

### [2-(2,6-dioxopiperidin-3-yl)-4-(oxan-4-yloxy)-3-oxo-2,3-dihydro-1H-isoindol-5-yl]methyl N-[4-(3,4-difluorophenoxy)-2-fluorophenyl]carbamate

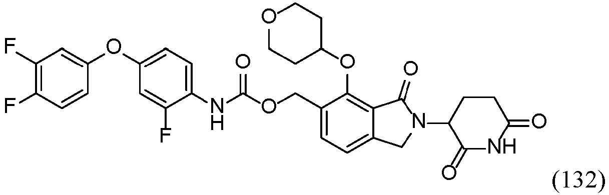

### Intermediate 132A: 3-(6-(hydroxymethyl)-1-oxo-7-((tetrahydro-2H-pyran-4-yl)oxy) isoindolin-2-yl)piperidine-2,6-dione

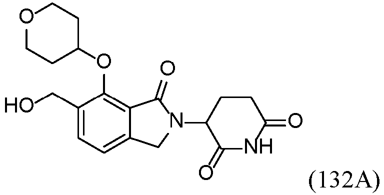

Prepared according to the general procedures described for Example 52, Intermediate 52D, replacing iodoethane in the first step with 4-iodotetrahydro-2H-pyran. LCMS (Method E): retention time 0.75 min, [M+H]⁺ 375.1; ¹H NMR (400 MHz, METHANOL-d₄) δ 7.72 (d, J=7.7 Hz, 1H), 7.28 (d, J=7.7 Hz, 1H), 5.09 (dd, J=13.3, 5.2 Hz, 1H), 4.82-4.77 (m, 1H), 4.74 (s, 2H), 4.51-4.34 (m, 2H), 3.97 (dt, *J=11.8,* 4.0 Hz, 2H), 3.51-3.39 (m, 2H), 2.96-2.86 (m, 1H), 2.82-2.71 (m, 1H), 2.48 (qd, J=13.2, 4.8 Hz, 1H), 2.21-2.10 (m, 1H), 2.02 (br dd, *J=11.4,* 1.4 Hz, 2H), 1.85-1.70 (m, 2H).

### Example 132:

Phosgene (15% in toluene, 19.8 mg, 0.040 mmol) was added to 3-(6-(hydroxymethyl)-1-oxo-7-((tetrahydro-2H-pyran-4-yl)oxy)isoindolin-2-yl)piperidine-2,6-dione (10 mg, 0.027 mmol) in THF (1 mL). The resulting mixture was stirred at room temperature for 1 h and then concentrated in vacuo. The residue was dissolved in THF (1 mL), then 4-(3,4-difluorophenoxy)-2-fluoroaniline (7.0 mg, 0.029 mmol) was added, followed by DIEA (10 µL, 0.059 mmol). The reaction mixture was stirred at room temperature for 1 h and purified via preparative HPLC (Column: XBridge C18, 19 mm x 200 mm, 5 µm particles; Mobile Phase A: acetonitrile/water (5:95) with 0.05% TFA, Mobile Phase B: acetonitrile/water (95:5) with 0.05% TFA; Temperature: 25 °C; Gradient: 35-65%B (0.0-20.0 min), 65-100%B (20.0-20.1 min), 100%B (20.1-24.0 min); Flow: 20 mL/min). Fractions containing the product were combined and dried via centrifugal evaporation to afford [2-(2,6-dioxopiperidin-3-yl)-4-(oxan-4-yloxy)-3-oxo-2,3-dihydro-1H-isoindol-5-yl] methyl N-[4-(3,4-difluorophenoxy)-2-fluorophenyl]carbamate (4.6 mg, 26% yield). LCMS (Method C): retention time 2.00 min, [M+H]⁺ 640.1; ¹H NMR (500 MHz, DMSO-d₆) δ 10.97 (s, 1H), 9.54-9.21 (m, 1H), 7.66 (br d, J=7.4 Hz, 1H), 7.59-7.50 (m, 1H), 7.49-7.39 (m, 1H), 7.31 (d, J=7.6 Hz, 1H), 7.24-7.18 (m, 1H), 7.00 (dd, *J=11.4,* 2.6 Hz, 1H), 6.92-6.80 (m, 2H), 5.21 (s, 2H), 5.07 (dd, J=13.3, 5.0 Hz, 1H), 4.84-4.72 (m, 1H), 4.47-4.36 (m, 1H), 4.34-4.22 (m, 1H), 3.89-3.80 (m, 2H), 3.36-3.25 (m, 2H), 2.93-2.80 (m, 1H), 2.61 (br d, J=17.4 Hz, 1H), 2.36 (qd, J=13.2, 4.7 Hz, 1H), 2.04-1.84 (m, 3H), 1.76-1.58 (m, 2H).

### EXAMPLES 133-136

The compounds in Table 12 were prepared according to the general procedures described for Example 132, replacing 4-(3,4-difluorophenoxy)-2-fluoroaniline with the appropriate aniline:

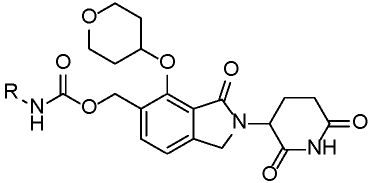

**TABLE 12**

| Ex. No. | R | LCMS [M+H]⁺ | RT (min) | HPLC Method |
|---|---|---|---|---|
| 133 | 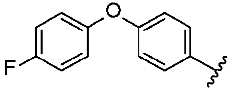 | 604.2 | 1.95 | C |
| 134 | 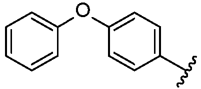 | 586.3 | 1.92 | C |
| 135 | 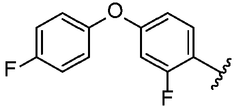 | 621.9 | 1.95 | D |
| 136 | 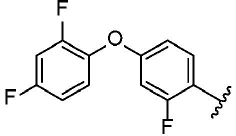 | 640.2 | 2.05 | D |

### EXAMPLE 137

### [4-(cyclohexyloxy)-2-(2,6-dioxopiperidin-3-yl)-3-oxo-2,3-dihydro-1H-isoindol-5-yl]methyl N-[4-(3,4-difluorophenoxy)phenyl]carbamate

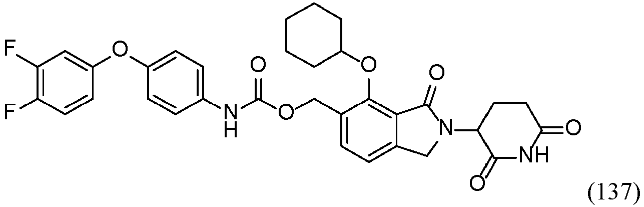

### Intermediate 137A: ethyl 3-bromo-2-(cyclohexyloxy)-6-methylbenzoate

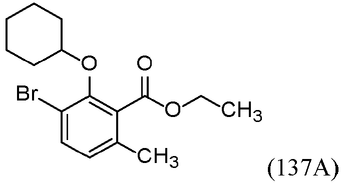

To a cooled (0 °C) solution of ethyl 3-bromo-2-hydroxy-6-methylbenzoate (10 g, 38.6 mmol), cyclohexanol (5.80 g, 57.9 mmol), and triphenylphosphine (20.25 g, 77 mmol) in THF (20 mL) was added diisopropyl azodicarboxylate (22.5 mL, 116 mmol) dropwise. The resulting mixture was stirred at 25 °C for 16 hours and concentrated. The crude material was purified by flash chromatography (SiO₂, 0-10% ethyl acetate/petroleum ether) to afford ethyl 3-bromo-2-(cyclohexyloxy)-6-methylbenzoate (11 g, 84% yield). ¹H NMR (400 MHz, DMSO-d₆) δ 7.58 (d, J=8.3 Hz, 1 H), 6.96 (dd, J=8.2, 0.7 Hz, 1 H), 4.31 (q, J=7.1 Hz, 2 H), 4.11 (tt, J=9.7, 3.9 Hz, 1 H), 2.19 (s, 3 H), 1.92-1.80 (m, 2 H), 1.71 (m, 2 H), 1.55-1.38 (m, 3 H), 1.30 (t, 3H), 1.26-1.14 (m, 3 H).

### Intermediate 137B: tert-butyl 5-amino-4-(6-bromo-7-(cyclohexyloxy)-1-oxoisoindolin-2-yl)-5-oxopentanoate

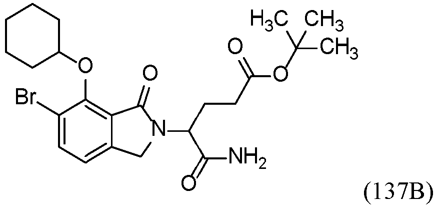

To a solution of ethyl 3-bromo-2-(cyclohexyloxy)-6-methylbenzoate (4 g, 11.72 mmol) and oxone (7.21 g, 11.72 mmol) in DCM (500 mL) and water (8.45 mL, 469 mmol) was added KBr (1.395 g, 11.72 mmol). The resulting mixture was stirred at 25 °C for 16 hours under LED light. The reaction was quenched with water and then extracted with DCM (2 x 50 mL). The combined organic layers were washed with brine, dried (Na₂SO₄), filtered, and concentrated to afford crude ethyl 3-bromo-6-(bromomethyl)-2-(cyclohexyloxy)benzoate (4.5 g).

To a solution of crude ethyl 3-bromo-6-(bromomethyl)-2-(cyclohexyloxy)benzoate (4.5 g) in DMF (10 mL) was added DIEA (5.6 mL, 32.1 mmol) and tert-butyl (S)-4,5-diamino-5-oxopentanoate (3.25 g, 16.07 mmol). The resulting mixture was stirred at 90 °C for 4 hours. The reaction was quenched with water and extracted with ethyl acetate (2x). The combined organic layers were washed with brine, dried (Na₂SO₄), filtered, and concentrated. The crude material was purified by flash chromatography (SiO₂, 5-100% ethyl acetate/petroleum ether) to afford tert-butyl 5-amino-4-(6-bromo-7-(cyclohexyloxy)-1-oxoisoindolin-2-yl)-5-oxopentanoate (4.4 g, 68% yield over two steps). The enantiomeric excess of this material and subsequent intermediates was not determined. LCMS (Method L): retention time 1.32 min, [M+H]⁺ 497.2.

### Intermediate 137C: tert-butyl 5-amino-4-(7-(cyclohexyloxy)-6-(hydroxymethyl)-1-oxoisoindolin-2-yl)-5-oxopentanoate

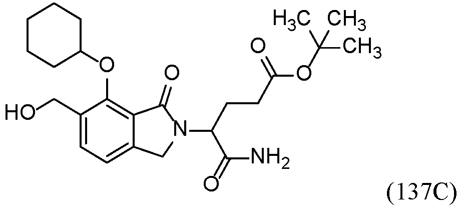

A solution of tert-butyl 5-amino-4-(6-bromo-7-(cyclohexyloxy)-1-oxoisoindolin-2-yl)-5-oxopentanoate (4.4 g, 8.88 mmol) and 1-(tributylstannyl)methanol (5.70 g, 17.76 mmol) in 1,4-dioxane (20 mL) was purged with nitrogen for 5 minutes. Tetrakis(triphenylphosphine)palladium (0.888 mg, 8.88 mmol) was added and the resulting mixture was stirred at 90 °C for 16 hours. The reaction was quenched with water. The reaction mixture was extracted with ethyl acetate (2x). The combined organic layers were washed with brine, dried (Na₂SO₄), filtered, and concentrated. The crude material was purified by flash chromatography (SiO₂, 5-100% ethyl acetate/petroleum ether) to afford tert-butyl 5-amino-4-(7-(cyclohexyloxy)-6-(hydroxymethyl)-1-oxoisoindolin-2-yl)-5-oxopentanoate (2.4 g, 4.92 mmol, 55% yield). LCMS (Method K): retention time 2.45 min, [M+H]⁺ 447.2.

### Intermediate 137D: tert-butyl 5-amino-4-(7-(cyclohexyloxy)-6-((((4-(3,4-difluorophenoxy)phenyl)carbamoyl)oxy)methyl)-1-oxoisoindolin-2-yl)-5-oxopentanoate

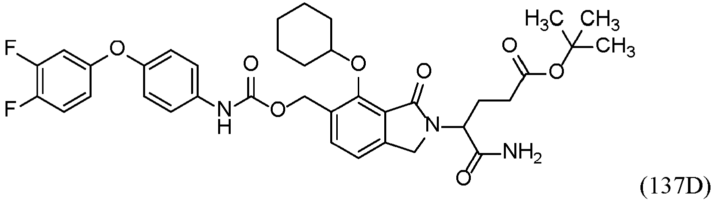

To a solution of tert-butyl 5-amino-4-(7-(cyclohexyloxy)-6-(hydroxymethyl)-1-oxoisoindolin-2-yl)-5-oxopentanoate (250 mg, 0.560 mmol) in DMF (1 mL) was added DIEA (0.293 mL, 1.680 mmol), followed by 4-nitrophenyl (4-(3,4-difluorophenoxy)phenyl) carbamate (433 mg, 1.120 mmol) in DMF (1 mL). The resulting mixture was stirred at 25 °C for 3 hours. The reaction was quenched with water. The reaction mixture was extracted with ethyl acetate (2x). The combined organic layers were washed with brine, dried (Na₂SO₄), filtered, and concentrated. The crude material was purified by flash chromatography (SiO₂, 5-100% ethyl acetate/petroleum ether) to afford tert-butyl 5-amino-4-(7-(cyclohexyloxy)-6-((((4-(3,4-difluorophenoxy)phenyl)carbamoyl)oxy)methyl)-1-oxoisoindolin-2-yl)-5-oxopentanoate (300 mg, 61% yield). LCMS (Method M): retention time 1.83 min, [M+H]⁺ 694.2.

### Example 137:

To a solution of tert-butyl 5-amino-4-(7-(cyclohexyloxy)-6-((((4-(3,4-difluorophenoxy)phenyl)carbamoyl)oxy)methyl)-1-oxoisoindolin-2-yl)-5-oxopentanoate (200 mg, 0.288 mmol) in 1,4-dioxane (0.5 mL) was added methanesulfonic acid (0.112 mL, 1.730 mmol). The resulting mixture was stirred at 70 °C for 1 hour. The reaction mixture was then filtered and concentrated. The crude material was purified via purified via preparative HPLC (Column: XBridge C18, 19 mm x 250 mm, 5 µm particles; Mobile Phase A: 5 mM ammonium formate, Mobile Phase B: acetonitrile; Temperature: 25 °C; Gradient: 60-90%B (0.0-7.0 min), 90%B (7.0-10.0 min); Flow: 15 mL/min). Fractions containing the product were combined and concentrated to afford (4-(cyclohexyloxy)-2-(2,6-dioxopiperidin-3-yl)-3-oxoisoindolin-5-yl)methyl (4-(3,4-difluorophenoxy)phenyl)carbamate (40 mg, 21. % yield). LCMS (Method P): retention time 2.71 min, [M-H]⁻ 618.2; ¹H NMR (400 MHz, DMSO-d₆) δ 10.97 (br s, 1H), 9.76 (br s, 1H), 7.65 (d, J=7.8 Hz, 1H), 7.47-7.53 (m, 2H), 7.37-7.46 (m, 1H), 7.29 (d, J=7.8 Hz, 1H), 7.09 (ddd, J=12.0, 6.8, 3.0 Hz, 1H), 7.02 (d, J=9.1 Hz, 2H), 6.77 (dtd, J=9.0, 3.3, 3.3, 1.8 Hz, 1H), 5.17-5.28 (m, 2H), 5.10 (dd, J=13.3, 5.1 Hz, 1H), 4.57-4.67 (m, 1H), 4.40-4.44 (m, 1H), 4.25-4.46 (m, 1H), 2.82-2.96 (m, 1H), 2.57-2.69 (m, 1H), 2.31-2.41 (m, 1H), 1.91-2.06 (m, 3H), 1.65-1.78 (m, 2H), 1.40-1.55 (m, 3H), 1.13-1.27 (m, 3H).

### EXAMPLES 138-140

The compounds in Table 13 were prepared according to the general procedures described for Example 137, replacing cyclohexanol in the first step with the appropriate alcohol, and replacing 4-nitrophenyl (4-(3,4-difluorophenoxy)phenyl)carbamate in the penultimate step with the appropriate 4-nitrophenyl carbamate (prepared in a manner similar to Intermediate I-7):

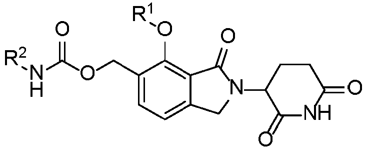

**TABLE 13**

| Ex. No. | R¹ | R² | LCMS [M+H]⁺ | RT (min) | HPLC Method |
|---|---|---|---|---|---|
| 138 | 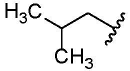 | 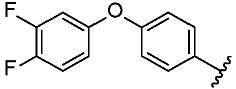 | 594.3 | 2.52 | K |
| 139 | 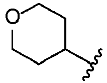 | 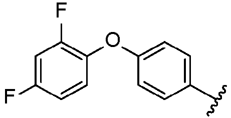 | 622.2 | 1.94 | C |
| 140 | 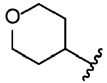 | 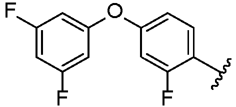 | 640.0 | 2.06 | D |

### EXAMPLE 141

### [2-(2,6-dioxopiperidin-3-yl)-4-[(3R)-oxan-3-yloxy]-3-oxo-2,3-dihydro-1H-isoindol-5-yl] methyl N-[4-(3,4-difluorophenoxy)phenyl]carbamate

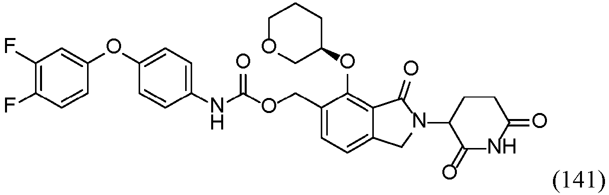

### Intermediate 141A: methyl (R)-3-bromo-6-methyl-2-((tetrahydro-2H-pyran-3-yl)oxy) benzoate

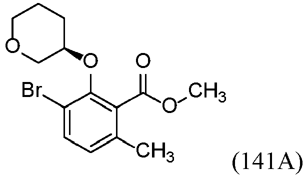

To a cooled (0 °C) mixture of methyl 3-bromo-2-hydroxy-6-methylbenzoate (2.44 g, 9.96 mmol) and (S)-tetrahydro-2H-pyran-3-ol (1.02 g, 9.96 mmol) in THF (30 mL) was added diethyl azodicarboxylate (40% in toluene, 4.7 mL, 12.0 mmol). The resulting mixture was stirred at room temperature for 2 h. The reaction mixture was diluted with ethyl acetate, washed with water, brine, dried (MgSO₄) and concentrated. The residue was purified by silica gel chromatography, eluting with 0 to 10% ethyl acetate in hexanes, to afford methyl (R)-3-bromo-6-methyl-2-((tetrahydro-2H-pyran-3-yl)oxy)benzoate (900 mg, 27% yield). LCMS (Method E): retention time 1.03 min, [M+H]⁺ 329.0; ¹H NMR (400 MHz, CHLOROFORM-d) δ 7.47 (d, J=8.2 Hz, 1H), 6.84 (dd, J=8.2, 0.7 Hz, 1H), 4.43-4.20 (m, 1H), 3.96 (dd, J=3.7, 1.7 Hz, 1H), 3.93 (s, 3H), 3.74 (dt, *J=11.1,* 4.5 Hz, 1H), 3.56-3.41 (m, 2H), 2.26 (s, 3H), 2.14-2.01 (m, 1H), 1.92-1.76 (m, 2H), 1.59-1.53 (m, 1H).

### Intermediate 141B: methyl (R)-3-bromo-6-(bromomethyl)-2-((tetrahydro-2H-pyran-3-yl) oxy)benzoate

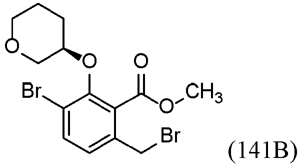

To a mixture of methyl (R)-3-bromo-6-methyl-2-((tetrahydro-2H-pyran-3-yl)oxy) benzoate (450 mg, 1.37 mmol), potassium bromide (211 mg, 1.78 mmol) and Oxone (1.09 g, 1.78 mmol) in DCM (4 mL) was added water (1.0 mL). The resulting mixture was stirred in a sealed tube under irradiation with a LED light at 5 cm distance for 2 h. The reaction was quenched with aqueous Na₂SO₃. The reaction mixture was diluted with ethyl acetate, washed with water, brine, dried (MgSO₄) and concentrated. The residue was purified by silica gel chromatography, eluting with 0 to 10% ethyl acetate in hexanes, to afford methyl (R)-3-bromo-6-(bromomethyl)-2-((tetrahydro-2H-pyran-3-yl)oxy)benzoate (400 mg, 72% yield). LCMS (Method E): retention time 1.05 min, [M+H]⁺ 408.8; ¹H NMR (400 MHz, CHLOROFORM-d) δ 7.56 (d, J=8.3 Hz, 1H), 7.04 (d, J=8.3 Hz, 1H), 4.55-4.31 (m, 2H), 4.27-4.16 (m, 1H), 3.95 (s, 3H), 3.91-3.85 (m, 1H), 3.74-3.64 (m, 1H), 3.55-3.39 (m, 2H), 2.11-1.95 (m, 1H), 1.87-1.72 (m, 2H), 1.62-1.47 (m, 1H).

### Intermediate 141C: 3-(6-bromo-1-oxo-7-(((R)-tetrahydro-2H-pyran-3-yl)oxy)isoindolin-2-yl) piperidine-2,6-dione

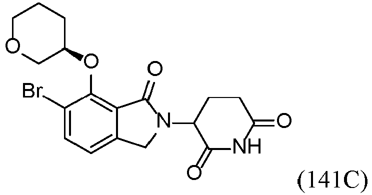

A mixture of ethyl (R)-3-bromo-6-(bromomethyl)-2-((tetrahydro-2H-pyran-3-yl)oxy) benzoate (400 mg, 0.948 mmol), 3-aminopiperidine-2,6-dione HCl salt (234 mg, 1.42 mmol) and DIEA (0.497 mL, 2.84 mmol) in acetonitrile (2 mL) was stirred at 70 °C for 15 h. The reaction mixture was allowed to cool to room temperature and concentrated. The residue was purified by silica gel chromatography, eluting with 0 to 10% MeOH in DCM to afford 3-(6-bromo-1-oxo-7-(((R)-tetrahydro-2H-pyran-3-yl)oxy)isoindolin-2-yl)piperidine-2,6-dione (350 mg, 84% yield). LCMS (Method E): retention time 0.87 min, [M+H]⁺ 423.9; ¹H NMR (400 MHz, METHANOL-d₄) δ 7.82 (d, J=8.0 Hz, 1H), 7.21 (d, J=8.0 Hz, 1H), 5.08 (dd, J=13.3, 4.9 Hz, 1H), 4.64 (dt, J=7.7, 3.9 Hz, 1H), 4.51-4.30 (m, 2H), 4.10 (q, J=7.1 Hz, 1H), 4.00-3.89 (m, 1H), 3.82-3.68 (m, 2H), 3.63-3.51 (m, 1H), 3.00-2.85 (m, 1H), 2.83-2.72 (m, 1H), 2.47 (ddd, J=13.0, 4.7, 2.0 Hz, 1H), 2.23-2.06 (m, 2H), 1.92-1.84 (m, 1H), 1.61-1.51 (m, 1H).

### Intermediate 141D: 3-(6-(hydroxymethyl)-1-oxo-7-(((R)-tetrahydro-2H-pyran-3-yl)oxy) isoindolin-2-yl)piperidine-2,6-dione

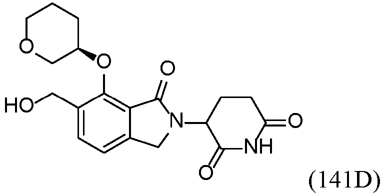

A mixture of 3-(6-bromo-1-oxo-7-(((R)-tetrahydro-2H-pyran-3-yl)oxy)isoindolin-2-yl)piperidine-2,6-dione (280 mg, 0.662 mmol), (tributylstannyl)methanol (255 mg, 0.794 mmol) and Pd(PPh₃)₄ (76 mg, 0.066 mmol) in 1,4-dioxane (4 mL) was stirred at 100 °C under nitrogen in a sealed tube for 15 h. The reaction mixture was allowed to cool to room temperature and purified by silica gel chromatography, eluting with 0 to 10% MeOH in DCM, to afford 3-(6-(hydroxymethyl)-1-oxo-7-(((R)-tetrahydro-2H-pyran-3-yl)oxy) isoindolin-2-yl)piperidine-2,6-dione (190 mg, 77% yield). LCMS (Method E): retention time 0.70 min, [M+H]⁺ 375.0; ¹H NMR (400 MHz, METHANOL-d₄) δ 7.70 (d, J=7.7 Hz, 1H), 7.28 (d, J=7.7 Hz, 1H), 5.08 (dd, J=13.3, 5.2 Hz, 1H), 4.82-4.77 (m, 1H), 4.74-4.65 (m, 1H), 4.48-4.37 (m, 2H), 3.86-3.57 (m, 4H), 2.98-2.71 (m, 2H), 2.55-2.37 (m, 1H), 2.22-2.11 (m, 1H), 2.01 (m, 4H), 1.61-1.47 (m, 1H).

### Example 141:

Phosgene (20% in toluene, 49.5 mg, 0.100 mmol) was added to 3-(6-(hydroxymethyl)-1-oxo-7-(((R)-tetrahydro-2H-pyran-3-yl)oxy)isoindolin-2-yl)piperidine-2,6-dione (25 mg, 0.067 mmol) in THF (1 mL). The resulting mixture was stirred at room temperature for 1 h and then concentrated in vacuo. The residue was dissolved in THF (1 mL), then 4-(3,4-difluorophenoxy)aniline (16.3 mg, 0.073 mmol) was added, followed by DIEA (0.026 mL, 0.147 mmol). The reaction mixture was stirred at room temperature for 1 h and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford [2-(2,6-dioxopiperidin-3-yl)-4-[(3R)-oxan-3-yloxy]-3-oxo-2,3-dihydro-1H-isoindol-5-yl]methyl N-[4-(3,4-difluorophenoxy)phenyl]carbamate (25.8 mg, 61% yield). LCMS (Method C): retention time 2.08 min, [M+H]⁺ 622.3; ¹H NMR (500 MHz, DMSO-d₆) δ 10.98 (s, 1H), 9.77 (br s, 1H), 7.66 (d, J=7.8 Hz, 1H), 7.49 (br d, J=8.6 Hz, 2H), 7.44-7.35 (m, 1H), 7.32 (d, J=7.7 Hz, 1H), 7.08 (ddd, *J=11.9,* 6.8, 2.9 Hz, 1H), 7.01 (d, J=8.9 Hz, 2H), 6.88-6.60 (m, 1H), 5.50-5.21 (m, 2H), 5.13-5.02 (m, 1H), 4.78-4.58 (m, 1H), 4.49-4.28 (m, 2H), 3.82-3.66 (m, 1H), 3.64-3.30 (m, 2H), 2.93-2.79 (m, 1H), 2.69-2.58 (m, 2H), 2.44-2.27 (m, 1H), 2.09-1.70 (m, 4H), 1.52-1.32 (m, 1H).

### EXAMPLES 142-150

The compounds in Table 14 were prepared according to the general procedures described for Example 141, replacing 4-(3,4-difluorophenoxy)aniline with the appropriate aniline:

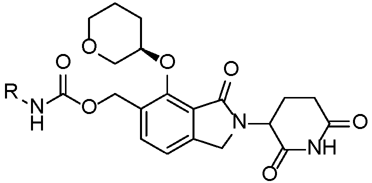

**TABLE 14**

| Ex. No. | R | LCMS [M+H]⁺ | RT (min) | HPLC Method |
|---|---|---|---|---|
| 142 | 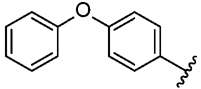 | 586.2 | 2.00 | C |
| 143 | 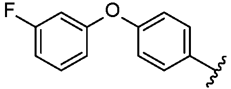 | 604.0 | 2.07 | D |
| 144 | 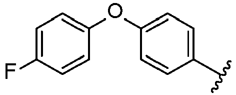 | 604.2 | 2.03 | C |
| 145 | 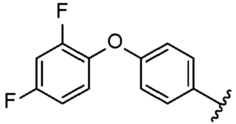 | 622.3 | 2.03 | D |
| 146 | 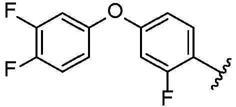 | 640.1 | 2.08 | C |
| 147 | 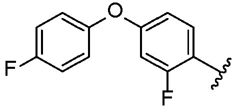 | 622.1 | 2.04 | C |
| 148 | 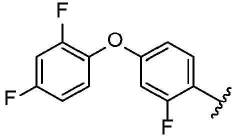 | 640.2 | 2.02 | D |
| 149 | 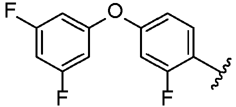 | 640.1 | 2.06 | C |
| 150 | 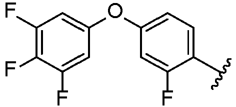 | 658.1 | 2.10 | C |

### EXAMPLE 151

### [4-(cyclopentyloxy)-2-(2,6-dioxopiperidin-3-yl)-3-oxo-2,3-dihydro-1H-isoindol-5-yl]methyl N-[4-(3,4-difluorophenoxy)phenyl]carbamate

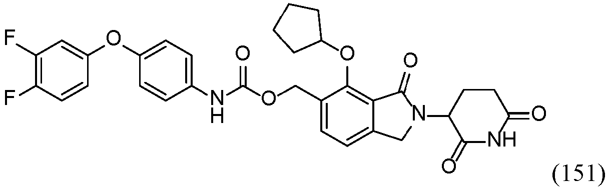

### Intermediate 151A: 3-(7-(cyclopentyloxy)-6-(hydroxymethyl)-1-oxoisoindolin-2-yl) piperidine-2,6-dione

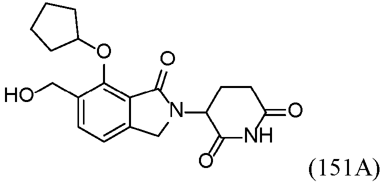

Prepared according to the general procedures described for Example 141, Intermediate 141D, replacing (S)-tetrahydro-2H-pyran-3-ol in the first step with cyclopentanol. LCMS (Method E): retention time 0.75 min, [M+H]⁺ 359.0; ¹H NMR (400 MHz, METHANOL-d₄) δ 7.70 (d, J=7.7 Hz, 1H), 7.24 (d, J=7.7 Hz, 1H), 5.35 (tt, *J=5.1,* 2.5 Hz, 1H), 5.08 (dd, J=13.3, 5.1 Hz, 1H), 4.70 (s, 2H), 4.48-4.30 (m, 2H), 2.97-2.71 (m, 2H), 2.47 (qd, J=13.2, 4.8 Hz, 1H), 2.20-2.11 (m, 1H), 1.96-1.81 (m, 4H), 1.79-1.68 (m, 2H), 1.68-1.55 (m, 2H).

### Example 151:

A mixture of 3-(7-(cyclopentyloxy)-6-(hydroxymethyl)-1-oxoisoindolin-2-yl) piperidine-2,6-dione (20 mg, 0.056 mmol), 4-(3,4-difluorophenoxy)benzoic acid (15.4 mg, 0.061 mmol), DIEA (0.013 mL, 0.073 mmol), and diphenyl phosphorazidate (18.4 mg, 0.067 mmol) in 1,4-dioxane (1 mL) was heated at 105 °C for 15 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford [4-(cyclopentyloxy)-2-(2,6-dioxopiperidin-3-yl)-3-oxo-2,3-dihydro-1H-isoindol-5-yl]methyl N-[4-(3,4-difluorophenoxy) phenyl]carbamate (11.2 mg, 33% yield). LCMS (Method C): retention time 2.27 min, [M+H]⁺ 606.0; ¹H NMR (500 MHz, DMSO-d₆) δ 9.75 (br s, 1H), 7.65 (d, J=7.7 Hz, 1H), 7.48 (br d, J=8.4 Hz, 2H), 7.42-7.33 (m, 1H), 7.29 (d, J=7.7 Hz, 1H), 7.17-7.05 (m, 1H), 7.00 (s, 1H), 6.82-6.66 (m, 1H), 5.40 (br s, 1H), 5.17 (s, 2H), 5.04 (dd, J=13.2, 5.0 Hz, 1H), 4.46-4.20 (m, 2H), 2.98-2.80 (m, 1H), 2.69-2.59 (m, 1H), 2.42-2.29 (m, 1H), 2.07-1.95 (m, 1H), 1.88-1.46 (m, 8H).

### EXAMPLE 152

### [2-(2,6-dioxopiperidin-3-yl)-3-oxo-4-[(3S)-oxolan-3-yloxy]-2,3-dihydro-1H-isoindol-5-yl]methyl N-[4-(3,4-difluorophenoxy)phenyl]carbamate

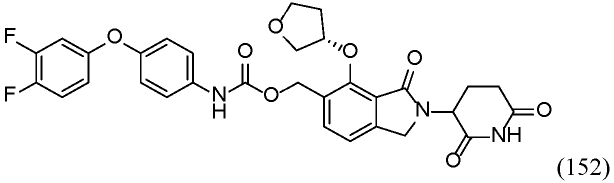

Prepared according to the general procedures described for Example 151, replacing cyclopentanol in the first step with (R)-tetrahydrofuran-3-ol. LCMS (Method C): retention time 2.02 min, [M+H]⁺ 608.2; ¹H NMR (500 MHz, DMSO-d₆) δ 10.99 (s, 1H), 9.77 (br s, 1H), 7.68 (d, J=7.7 Hz, 1H), 7.49 (br d, J=8.8 Hz, 2H), 7.44-7.39 (m, 1H), 7.37-7.30 (m, 1H), 7.26-7.18 (m, 1H), 7.01 (d, J=8.9 Hz, 2H), 6.82-6.70 (m, 1H), 5.81-5.50 (m, 1H), 5.31-5.15 (m, 2H), 5.06 (dd, J=13.3, 5.0 Hz, 1H), 4.48-4.27 (m, 2H), 4.02-3.65 (m, 4H), 2.92-2.80 (m, 1H), 2.61 (br d, J=19.1 Hz, 1H), 2.42-2.27 (m, 1H), 2.15-1.93 (m, 3H).

### EXAMPLE 153

### [2-(2,6-dioxopiperidin-3-yl)-3-oxo-4-[(3R)-oxolan-3-yloxy]-2,3-dihydro-1H-isoindol-5-yl] methyl N-[4-(3,4-difluorophenoxy)phenyl]carbamate

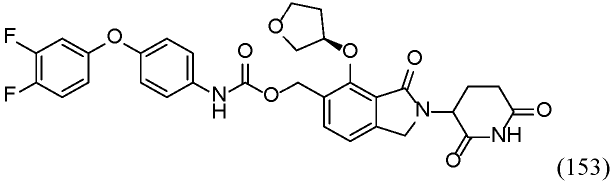

Prepared according to the general procedures described for Example 151, replacing cyclopentanol in the first step with (S)-tetrahydrofuran-3-ol. LCMS (Method D): retention time 1.95 min, [M+H]⁺ 608.3; ¹H NMR (500 MHz, DMSO-d₆) ¹H NMR (500 MHz, DMSO-d₆) δ 9.76 (br s, 1H), 7.67 (d, J=7.8 Hz, 1H), 7.48 (br d, J=8.7 Hz, 2H), 7.44-7.35 (m, 1H), 7.34 (d, J=7.7 Hz, 1H), 7.07 (ddd, *J=11.9,* 6.8, 3.0 Hz, 1H), 7.01 (d, J=9.0 Hz, 2H), 6.80-6.73 (m, 1H), 5.60-5.51 (m, 1H), 5.26-5.12 (m, 2H), 5.05 (dd, J=13.2, 5.1 Hz, 1H), 4.50-4.39 (m, 1H), 4.36-4.27 (m, 1H), 4.01-3.63 (m, 4H), 2.93-2.81 (m, 1H), 2.66-2.57 (m, 1H), 2.45-2.31 (m, 1H), 2.17-1.94 (m, 3H).

### EXAMPLES 154-158

The compounds in Table 15 were prepared according to the general procedures described for Example 141, replacing (S)-tetrahydro-2H-pyran-3-ol in the first step with (S)-tetrahydrofuran-3-ol, and replacing 4-(3,4-difluorophenoxy)aniline in the final step with the appropriate aniline:

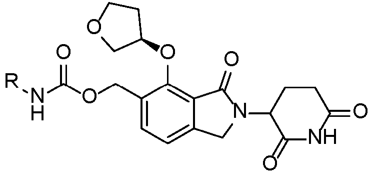

**TABLE 15**

| Ex. No. | R | LCMS [M+H]⁺ | RT (min) | HPLC Method |
|---|---|---|---|---|
| 154 | 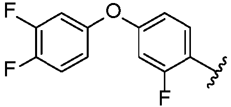 | 626.2 | 2.04 | D |
| 155 | 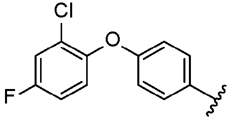 | 624.1 | 2.04 | C |
| 156 | 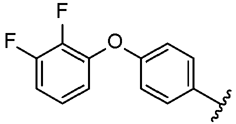 | 608.2 | 1.97 | D |
| 157 | 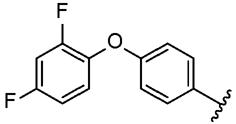 | 608.1 | 1.90 | C |
| 158 | 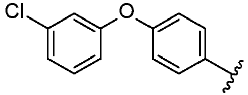 | 606.0 | 2.05 | C |

### EXAMPLE 159

### [2-(2,6-dioxopiperidin-3-yl)-4-(2-hydroxy-2-methylpropoxy)-3-oxo-2,3-dihydro-1H-isoindol-5-yl]methyl N-[4-(3,4-difluorophenoxy)phenyl]carbamate

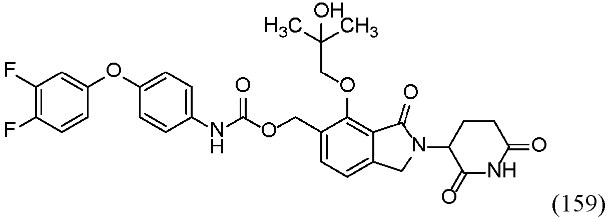

### Intermediate 159A: ethyl 3-bromo-2-(2-hydroxy-2-methylpropoxy)-6-methylbenzoate

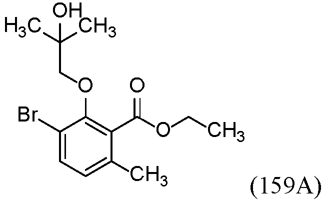

A mixture of ethyl 3-bromo-2-hydroxy-6-methylbenzoate (2.00 g, 7.72 mmol), 2,2-dimethyloxirane (0.612 g, 8.49 mmol) and potassium carbonate (2.67 g, 19.3 mmol) in DMF (10 mL) was stirred at 100 °C for 15 h. The reaction mixture was allowed to cool to room temperature, diluted with ethyl acetate, washed with water, brine, dried (MgSO₄) and concentrated. The residue was purified by silica gel chromatography, eluting with 0 to 20% ethyl acetate in hexanes to afford ethyl 3-bromo-2-(2-hydroxy-2-methylpropoxy)-6-methylbenzoate (1.60 g, 63% yield). LCMS (Method E): retention time 1.02 min, [M+H]⁺ 331.8; 333.9; ¹H NMR (400 MHz, CHLOROFORM-d) δ 7.47 (d, J=8.3 Hz, 1H), 6.93-6.80 (m, 1H), 4.49-4.35 (m, 2H), 3.92 (s, 2H), 2.28 (s, 3H), 1.40 (t, J=7.2 Hz, 3H), 1.32 (s, 6H).

### Example 159:

Prepared according to the general procedures described for Example 141, replacing (R)-3-bromo-6-methyl-2-((tetrahydro-2H-pyran-3-yl)oxy)benzoate (Intermediate 141A) in the second step with ethyl 3-bromo-2-(2-hydroxy-2-methylpropoxy)-6-methylbenzoate (Intermediate 159A). LCMS (Method D): retention time 2.05 min, [M+H]⁺ 610.2; ¹H NMR (500 MHz, DMSO-d₆) δ 9.75 (br s, 1H), 7.65 (br d, J=7.7 Hz, 1H), 7.47 (br d, J=8.2 Hz, 2H), 7.44-7.38 (m, 1H), 7.36-7.28 (m, 1H), 7.11-7.05 (m, 1H), 7.00 (br d, J=8.9 Hz, 2H), 6.76 (br d, J=9.1 Hz, 1H), 5.28 (s, 2H), 5.05 (br dd, J=13.1, 4.6 Hz, 1H), 4.81 (s, 1H), 4.50-4.23 (m, 2H), 4.16-3.95 (m, 2H), 2.91-2.78 (m, 1H), 2.64-2.58 (m, 1H), 2.45-2.27 (m, 1H), 2.00 (br dd, J=10.6, 4.9 Hz, 1H), 1.21 (s, 6H).

### EXAMPLES 160-165

The compounds in Table 16 were prepared according to the general procedures described for Example 159, replacing 4-(3,4-difluorophenoxy)aniline with the appropriate aniline:

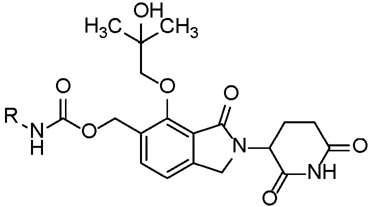

**TABLE 16**

| Ex. No. | R | LCMS [M+H]⁺ | RT (min) | HPLC Method |
|---|---|---|---|---|
| 160 | 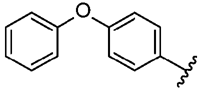 | 574.2 | 1.87 | C |
| 161 | 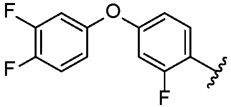 | 628.2 | 2.11 | D |
| 162 | 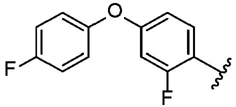 | 610.2 | 1.95 | D |
| 163 | 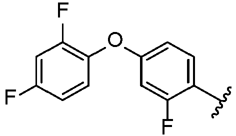 | 627.9 | 1.96 | C |
| 164 | 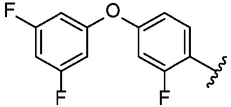 | 628.2 | 2.11 | D |
| 165 | 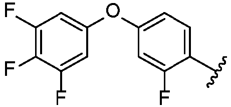 | 646.2 | 2.09 | C |

### INTERMEDIATE I-10

### (1S,4S)-4-((tert-butyldimethylsilyl)oxy)cyclohexan-1-ol

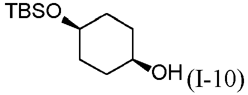

To a solution of (1S,4S)-cyclohexane-1,4-diol (500 mg, 4.30 mmol) in DMF (5 mL) was added imidazole (293 mg, 4.30 mmol). The resulting mixture was cooled to 0 °C, then tert-butyldimethylsilyl chloride (649 mg, 4.30 mmol) was added portion wise. The reaction mixture was stirred at 0 °C for 6 h. The reaction mixture was poured into ice-cold water (20 mL) and extracted with EtOAc (2 x 30 mL). The combined organic layers were washed with brine (20 mL), dried (Na₂SO₄), filtered, and concentrated. The crude material was purified by flash chromatography (SiO₂, 25-50% ethyl acetate/petroleum ether) to afford (1S,4S)-4-((tert-butyldimethylsilyl)oxy)cyclohexan-1-ol (410 mg, 41% yield) as a white semi-solid. ¹H NMR (DMSO-d₆, 400 MHz) δ 4.36 (br s, 1H), 3.77-3.73 (m, 1H), 3.47 (br s, 1H), 1.4-1.7 (m, 8H), 0.87 (s, 9H), 0.03 (s, 6H).

### INTERMEDIATE I-11

### (1R,4R)-4-((tert-butyldimethylsilyl)oxy)cyclohexan-1-ol

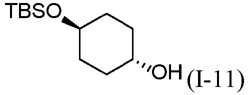

To a solution of (1R,4R)-cyclohexane-1,4-diol (500 mg, 4.30 mmol) in DMF (5 mL) was added imidazole (293 mg, 4.30 mmol). The resulting mixture was cooled to 0 °C, then tert-butyldimethylsilyl chloride (649 mg, 4.30 mmol) was added portion wise. The reaction mixture was stirred at 0 °C for 6 h. The reaction mixture was poured into ice-cold water (20 mL) and extracted with EtOAc (2 x 30 mL). The combined organic layers were washed with brine (20 mL), dried (Na₂SO₄), filtered, and concentrated. The crude material was purified by flash chromatography (SiO₂, 25-50% ethyl acetate/petroleum ether) to afford (1R,4R)-4-((tert-butyldimethylsilyl)oxy)cyclohexan-1-ol (400 mg, 40% yield) as a white semi-solid. ¹H NMR (400 MHz, DMSO-d₆) δ 4.43 (d, *J =* 4.1 Hz, 1H), 3.67-3.51 (m, 1H), 3.47-3.35 (m, 1H), 1.74 (td, *J* = 3.6, 8.9 Hz, 4H), 1.30-1.11 (m, 4H), 0.84 (s, 9H), 0.02 (s, 6H).

### EXAMPLE 166

### [2-(2,6-dioxopiperidin-3-yl)-3-oxo-4-{[(1R,4R)-4-hydroxycyclohexyl]oxy}-2,3-dihydro-1H-isoindol-5-yl]methyl N-[4-(3,4-difluorophenoxy)phenyl]carbamate

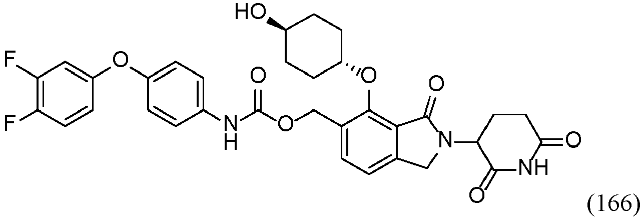

### Intermediate 166A: ethyl 3-bromo-6-methyl-2-(((trifluoromethyl)sulfonyl)oxy)benzoate

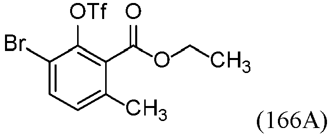

To a solution of ethyl 3-bromo-2-hydroxy-6-methylbenzoate (15 g, 57.9 mmol) in DCM (20 mL) was added DIEA (30.3 mL, 174 mmol). The mixture was cooled to -78 °C, then triflic anhydride (14.7 mL, 87.0 mmol) was added. The reaction mixture was stirred at -78 °C overnight. The mixture was then diluted with dichloromethane, washed with 1 N HCl (100 mL), 10% NaOH (100 mL), dried (MgSO₄), and concentrated to afford ethyl 3-bromo-6-methyl-2-(((trifluoromethyl)sulfonyl)oxy)benzoate (21 g, 93 % yield) as a dark yellow liquid. ¹H NMR (400 MHz, DMSO-d₆) δ 7.94 (d, J=8.3 Hz, 1H), 7.43 (dd, J=0.7, 8.3 Hz, 1H), 4.34 (q, J=7.1 Hz, 2H), 2.36 (s, 3H), 1.31 (t, J=7.1 Hz, 3H); ¹⁹F NMR (400 MHz, DMSO-d₆) δ -72.95.

### Intermediate 166B: tert-butyl 5-amino-4-(6-bromo-1-oxo-7-(((trifluoromethyl)sulfonyl)oxy) isoindolin-2-yl)-5-oxopentanoate

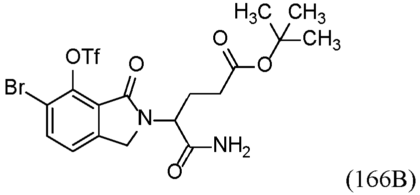

To a vial containing a colorless mixture of ethyl 3-bromo-6-methyl-2-(((trifluoromethyl)sulfonyl)oxy)benzoate (2.00 g, 5.11 mmol), potassium bromide (0.608 g, 5.11 mmol), and Oxone (3.14 g, 5.11 mmol) in DCM (10 mL) was added water (3.68 mL), resulting in the appearance of a dull orange color. The vial was immediately sealed with a Teflon-lined cap, and the reaction mixture was stirred vigorously at room temperature under irradiation with a 23W CFL lamp. After 18 h, the reaction mixture was quenched with solid sodium sulfite. Water was then added and the mixture was extracted with DCM (3x). The organic layers were combined, dried (Na₂SO₄), filtered, and concentrated in vacuo. A total of ten batches were combined to give 23.7 g of crude ethyl 3-bromo-6-(bromomethyl)-2-(((trifluoromethyl)sulfonyl)oxy)benzoate, which was carried into the next step without further purification.

To a solution of crude ethyl 3-bromo-6-(bromomethyl)-2-(((trifluoromethyl)sulfonyl) oxy)benzoate (23.7 g) in DMF (150 mL) were added tert-butyl (S)-4,5-diamino-5-oxopentanoate (10.20 g, 50.4 mmol) and DIEA (44.0 mL, 252 mmol). The resulting mixture was stirred at 85 °C for 3 h. The reaction mixture was diluted with ice cold water (100 mL) and EtOAc (100 mL), then the layers were separated, and the aqueous layer was back-extracted with EtOAc (100 mL). The combined organic layers were dried (Na₂SO₄) and concentrated. The crude material was purified by reversed phase chromatography to afford tert-butyl 5-amino-4-(6-bromo-1-oxo-7-(((trifluoromethyl)sulfonyl)oxy)isoindolin-2-yl)-5-oxopentanoate (12.9 g, 45% yield over two steps) as a pale brown solid. The enantiomeric excess of this material and subsequent intermediates was not determined. LCMS (Method K): retention time 2.97 min, [M+Na]⁺ 567.0, 569.0.

### Intermediate 166C: tert-butyl 5-amino-4-(6-bromo-7-hydroxy-1-oxoisoindolin-2-yl)-5-oxopentanoate

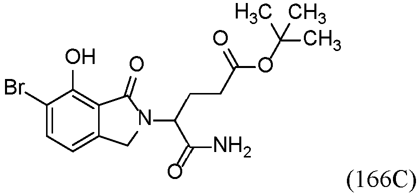

To a solution of tert-butyl 5-amino-4-(6-bromo-1-oxo-7-(((trifluoromethyl)sulfonyl) oxy)isoindolin-2-yl)-5-oxopentanoate (1.5g, 2.75 mmol) in THF (5 mL) was added tetramethylammonium fluoride (0.769 g, 8.25 mmol). The resulting mixture was stirred at room temperature for 16 h. The reaction mixture was concentrated and the crude product was purified by reversed phase chromatography (150 g RediSep C18 column, 30% acetonitrile/70% 5 mM ammonium formate in water, 60 mL/min). Fractions containing the product were combined and concentrated to afford tert-butyl 5-amino-4-(6-bromo-7-hydroxy-1-oxoisoindolin-2-yl)-5-oxopentanoate (800 mg, 63% yield) as a pale yellow solid. LCMS (Method Q): retention time 2.25 min, [M+H]⁺ 413.1, 415.1.

### Intermediate 166D: tert-butyl 5-amino-4-(6-bromo-7-(((1R,4R)-4-((tert-butyldimethylsilyl) oxy)cyclohexyl)oxy)-1-oxoisoindolin-2-yl)-5-oxopentanoate

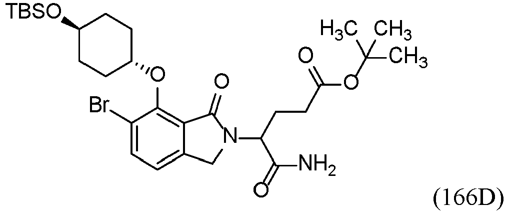

Diisopropyl azodicarboxylate (0.565 mL, 2.90 mmol) was added dropwise to a cooled (0 °C) suspension of tert-butyl 5-amino-4-(6-bromo-7-hydroxy-1-oxoisoindolin-2-yl)-5-oxopentanoate (400 mg, 0.968 mmol), (1S,4S)-4-((tert-butyldimethylsilyl)oxy)cyclohexan-1-ol (268 mg, 1.161 mmol), and triphenylphosphine (508 mg, 1.936 mmol) in THF (15 mL). The resulting mixture was stirred at 25 °C for 16 h. The reaction mixture was then evaporated to dryness under reduced pressure. The crude material was purified by flash chromatography (SiO₂, 30-50% ethyl acetate/petroleum ether) to afford tert-butyl 5-amino-4-(6-bromo-7-(((1R,4R)-4-((tert-butyldimethylsilyl)oxy)cyclohexyl)oxy)-1-oxoisoindolin-2-yl)-5-oxopentanoate (330 mg, 50% yield) as a white solid. LCMS (Method R): retention time 2.17 min, [M+H]⁺ 625.2, 627.1.

### Intermediate 166E: tert-butyl 5-amino-4-(7-(((1R,4R)-4-((tert-butyldimethylsilyl)oxy) cyclohexyl)oxy)-6-(hydroxymethyl)-1-oxoisoindolin-2-yl)-5-oxopentanoate

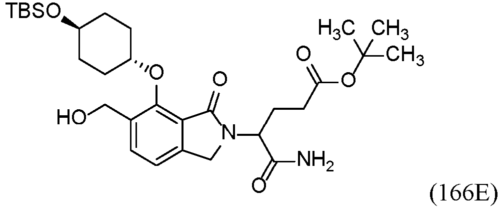

A solution of tert-butyl 5-amino-4-(6-bromo-7-(((1R,4R)-4-((tert-butyldimethylsilyl) oxy)cyclohexyl)oxy)-1-oxoisoindolin-2-yl)-5-oxopentanoate (200 mg, 0.320 mmol) and tetrakis(triphenylphosphine)palladium (44.3 mg, 0.038 mmol) in 1,4-dioxane (6 mL) was purged with nitrogen for 5 min. 1-(Tributylstannyl)methanol (308 mg, 0.959 mmol) was added and the resulting mixture was stirred at 100 °C for 16 h. The reaction mixture was then evaporated to dryness under reduced pressure. The crude material was purified by flash chromatography (SiO₂, 50-80% ethyl acetate/petroleum ether) to afford tert-butyl 5-amino-4-(7-(((1R,4R)-4-((tert-butyldimethylsilyl)oxy)cyclohexyl)oxy)-6-(hydroxymethyl)-1-oxoisoindolin-2-yl)-5-oxopentanoate (70 mg, ~76% purity by HPLC) as a pale yellow solid. LCMS (Method Q): retention time 3.49 min, [M+H]⁺ 577.2.

### Intermediate 166F: tert-butyl 5-amino-4-(7-(((1R,4R)-4-((tert-butyldimethylsilyl)oxy) cyclohexyl)oxy)-6-((((4-(3,4-difluorophenoxy)phenyl)carbamoyl)oxy)methyl)-1-oxoisoindolin-2-yl)-5-oxopentanoate

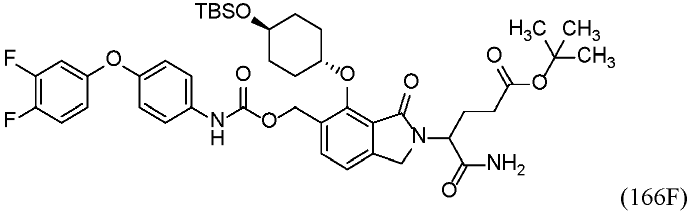

To a solution of tert-butyl 5-amino-4-(7-(((1r,4r)-4-((tert-butyldimethylsilyl)oxy) cyclohexyl)oxy)-6-(hydroxymethyl)-1-oxoisoindolin-2-yl)-5-oxopentanoate (70 mg, ~76% purity by HPLC) in DMF (1 mL) was added DIEA (0.064 mL, 0.364 mmol). The resulting mixture was stirred at room temperature for 10 min, then a solution of 4-nitrophenyl (4-(3,4-difluorophenoxy)phenyl)carbamate (70.3 mg, 0.182 mmol) in DMF (0.5 mL) was added. The reaction mixture was stirred at room temperature for 3 h. The mixture was then evaporated to dryness under reduced pressure. The crude material was purified by flash chromatography (SiO₂, 50-80% ethyl acetate/petroleum ether) to afford tert-butyl 5-amino-4-(7-(((1R,4R)-4-((tert-butyldimethylsilyl)oxy)cyclohexyl)oxy)-6-((((4-(3,4-difluorophenoxy)phenyl) carbamoyl)oxy)methyl)-1-oxoisoindolin-2-yl)-5-oxopentanoate as a pale yellow gum. LCMS (Method K): retention time 4.59 min, [M-H]⁻ 822.2.

### Example 166:

To a solution of tert-butyl 5-amino-4-(7-(((1R,4R)-4-((tert-butyldimethylsilyl)oxy) cyclohexyl)oxy)-6-((((4-(3,4-difluorophenoxy)phenyl)carbamoyl)oxy)methyl)-1-oxoisoindolin-2-yl)-5-oxopentanoate (40 mg, ~83% purity by HPLC) in 1,4-dioxane (1 mL) was added methanesulfonic acid (0.032 mL, 0.485 mmol). The reaction mixture was stirred at 80 °C for 2 h. The mixture was then purified by preparatory HPLC (Column: XBridge C18, 19 mm x 150 mm, 5 µm particles; Mobile Phase A: water with 5 mM ammonium formate, Mobile Phase B: acetonitrile; Temperature: 25 °C; Gradient: 20-60%B (0.0-15.0 min); Flow: 15 mL/min). Fractions containing the product were combined and lyophilized to dryness to afford [2-(2,6-dioxopiperidin-3-yl)-3-oxo-4-{[(1R,4R)-4-hydroxycyclohexyl] oxy}-2,3-dihydro-1H-isoindol-5-yl]methyl N-[4-(3,4-difluorophenoxy)phenyl]carbamate (2.0 mg, 6% yield) as an off-white solid. LCMS (Method K): retention time 2.72 min, [M-H]⁻ 634.2; ¹H NMR (400 MHz, DMSO-d₆) δ 10.98 (s, 1H), 9.77 (s, 1H), 7.66 (d, J=7.6 Hz, 1H), 7.50 (d, J=9.2 Hz, 2H), 7.38-7.46 (m, 1H), 7.30 (d, J=7.6 Hz, 1H), 7.10-7.13 (m, 1H), 7.03 (dd, J=2.0, 6.8 Hz, 2H), 6.77-6.80 (m, 1H), 5.22 (s, 2H), 5.12 (dd, J=5.2, 13.4 Hz, 1H), 4.61 (m, 1H), 4.50 (d, *J=*4.4 Hz, 1H), 4.43 (d, J=17.6 Hz, 1H), 4.30 (d, J=17.6 Hz, 1H), 3.46-3.47 (m, 1H), 2.86-2.89 (m, 1H), 2.59-2.63 (m, 1H), 2.36-2.41 (m, 1H), 1.99-2.03 (m, 3H), 1.82-1.79 (m, 1H), 1.47-1.54 (m, 2H), 1.43-1.48 (m, 2H), 1.24 (s, 1H).

### EXAMPLE 167

### [2-(2,6-dioxopiperidin-3-yl)-3-oxo-4-{[(1S,4S)-4-hydroxycyclohexyl]oxy}-2,3-dihydro-1H-isoindol-5-yl]methyl N-[4-(3,4-difluorophenoxy)phenyl]carbamate

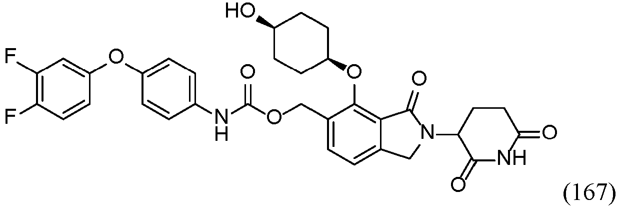

### Intermediate 167A: tert-butyl 5-amino-4-(6-bromo-7-(((1S,4S)-4-((tert-butyldimethylsilyl) oxy)cyclohexyl)oxy)-1-oxoisoindolin-2-yl)-5-oxopentanoate

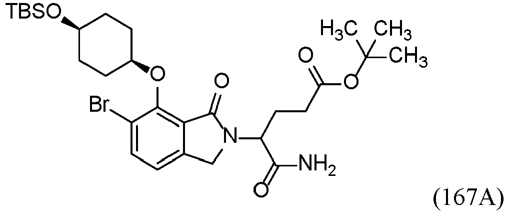

Diisopropyl azodicarboxylate (0.847 mL, 4.36 mmol) was added dropwise to a cooled (0 °C) suspension of tert-butyl 5-amino-4-(6-bromo-7-hydroxy-1-oxoisoindolin-2-yl)-5-oxopentanoate (600 mg, 1.45 mmol), (1r,4r)-4-((tert-butyldimethylsilyl)oxy)cyclohexan-1-ol (401 mg, 1.742 mmol), and triphenylphosphine (762 mg, 2.90 mmol) in THF (15 mL). The resulting mixture was stirred at 25 °C for 16 h. The reaction mixture was then evaporated to dryness under reduced pressure. The crude material was purified by flash chromatography (SiO₂, 30-50% ethyl acetate/petroleum ether) to afford tert-butyl 5-amino-4-(6-bromo-7-(((1S,4S)-4-((tert-butyldimethylsilyl)oxy)cyclohexyl)oxy)-1-oxoisoindolin-2-yl)-5-oxopentanoate (410 mg, ~61% purity by HPLC) as a white solid. LCMS (Method Q): retention time 4.40 min, [M+H]⁺ 625.3, 627.2.

### Intermediate 167B: tert-butyl 5-amino-4-(7-(((1S,4S)-4-((tert-butyldimethylsilyl)oxy) cyclohexyl)oxy)-6-(hydroxymethyl)-1-oxoisoindolin-2-yl)-5-oxopentanoate

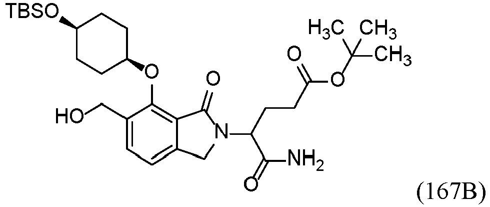

A solution of tert-butyl 5-amino-4-(6-bromo-7-(((1S,4S)-4-((tert-butyldimethylsilyl) oxy)cyclohexyl)oxy)-1-oxoisoindolin-2-yl)-5-oxopentanoate (410 mg, 0.655 mmol) and tetrakis(triphenylphosphine)palladium (91 mg, 0.079 mmol) in 1,4-dioxane (6 mL) was purged with nitrogen for 5 min. 1-(Tributylstannyl)methanol (631 mg, 1.966 mmol) was added and the resulting mixture was stirred at 100 °C for 16 h. The reaction mixture was then evaporated to dryness under reduced pressure. The crude material was purified by flash chromatography (SiO₂, 50-80% ethyl acetate/petroleum ether) to afford tert-butyl 5-amino-4-(7-(((1s,4s)-4-((tert-butyldimethylsilyl)oxy)cyclohexyl)oxy)-6-(hydroxymethyl)-1-oxoisoindolin-2-yl)-5-oxopentanoate (200 mg, ~65% purity by HPLC) as a pale yellow solid. LCMS (Method K): retention time 3.90 min, [M+H]⁺ 577.2.

### Intermediate 167C: tert-butyl 5-amino-4-(7-(((1S,4S)-4-((tert-butyldimethylsilyl)oxy) cyclohexyl)oxy)-6-((((4-(3,4-difluorophenoxy)phenyl)carbamoyl)oxy)methyl)-1-oxoisoindolin-2-yl)-5-oxopentanoate

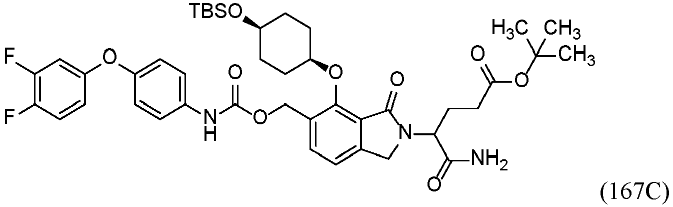

To a solution of tert-butyl 5-amino-4-(7-(((1s,4s)-4-((tert-butyldimethylsilyl)oxy) cyclohexyl)oxy)-6-(hydroxymethyl)-1-oxoisoindolin-2-yl)-5-oxopentanoate (200 mg, ~65% purity by HPLC) in DMF (2 mL) was added DIEA (0.182 mL, 1.040 mmol). The resulting mixture was stirred at room temperature for 10 min, then a solution of 4-nitrophenyl (4-(3,4-difluorophenoxy)phenyl)carbamate (201 mg, 0.520 mmol) in DMF (0.5 mL) was added. The reaction mixture was stirred at room temperature for 3 h. The mixture was then evaporated to dryness under reduced pressure. The crude material was purified by flash chromatography (SiO₂, 50-80% ethyl acetate/petroleum ether). The product was further purified by reversed phase chromatography (30 g RediSep C18 column, 100% acetonitrile, 30 mL/min) to afford tert-butyl 5-amino-4-(7-(((1S,4S)-4-((tert-butyldimethylsilyl)oxy)cyclohexyl)oxy)-6-((((4-(3,4-difluorophenoxy)phenyl)carbamoyl)oxy)methyl)-1-oxoisoindolin-2-yl)-5-oxopentanoate (80 mg, 26% yield) as a pale yellow gum. LCMS (Method R): retention time 2.56 min, [M-H]⁻ 822.4.

### Example 167:

To a solution of tert-butyl 5-amino-4-(7-(((1S,4S)-4-((tert-butyldimethylsilyl)oxy) cyclohexyl)oxy)-6-((((4-(3,4-difluorophenoxy)phenyl)carbamoyl)oxy)methyl)-1-oxoisoindolin-2-yl)-5-oxopentanoate (80 mg, 0.097 mmol) in 1,4-dioxane (2 mL) was added methanesulfonic acid (0.063 mL, 0.971 mmol). The reaction mixture was stirred at 80 °C for 2 h. The mixture was then purified by preparatory HPLC (Column: XBridge C18, 19 mm x 150 mm, 5 µm particles; Mobile Phase A: water with 5 mM ammonium formate, Mobile Phase B: acetonitrile; Temperature: 25 °C; Gradient: 20-60%B (0.0-15.0 min); Flow: 15 mL/min). Fractions containing the product were combined and lyophilized to dryness to afford [2-(2,6-dioxopiperidin-3-yl)-3-oxo-4-{[(1S,4S)-4-hydroxycyclohexyl]oxy}-2,3-dihydro-1H-isoindol-5-yl]methyl N-[4-(3,4-difluorophenoxy)phenyl]carbamate (3.8 mg, 6% yield) as an off-white solid. LCMS (Method K): retention time 2.71 min, [M-H]⁻ 634.2; ¹H NMR (400 MHz, DMSO-d₆) δ 10.98 (s, 1H), 9.77 (s, 1H), 7.66 (d, J=7.6 Hz, 1H), 7.50 (d, J=9.2 Hz, 2H), 7.38-7.46 (m, 1H), 7.30 (d, J=7.6 Hz, 1H), 7.10-7.13 (m, 1H), 7.03 (dd, J=2.0, 6.8 Hz, 2H), 6.77-6.80 (m, 1H), 5.22 (s, 2H), 5.12 (dd, J=5.2, 13.4 Hz, 1H), 4.61 (m, 1H), 4.50 (d, *J=4.4* Hz, 1H), 4.43 (d, J=17.6 Hz, 1H), 4.30 (d, J=17.6 Hz, 1H), 3.46-3.47 (m, 1H), 2.86-2.89 (m, 1H), 2.59-2.63 (m, 1H), 2.36-2.41 (m, 1H), 1.99-2.03 (m, 3H), 1.82-1.79 (m, 1H), 1.47-1.54 (m, 2H), 1.43-1.48 (m, 2H), 1.24 (s, 1H).

### EXAMPLE 168

### [2-(2,6-dioxopiperidin-3-yl)-4-(2-methoxyethoxy)-3-oxo-2,3-dihydro-1H-isoindol-5-yl] methyl N-[4-(3,4-difluorophenoxy)phenyl]carbamate

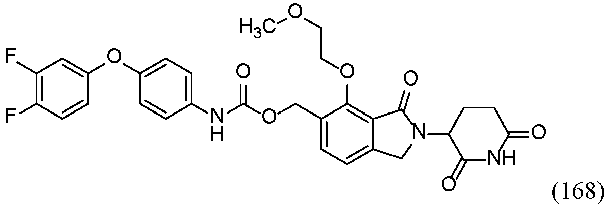

### Intermediate 168A: ethyl 3-bromo-2-(2-methoxyethoxy)-6-methylbenzoate

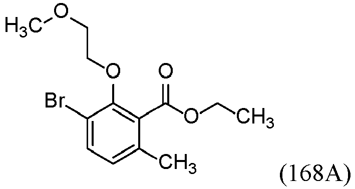

A mixture of ethyl 3-bromo-2-hydroxy-6-methylbenzoate (1.30 g, 5.02 mmol), 1-bromo-2-methoxyethane (0.767 g, 5.52 mmol), and potassium carbonate (1.73 g, 12.5 mmol) in acetone (10 mL) was stirred at 80 °C for 2 h. The reaction mixture was allowed to cool to room temperature, diluted with ethyl acetate, washed with water, brine, dried (MgSO₄), and concentrated. The residue was purified by silica gel chromatography, eluting with 0 to 20% ethyl acetate in hexanes to afford ethyl 3-bromo-2-(2-methoxyethoxy)-6-methylbenzoate (1.15 g, 72% yield). LCMS (Method E): retention time 1.02 min, [M+H]⁺ 316.8; 318.9; ¹H NMR (400 MHz, CHLOROFORM-d) δ 7.46 (d, J=8.2 Hz, 1H), 6.86 (d, J=8.2 Hz, 1H), 4.50-4.33 (m, 2H), 4.26-4.11 (m, 2H), 3.80-3.69 (m, 2H), 3.45 (s, 3H), 2.28 (s, 3H), 1.42-1.35 (m, 3H).

### Intermediate 168B: 3-(6-(hydroxymethyl)-7-(2-methoxyethoxy)-1-oxoisoindolin-2-yl) piperidine-2,6-dione

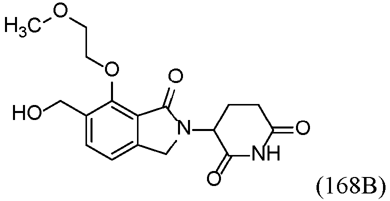

Prepared according to the general procedures described for Example 141, Intermediate 141D, replacing (R)-3-bromo-6-methyl-2-((tetrahydro-2H-pyran-3-yl)oxy) benzoate (Intermediate 141A) in the second step with ethyl 3-bromo-2-(2-methoxyethoxy)-6-methylbenzoate (Intermediate 168A). LCMS (Method E): retention time 0.67 min, [M+H]⁺ 349.0; ¹H NMR (400 MHz, METHANOL-d₄) δ 7.66 (d, J=7.7 Hz, 1H), 7.26 (d, J=7.6 Hz, 1H), 5.13-5.01 (m, 1H), 4.74 (s, 2H), 4.52-4.44 (m, 2H), 4.43 (d, J=6.9 Hz, 2H), 3.75-3.69 (m, 2H), 3.41 (s, 3H), 2.97-2.87 (m, 1H), 2.80-2.71 (m, 1H), 2.51-2.38 (m, 1H), 2.18-2.08 (m, 1H).

### Example 168:

A mixture of 3-(6-(hydroxymethyl)-7-(2-methoxyethoxy)-1-oxoisoindolin-2-yl) piperidine-2,6-dione (20 mg, 0.057 mmol), 4-(3,4-difluorophenoxy)benzoic acid (15.8 mg, 0.063 mmol), DIEA (0.013 mL, 0.075 mmol), and diphenyl phosphorazidate (19.0 mg, 0.069 mmol) in 1,4-dioxane (1 mL) was heated at 105 °C for 15 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford [2-(2,6-dioxopiperidin-3-yl)-4-(2-methoxyethoxy)-3-oxo-2,3-dihydro-1H-isoindol-5-yl]methyl N-[4-(3,4-difluorophenoxy) phenyl]carbamate (20 mg, 56% yield). LCMS (Method D): retention time 1.98 min, [M+H]⁺ 596.2; ¹H NMR (500 MHz, DMSO-d₆) δ 10.98 (s, 1H), 9.76 (br s, 1H), 7.65 (d, J=7.8 Hz, 1H), 7.49 (br s, 1H), 7.42-7.31 (m, 2H), 7.27-7.15 (m, 2H), 7.07 (ddd, *J=12.0,* 6.8, 2.9 Hz, 1H), 7.02-6.93 (m, 2H), 6.82-6.70 (m, 1H), 5.25 (s, 2H), 5.04 (br dd, *J=13.1,* 4.9 Hz, 1H), 4.57-4.27 (m, 4H), 3.92 (d, *J=11.6* Hz, 1H), 3.61 (br t, *J=4.5* Hz, 1H), 3.27 (s, 3H), 2.97-2.83 (m, 1H), 2.61 (br d, J=16.0 Hz, 1H), 2.41-2.31 (m, 1H), 2.06-1.94 (m, 1H).

### EXAMPLE 169

### [2-(2,6-dioxopiperidin-3-yl)-4-(2-methoxyethoxy)-3-oxo-2,3-dihydro-1H-isoindol-5-yl] methyl N-[4-(3,4-difluorophenoxy)-2-fluorophenyl]carbamate

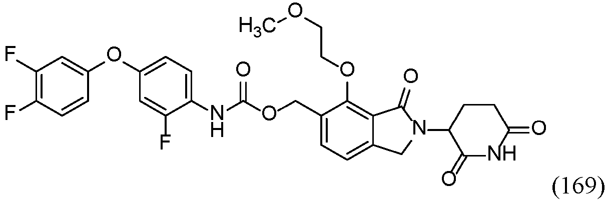

Prepared according to the general procedures described for Example 168, replacing 4-(3,4-difluorophenoxy)benzoic acid with 4-(3,4-difluorophenoxy)-2-fluorobenzoic acid. LCMS (Method D): retention time 2.01 min, [M+H]⁺ 613.9; ¹H NMR (500 MHz, DMSO-d₆) δ 10.98 (s, 1H), 9.38 (br d, *J=1.1* Hz, 1H), 7.65 (br d, J=7.6 Hz, 1H), 7.61-7.54 (m, 1H), 7.49-7.42 (m, 1H), 7.32 (d, J=7.7 Hz, 1H), 7.24 (ddd, *J=11.7,* 6.9, 2.9 Hz, 1H), 7.01 (dd, *J=11.5,* 2.6 Hz, 1H), 6.94-6.80 (m, 2H), 5.25 (s, 2H), 5.05 (dd, J=13.3, 5.0 Hz, 1H), 4.59-4.24 (m, 4H), 3.61 (t, J=4.6 Hz, 1H), 3.27 (s, 3H), 3.00-2.82 (m, 2H), 2.60 (br d, J=18.2 Hz, 1H), 2.43-2.32 (m, 1H), 2.07-1.94 (m, 1H).

### EXAMPLE 170

### [2-(2,6-dioxopiperidin-3-yl)-4-(oxepan-4-yloxy)-3-oxo-2,3-dihydro-1H-isoindol-5-yl]methyl N-[4-(3,4-difluorophenoxy)phenyl]carbamate

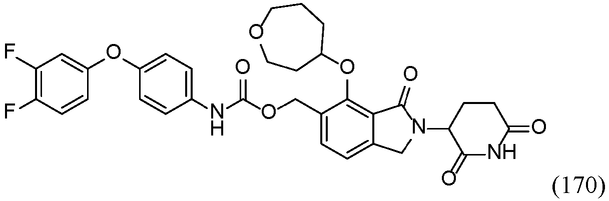

Prepared according to the general procedures described for Example 141, replacing (S)-tetrahydro-2H-pyran-3-ol with racemic oxepan-4-ol. LCMS (Method D): retention time 2.14 min, [M+H]⁺ 636.2; ¹H NMR (500 MHz, DMSO-d6) δ 10.96 (s, 1H), 9.73 (br s, 1H), 7.65 (d, J=7.6 Hz, 1H), 7.47 (br d, J=8.2 Hz, 2H), 7.41-7.33 (m, 1H), 7.30 (br d, J=7.9 Hz, 1H), 7.12-6.93 (m, 3H), 6.80-6.64 (m, 1H), 5.20 (s, 2H), 5.05 (dd, J=13.3, 4.9 Hz, 1H), 4.92-4.79 (m, 1H), 4.47-4.21 (m, 2H), 3.73-3.40 (m, 3H), 2.90-2.80 (m, 1H), 2.69-2.59 (m, 1H), 2.42-2.28 (m, 1H), 2.22-2.10 (m, 1H), 2.07-1.68 (m, 6H), 1.57-1.38 (m, 1H).

### EXAMPLE 171

### [2-(2,6-dioxopiperidin-3-yl)-3-oxo-4-(pyridin-3-yloxy)-2,3-dihydro-1H-isoindol-5-yl]methyl N-(4-phenoxyphenyl)carbamate

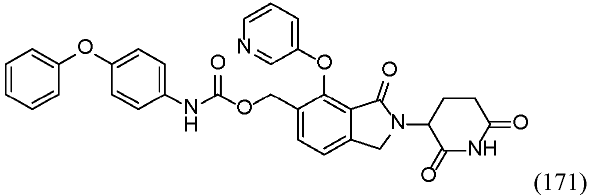

### Intermediate 171A: 3-bromo-2-fluoro-6-methylbenzoic acid

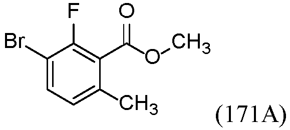

To a solution of 3-bromo-2-fluorobenzoic acid (7.0 g, 32.0 mmol) in HFIP (350 mL) was added potassium carbonate (14 g, 101 mmol), silver carbonate (14 g, 50.8 mmol), pentamethylcyclopentadienyliridium(III) chloride dimer (2.1 g, 3.94 mmol), and potassium methyltrifluoroborate (14 g, 115 mmol) at room temperature. The resulting mixture was stirred at 100 °C for 12 h. The reaction mixture was diluted with methanol, filtered, and the filtrate was concentrated under reduced pressure to afford crude 3-bromo-2-fluoro-6-methylbenzoic acid (7.0 g).

To a cooled (0 °C) solution of crude 3-bromo-2-fluoro-6-methylbenzoic acid (7.0 g) in DMF (50 mL) was added potassium carbonate (4.15 g, 30.0 mmol) and iodomethane (1.9 mL, 30.0 mmol). The resulting mixture was stirred at room temperature for 12 h. The reaction was quenched with cold water (100 mL). The reaction mixture was extracted with ethyl acetate (3 x 90 mL). The organic layer was washed with brine (90 mL), dried (Na₂SO₄), filtered, and concentrated. The crude material was purified by flash chromatography (SiO₂, 10% ethyl acetate/petroleum ether) to afford methyl 3-bromo-2-fluoro-6-methylbenzoate (6.0 g, 76% yield over two steps). ¹H NMR (400 MHz, CDCl₃) δ 7.50 (dd, J=7.0, 8.0 Hz, 1 H), 6.90 (d, J=8.0 Hz, 1 H), 3.96 (s, 3 H), 2.35 (s, 3 H).

### Intermediate 171B: tert-butyl 5-amino-4-(6-bromo-7-fluoro-1-oxoisoindolin-2-yl)-5-oxopentanoate

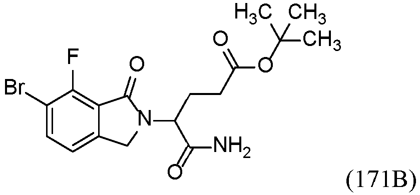

To a solution of methyl 3-bromo-2-fluoro-6-methylbenzoate (5.0 g, 20.24 mmol) in DCE (50 mL) was added NBS (4.32 g, 24.29 mmol) and AIBN (0.332 g, 2.024 mmol. The resulting mixture was stirred at 85 °C for 2 h. The reaction mixture was then concentrated. The residue was treated with water (50 mL) and extracted with dichloromethane (2 x 30 mL). The combined organic layers were washed with brine (50 mL), dried (Na₂SO₄), filtered, and concentrated to afford crude methyl 3-bromo-6-(bromomethyl)-2-fluorobenzoate (6.0 g).

To a solution of crude methyl 3-bromo-6-(bromomethyl)-2-fluorobenzoate (6.0) in DMF (150 mL) was added tert-butyl (S)-4,5-diamino-5-oxopentanoate hydrochloride salt (4.22 g, 17.67 mmol) and DIEA (6.2 mL, 35.3 mmol). The resulting mixture was stirred at 100 °C for 12 h. The reaction was quenched with cold water (100 mL). The reaction mixture was extracted with ethyl acetate (3 x 100 mL). The organic layer was washed with brine (100 mL), dried (Na₂SO₄), filtered, and concentrated. The crude product was purified by flash chromatography (SiO₂, 0-50% EtOAc/petroleum ether) to afford tert-butyl 5-amino-4-(6-bromo-7-fluoro-1-oxoisoindolin-2-yl)-5-oxopentanoate (4.0 g, 43% yield over two steps). The enantiomeric excess of this material and subsequent intermediates was not determined. ¹H NMR (400 MHz, DMSO-d₆) δ 7.91 (dd, *J*=8.3, 6.3 Hz, 1 H), 7.58 (bs, 1 H), 7.41 (d, *J*=8.0 Hz, 1 H), 7.21 (bs, 1 H), 4.70-4.67 (m, 1 H), 4.61-4.11 (m, 2 H), 2.21-2.10 (m, 3 H), 2.03-1.90 (m, 1 H), 1.33 (s, 9 H).

### Intermediate 171C: tert-butyl 5-amino-4-(6-bromo-1-oxo-7-(pyridin-3-yloxy)isoindolin-2-yl)-5-oxopentanoate

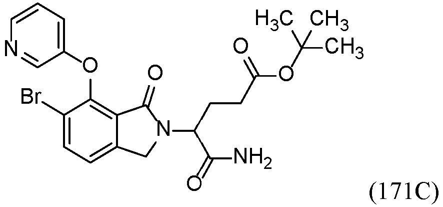

To a solution of tert-butyl 5-amino-4-(6-bromo-7-fluoro-1-oxoisoindolin-2-yl)-5-oxopentanoate (2.0 g, 4.33 mmol) in acetonitrile (5 mL) was added potassium carbonate (1.198 g, 8.67 mmol) and pyridin-3-ol (1.237 g, 13.00 mmol). The resulting mixture was stirred at 100 °C for 12 h. The reaction mixture was passed through a pad of Celite, washed with ethyl acetate (150 mL), and the filtrate was concentrated. The crude product was purified by flash chromatography (SiO₂, 0-100% EtOAc/petroleum ether) to afford tert-butyl 5-amino-4-(6-bromo-1-oxo-7-(pyridin-3-yloxy)isoindolin-2-yl)-5-oxopentanoate (1.0 g, 45% yield). LCMS (Method M): retention time 1.24 min, [M+H]⁺ 490.2, 492.0.

### Intermediate 171D: tert-butyl 5-amino-4-(6-(hydroxymethyl)-1-oxo-7-(pyridin-3-yloxy) isoindolin-2-yl)-5-oxopentanoate

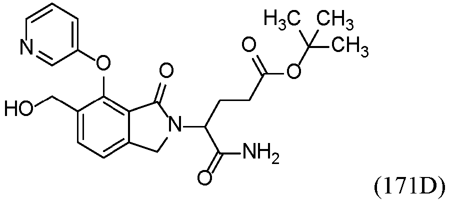

To a solution of tert-butyl 5-amino-4-(6-bromo-1-oxo-7-(pyridin-3-yloxy)isoindolin-2-yl)-5-oxopentanoate (1.0 g, 1.958 mmol) in 1,4-dioxane (10 mL) was added Pd(PPh₃)₄ (0.113 g, 0.098 mmol) and 1-(tributylstannyl)methanol (0.629 g, 1.958 mmol). The resulting mixture was purged with nitrogen for 5 min, then it was stirred at 100 °C for 12 h. The reaction mixture was then concentrated. The crude material was purified by reversed phase chromatography (150 g RediSep C18 column, 45% acetonitrile/5 mM ammonium formate in water, 40 mL/min) to afford tert-butyl 5-amino-4-(6-(hydroxymethyl)-1-oxo-7-(pyridin-3-yloxy)isoindolin-2-yl)-5-oxopentanoate (350 mg, 38% yield). LCMS (Method S): retention time 1.69 min, [M+H]⁺ 442.2; ¹H NMR (400 MHz, DMSO-d₆) δ 8.20-8.21 (m, 2H), 7.81 (d, J=7.6 Hz, 1H), 7.51-7.55 (m, 2H), 7.26-7.30 (m, 1H), 7.10-7.14 (m, 2H), 5.27-5.29 (m, 1H), 4.44-4.62 (m, 5H), 2.07-2.14 (m, 3H), 1.91-1.96 (m, 1H), 1.32 (s, 9H).

### Example 171:

To a solution of tert-butyl 5-amino-4-(6-(hydroxymethyl)-1-oxo-7-(pyridin-3-yloxy) isoindolin-2-yl)-5-oxopentanoate (100 mg, 0.227 mmol) in tetrahydrofuran (10 mL), was added potassium carbonate (62.6 mg, 0.453 mmol) and 4-nitrophenyl (4-phenoxyphenyl) carbamate (238 mg, 0.680 mmol). The resulting mixture was stirred at room temperature for 12 h. The reaction mixture was passed through a pad of Celite, washed with methanol, and concentrated. The crude material was purified by reversed phase chromatography to afford tert-butyl 5-amino-5-oxo-4-(1-oxo-6-((((4-phenoxyphenyl)carbamoyl)oxy)methyl)-7-(pyridin-3-yloxy)isoindolin-2-yl)pentanoate in low purity (60 mg). This material was carried into the next step without further purification.

To a solution of partially purified tert-butyl 5-amino-5-oxo-4-(1-oxo-6-((((4-phenoxyphenyl)carbamoyl)oxy)methyl)-7-(pyridin-3-yloxy)isoindolin-2-yl)pentanoate (60 mg) in 1,4-dioxane (5 mL) was added methanesulfonic acid (0.96 µL, 0.015 mmol). The resulting mixture was stirred at 80 °C for 2 h. The reaction mixture was then concentrated in vacuo. The crude material was purified by preparative HPLC to afford (2-(2,6-dioxopiperidin-3-yl)-3-oxo-4-(pyridin-3-yloxy)isoindolin-5-yl)methyl (4-phenoxyphenyl) carbamate (6.50 mg, 4% yield over two steps). LCMS (Method S): retention time 2.20 min, [M+H]⁺ 579.2; ¹H NMR (400 MHz, DMSO-d₆) δ 10.95 (s, 1H), 9.69 (br s, 1H), 8.29 (d, J=2.8 Hz, 1H), 8.25 (d, J=3.8 Hz, 1H), 7.87 (d, J=7.8 Hz, 1H), 7.60 (d, J=7.8 Hz, 1H), 7.44-7.30 (m, 5H), 7.27-7.21 (m, 1H), 7.10-7.06 (m, 1H), 6.97-6.92 (m, 4H), 5.20 (s, 2H), 4.94 (dd, *J=5.1,* 13.3 Hz, 1H), 4.53-4.31 (m, 2H), 2.90-2.75 (m, 1H), 2.60-2.53 (m, 1H) 2.36-2.26 (m, 1H), 2.00-1.88 (m, 1H).

### EXAMPLE 172

### [2-(2,6-dioxopiperidin-3-yl)-3-oxo-4-(pyridin-3-yloxy)-2,3-dihydro-1H-isoindol-5-yl]methyl N-[4-(3,4-difluorophenoxy)phenyl]carbamate

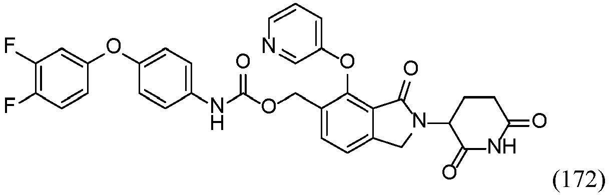

### Intermediate 172A: tert-butyl 5-amino-4-(6-((((4-(3,4-difluorophenoxy)phenyl)carbamoyl) oxy)methyl)-1-oxo-7-(pyridin-3-yloxy)isoindolin-2-yl)-5-oxopentanoate

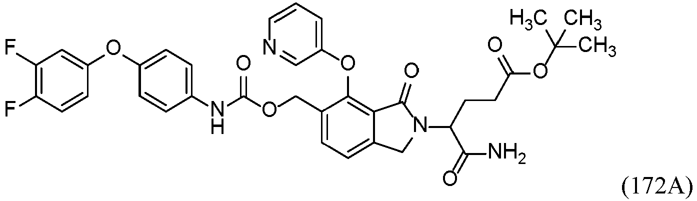

A mixture of tert-butyl 5-amino-4-(6-(hydroxymethyl)-1-oxo-7-(pyridin-3-yloxy) isoindolin-2-yl)-5-oxopentanoate (60 mg, 0.136 mmol), 4-nitrophenyl (4-(3,4-difluorophenoxy)phenyl)carbamate (68.3 mg, 0.177 mmol), and potassium carbonate (56.3 mg, 0.408 mmol) in acetone (3 mL) was stirred at 70 °C for 2 h. The reaction mixture was allowed to cool to room temperature and filtered. The filtrate was concentrated and the resulting residue was purified by silica gel chromatography, eluting with 0 to 10% MeOH in DCM, to afford tert-butyl 5-amino-4-(6-((((4-(3,4-difluorophenoxy)phenyl)carbamoyl)oxy) methyl)-1-oxo-7-(pyridin-3-yloxy)isoindolin-2-yl)-5-oxopentanoate (60 mg, 64% yield). LCMS (Method E): retention time 0.96 min, [M+H]⁺ 689.1; ¹H NMR (400 MHz, CHLOROFORM-d) δ 8.36-8.20 (m, 2H), 7.79 (d, J=7.7 Hz, 1H), 7.41 (d, J=7.7 Hz, 1H), 7.26-7.18 (m, 2H), 7.09 (q, J=9.0 Hz, 1H), 6.97-6.90 (m, 2H), 6.78 (ddd, *J=11.3,* 6.6, 2.9 Hz, 1H), 6.68 (dtd, J=8.9, 3.2, 1.8 Hz, 1H), 6.23 (br s, 1H), 5.32 (br s, 2H), 4.78-4.70 (m, 1H), 4.65-4.40 (m, 2H), 2.39-2.12 (m, 4H), 1.38 (s, 9H).

### Example 172:

A mixture of tert-butyl 5-amino-4-(6-((((4-(3,4-difluorophenoxy)phenyl)carbamoyl) oxy)methyl)-1-oxo-7-(pyridin-3-yloxy)isoindolin-2-yl)-5-oxopentanoate (60 mg, 0.087 mmol) and methanesulfonic acid (0.023 mL, 0.348 mmol) in 1,4-dioxane (2 mL) was stirred at 70 °C for 2 h. The reaction mixture was allowed to cool to room temperature and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford [2-(2,6-dioxopiperidin-3-yl)-3-oxo-4-(pyridin-3-yloxy)-2,3-dihydro-1H-isoindol-5-yl]methyl N-[4-(3,4-difluorophenoxy)phenyl]carbamate (31 mg, 55% yield). LCMS (Method D): retention time 1.71 min, [M+H]⁺ 615.2; ¹H NMR (500 MHz, DMSO-d₆) δ 9.69 (br s, 1H), 8.25-8.12 (m, 2H), 7.85 (br d, *J*=7.9 Hz, 1H), 7.58 (br d, *J*=7.8 Hz, 1H), 7.47-7.32 (m, 3H), 7.28 (br dd, *J*=8.0, 4.4 Hz, 1H), 7.14 (br d, *J*=8.0 Hz, 1H), 7.06 (ddd, *J*=11.6*,* 6.6, 2.4 Hz, 1H), 6.98 (br d, *J*=8.6 Hz, 2H), 6.75 (br d, *J*=9.5 Hz, 1H), 5.18 (s, 2H), 4.91 (br dd, *J*=12.9, 4.5 Hz, 1H), 4.53-4.42 (m, 1H), 4.41-4.29 (m, 1H), 2.87-2.72 (m, 1H), 2.60-2.56 (m, 1H), 2.38-2.23 (m, 1H), 2.00-1.88 (m, 1H).

### EXAMPLE 173

### {2-[(3S)-2,6-dioxopiperidin-3-yl]-3-oxo-4-(3-phenylpropoxy)-2,3-dihydro-1H-isoindol-5-yl} methyl N-[4-(3,4-difluorophenoxy)phenyl]carbamate

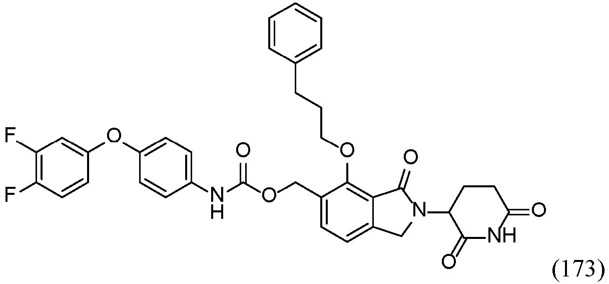

### Intermediate 173A: tert-butyl 5-amino-4-(7-hydroxy-1-oxo-6-vinylisoindolin-2-yl)-5-oxopentanoate

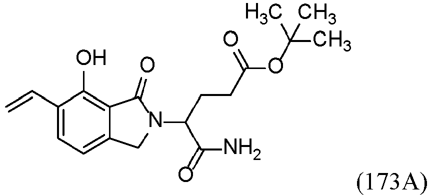

To a solution of tert-butyl 5-amino-4-(6-bromo-7-hydroxy-1-oxoisoindolin-2-yl)-5-oxopentanoate (30.0 g, 61.7 mmol) in 1,4-dioxane (100 mL) was added potassium vinyltrifluoroborate (16.53 g, 123 mmol), aqueous potassium phosphate tribasic (2 M, 93 mL, 185 mmol), and Pd(dppf)Cl₂ (4.51 g, 6.17 mmol). The resulting mixture was purged with nitrogen for 10 min and stirred at 100 °C for 12 h. The reaction mixture was diluted with water (100 mL) and extracted with ethyl acetate (3 x 70 mL). The combined organic layers were washed with brine (100 mL), dried (Na₂SO₄), filtered, and concentrated. The crude material was purified by flash chromatography (SiO₂, 0-40% ethyl acetate/petroleum ether) to afford tert-butyl 5-amino-4-(7-hydroxy-1-oxo-6-vinylisoindolin-2-yl)-5-oxopentanoate (19 g, 76% yield). LCMS (Method S): retention time 2.51 min, [M+2H-tBu]⁺ 305.0.

### Intermediate 173B: tert-butyl 5-amino-4-(7-hydroxy-6-(hydroxymethyl)-1-oxoisoindolin-2-yl)-5-oxopentanoate

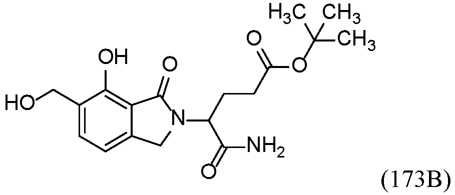

To a cooled (0 °C) solution of tert-butyl 5-amino-4-(7-hydroxy-1-oxo-6-vinylisoindolin-2-yl)-5-oxopentanoate (19 g, 46.9 mmol) in THF (100 mL) and water (20 mL) was added osmium tetroxide solution (4 wt. % in water, 29.8 g, 4.69 mmol) under nitrogen atmosphere. The resulting mixture was stirred at 0 °C for 30 min and then for 1 h at room temperature. The reaction mixture was cooled again to 0 °C and sodium periodate (30.1 g, 141 mmol) was added slowly over a period of 10 min. The mixture was then stirred at room temperature for 12 h. The reaction mixture was diluted with cold water (30 mL) and extracted with ethyl acetate (3 x 30 mL). The organic layers were washed with brine (30 mL), dried (Na₂SO₄), filtered, and concentrated to afford crude tert-butyl 5-amino-4-(6-formyl-7-hydroxy-1-oxoisoindolin-2-yl)-5-oxopentanoate (20 g).

To a cooled (0 °C) solution of crude tert-butyl 5-amino-4-(6-formyl-7-hydroxy-1-oxoisoindolin-2-yl)-5-oxopentanoate (20 g) in methanol (30 mL) was added sodium borohydride (7.10 g, 188 mmol). The resulting mixture was stirred at 0 °C for 4 h. The reaction mixture was then concentrated and the crude product was purified twice by reversed phase chromatography (415 g RediSep C18 column, 35% acetonitrile/water with 5 mM ammonium formate, 100 mL/min) to afford tert-butyl 5-amino-4-(7-hydroxy-6-(hydroxymethyl)-1-oxoisoindolin-2-yl)-5-oxopentanoate (6.2 g, 34% yield over two steps). LCMS (Method K): retention time 1.55 min, [M+H]⁺ 365.0.

### Intermediate 173C: tert-butyl 5-amino-4-(6-(hydroxymethyl)-1-oxo-7-(3-phenylpropoxy) isoindolin-2-yl)-5-oxopentanoate

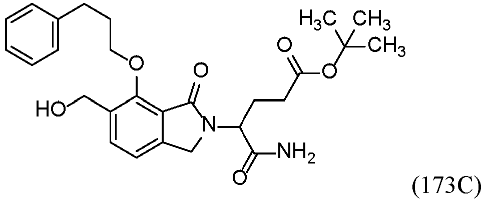

A mixture of tert-butyl 5-amino-4-(7-hydroxy-6-(hydroxymethyl)-1-oxoisoindolin-2-yl)-5-oxopentanoate (50 mg, 0.137 mmol), (3-bromopropyl)benzene (41.0 mg, 0.206 mmol), and potassium carbonate (56.9 mg, 0.412 mmol) in DMF (1 mL) was stirred at 80 °C for 2 h. The reaction mixture was allowed to cool to room temperature, diluted with water, and extracted with ethyl acetate. The combined organic layers were dried (MgSO₄), filtered, and concentrated. The residue was purified by silica gel chromatography, eluting with 20 to 100% ethyl acetate in hexanes, to afford tert-butyl 5-amino-4-(6-(hydroxymethyl)-1-oxo-7-(3-phenylpropoxy)isoindolin-2-yl)-5-oxopentanoate (49 mg, 74%). LCMS (Method E): retention time 0.95 min, [M+H]⁺ 483.2; ¹H NMR (500 MHz, CHLOROFORM-d) δ 7.57 (d, *J*=7.6 Hz, 1H), 7.34-7.29 (m, 2H), 7.27-7.23 (m, 2H), 7.23-7.19 (m, 1H), 7.16 (d, *J*=7.6 Hz, 1H), 6.33 (br s, 1H), 5.41 (br s, 1H), 4.88 (dd, *J*=8.7, 6.4 Hz, 1H), 4.83-4.72 (m, 2H), 4.53-4.37 (m, 3H), 4.33 (dt, *J*=9.3, 6.6 Hz, 1H), 2.89-2.83 (m, 2H), 2.43-2.08 (m, 7H), 1.44 (s, 9H).

### Intermediate 173D: 3-(6-(hydroxymethyl)-1-oxo-7-(3-phenylpropoxy)isoindolin-2-yl) piperidine-2,6-dione

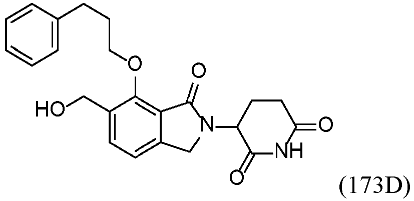

A mixture of tert-butyl 5-amino-4-(6-(hydroxymethyl)-1-oxo-7-(3-phenylpropoxy) isoindolin-2-yl)-5-oxopentanoate (45 mg, 0.093 mmol) and methanesulfonic acid (0.036 mL, 0.559 mmol) in 1,4-dioxane (1 mL) was stirred at 80 °C for 2 h. The reaction mixture was allowed to cool to room temperature and purified by reversed phase chromatography (30% acetonitrile in water with 0.1% TFA) to afford 3-(6-(hydroxymethyl)-1-oxo-7-(3-phenylpropoxy)isoindolin-2-yl)piperidine-2,6-dione (14 mg, 37%). LCMS (Method E): retention time 0.83 min, [M+H]⁺ 409.1.

### Example 173:

Phosgene (20% in toluene, 36.3 mg, 0.073 mmol) was added dropwise to 3-(6-(hydroxymethyl)-1-oxo-7-(3-phenylpropoxy)isoindolin-2-yl)piperidine-2,6-dione (20 mg, 0.049 mmol) in THF (1 mL). The resulting mixture was stirred at room temperature for 1 h and then concentrated in vacuo. The residue was dissolved in THF (1 mL), then 4-(3,4-difluorophenoxy)aniline (11.9 mg, 0.054 mmol) was added, followed by DIEA (0.019 mL, 0.108 mmol). The reaction mixture was stirred at room temperature for 2 h and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 49-79%B (0.0-20.0 min), 79-100%B (20.0-20.1 min), 100%B (20.1-24.0 min); Flow: 20 mL/min). Fractions containing the product were combined and dried via centrifugal evaporation to afford {2-[(3S)-2,6-dioxopiperidin-3-yl]-3-oxo-4-(3-phenylpropoxy)-2,3-dihydro-1H-isoindol-5-yl}methyl N-[4-(3,4-difluorophenoxy)phenyl]carbamate (2.5 mg, 8% yield). LCMS (Method D): retention time 2.51 min, [M+H]⁺ 656.0; ¹H NMR (500 MHz, DMSO-d₆) δ 9.77 (br s, 1H), 7.68 (d, *J*=7.6 Hz, 1H), 7.49 (br d, *J*=8.2 Hz, 2H), 7.44-7.35 (m, 1H), 7.32 (d, *J*=7.9 Hz, 1H), 7.26-7.10 (m, 5H), 7.07 (ddd, *J=*11.9*,* 6.8, 3.1 Hz, 1H), 7.01 (d, *J*=8.9 Hz, 2H), 6.79-6.72 (m, 1H), 5.25 (s, 2H), 5.06 (dd, *J=*13.0, 5.0 Hz, 1H), 4.47-4.38 (m, 1H), 4.38-4.24 (m, 3H), 2.95-2.83 (m, 1H), 2.78-2.69 (m, 2H), 2.66-2.56 (m, 1H), 2.38 (qd, *J*=13.4, 4.7 Hz, 1H), 2.10-1.94 (m, 3H).

### EXAMPLE 174

### Methyl 2-({5-[({[4-(3,4-difluorophenoxy)phenyl]carbamoyl}oxy)methyl]-2-(2,6-dioxopiperidin-3-yl)-3-oxo-2,3-dihydro-1H-isoindol-4-yl}oxy)acetate

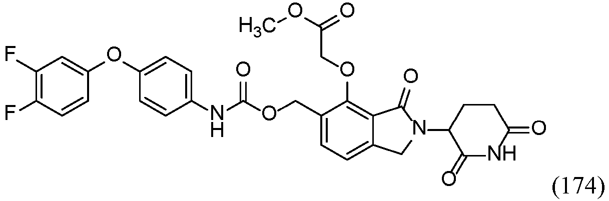

### Intermediate 174A: tert-butyl 2-((5-((((4-(3,4-difluorophenoxy)phenyl)carbamoyl)oxy) methyl)-2-(2,6-dioxopiperidin-3-yl)-3-oxoisoindolin-4-yl)oxy)acetate

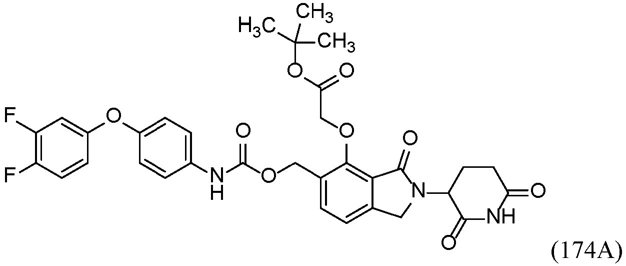

Intermediate 174A was prepared according to the general procedures described for Example 52, replacing iodoethane in the first step with tert-butyl 2-bromoacetate, and replacing 4-phenoxybenzoic acid in the final step with 4-(3,4-difluorophenoxy)benzoic acid. LCMS (Method E): retention time 1.09 min, [M+H]⁺ 652.1; ¹H NMR (400 MHz, METHANOL-d₄) δ 7.70 (d, *J*=7.7 Hz, 1H), 7.46 (br d, *J*=8.7 Hz, 2H), 7.30 (d, *J*=7.7 Hz, 1H), 7.21 (dt, *J*=10.2, 9.2 Hz, 1H), 6.96 (d, J=9.0 Hz, 2H), 6.86 (ddd, *J=*11.8*,* 6.7, 2.9 Hz, 1H), 6.73 (dtd, *J*=9.0, 3.2, 1.7 Hz, 1H), 5.43 (s, 2H), 5.11 (dd, *J=*13.4*,* 5.2 Hz, 1H), 5.01 (d, *J*=2.1 Hz, 2H), 4.54-4.37 (m, 2H), 2.99-2.85 (m, 1H), 2.82-2.70 (m, 1H), 2.47 (qd, *J=*13.2*,* 4.7 Hz, 1H), 2.16 (dtd, *J=*12.7, 5.2, 2.4 Hz, 1H), 1.45 (s, 9H).

### Intermediate 174B: 2-((5-((((4-(3,4-difluorophenoxy)phenyl)carbamoyl)oxy)methyl)-2-(2,6-dioxopiperidin-3-yl)-3-oxoisoindolin-4-yl)oxy)acetic acid

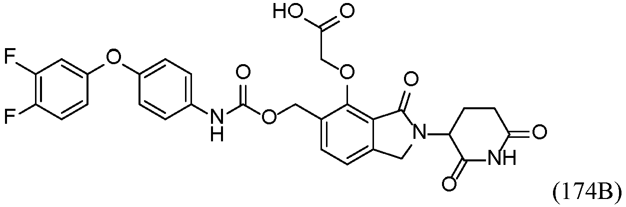

HCl solution (4 N in 1,4-dioxane, 0.192 mL, 0.767 mmol) was added to tert-butyl 2-((5-((((4-(3,4-difluorophenoxy)phenyl)carbamoyl)oxy)methyl)-2-(2,6-dioxopiperidin-3-yl)-3-oxoisoindolin-4-yl)oxy)acetate (25 mg, 0.038 mmol) in DCM (1 mL). The resulting mixture was stirred at room temperature for 15 h. The reaction mixture was concentrated in vacuo to afford 2-((5-((((4-(3,4-difluorophenoxy)phenyl)carbamoyl)oxy)methyl)-2-(2,6-dioxopiperidin-3-yl)-3-oxoisoindolin-4-yl)oxy)acetic acid (22 mg, 87% yield). LCMS (Method E): retention time 0.97 min, [M+H]⁺ 596.1; ¹H NMR (400 MHz, METHANOL-d₄) δ 7.72 (d, *J*=7.7 Hz, 1H), 7.46 (br d, *J*=8.8 Hz, 2H), 7.32 (d, *J*=7.7 Hz, 1H), 7.26-7.14 (m, 1H), 6.96 (d, *J*=8.9 Hz, 2H), 6.86 (ddd, *J=*11.8*,* 6.7, 2.9 Hz, 1H), 6.73 (dtd, *J*=9.0, 3.2, 1.9 Hz, 1H), 5.44 (s, 2H), 5.17-5.05 (m, 3H), 4.55-4.38 (m, 2H), 2.97-2.85 (m, 1H), 2.82-2.70 (m, 1H), 2.56-2.42 (m, 1H), 2.17 (ddt, *J=*12.7, 5.2, 2.6 Hz, 1H).

### Example 174:

A solution of TMS-diazomethane (2 M in hexanes, 0.020 mL, 0.040 mmol) was added to 2-((5-((((4-(3,4-difluorophenoxy)phenyl)carbamoyl)oxy)methyl)-2-(2,6-dioxopiperidin-3-yl)-3-oxoisoindolin-4-yl)oxy)acetic acid (8 mg, 0.013 mmol) in methanol (0.5 mL) and toluene (0.5 mL). The resulting mixture was stirred at room temperature for 1 h and concentrated in vacuo to afford methyl 2-({5-[({[4-(3,4-difluorophenoxy)phenyl] carbamoyl}oxy)methyl]-2-(2,6-dioxopiperidin-3-yl)-3-oxo-2,3-dihydro-1H-isoindol-4-yl} oxy)acetate (6 mg, 66% yield). LCMS (Method E): retention time 1.01 min, [M+H]⁺ 610.1; ¹H NMR (400 MHz, METHANOL-d₄) δ 7.71 (d, *J*=7.7 Hz, 1H), 7.46 (br d, *J*=8.6 Hz, 1H), 7.32 (d, *J*=7.7 Hz, 1H), 7.25-7.08 (m, 3H), 7.01-6.93 (m, 2H), 6.88-6.80 (m, 1H), 6.73 (dtd, *J*=9.0, 3.2, 1.9 Hz, 1H), 5.43 (s, 2H), 5.19-5.06 (m, 3H), 4.46 (d, *J*=7.4 Hz, 2H), 3.73 (s, 3H), 2.98-2.74 (m, 2H), 2.54-2.41 (m, 1H), 2.22-2.10 (m, 1H).

### EXAMPLE 175

### {4-[(dimethylcarbamoyl)methoxy]-2-(2,6-dioxopiperidin-3-yl)-3-oxo-2,3-dihydro-1H-isoindol-5-yl}methyl N-[4-(3,4-difluorophenoxy)phenyl]carbamate

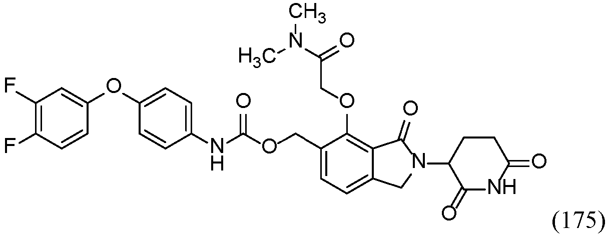

A mixture of 2-((5-((((4-(3,4-difluorophenoxy)phenyl)carbamoyl)oxy)methyl)-2-(2,6-dioxopiperidin-3-yl)-3-oxoisoindolin-4-yl)oxy)acetic acid (30 mg, 0.050 mmol), dimethylamine solution (11% in 2-propanol, 20.7 mg, 0.050 mmol), HATU (28.7 mg, 0.076 mmol), and DIEA (0.018 mL, 0.101 mmol) in DMF (1 mL) was stirred at room temperature for 1 h. The reaction mixture was purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford {4-[(dimethylcarbamoyl)methoxy]-2-(2,6-dioxopiperidin-3-yl)-3-oxo-2,3-dihydro-1H-isoindol-5-yl}methyl N-[4-(3,4-difluorophenoxy)phenyl]carbamate (17.1 mg, 53% yield). LCMS (Method C): retention time 1.88 min, [M+H]⁺ 623.2; ¹H NMR (500 MHz, DMSO-d₆) δ 9.76 (br s, 1H), 7.65 (d, *J*=7.7 Hz, 1H), 7.48 (br d, *J*=8.5 Hz, 2H), 7.44-7.35 (m, 1H), 7.30 (d, *J*=7.6 Hz, 1H), 7.16-7.05 (m, 1H), 7.01 (br d, *J*=8.9 Hz, 2H), 6.85-6.70 (m, 1H), 5.46-5.34 (m, 2H), 5.30 (s, 2H), 5.07 (dd, *J=*13.0*,* 5.1 Hz, 1H), 4.52-4.21 (m, 2H), 2.90 (s, 3H), 2.86-2.83 (m, 1H), 2.78 (s, 3H), 2.64-2.55 (m, 1H), 2.43-2.30 (m, 1H), 2.05-1.96 (m, 1H).

### EXAMPLES 176-178

The compounds in Table 17 were prepared according to the general procedures described for Example 175, replacing dimethylamine with the appropriate amine:

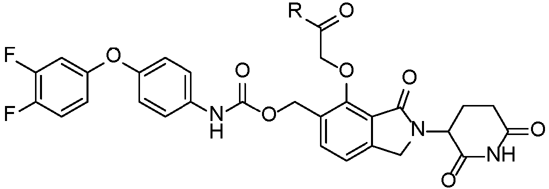

**TABLE 17**

| Ex. No. | R | LCMS [M+H]⁺ | RT (min) | HPLC Method |
|---|---|---|---|---|
| 176 | 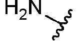 | 595.4 | 1.76 | D |
| 177 | 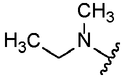 | 637.0 | 1.91 | C |
| 178 | 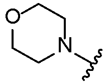 | 665.0 | 1.83 | C |

### EXAMPLE 179

### {4-[(1-acetylpiperidin-4-yl)oxy]-2-(2,6-dioxopiperidin-3-yl)-3-oxo-2,3-dihydro-1H-isoindol-5-yl}methyl N-[4-(3,4-difluorophenoxy)phenyl]carbamate

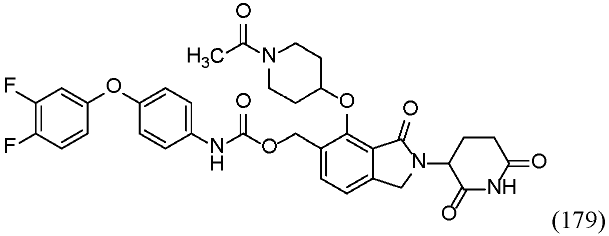

### Intermediate 179A: tert-butyl 4-((2-(2,6-dioxopiperidin-3-yl)-5-(hydroxymethyl)-3-oxoisoindolin-4-yl)oxy)piperidine-1-carboxylate

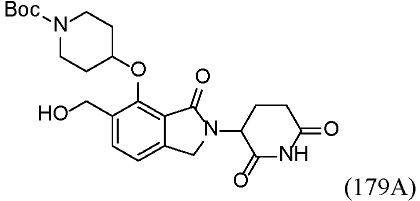

Intermediate 79A was prepared according to the general procedures described for Example 141, Intermediate 141D, replacing (S)-tetrahydro-2H-pyran-3-ol with tert-butyl 4-hydroxypiperidine-1-carboxylate. LCMS (Method E): retention time 0.79 min, [M+H]⁺ 474.0; ¹H NMR (400 MHz, METHANOL-d₄) δ 7.72 (d, *J*=7.6 Hz, 1H), 7.28 (d, *J*=7.7 Hz, 1H), 5.13-5.03 (m, 1H), 4.82-4.75 (m, 1H), 4.72 (s, 2H), 4.48-4.32 (m, 2H), 3.96-3.84 (m, 2H), 3.03 (m, 2H), 2.95-2.71 (m, 2H), 2.52-2.35 (m, 1H), 2.18-2.11 (m, 1H), 2.02-1.93 (m, 2H), 1.74-1.63 (m, 2H), 1.47 (s, 9H).

### Intermediate 179B: tert-butyl 4-((2-(2,6-dioxopiperidin-3-yl)-5-(hydroxymethyl)-3-oxoisoindolin-4-yl)oxy)piperidine-1-carboxylate

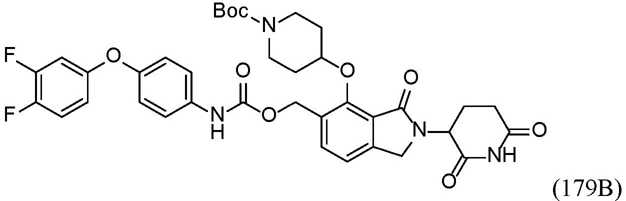

A mixture of tert-butyl 4-((2-(2,6-dioxopiperidin-3-yl)-5-(hydroxymethyl)-3-oxoisoindolin-4-yl)oxy)piperidine-1-carboxylate (40 mg, 0.084 mmol), 4-(3,4-difluorophenoxy)benzoic acid (23.3 mg, 0.093 mmol), DIEA (0.019 mL, 0.110 mmol), and diphenyl phosphorazidate (27.9 mg, 0.101 mmol) in 1,4-dioxane (1 mL) was heated at 105 °C for 15 h. The reaction mixture was allowed to cool to room temperature and purified by silica gel chromatography, eluting with 0 to 10% MeOH in DCM, to afford tert-butyl 4-((5-((((4-(3,4-difluorophenoxy)phenyl)carbamoyl)oxy)methyl)-2-(2,6-dioxopiperidin-3-yl)-3-oxoisoindolin-4-yl)oxy)piperidine-1-carboxylate (40 mg, 66% yield). LCMS (Method E): retention time 1.10 min, [M+H]⁺ 721.1; ¹H NMR (400 MHz, CHLOROFORM-d) δ 8.07 (s, 1H), 7.66 (d, *J*=7.7 Hz, 1H), 7.40 (br d, *J*=8.4 Hz, 1H), 7.17 (s, 1H), 7.13-7.04 (m, 1H), 6.97 (d, *J*=9.0 Hz, 2H), 6.88-6.76 (m, 2H), 6.74-6.63 (m, 1H), 5.33 (s, 2H), 5.18 (dd, *J*=13.2, 5.2 Hz, 1H), 4.99-4.88 (m, 1H), 4.58-4.27 (m, 2H), 3.96 (br d, *J*=7.6 Hz, 2H), 3.15-2.94 (m, 2H), 2.92-2.73 (m, 2H), 2.34 (dd, *J=*13.2*,* 4.8 Hz, 1H), 2.23-2.11 (m, 1H), 2.03-1.93 (m, 2H), 1.83-1.72 (m, 2H), 1.47 (s, 9H).

### Intermediate 179C: tert-butyl 4-((2-(2,6-dioxopiperidin-3-yl)-5-(hydroxymethyl)-3-oxoisoindolin-4-yl)oxy)piperidine-1-carboxylate

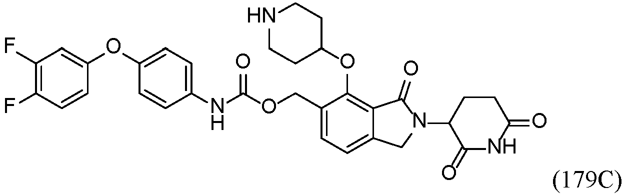

HCl solution (4 N in 1,4-dioxane, 0.132 mL, 0.527 mmol) was added to tert-butyl 4-((5-((((4-(3,4-difluorophenoxy)phenyl)carbamoyl)oxy)methyl)-2-(2,6-dioxopiperidin-3-yl)-3-oxoisoindolin-4-yl)oxy)piperidine-1-carboxylate (38 mg, 0.053 mmol) in DCM (0.5 mL) at room temperature. The resulting mixture was stirred for 15 h and then concentrated in vacuo to afford (2-(2,6-dioxopiperidin-3-yl)-3-oxo-4-(piperidin-4-yloxy)isoindolin-5-yl)methyl (4-(3,4-difluorophenoxy)phenyl)carbamate as the HCl salt (34 mg, 93% yield). LCMS (Method E): retention time 0.88 min, [M+H]⁺ 621.2; ¹H NMR (400 MHz, CHLOROFORM-d) δ 7.39 (d, *J*=7.7 Hz, 1H), 7.22-7.07 (m, 2H), 7.00 (d, *J*=7.7 Hz, 1H), 6.91-6.79 (m, 1H), 6.69-6.57 (m, 2H), 6.53-6.46 (m, 1H), 6.44-6.35 (m, 1H), 4.98 (s, 2H), 4.74 (dd, *J=*13.3, 5.2 Hz, 1H), 4.59 (tt, *J*=7.4, 3.8 Hz, 1H), 4.21-4.03 (m, 2H), 3.19-3.07 (m, 2H), 2.88-2.72 (m, 2H), 2.59-2.36 (m, 2H), 2.15 (qd, *J*=13.2, 4.9 Hz, 1H), 1.99-1.73 (m, 5H).

### Example 179:

To a cooled (0 °C) mixture of (2-(2,6-dioxopiperidin-3-yl)-3-oxo-4-(piperidin-4-yloxy)isoindolin-5-yl)methyl (4-(3,4-difluorophenoxy)phenyl)carbamate HCl salt (15 mg, 0.023 mmol) in DCM (0.5 mL) was added acetic anhydride (2.2 µL, 0.023 mmol). The reaction mixture was stirred for 1 h and purified via preparative HPLC (Column: XBridge C18, 19 mm x 200 mm, 5 µm particles; Mobile Phase A: acetonitrile/water (5:95) with 10 mM ammonium acetate, Mobile Phase B: acetonitrile/water (95:5) with 10 mM ammonium acetate; Temperature: 25 °C; Gradient: 42-62%B (0.0-30.0 min), 62-100%B (30.0-30.1 min), 100%B (30.1-34.0 min); Flow: 20 mL/min). Fractions containing the product were combined and dried via centrifugal evaporation to afford {4-[(1-acetylpiperidin-4-yl)oxy]-2-(2,6-dioxopiperidin-3-yl)-3-oxo-2,3-dihydro-1H-isoindol-5-yl}methyl N-[4-(3,4-difluorophenoxy)phenyl]carbamate (7.6 mg, 50% yield). LCMS (Method C): retention time 1.85 min, [M+1]⁺ 663.0; ¹H NMR (500 MHz, DMSO-d₆) δ 9.74 (br s, 1H), 7.72-7.57 (m, 2H), 7.48 (br d, J=8.5 Hz, 2H), 7.43-7.37 (m, 1H), 7.33-7.28 (m, 1H), 7.19 (br s, 1H), 7.08 (ddd, J=11.7, 6.8, 2.7 Hz, 1H), 7.01 (br d, J=8.9 Hz, 2H), 6.81-6.72 (m, 1H), 5.32-5.19 (m, 2H), 4.85-4.80 (m, 1H), 4.73 (dd, J=10.0, 4.8 Hz, 1H), 4.59-4.39 (m, 2H), 4.38-4.22 (m, 1H), 4.09-3.93 (m, 1H), 3.79-3.66 (m, 1H), 3.47-3.33 (m, 1H), 3.20-3.06 (m, 1H), 2.99-2.83 (m, 1H), 2.28-2.12 (m, 2H), 2.04 (s, 3H), 1.97-1.84 (m, 2H), 1.75-1.66 (m, 1H), 1.63-1.45 (m, 1H).

### INTERMEDIATE I-12

### tert-butyl (S)-4,5-diamino-5-oxopentanoate-2,2,3,3,4-ds hydrochloride

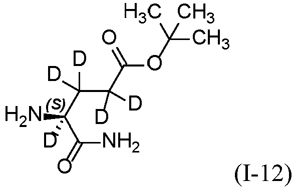

tert-Butyl (S)-4,5-diamino-5-oxopentanoate-2,2,3,3,4-ds hydrochloride was prepared from commercially available L-glutamic acid-2,3,3,4,4-d₅ according to the general methods disclosed in WO2019040109 A1 for the preparation of tert-butyl (S)-4,5-diamino-5-oxopentanoate.

### EXAMPLE 180

### {2-[(3S)-2,6-dioxo(3,4,4,5,5-²H₅)piperidin-3-yl]-4-methoxy-3-oxo-2,3-dihydro-1H-isoindol-5-yl}methyl N-[4-(3,4-difluorophenoxy)phenyl]carbamate

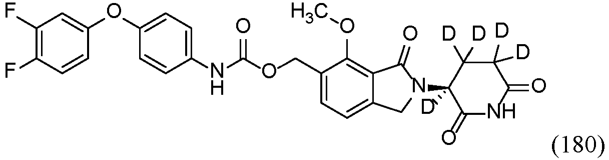

### Intermediate 180A: tert-butyl (S)-5-amino-4-(6-bromo-7-methoxy-1-oxoisoindolin-2-yl)-5-oxopentanoate-2,2,3,3,4-d₅

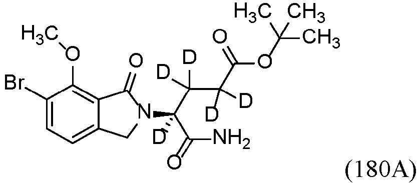

A mixture of methyl 3-bromo-6-(bromomethyl)-2-methoxybenzoate (575 mg, 1.70 mmol), tert-butyl (S)-4,5-diamino-5-oxopentanoate-2,2,3,3,4-ds hydrochloride (431 mg, 1.77 mmol), and DIEA (0.743 mL, 4.25 mmol) in acetonitrile (3.75 mL) was stirred at 60 °C for 15 h. The reaction mixture was concentrated, re-dissolved in ethyl acetate, washed with water, then brine. The organics were dried with MgSO₄. After filtration and concentration, the crude product was re-dissolved in a minimum amount of ethyl acetate. Hexane was then added to precipitate the product, giving tert-butyl (S)-5-amino-4-(6-bromo-7-methoxy-1-oxoisoindolin-2-yl)-5-oxopentanoate-2,2,3,3,4-d5 (0.66 g, 90 % yield) as a white solid. LCMS (Method E): retention time 0.92 min, [M+H]⁺ 432.1, 434.1; ¹H NMR (400 MHz, CHLOROFORM-d) δ 7.75 (d, *J*=8.1 Hz, 1H), 7.09 (d, *J*=8.0 Hz, 1H), 6.38 (br s, 1H), 5.43 (br s, 1H), 4.62-4.34 (m, 2H), 4.12 (s, 3H), 1.45 (s, 9H).

### Intermediate 180B: tert-butyl (S)-5-amino-4-(6-(hydroxymethyl)-7-methoxy-1-oxoisoindolin-2-yl)-5-oxopentanoate-2,2,3,3,4-d₅

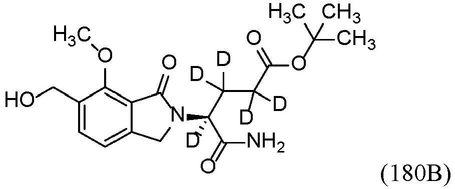

A mixture of tert-butyl (S)-5-amino-4-(6-bromo-7-methoxy-1-oxoisoindolin-2-yl)-5-oxopentanoate-2,2,3,3,4-d₅ (650 mg, 1.50 mmol), 1-(tributylstannyl)methanol (724 mg, 2.26 mmol) and Pd(PPh₃)₄ (174 mg, 0.150 mmol) in 1,4-dioxane (10.7 mL) was degassed with nitrogen, then heated to 100 °C for 5 h. The reaction mixture was concentrated and purified by flash chromatography (80 g SiO₂ column, 0-20% MeOH/DCM) to afford tert-butyl (S)-5-amino-4-(6-(hydroxymethyl)-7-methoxy-1-oxoisoindolin-2-yl)-5-oxopentanoate-2,2,3,3,4-d₅ (0.43 g, 75% yield) as a pale foam. LCMS (Method E): retention time 0.76 min, [M+H]⁺ 384.1; ¹H NMR (400 MHz, CHLOROFORM-d) δ 7.57 (d, *J*=7.6 Hz, 1H), 7.18 (d, *J*=7.6 Hz, 1H), 6.40 (br s, 1H), 5.49-5.33 (m, 1H), 4.78 (dd, *J*=6.0, 4.9 Hz, 2H), 4.58-4.34 (m, 2H), 4.17 (s, 3H), 2.28 (t, *J*=6.3 Hz, 1H), 1.44 (s, 9H).

### Intermediate 180C: tert-butyl (S)-5-amino-4-(6-((((4-(3,4-difluorophenoxy)phenyl) carbamoyl)oxy)methyl)-7-methoxy-1-oxoisoindolin-2-yl)-5-oxopentanoate-2,2,3,3,4-d₅

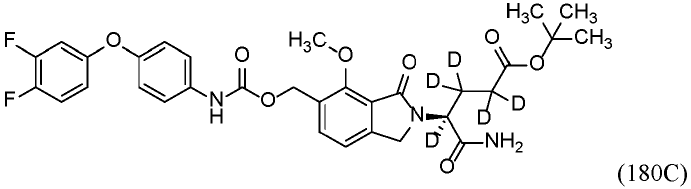

A mixture of tert-butyl (S)-5-amino-4-(6-(hydroxymethyl)-7-methoxy-1-oxoisoindolin-2-yl)-5-oxopentanoate-2,2,3,3,4-d₅ (400 mg, 1.04 mmol), 4-(3,4-difluorophenoxy)benzoic acid (522 mg, 2.09 mmol), diphenyl phosphoryl azide (718 mg, 2.61 mmol), and DIEA (0.455 mL, 2.61 mmol) in 1,4-dioxane (2.65 mL) was heated to 90-98 °C for 1-2 h. (A significant exotherm was observed once internal temperature reached ~80 °C; heating was stopped to avoid overheating; gas evolution was observed). The reaction mixture was cooled to room temperature and concentrated to remove all volatiles. The crude material was purified by flash chromatography (40 g SiO₂ column, 0-80% ethyl acetate/hexane) to afford tert-butyl (S)-5-amino-4-(6-((((4-(3,4-difluorophenoxy)phenyl) carbamoyl)oxy)methyl)-7-methoxy-1-oxoisoindolin-2-yl)-5-oxopentanoate-2,2,3,3,4-d₅ (498 mg, 76% yield) as a pale foam. LCMS (Method E): retention time 1.06 min, [M+H]⁺ 631.3, [M+K]⁺ 669.3; ¹H NMR (499 MHz, CHLOROFORM-d) δ 7.65 (d, *J*=7.8 Hz, 1H), 7.39 (br d, *J*=8.5 Hz, 2H), 7.20 (d, *J*=7.6 Hz, 1H), 7.16-7.05 (m, 1H), 6.98 (d, *J*=9.0 Hz, 2H), 6.80 (ddd, *J=*11.4, 6.7, 2.9 Hz, 1H), 6.74-6.62 (m, 2H), 6.30 (br s, 1H), 5.34 (d, *J*=2.8 Hz, 2H), 5.29 (br s, 1H), 4.58-4.38 (m, 2H), 4.19 (s, 3H), 1.45 (s, 9H).

### Intermediate 180D: (S)-5-amino-4-(6-((((4-(3,4-difluorophenoxy)phenyl)carbamoyl)oxy) methyl)-7-methoxy-1-oxoisoindolin-2-yl)-5-oxopentanoic-2,2,3,3,4-ds acid

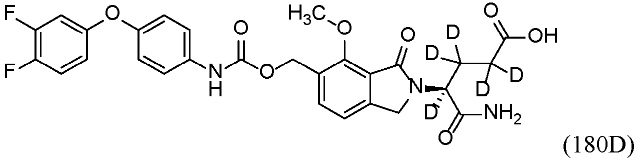

TFA (2.13 mL, 27.6 mmol) was added to tert-butyl (S)-5-amino-4-(6-((((4-(3,4-difluorophenoxy)phenyl)carbamoyl)oxy)methyl)-7-methoxy-1-oxoisoindolin-2-yl)-5-oxopentanoate-2,2,3,3,4-d₅ (498 mg, 0.790 mmol) in DCM (4.4 mL). The resulting mixture was stirred at room temperature for 2 h and then concentrated in vacuo to afford (S)-5-amino-4-(6-((((4-(3,4-difluorophenoxy)phenyl)carbamoyl)oxy)methyl)-7-methoxy-1-oxoisoindolin-2-yl)-5-oxopentanoic-2,2,3,3,4-ds acid (450 mg, 99% yield) as an off-white foam. LCMS (Method E): retention time 0.95 min, [M+H]⁺ 575.2, [M+K]⁺ 613.2; ¹H NMR (400 MHz, CHLOROFORM-d) δ 7.83-7.74 (m, 1H), 7.68 (d, *J*=7.7 Hz, 1H), 7.38 (br d, *J*=8.0 Hz, 2H), 7.24 (d, *J*=7.7 Hz, 1H), 7.16-7.05 (m, 1H), 6.97 (d, *J*=8.9 Hz, 2H), 6.80 (ddd, *J=*11.4, 6.6, 2.9 Hz, 2H), 6.74-6.66 (m, 1H), 6.61 (br d, *J*=1.5 Hz, 1H), 5.57-5.46 (m, 1H), 5.33 (d, *J*=2.2 Hz, 2H), 4.78 (d, *J*=18.0 Hz, 1H), 4.48 (d, *J*=18.0 Hz, 1H), 4.11 (s, 3H).

### Example 180:

Thionyl chloride (0.380 mL, 5.21 mmol) was diluted with anhydrous 1,4-dioxane and added slowly to a cooled (-55 °C) solution of (S)-5-amino-4-(6-((((4-(3,4-difluorophenoxy) phenyl)carbamoyl)oxy)methyl)-7-methoxy-1-oxoisoindolin-2-yl)-5-oxopentanoic-2,2,3,3,4-ds acid (374 mg, 0.651 mmol) in DMF (8.54 mL). The resulting mixture was stirred at -55 °C for 15 minutes, then pyridine (0.948 mL, 11.72 mmol) was added slowly. The reaction mixture was stirred at -55 °C for ~20 minutes. The reaction was then quenched with ice, followed by ice water (3x volume of DMF), resulting in the formation of a gum that later solidified with prolonged stirring. The suspension was stirred at 0-5 °C for 1- 2 h and then filtered. The solid was thoroughly rinsed with water and dried overnight. The material was purified by SFC (Column: CHIRALPAK AS-H, 5 x 25 cm, 5 µm particles; Mobile Phase: carbon dioxide/1:1 v/v MeOH:acetonitrile (59:41); Temperature: 35 °C; BPR Pressure: 100 bar; Flow: 320 mL/min) to afford {2-[(3S)-2,6-dioxo(3,4,4,5,5-²H5)piperidin-3-yl]-4-methoxy-3-oxo-2,3-dihydro-1H-isoindol-5-yl}methyl N-[4-(3,4-difluorophenoxy)phenyl] carbamate (280 mg, 77 % yield) as an off-white solid. SFC (Method T): retention time: 5.65 min (≥99.9% ee). LCMS (Method E): retention time 0.99 min, [M+H]⁺ 557.3, [M+K]⁺ 595.4; ¹H NMR (400 MHz, DMSO-d₆) δ 10.98 (s, 1H), 9.78 (s, 1H), 7.68 (d, *J*=7.7 Hz, 1H), 7.56-7.30 (m, 4H), 7.10 (ddd, *J*=12.0, 6.8, 2.9 Hz, 1H), 7.02 (d, *J*=9.0 Hz, 2H), 6.88-6.63 (m, 1H), 5.23 (s, 2H), 4.52-4.25 (m, 2H), 4.05 (s, 3H); ¹⁹F NMR (376 MHz, DMSO-d₆) δ -135.53, -146.01.

### BIOLOGICAL ASSAYS

The pharmacological properties of the compounds of this invention may be confirmed by a number of biological assays. The exemplified biological assays, which follow, have been compounds with at least one compound of the invention.

### Cyclin E1 Cellular Degradation Assay (MSD)

Culturing cells: On Day 0, OVCAR3 cells were plated at 15,000 cells/well in 40 uL of culture medium (RPMI1640, 20% FBS, 0.01 insulin, 1% Anti/Anti) in a 384 well cell culture plate. Cell cultures were incubated for 24 hours at 37 °C and 5% CO₂.

Coating MSD plates: On Day 1, rabbit anti-CCNE1 antibody (Abcam ab33911, 1 ug/mL in DPBS, 10 nµL/well) was dispensed to a Meso Scale Discovery plate (MSD L21XA). The MSD plate was spun briefly to ensure the solution coated the bottom of the well and incubated overnight at 4 °C. On Day 2, the plate was washed 3 times with TBS + 0.2% TWEEN, blocked for 1 hour with 3% MSD Blocker A (MSD R93AA-1, 30 uL/well), then washed again 3 times with TBS + 0.2% TWEEN.

Treating and lysing cells: On Day 1, compounds of interest were dispensed into the cell cultures by acoustic dispensing technology. The cell cultures were then spun at 400 g for 4 minutes to disperse the compounds and incubated at 37 °C and 5% CO₂ for 24 hours. After incubation, 40 uL of DPBS was added to the cell cultures to dilute the media. All liquid was gently discarded and immediately replaced with 30 µL of lysis buffer (MSD Tris Lysis buffer (MSD R60TX-2), Halt Protease inhibitor (ThermoFisher 78438), Phosphatase Inhibitor II (Sigma-Aldrich P5726), Phosphatase Inhibitor III (Sigma-Aldrich P0044), 0.02M PMSF (Sigma-Aldrich 7626), 0.1% SDS (VWR E719-100ML)). The plate was then sealed, shaken at 300 rpm at room temperature for 5 minutes, and freeze-thawed for optimum cell lysis.

Collecting data: After thawing, 25 µL of lysate was transferred to a coated MSD plate. The plate was then sealed and shaken at 300 rpm overnight in 4 °C. On Day 3, the MSD plate was washed 3 times with TBS + 0.2% TWEEN. Mouse anti-CCNE1 antibody (Abcam ab238081, 0.5 µg/mL in 1% MSD Blocker A, 10 µL/well) dispensed to the MSD plate, which was then sealed and shaken at room temperature for 3 hours. After the first antibody incubation, the plate was washed 3 times with TBS + 0.2% TWEEN, then goat anti-mouse Sulfo-Tag antibody (MSD R32AC, 1 µg/mL in 1% MSD Blocker A, 10 µL/well) was dispensed to the MSD plate. The plate was sealed again and shaken at room temperature for 1 hour, then washed 3 times with TBS + 0.2% TWEEN. After the final wash, 35 µL of 1x Read Buffer (MSD R92TC-1) was added to each well and the data was captured using the MSD plate reader. The reference compound, [2-(2,6-dioxopiperidin-3-yl)-4-methoxy-3-oxo-2,3-dihydro-1H-isoindol-5-yl]methyl N-[4-(3,4-difluorophenoxy)phenyl]carbamate, at 10 µM was used to define 110% response. Assay results for each compound were reported as DC₅₀, the concentration giving 50% response, and Dₘₐₓ, the maximum response observed with the compound.

### Phospho-Retinoblastoma (pRB) Cellular Assay

NIH OVCAR3 (HTB-161, American Type Culture Collection, Manassas VA) cells were grown up to passage 10 in culture media made up of RPMI (11835-030 Gibco, Grand Island NY), 20% HI FBS (16140-071, Gibco, Grand Island NY), and 0.01 mg/mL insulin from bovine pancreas (I6634-1G, Sigma Aldrich, St. Louis, MO).

The cells were seeded in a 384-well CulturPlate (6007688, Perkin Elmer, Waltham MA) at a density of 10,000 cells/well in 20 µL of the above culture media and incubated overnight at 37 °C and 5% CO₂.

The next day, cells were treated using an Echo 650 (Beckman Coulter, Indianapolis IN) acoustic dispenser with 40 nL of compounds dissolved in DMSO at the appropriate serial dilutions (10 mM to 1.7 µM). Cells were then incubated for 24 hours as above.

After 24 hours elapsed, media was removed from each well and shortly after 20 µL of the following mix was added: 12 µL deionized water, 4 µL lysis buffer, 4 µL of reconstituted antibody mix, and 1:100 phosphatase inhibitor. These components belong to the Phospho-Rb (Ser807/811) Cellular Kit (64RBS807PEY, Perkin Elmer, Waltham MA).

Plates were sealed and incubated away from light at room temperature for at least 4 hours before reading them on an EnVision plate reader (Perkin Elmer, Waltham MA) according to the kit manufacturer's protocol. The reference compound, N-(5-{[(5-tert-butyl-1,3-oxazol-2-yl)methyl]sulfanyl}-1,3-thiazol-2-yl)piperidine-4-carboxamide, at 10 µM was used to define 100% response. Assay results for each compound were reported as IC₅₀, the concentration giving 50% response, and Yₘₐₓ, the maximum response observed with the compound.

### GSPT1 Cellular Degradation Assay

DF15-GSPT1-ePL was used for intracellular GSPT1 protein degradation detection in form of chemiluminescent signal, which was built on a human DF15 cell line (CVCL_Y429) with conditional co-expression of chimeric GSPT1 (target) and ePL/donor (enhanced ProLabel^{®} by DiscoverX for enzyme fragment complementation with donor-acceptor pairing). The cells used the culture medium in two arrangements for assay and maintenance. During assay, it used Core-RPMI 1640 medium with 10% Fetal Bovine Serum (Heat Inactivated) and 1X Penicillin/Streptomycin; and during passage maintenance, used added ingredients: 0.1 mM NEAA, 1 mM sodium pyruvate, 25 mM Hepes, 0.1% Pluronic F-68, 1X Glutamin, 4 µg/mL Blasticidin (selection agent). Detection was performed on a 1536 well tissue culture plate (Corning# 3727) with cells seeded in 3.5 µL core-RPMI 1640 at density of 0.25 million/mL after the pre-titrated test compounds in DMSO on ECHO plate were dispensed onto the assay plate by use of ECHO (liquid handing system via acoustic energy from Labcyte). Assay incubation lasted 20 hours in 37 °C incubator with 5% CO₂; then followed by 30 minutes of plate-cooling at room temperature; then detection-reagent-mix was added in 3.5 µL/well with pre-defined proportion of enzyme acceptor, substrate, and lysis buffer. Plate signal reading for luminescence on an EnVision plate reader (PerkinElmer) was conducted after 60 minutes of former step at room temperature. Raw data was collected and processed with Dotmatics (data analyzer) for test compound's potency values. The reference compound, 3-[5-(3,6-dimethoxyisoquinolin-1-yl)-1-oxo-2,3-dihydro-1H-isoindol-2-yl]piperidine-2,6-dione, at 1.43 µM was used to define 75% response. Assay results for each compound were reported as DC₅₀, the concentration giving 50% response, and Dₘₐₓ, the maximum response observed with the compound.

**TABLE 18**

| Cyclin E1 Degradation, pRB Inhibition, and GSPT1 Degradation Activity | | | | | | | |
|---|---|---|---|---|---|---|---|
| Ex. No. | Cyclin E1 | | pRB | | GSPT1 | | Ratio of GSPT1 DC₅₀/Cyclin E1 DC₅₀ |
| | DC₅₀ (nM) | Dₘₐₓ (%) | IC₅₀ (nM) | Yₘₐₓ (%) | DC₅₀ (nM) | Dₘₐₓ (%) | |
| 1 | 199 | 110 | 133 | 104 | >14,000 | 27 | >70 |

| Ex. No. | Cyclin E1 | | pRB | | GSPT1 | | Ratio of GSPT1 DC₅₀/Cyclin E1 DC₅₀ |
|---|---|---|---|---|---|---|---|
| | DC₅₀ (nM) | Dₘₐₓ (%) | IC₅₀ (nM) | Yₘₐₓ (%) | DC₅₀ (nM) | Dₘₐₓ (%) | |
| 2 | 78 | 108 | 83 | 123 | >14,000 | 32 | >180 |
| 3 | 91 | 112 | 103 | 120 | >14,000 | 34 | >153 |
| 4 | 39 | 108 | 89 | 109 | >14,000 | 35 | >355 |
| 5 | 299 | 118 | 3 | 131 | >14,000 | 33 | >47 |
| 6 | 62 | 94 | 149 | 117 | >14,000 | 6 | >226 |
| 7 | 203 | 90 | 1,783 | 101 | >14,000 | 21 | >69 |
| 8 | 353 | 104 | 1,230 | 99 | >14,000 | 15 | >40 |
| 9 | 648 | 86 | 3,010 | 85 | >14,000 | 38 | >22 |
| 10 | 114 | 106 | 352 | 105 | >14,000 | 54 | >123 |
| 11 | 61 | 112 | 356 | 98 | >14,000 | 33 | >228 |
| 12 | 82 | 100 | 483 | 111 | >14,000 | 11 | >171 |
| 13 | 55 | 113 | 278 | 97 | >14,000 | 7 | >254 |
| 14 | 59 | 108 | 363 | 96 | >14,000 | 43 | >239 |
| 15 | 35 | 109 | 127 | 109 | >14,000 | 48 | >395 |
| 16 | 161 | 107 | 130 | 105 | >14,000 | 46 | >87 |
| 17 | 447 | 99 | 225 | 114 | >14,000 | 15 | >31 |
| 18 | 89 | 100 | 85 | 110 | >14,000 | 13 | >157 |
| 19 | 146 | 105 | 701 | 92 | >14,000 | 9 | >96 |
| 20 | 96 | 101 | 440 | 103 | >14,000 | 11 | >146 |
| 21 | 40 | 101 | 818 | 92 | >14,000 | 14 | >353 |
| 22 | 26 | 109 | 263 | 97 | 11,400 | 57 | 431 |
| 23 | 71 | 105 | 158 | 97 | >14,000 | 27 | >196 |
| 24 | 39 | 107 | 229 | 105 | >14,000 | 10 | >358 |
| 25 | 184 | 107 | 187 | 96 | >14,000 | 37 | >76 |
| 26 | 97 | 106 | 428 | 96 | >14,000 | 32 | >145 |
| 27 | 93 | 104 | 1,183 | 86 | >14,000 | 30 | >151 |
| 28 | 110 | 103 | 100 | 106 | >14,000 | 45 | >127 |
| 29 | 1,434 | 92 | 865 | 99 | >14,000 | 45 | >10 |
| 30 | 672 | 103 | 442 | 107 | >14,000 | 31 | >21 |
| 31 | 735 | 83 | 2,306 | 81 | 6,600 | 64 | 9 |
| 32 | 67 | 96 | 498 | 83 | >14,000 | 24 | >208 |
| 33 | 1,631 | 85 | 809 | 87 | >14,000 | 8 | >9 |
| 34 | 194 | 107 | 77 | 122 | >14,000 | 19 | >72 |
| 35 | 159 | 107 | 85 | 114 | >14,000 | 46 | >88 |
| 36 | 257 | 108 | 339 | 98 | >14,000 | 28 | >54 |
| 37 | 107 | 102 | 289 | 111 | >14,000 | 28 | >131 |
| 38 | 91 | 107 | 128 | 111 | 4,000 | 46 | 44 |
| 39 | 103 | 108 | 127 | 111 | >14,000 | 20 | >136 |
| 40 | 41 | 108 | 128 | 110 | >14,000 | 9 | >339 |
| 41 | 336 | 92 | 248 | 100 | >14,000 | 13 | >42 |
| 42 | 96 | 106 | 132 | 110 | >14,000 | 20 | >146 |
| 43 | 165 | 111 | 131 | 121 | >14,000 | 33 | >85 |
| 44 | 273 | 111 | 109 | 111 | >14,000 | 42 | >51 |
| 45 | 129 | 112 | 112 | 116 | >14,000 | 38 | >108 |
| 46 | 357 | 105 | 376 | 102 | >14,000 | 42 | >39 |
| 47 | 320 | 101 | 361 | 107 | >14,000 | 20 | >44 |
| 48 | 139 | 111 | 69 | 108 | >14,000 | 46 | >101 |
| 49 | 77 | 106 | 171 | 116 | >14,000 | 24 | >181 |
| 50 | 463 | 77 | 2,085 | 77 | >14,000 | 25 | >30 |
| 51 | 363 | 106 | 188 | 109 | >14,000 | 16 | >39 |
| 52 | 134 | 107 | 81 | 111 | >14,000 | 13 | >105 |
| 53 | 176 | 109 | 86 | 99 | >14,000 | 37 | >80 |
| 54 | 58 | 116 | 624 | 102 | >14,000 | 22 | >241 |
| 55 | 141 | 108 | 185 | 114 | >14,000 | 21 | >99 |
| 56 | 159 | 106 | 113 | 127 | >14,000 | 19 | >88 |
| 57 | 128 | 102 | 212 | 114 | >14,000 | 36 | >109 |
| 58 | 139 | 104 | 298 | 109 | >14,000 | 48 | >100 |
| 59 | 131 | 104 | 175 | 113 | >14,000 | 48 | >107 |
| 60 | 184 | 104 | 503 | 109 | >14,000 | 19 | >76 |
| 61 | 157 | 104 | 412 | 107 | >14,000 | 22 | >89 |
| 62 | 98 | 102 | 676 | 94 | >14,000 | 33 | >143 |
| 63 | 113 | 104 | 136 | 121 | >14,000 | 41 | >124 |
| 64 | 133 | 109 | 83 | 111 | >14,000 | 22 | >105 |
| 65 | 164 | 101 | 59 | 127 | >14,000 | 0 | >85 |
| 66 | 111 | 103 | 55 | 148 | >14,000 | 19 | >126 |
| 67 | 112 | 99 | 144 | 127 | >14,000 | 10 | >125 |
| 68 | 179 | 107 | 83 | 127 | >14,000 | 16 | >78 |
| 69 | 263 | 105 | 94 | 125 | >14,000 | 11 | >53 |
| 70 | 185 | 94 | 223 | 114 | >14,000 | 9 | >76 |
| 71 | 241 | 105 | 234 | 97 | >14,000 | 27 | >58 |
| 72 | 394 | 103 | 364 | 120 | >14,000 | 5 | >36 |
| 73 | 312 | 102 | 272 | 118 | >14,000 | 27 | >45 |
| 74 | 222 | 111 | 53 | 116 | >14,000 | 37 | >63 |
| 75 | 328 | 109 | 287 | 120 | >14,000 | 23 | >43 |
| 76 | 267 | 111 | 104 | 122 | >14,000 | 33 | >52 |
| 77 | 284 | 113 | 163 | 94 | >14,000 | 32 | >49 |
| 78 | 316 | 113 | 165 | 126 | >14,000 | 2 | >44 |
| 79 | 375 | 112 | 128 | 120 | >14,000 | 31 | >37 |
| 80 | 181 | 115 | 73 | 119 | >14,000 | 40 | >77 |
| 81 | 92 | 110 | 64 | 103 | >14,000 | 37 | >152 |
| 82 | 327 | 109 | 354 | 97 | >14,000 | 25 | >43 |
| 83 | 136 | 110 | 172 | 117 | >14,000 | 31 | >103 |
| 84 | 94 | 108 | 254 | 95 | >14,000 | 22 | >149 |
| 85 | 181 | 105 | 117 | 98 | >14,000 | 35 | >77 |
| 86 | 205 | 107 | 176 | 112 | >14,000 | 33 | >68 |
| 87 | 414 | 100 | 272 | 98 | >14,000 | 39 | >34 |
| 88 | 203 | 104 | 227 | 101 | >14,000 | 30 | >69 |
| 89 | 289 | 101 | 159 | 108 | >14,000 | 42 | >48 |
| 90 | 200 | 100 | 479 | 103 | >14,000 | 10 | >70 |
| 91 | 223 | 106 | 160 | 101 | >14,000 | 20 | >63 |
| 92 | 170 | 114 | 34 | 112 | >14,000 | 23 | >82 |
| 93 | 338 | 103 | 159 | 111 | >14,000 | 5 | >41 |
| 94 | 198 | 106 | 287 | 107 | >14,000 | 25 | >71 |
| 95 | 274 | 110 | 63 | 125 | >14,000 | 36 | >51 |
| 96 | 129 | 106 | 100 | 117 | >14,000 | 27 | >109 |
| 97 | 357 | 106 | 326 | 101 | >14,000 | 24 | >39 |
| 98 | 97 | 109 | 92 | 106 | >14,000 | 28 | >144 |
| 99 | 118 | 109 | 221 | 114 | >14,000 | 22 | >118 |
| 100 | 327 | 112 | 140 | 118 | >14,000 | 33 | >43 |
| 101 | 72 | 108 | 61 | 111 | >14,000 | 18 | >194 |
| 102 | 59 | 108 | 121 | 120 | >14,000 | 35 | >236 |
| 103 | 148 | 107 | 86 | 100 | >14,000 | 22 | >95 |
| 104 | 37 | 108 | 50 | 118 | >14,000 | 7 | >380 |
| 105 | 52 | 107 | 33 | 120 | >14,000 | 50 | >268 |
| 106 | 57 | 106 | 178 | 113 | >14,000 | 47 | >245 |
| 107 | 69 | 108 | 106 | 124 | 10,019 | 63 | 146 |
| 108 | 99 | 106 | 111 | 112 | >14,000 | 24 | >142 |
| 109 | 111 | 107 | 66 | 114 | >14,000 | 42 | >127 |
| 110 | 173 | 106 | 72 | 99 | >14,000 | 20 | >81 |
| 111 | 95 | 106 | 74 | 99 | >14,000 | 15 | >147 |
| 112 | 226 | 105 | 46 | 115 | >14,000 | 26 | >62 |
| 113 | 190 | 113 | 99 | 93 | >14,000 | 37 | >74 |
| 114 | 282 | 106 | 640 | 110 | >14,000 | 29 | >50 |
| 115 | 217 | 108 | 247 | 114 | 10,344 | 63 | 48 |
| 116 | 87 | 110 | 73 | 107 | >14,000 | 43 | >160 |
| 117 | 104 | 108 | 115 | 108 | >14,000 | 28 | >135 |
| 118 | 417 | 96 | 299 | 105 | >14,000 | 24 | >34 |
| 119 | 116 | 107 | 54 | 111 | >14,000 | 10 | >120 |
| 120 | 148 | 108 | 55 | 121 | >14,000 | 6 | >95 |
| 121 | 53 | 107 | 40 | 125 | >14,000 | 9 | >263 |
| 122 | 61 | 106 | 54 | 122 | >14,000 | 16 | >230 |
| 123 | 131 | 107 | 110 | 121 | >14,000 | 4 | >107 |
| 124 | 195 | 103 | 163 | 121 | >14,000 | 15 | >72 |
| 125 | 263 | 107 | 98 | 138 | >14,000 | 7 | >53 |
| 126 | 140 | 107 | 117 | 129 | >14,000 | 9 | >100 |
| 127 | 126 | 105 | 48 | 121 | >14,000 | 8 | >111 |
| 128 | 101 | 106 | 136 | 124 | >14,000 | 10 | >139 |
| 129 | 221 | 107 | 187 | 117 | >14,000 | 19 | >63 |
| 130 | 19 | 106 | 22 | 119 | >14,000 | 19 | >738 |
| 131 | 31 | 102 | 28 | 129 | >14,000 | 0 | >450 |
| 132 | 20 | 107 | 66 | 142 | >14,000 | 10 | >693 |
| 133 | 25 | 106 | 42 | 145 | >14,000 | 16 | >569 |
| 134 | 13 | 107 | 14 | 144 | >14,000 | 0 | >1,066 |
| 135 | 17 | 110 | 27 | 148 | >14,000 | 5 | >847 |
| 136 | 38 | 110 | 24 | 144 | >14,000 | 11 | >368 |
| 137 | 26 | 108 | 19 | 123 | >14,000 | 1 | >547 |
| 138 | 173 | 108 | 45 | 120 | >14,000 | 9 | >81 |
| 139 | 67 | 105 | 27 | 170 | >14,000 | 8 | >210 |
| 140 | 30 | 105 | 16 | 132 | >14,000 | 0 | >474 |
| 141 | 34 | 112 | 55 | 136 | >14,000 | 9 | >418 |
| 142 | 22 | 108 | 145 | 139 | >14,000 | 4 | >635 |
| 143 | 31 | 110 | 66 | 153 | >14,000 | 8 | >445 |
| 144 | 106 | 103 | 23 | 145 | >14,000 | 4 | >133 |
| 145 | 57 | 111 | 37 | 145 | >14,000 | 9 | >247 |
| 146 | 47 | 112 | 38 | 133 | >14,000 | 8 | >296 |
| 147 | 12 | 110 | 43 | 137 | >14,000 | 2 | >1,121 |
| 148 | 33 | 110 | 75 | 136 | >14,000 | 10 | >423 |
| 149 | 67 | 109 | 71 | 144 | >14,000 | 15 | >209 |
| 150 | 182 | 100 | 96 | 137 | >14,000 | 0 | >77 |
| 151 | 71 | 108 | 31 | 113 | >14,000 | 10 | >196 |
| 152 | 152 | 108 | 152 | 120 | >14,000 | 32 | >92 |
| 153 | 84 | 107 | 37 | 124 | >14,000 | 19 | >166 |
| 154 | 147 | 100 | 225 | 123 | >14,000 | 26 | >95 |
| 155 | 176 | 100 | 188 | 116 | - | - | - |
| 156 | 101 | 103 | 39 | 116 | - | - | - |
| 157 | 116 | 99 | 65 | 119 | - | - | - |
| 158 | 74 | 104 | 175 | 109 | - | - | - |
| 159 | 15 | 101 | 47 | 145 | >14,000 | 21 | >937 |
| 160 | 23 | 98 | 131 | 145 | >14,000 | 16 | >615 |
| 161 | 62 | 102 | 37 | 129 | >14,000 | 10 | >225 |
| 162 | 27 | 107 | 24 | 133 | >14,000 | 8 | >521 |
| 163 | 15 | 110 | 112 | 137 | >14,000 | 7 | >921 |
| 164 | 75 | 118 | 25 | 142 | >14,000 | 2 | >187 |
| 165 | 42 | 107 | 28 | 133 | >14,000 | 10 | >332 |
| 166 | 87 | 108 | 95 | 134 | >14,000 | 27 | >160 |
| 167 | 13 | 111 | 8 | 148 | >14,000 | 6 | >1,042 |
| 168 | 62 | 110 | 19 | 118 | >14,000 | 33 | >226 |
| 169 | 88 | 104 | 36 | 120 | >14,000 | 7 | >159 |
| 170 | 15 | 109 | 52 | 142 | >14,000 | 0 | >930 |
| 171 | 170 | 107 | 160 | 130 | >14,000 | 0 | >82 |
| 172 | 79 | 100 | 118 | 120 | >14,000 | 6 | >177 |
| 173 | 156 | 105 | 129 | 127 | >14,000 | 14 | >89 |
| 174 | 204 | 100 | 473 | 99 | >14,000 | 16 | >69 |
| 175 | 45 | 110 | 30 | 128 | >14,000 | 11 | >308 |
| 176 | 179 | 101 | 44 | 112 | >14,000 | 20 | >78 |
| 177 | 299 | 108 | 161 | 126 | >14,000 | 0 | >47 |
| 178 | 110 | 105 | 131 | 126 | >14,000 | 16 | >127 |
| 179 | 224 | 104 | 42 | 140 | >14,000 | 10 | >62 |
| 180 | 60 | 111 | 54 | 108 | >14,000 | 48 | >233 |

Table 18 shows the activities of Examples 1 to 180 for the degradation of Cyclin E1, inhibition of pRB, and degradation of GSPT1. A smaller DC₅₀ value in the Cyclin E1 assay indicated greater activity for the compound in the Cyclin E1 assay. A larger DC₅₀ value in the Cyclin E1 assay indicated less activity for the compound in the Cyclin E1 assay. A smaller IC₅₀ value in the pRB assay indicated greater activity for the compound in the pRB assay. A larger IC₅₀ value in the pRB assay indicated less activity for the compound in the pRB assay. A smaller DC₅₀ value in the GSPT1 assay indicated greater activity for the compound in the GSPT1 assay. A larger DC₅₀ value in the GSPT1 assay indicated less activity for the compound in the GSPT1 assay. A larger value for the ratio of GSPT1 DC₅₀/Cyclin E1 DC₅₀ indicated that a compound had greater selectivity for activity towards Cyclin E1 degradation than activity towards GSPT1 degradation, and thus was more selective over GSPT1 degradation.

In the study reported in Table 18, the exemplified compounds, Examples 1-154 and 159-180, showed selectivity of at least 9 for activity towards degradation of Cyclin E1 over activity towards degradation of GSPT1.

The compounds of the present invention possess activity as degraders of Cyclin E1 and are selective over degradation of GSPT1. Thus, the compounds of the present invention may be used in the treatment of cancer and other proliferative diseases while reducing or minimizing side effects associated with degradation of GSPT1. Thus, the present invention fills the foregoing need by providing compounds that are useful to decrease the level of Cyclin E1 and are selective against degradation of GSPT1.

### Cellular Degradation of Cyclin E1 and Inhibition of RB1 phosphorylation (Western blot)

The assay measured the ability of the compound to degrade Cyclin E1, inhibit RB phosphorylation, and to determine the selectivity against degradation of GSPT1 in cancer cells in vitro. HCC1569 cells (ATCC#CRL-2330) were cultured in RPMI media (Gibco, catalog A104910) supplemented with 10% FBS (Gibco, catalog 10091148). Cells were plated in tissue culture-treated 6 well plates at a density of 0.5 million cells per well in 2 mL media. The cells were then incubated overnight in a cell culture incubator (5% CO₂ and 37 °C) and allowed to attach to the plate. The following day cells were dosed with compounds at the desired concentrations. Compounds were first resuspended in DMSO to make 10 mM stock, and 20 µM stock was prepared by diluting 10 mM stock in culture media. Cells were treated with different concentrations of compound (10 µM to 0.05 µM range) and DMSO was used as a negative control. After dosing with compounds, the cell plates were incubated at 37 °C and 5% CO₂ for 24 h. After 24 h, the cell plates were placed on ice, the growth media was carefully removed and washed once with cold 1X PBS. Cells were lysed by adding 50 µL of ice-cold RIPA buffer containing Protease and Phosphatase inhibitors. Cells were scrapped using a cell scrapper and the extract was transferred into a microcentrifuge tube and kept on ice. For complete lysis, the cell extract was sonicated twice for 10 seconds at 20 kHz. Tubes were spun at 13K RPM for 15 min at 4 °C to remove the cell debris. The supernatant was then transferred into new microcentrifuge tubes and kept on ice. Total protein concentration was measured using a BCA assay kit (Thermo Scientific, cat# PI23227). Next, 100 µL of 1µg/µL protein sample was prepared by mixing the desired amount of cell lysate, 3X LDS sample buffer (Invitrogen, cat # B0007 added DTT as a reducing agent), and RIPA buffer. Protein samples were reduced and denatured by boiling them at 90 °C for 5 min. Next, 10 µg of total protein was loaded onto 4 to 20% TGX gels (From Bio-Rad) along with a prestained molecular weight marker. Gel electrophoresis was done at 90 to 130V, then the separated proteins were electrotransferred onto the 0.2 µm nitrocellulose membrane (Bio-Rad cat#1704159). Blocking buffer was prepared in 1X TBST with 5% w/v nonfat dry milk. After transfer, the membrane blocking was performed by incubating the membrane with 25 mL blocking buffer for 1 hr. After blocking, blots were washed once with 1X TBST for 5 min and then incubated with the primary antibody diluted (1:2,000 for Cyclin E1 and GSPT1; 1:10,000 for β-Actin) in blocking buffer overnight with gentle agitation on a shaker. The following day, incubated blots were washed three times for 5 min each in 15 mL of 1X TBST and then incubated with species-appropriate HRP-conjugated secondary antibody diluted in blocking buffer (1:10,000 dilution) for 1 hr at room temperature with gentle agitation. Blots were then washed three times for 5 min in 1X TBST before proceeding with protein detection. Protein was then detected by incubating blots with 2 mL of HRP detection reagent (Thermo scientific cat# A38556) for one minute at room temperature. Excess reagent was drained and gently removed on a Kimwipe and the chemiluminescence signal was detected using Fluorchem imaging system. List of primary antibodies: Cyclin E1-Cell Signaling, catalog 20808; GSPT1-Cell Signaling, catalog 14990; pS807/811 Rb1-Cell Signaling, catalog 8516; β-Actin-Cell Signaling, catalog 5125. Relative quantification of western blots was performed using ImageJ software, data was then normalized to loading control (β-Actin) first and then to the DMSO-treated sample. Nonlinear regression and sigmoidal dose-response curves are used to calculate the DC₅₀ or EC₅₀ with GraphPad Prism software.

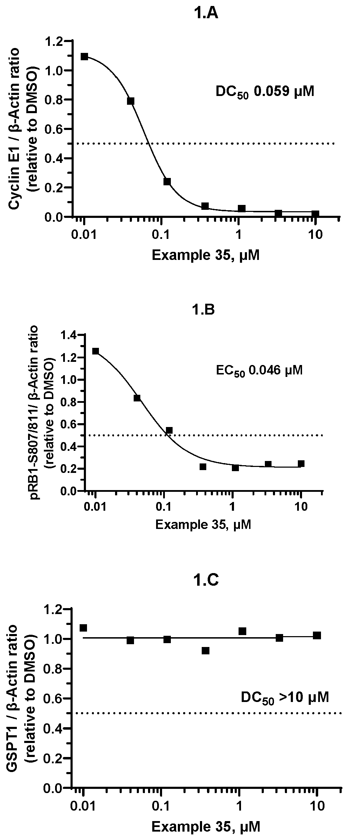

Figure 1 shows that Example 35 (i) induced degradation of Cyclin E1 protein in a dose dependent manner (Fig.1A); (ii) inhibited RB1 phosphorylation (Fig.1B) in a dose dependent manner; and (iii) did not significantly degrade GSPT1 protein up to a concentration of 10 µM (Fig.1C) in HCC1569 cancer cells.

### In vivo Xenograft Tumor Efficacy Studies

The anti-cancer efficacy of Example 35 was evaluated in xenograft models by measuring the reduction in tumor volume in animals treated with Example 35 compared to vehicle control animals. First, 5-10 x 10⁶ tumor cells/animal in a 1:1 Matrigel^{®} mix (0.2 mL total volume) were injected subcutaneously into female NSG mice (for NIHOVCAR3) and SCID mice (for HCC1569). Both strains of mice were purchased from Jackson Laboratory. Once tumors reached ~150-200 mm³, animals were randomized into groups of 8-10 animals/treatment arm for efficacy studies. Example 35 was administered via oral gavage (PO) at indicated doses and regimens (Table 7). Tumor growth and body weight were measured twice/week during the course of the study to evaluate efficacy and signs of toxicity.

Example 35 was formulated in 10% ethanol, 10% Vitamin E - TPGS, 30% propylene glycol, 50% PEG400 and administered by oral gavage (final volume 0.2 mL) at the doses indicated in Table 7. Example 35 was formulated on a weekly basis and stored at room temperature. Vehicle was administered to the control group according to the schedules used for Example 35 dosed at a volume of 0.2 mL/mouse.

The efficacy for each treated arm at different dose levels were calculated using the following formula: %ΔT/ΔC = [(TV_{T}-TV_{T0})/(TV_{C}-TV_{C0})] x 100%. Here, TV_{T} and TV_{C} are mean endpoint tumor volumes in the treated and control groups, respectively. TV_{T0} and TV_{C0} are mean baseline tumor volumes in those groups. The percentage of tumor growth inhibition was calculated from the formula: %TGI = [100% - (%ΔT/ΔC)]. When %ΔT/ΔC becomes negative, the following formula was used to calculate the percentage of tumor growth regression: %TGR = [(TV_{T}-TV_{T0})/TV_{T0}] x 100%.

Example 35 was evaluated for *in vivo* single-agent efficacy against the HCC1569 breast carcinoma xenograft model and NIHOVCAR3 ovarian adenocarcinoma xenograft model. A summary of the study and results are shown in Table 19.

**TABLE 19**

| Model | Histology | Example 35 dose (mg/kg) | Schedule | Tumor efficacy |
|---|---|---|---|---|
| HCC1569 | Breast | 10 | QDx28 | 70% TGI |
| | | 30 | QDx28 | 100% TGI |
| | | 100 | QDx28 | 100% TGI |
| NIHOVCAR3 | Ovary | 30 | QDx28 | 67% TGI |
| | | 100 | QDx28 | 90% TGI |
| | | 250 | QDx28 | -11% TGR |
| MKN1 | Stomach | 3 | QDx28 | 58% TGI |
| | | 10 | QDx28 | 95% TGI |
| | | 30 | QDx28 | -4% TGR |

| | | | | |
|---|---|---|---|---|
| TGI: tumor growth inhibition; TGR: tumor growth regression | | | | |

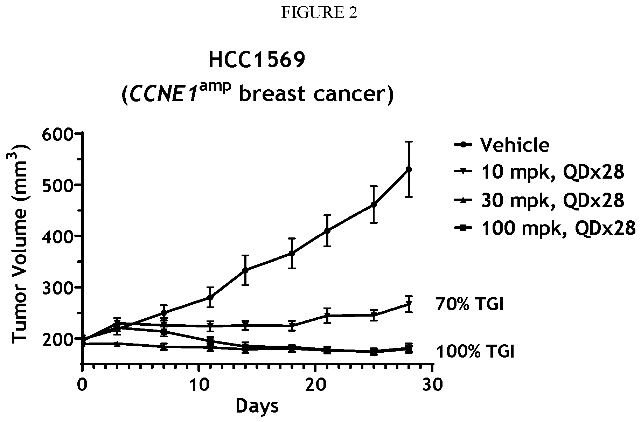

In the study reported in Table 19 and FIG. 2, Example 35 was active against all three xenograft models in mice. Example 35 demonstrated dose-dependent anti-tumor activity in the HCC1569, OVCAR3, and MKN1 xenografts upon repeated dosing. Example 35 had tumor growth inhibition (TGI) values of 70% at a dosage of 10 mpk, QDx28; and 100% at dosages of 30 mpk and 100 mpk, QDx28 in the HCC1569 breast carcinoma xenograft model. Example 35 had tumor growth inhibition (TGI) values of 67% at a dosage of 30 mpk, QDx28; and 90% at a dosage of 100 mpk QDx28; and tumor growth regression (TGR) of -11% at a dosage of 250 mpk QDx28 in the NIHOVCAR3 ovarian adenocarcinoma xenograft model. Example 35 had tumor growth inhibition (TGI) values of 58% at a dosage of 3 mpk, QDx28; and 95% at a dosage of 30 mpk QDx28; and tumor growth regression (TGR) of -4% at a dosage of 30 mpk QDx28 in the MKN1 gastric carcinoma xenograft model.

## Claims

1. A compound of Formula (I): 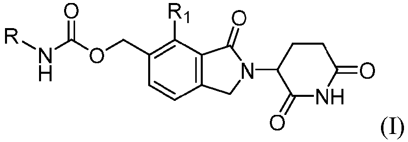 or stereoisomers, tautomers, or salts thereof, wherein:
R is 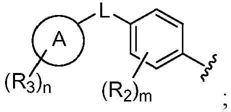
L is -O-, -CH₂-, -C(CH₃)₂-, -CF₂-, -NH-, -N(CH₃)-, or ;
Ring A is phenyl, naphthalenyl, or benzo[b]thiophenyl;
R₁ is -OCH₃, -OCH₂CH₃, -OCH(CH₃)₂, -OCH₂CH(CH₃)₂, -OCHF₂, -OCH₂C(CH₃)₂OH, -OCH₂CH₂OCH₃, -OCH₂C(O)OCH₃, -OCH₂C(O)NH₂, -OCH₂C(O)N(CH₃)₂, -OCH₂C(O)N(CH₃)(CH₂CH₃), -OCH₂CH₂CH₂(phenyl), -OCH₂C(O)(morpholinyl), -O(C₄₋₆ cycloalkyl), -O(hydroxycyclohexyl), -O(oxetanyl), -O(tetrahydrofuranyl), -O(tetrahydropyranyl), -O(oxepanyl), -O(phenyl), -O(pyridinyl), or -O(acetylpiperidinyl);
R₂ is F, Cl, -CH₃, or -OCH₃;
each R₃ is independently F, Cl, Br, -CH₃, -CD₃, -CHF₂, -CF₃, -OCH₃, or cyclopropyl; m is zero or 1; and
n is zero, 1, 2, or 3.

2. The compound according to claim 1, or stereoisomers, tautomers, or salts thereof, wherein
R is 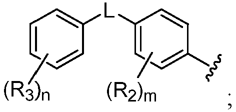 R₁ is -OCH₃, -OCH₂CH₃, -OCH(CH₃)₂, -OCHF₂, or -O(phenyl); and each R₃ is independently F, Cl, Br, -CH₃, or -OCH₃.

3. The compound according to claim 1, or stereoisomers, tautomers, or salts thereof, wherein Ring A is phenyl; and/or L is -O-.

4. The compound according to claim 1, or stereoisomers, tautomers, or salts thereof, wherein R₁ is -OCH₃, -OCH₂CH₃, or -OCH(CH₃)₂.

5. The compound according to claim 1, or stereoisomers, tautomers, or salts thereof, wherein Ring A is phenyl;
L is -O-; and
R₁ is -OCH₃.

6. The compound according to claim 1, or stereoisomers or tautomers thereof, having the structure: 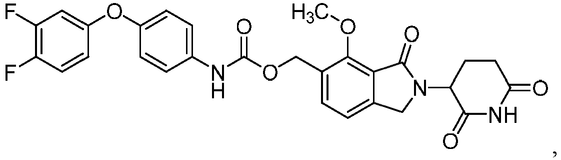 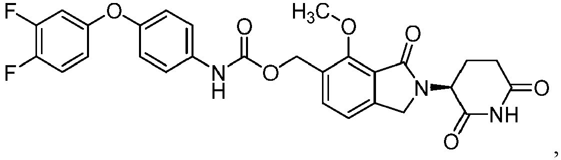 or

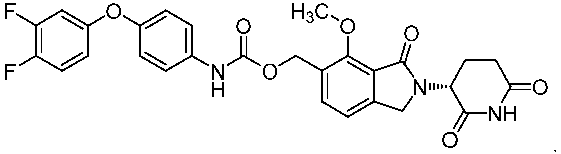

7. The compound according to claim 1, or stereoisomers or tautomers thereof, having the structure: 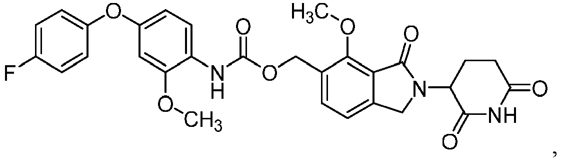 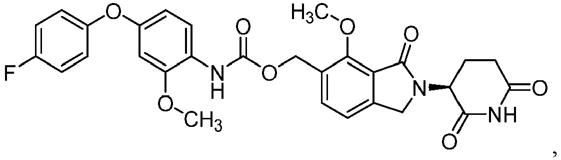 or

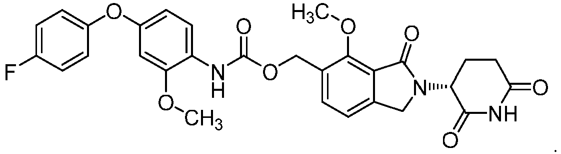

8. The compound according to claim 1, or stereoisomers or tautomers thereof, having the structure: 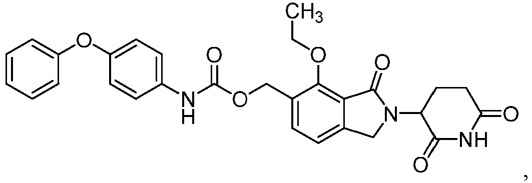 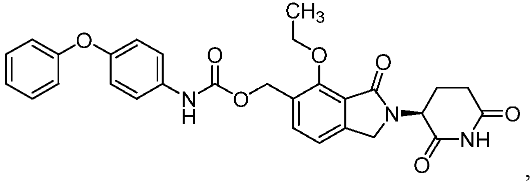 or

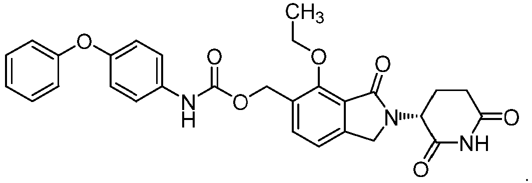

9. The compound according to claim 1, or tautomers or pharmaceutically acceptable salts thereof, wherein said compound is:
[2-(2,6-dioxopiperidin-3-yl)-4-methoxy-3-oxo-2,3-dihydro-1H-isoindol-5-yl]methyl N-[4-(3,4-difluorophenoxy)phenyl]carbamate (1);
[2-(2,6-dioxopiperidin-3-yl)-4-methoxy-3-oxo-2,3-dihydro-1H-isoindol-5-yl]methyl N-(4-phenoxyphenyl)carbamate (2);
[2-(2,6-dioxopiperidin-3-yl)-4-methoxy-3-oxo-2,3-dihydro-1H-isoindol-5-yl]methyl N-[4-(3-chlorophenoxy)phenyl]carbamate (3);
[2-(2,6-dioxopiperidin-3-yl)-4-methoxy-3-oxo-2,3-dihydro-1H-isoindol-5-yl]methyl N-[4-(3-fluorophenoxy)phenyl]carbamate (4);
[2-(2,6-dioxopiperidin-3-yl)-4-methoxy-3-oxo-2,3-dihydro-1H-isoindol-5-yl]methyl N-[4-(2,4-difluorophenoxy)phenyl]carbamate (5);
[2-(2,6-dioxopiperidin-3-yl)-4-methoxy-3-oxo-2,3-dihydro-1H-isoindol-5-yl]methyl N-{ 4-[(3-fluorophenyl)methyl]phenyl } carbamate (6);
[2-(2,6-dioxopiperidin-3-yl)-4-methoxy-3-oxo-2,3-dihydro-1H-isoindol-5-yl]methyl N-[4-(1-phenylcyclopropyl)phenyl]carbamate (7);
[2-(2,6-dioxopiperidin-3-yl)-4-methoxy-3-oxo-2,3-dihydro-1H-isoindol-5-yl]methyl N-{4-[(3-fluorophenyl)(methyl)amino]phenyl}carbamate (8);
[2-(2,6-dioxopiperidin-3-yl)-4-methoxy-3-oxo-2,3-dihydro-1H-isoindol-5-yl]methyl N-{4-[(3-fluorophenyl)amino]phenyl}carbamate (9);
[2-(2,6-dioxopiperidin-3-yl)-4-methoxy-3-oxo-2,3-dihydro-1H-isoindol-5-yl]methyl N-[4-(3-bromophenoxy)phenyl]carbamate (10);
[2-(2,6-dioxopiperidin-3-yl)-4-methoxy-3-oxo-2,3-dihydro-1H-isoindol-5-yl]methyl N-[4-(3-methylphenoxy)phenyl]carbamate (11);
[2-(2,6-dioxopiperidin-3-yl)-4-methoxy-3-oxo-2,3-dihydro-1H-isoindol-5-yl]methyl N-[4-(3,5-dimethylphenoxy)phenyl]carbamate (12);
[2-(2,6-dioxopiperidin-3-yl)-4-methoxy-3-oxo-2,3-dihydro-1H-isoindol-5-yl]methyl N-[4-(3,5-difluorophenoxy)phenyl]carbamate (13);
[2-(2,6-dioxopiperidin-3-yl)-4-methoxy-3-oxo-2,3-dihydro-1H-isoindol-5-yl]methyl N-[4-(3-chloro-4-fluorophenoxy)phenyl]carbamate (14);
[2-(2,6-dioxopiperidin-3-yl)-4-methoxy-3-oxo-2,3-dihydro-1H-isoindol-5-yl]methyl N-[2-fluoro-4-(3,4,5-trifluorophenoxy)phenyl]carbamate (15);
[2-(2,6-dioxopiperidin-3-yl)-4-methoxy-3-oxo-2,3-dihydro-1H-isoindol-5-yl]methyl N-[2-fluoro-4-(3-fluorophenoxy)phenyl]carbamate (16);
[2-(2,6-dioxopiperidin-3-yl)-4-methoxy-3-oxo-2,3-dihydro-1H-isoindol-5-yl]methyl N-{ 4-[(3,4-difluorophenyl)methyl]phenyl } carbamate (17);
[2-(2,6-dioxopiperidin-3-yl)-4-methoxy-3-oxo-2,3-dihydro-1H-isoindol-5-yl]methyl N-{4-[(3,4-difluorophenyl)methyl]-2-methylphenyl}carbamate (18);
[2-(2,6-dioxopiperidin-3-yl)-4-methoxy-3-oxo-2,3-dihydro-1H-isoindol-5-yl]methyl N-{4-[(3,4,5-trifluorophenyl)methyl]phenyl}carbamate (19);
[2-(2,6-dioxopiperidin-3-yl)-4-methoxy-3-oxo-2,3-dihydro-1H-isoindol-5-yl]methyl N-{2-fluoro-4-[(3-fluorophenyl)methyl]phenyl}carbamate (20);
[2-(2,6-dioxopiperidin-3-yl)-4-methoxy-3-oxo-2,3-dihydro-1H-isoindol-5-yl]methyl N-{4-[(3-chloro-4-fluorophenyl)methyl]phenyl}carbamate (21);
[2-(2,6-dioxopiperidin-3-yl)-4-methoxy-3-oxo-2,3-dihydro-1H-isoindol-5-yl]methyl N-[4-(2,3-difluorophenoxy)-2-fluorophenyl]carbamate (22);
[2-(2,6-dioxopiperidin-3-yl)-4-methoxy-3-oxo-2,3-dihydro-1H-isoindol-5-yl]methyl N-[4-(3,4-difluorophenoxy)-2-fluorophenyl]carbamate (23);
[2-(2,6-dioxopiperidin-3-yl)-4-methoxy-3-oxo-2,3-dihydro-1H-isoindol-5-yl]methyl N-[4-(3,5-difluorophenoxy)-2-fluorophenyl]carbamate (24);
[2-(2,6-dioxopiperidin-3-yl)-4-methoxy-3-oxo-2,3-dihydro-1H-isoindol-5-yl]methyl N-[2-chloro-4-(3,4,5-trifluorophenoxy)phenyl]carbamate (25);
[2-(2,6-dioxopiperidin-3-yl)-4-methoxy-3-oxo-2,3-dihydro-1H-isoindol-5-yl]methyl N-[4-(3-chlorophenoxy)-2-fluorophenyl]carbamate (26);
[2-(2,6-dioxopiperidin-3-yl)-4-methoxy-3-oxo-2,3-dihydro-1H-isoindol-5-yl]methyl N-[2-chloro-4-(3-fluorophenoxy)phenyl]carbamate (27);
[2-(2,6-dioxopiperidin-3-yl)-4-methoxy-3-oxo-2,3-dihydro-1H-isoindol-5-yl]methyl N-[2-fluoro-4-(4-fluorophenoxy)phenyl]carbamate (28);
[2-(2,6-dioxopiperidin-3-yl)-4-methoxy-3-oxo-2,3-dihydro-1H-isoindol-5-yl]methyl N-{4-[difluoro(phenyl)methyl]phenyl}carbamate (29);
[2-(2,6-dioxopiperidin-3-yl)-4-methoxy-3-oxo-2,3-dihydro-1H-isoindol-5-yl]methyl N-[4-(2,4-difluorophenoxy)-2-fluorophenyl]carbamate (30);
[2-(2,6-dioxopiperidin-3-yl)-4-methoxy-3-oxo-2,3-dihydro-1H-isoindol-5-yl]methyl N-[4-(2-methoxyphenoxy)phenyl]carbamate (31);
[2-(2,6-dioxopiperidin-3-yl)-4-methoxy-3-oxo-2,3-dihydro-1H-isoindol-5-yl]methyl N-[4-(3-methoxyphenoxy)phenyl]carbamate (32);
[2-(2,6-dioxopiperidin-3-yl)-4-methoxy-3-oxo-2,3-dihydro-1H-isoindol-5-yl]methyl N-[4-(4,5-difluoro-2-methoxyphenoxy)-2-methylphenyl]carbamate (33);
[2-(2,6-dioxopiperidin-3-yl)-4-methoxy-3-oxo-2,3-dihydro-1H-isoindol-5-yl]methyl N-[2-methoxy-4-(2,4,5-trifluorophenoxy)phenyl]carbamate (34);
{2-[(3S)-2,6-dioxopiperidin-3-yl]-4-methoxy-3-oxo-2,3-dihydro-1H-isoindol-5-yl} methyl N-[4-(3,4-difluorophenoxy)phenyl]carbamate (35);
{2-[(3R)-2,6-dioxopiperidin-3-yl]-4-methoxy-3-oxo-2,3-dihydro-1H-isoindol-5-yl} methyl N-[4-(3,4-difluorophenoxy)phenyl]carbamate (36);
[2-(2,6-dioxopiperidin-3-yl)-4-methoxy-3-oxo-2,3-dihydro-1H-isoindol-5-yl]methyl N-[4-(4-fluorophenoxy)-2-methoxyphenyl]carbamate (37);
[2-(2,6-dioxopiperidin-3-yl)-4-methoxy-3-oxo-2,3-dihydro-1H-isoindol-5-yl]methyl N-[4-(3,4-difluorophenoxy)-2-methylphenyl]carbamate (38);
[2-(2,6-dioxopiperidin-3-yl)-4-methoxy-3-oxo-2,3-dihydro-1H-isoindol-5-yl]methyl N-[4-(3-fluorophenoxy)-2-methylphenyl]carbamate (39);
[2-(2,6-dioxopiperidin-3-yl)-4-methoxy-3-oxo-2,3-dihydro-1H-isoindol-5-yl]methyl N-[2-methyl-4-(3,4,5-trifluorophenoxy)phenyl]carbamate (40);
[2-(2,6-dioxopiperidin-3-yl)-4-methoxy-3-oxo-2,3-dihydro-1H-isoindol-5-yl]methyl N-[4-(2-phenylpropan-2-yl)phenyl]carbamate (41);
[2-(2,6-dioxopiperidin-3-yl)-4-methoxy-3-oxo-2,3-dihydro-1H-isoindol-5-yl]methyl N-[4-(2,4-difluorophenoxy)-2-methylphenyl]carbamate (42);
[2-(2,6-dioxopiperidin-3-yl)-4-methoxy-3-oxo-2,3-dihydro-1H-isoindol-5-yl]methyl N-[2-fluoro-4-(2,4,5-trifluorophenoxy)phenyl]carbamate (43);
[2-(2,6-dioxopiperidin-3-yl)-4-methoxy-3-oxo-2,3-dihydro-1H-isoindol-5-yl]methyl N-[4-(2,4,5-trifluorophenoxy)phenyl]carbamate (44);
[2-(2,6-dioxopiperidin-3-yl)-4-methoxy-3-oxo-2,3-dihydro-1H-isoindol-5-yl]methyl N-[2-methyl-4-(2,4,5-trifluorophenoxy)phenyl]carbamate (45);
[2-(2,6-dioxopiperidin-3-yl)-4-methoxy-3-oxo-2,3-dihydro-1H-isoindol-5-yl]methyl N-[2-chloro-4-(3,4-difluorophenoxy)phenyl]carbamate (46);
[2-(2,6-dioxopiperidin-3-yl)-4-methoxy-3-oxo-2,3-dihydro-1H-isoindol-5-yl]methyl N-[4-(3,4-difluorophenoxy)-2-methoxyphenyl]carbamate (47);
[2-(2,6-dioxopiperidin-3-yl)-4-methoxy-3-oxo-2,3-dihydro-1H-isoindol-5-yl]methyl N-[4-(3-fluorophenoxy)-2-methoxyphenyl]carbamate (48);
[2-(2,6-dioxopiperidin-3-yl)-4-methoxy-3-oxo-2,3-dihydro-1H-isoindol-5-yl]methyl N-[4-(2,4-difluorophenoxy)-2-methoxyphenyl]carbamate (49);
[2-(2,6-dioxopiperidin-3-yl)-4-methoxy-3-oxo-2,3-dihydro-1H-isoindol-5-yl]methyl N-[4-(4-fluoro-2-methoxyphenoxy)-2-methoxyphenyl]carbamate (50);
[2-(2,6-dioxopiperidin-3-yl)-4-methoxy-3-oxo-2,3-dihydro-1H-isoindol-5-yl]methyl N-[4-(4-fluorophenoxy)-2-methylphenyl]carbamate (51);
[2-(2,6-dioxopiperidin-3-yl)-4-ethoxy-3-oxo-2,3-dihydro-1H-isoindol-5-yl]methyl N-(4-phenoxyphenyl)carbamate (52);
[2-(2,6-dioxopiperidin-3-yl)-4-ethoxy-3-oxo-2,3-dihydro-1H-isoindol-5-yl]methyl N-[4-(3,4-difluorophenoxy)phenyl]carbamate (53);
[2-(2,6-dioxopiperidin-3-yl)-4-ethoxy-3-oxo-2,3-dihydro-1H-isoindol-5-yl]methyl N-[4-(3-chlorophenoxy)phenyl]carbamate (54);
[2-(2,6-dioxopiperidin-3-yl)-4-ethoxy-3-oxo-2,3-dihydro-1H-isoindol-5-yl]methyl N-[4-(3-fluorophenoxy)phenyl]carbamate (55);
[2-(2,6-dioxopiperidin-3-yl)-3-oxo-4-(propan-2-yloxy)-2,3-dihydro-1H-isoindol-5-yl] methyl N-[4-(3,4-difluorophenoxy)phenyl]carbamate (56);
[2-(2,6-dioxopiperidin-3-yl)-3-oxo-4-(propan-2-yloxy)-2,3-dihydro-1H-isoindol-5-yl] methyl N-(4-phenoxyphenyl)carbamate (57);
[2-(2,6-dioxopiperidin-3-yl)-3-oxo-4-(propan-2-yloxy)-2,3-dihydro-1H-isoindol-5-yl] methyl N-[4-(3-chlorophenoxy)phenyl]carbamate (58);
[2-(2,6-dioxopiperidin-3-yl)-3-oxo-4-(propan-2-yloxy)-2,3-dihydro-1H-isoindol-5-yl] methyl N-[4-(4-fluorophenoxy)phenyl]carbamate (59);
[4-(difluoromethoxy)-2-(2,6-dioxopiperidin-3-yl)-3-oxo-2,3-dihydro-1H-isoindol-5-yl] methyl N-(4-phenoxyphenyl)carbamate (60);
[4-(difluoromethoxy)-2-(2,6-dioxopiperidin-3-yl)-3-oxo-2,3-dihydro-1H-isoindol-5-yl] methyl N-[4-(3,4-difluorophenoxy)phenyl]carbamate (61);
[2-(2,6-dioxopiperidin-3-yl)-3-oxo-4-phenoxy-2,3-dihydro-1H-isoindol-5-yl]methyl N-(4-phenoxyphenyl)carbamate (62);
[2-(2,6-dioxopiperidin-3-yl)-3-oxo-4-phenoxy-2,3-dihydro-1H-isoindol-5-yl]methyl N-[4-(3,4-difluorophenoxy)phenyl]carbamate (63);
[2-(2,6-dioxopiperidin-3-yl)-3-oxo-4-phenoxy-2,3-dihydro-1H-isoindol-5-yl]methyl N-[4-(3-fluorophenoxy)phenyl]carbamate (64);
[2-(2,6-dioxopiperidin-3-yl)-3-oxo-4-phenoxy-2,3-dihydro-1H-isoindol-5-yl]methyl N-[4-(2-fluorophenoxy)phenyl]carbamate (65);
[2-(2,6-dioxopiperidin-3-yl)-3-oxo-4-phenoxy-2,3-dihydro-1H-isoindol-5-yl]methyl N-[4-(2-chlorophenoxy)phenyl]carbamate (66);
[2-(2,6-dioxopiperidin-3-yl)-3-oxo-4-phenoxy-2,3-dihydro-1H-isoindol-5-yl]methyl N-[4-(2,4-difluorophenoxy)phenyl]carbamate (67);
[2-(2,6-dioxopiperidin-3-yl)-3-oxo-4-phenoxy-2,3-dihydro-1H-isoindol-5-yl]methyl N-[4-(2,3-difluorophenoxy)phenyl]carbamate (68);
[2-(2,6-dioxopiperidin-3-yl)-3-oxo-4-phenoxy-2,3-dihydro-1H-isoindol-5-yl]methyl N-[4-(3,4-difluorophenoxy)-2-fluorophenyl]carbamate (69);
[2-(2,6-dioxopiperidin-3-yl)-3-oxo-4-phenoxy-2,3-dihydro-1H-isoindol-5-yl]methyl N-[4-(2-chloro-4-fluorophenoxy)phenyl]carbamate (70);
[2-(2,6-dioxopiperidin-3-yl)-4-methoxy-3-oxo-2,3-dihydro-1H-isoindol-5-yl]methyl N-[4-(4-chloro-3-fluorophenoxy)phenyl]carbamate (71);
[2-(2,6-dioxopiperidin-3-yl)-4-methoxy-3-oxo-2,3-dihydro-1H-isoindol-5-yl]methyl N-[2-methoxy-4-(3,4,5-trifluorophenoxy)phenyl]carbamate (72);
[2-(2,6-dioxopiperidin-3-yl)-4-methoxy-3-oxo-2,3-dihydro-1H-isoindol-5-yl]methyl N-[4-(4,5-difluoro-2-methylphenoxy)-2-methoxyphenyl]carbamate (73);
[2-(2,6-dioxopiperidin-3-yl)-4-methoxy-3-oxo-2,3-dihydro-1H-isoindol-5-yl]methyl N-[4-(4,5-difluoro-2-methylphenoxy)-2-methylphenyl]carbamate (74);
[2-(2,6-dioxopiperidin-3-yl)-4-methoxy-3-oxo-2,3-dihydro-1H-isoindol-5-yl]methyl N-[4-(4-fluoro-2-methylphenoxy)-2-methoxyphenyl]carbamate (75);
[2-(2,6-dioxopiperidin-3-yl)-4-methoxy-3-oxo-2,3-dihydro-1H-isoindol-5-yl]methyl N-[4-(4-fluoro-2-methylphenoxy)-2-methylphenyl]carbamate (76);
[2-(2,6-dioxopiperidin-3-yl)-4-methoxy-3-oxo-2,3-dihydro-1H-isoindol-5-yl]methyl N-[4-(3,4,5-trifluorophenoxy)phenyl]carbamate (77);
[2-(2,6-dioxopiperidin-3-yl)-4-methoxy-3-oxo-2,3-dihydro-1H-isoindol-5-yl]methyl N-[4-(4,5-difluoro-2-methylphenoxy)phenyl]carbamate (78);
[2-(2,6-dioxopiperidin-3-yl)-4-methoxy-3-oxo-2,3-dihydro-1H-isoindol-5-yl]methyl N-[4-(2-fluorophenoxy)phenyl]carbamate (79);
[2-(2,6-dioxopiperidin-3-yl)-4-methoxy-3-oxo-2,3-dihydro-1H-isoindol-5-yl]methyl N-[4-(2-chlorophenoxy)phenyl]carbamate (80);
[2-(2,6-dioxopiperidin-3-yl)-4-methoxy-3-oxo-2,3-dihydro-1H-isoindol-5-yl]methyl N-[4-(4-fluoro-2-methylphenoxy)phenyl]carbamate (81);
[2-(2,6-dioxopiperidin-3-yl)-4-methoxy-3-oxo-2,3-dihydro-1H-isoindol-5-yl]methyl N-[4-(4-fluoro-3-methylphenoxy)-2-methylphenyl]carbamate (82);
[2-(2,6-dioxopiperidin-3-yl)-4-methoxy-3-oxo-2,3-dihydro-1H-isoindol-5-yl]methyl N-[4-(3-chloro-4-fluorophenoxy)-2-methylphenyl]carbamate (83);
[2-(2,6-dioxopiperidin-3-yl)-4-methoxy-3-oxo-2,3-dihydro-1H-isoindol-5-yl]methyl N-[4-(4-fluorophenoxy)phenyl]carbamate (84);
[2-(2,6-dioxopiperidin-3-yl)-4-methoxy-3-oxo-2,3-dihydro-1H-isoindol-5-yl]methyl N-{4-[2-(difluoromethyl)phenoxy]phenyl}carbamate (85);
[2-(2,6-dioxopiperidin-3-yl)-4-methoxy-3-oxo-2,3-dihydro-1H-isoindol-5-yl]methyl N-{4-[2-(difluoromethyl)phenoxy]-2-fluorophenyl}carbamate (86);
[2-(2,6-dioxopiperidin-3-yl)-4-methoxy-3-oxo-2,3-dihydro-1H-isoindol-5-yl]methyl N-[4-(2-cyclopropylphenoxy)phenyl]carbamate (87);
[2-(2,6-dioxopiperidin-3-yl)-4-methoxy-3-oxo-2,3-dihydro-1H-isoindol-5-yl]methyl N-{4-[2-(difluoromethyl)phenoxy]-2-methoxyphenyl}carbamate (88);
[2-(2,6-dioxopiperidin-3-yl)-4-methoxy-3-oxo-2,3-dihydro-1H-isoindol-5-yl]methyl N-{4-[2-(difluoromethyl)-4-fluorophenoxy]phenyl}carbamate (89);
[2-(2,6-dioxopiperidin-3-yl)-4-methoxy-3-oxo-2,3-dihydro-1H-isoindol-5-yl]methyl N-{2-chloro-4-[2-(trifluoromethyl)phenoxy]phenyl}carbamate (90);
[2-(2,6-dioxopiperidin-3-yl)-4-methoxy-3-oxo-2,3-dihydro-1H-isoindol-5-yl]methyl N-[4-(4-chloro-3-fluorophenoxy)-2-fluorophenyl]carbamate (91);
[2-(2,6-dioxopiperidin-3-yl)-4-methoxy-3-oxo-2,3-dihydro-1H-isoindol-5-yl]methyl N-{4-[2-(²H3)methylphenoxy]phenyl}carbamate (92);
[2-(2,6-dioxopiperidin-3-yl)-4-methoxy-3-oxo-2,3-dihydro-1H-isoindol-5-yl]methyl N-(2-methoxy-4-phenoxyphenyl)carbamate (93);
[2-(2,6-dioxopiperidin-3-yl)-4-methoxy-3-oxo-2,3-dihydro-1H-isoindol-5-yl]methyl N-[4-(4,5-difluoro-2-methylphenoxy)-2-fluorophenyl]carbamate (94);
[2-(2,6-dioxopiperidin-3-yl)-4-methoxy-3-oxo-2,3-dihydro-1H-isoindol-5-yl]methyl N-[2-fluoro-4-(4-fluoro-2-methylphenoxy)phenyl]carbamate (95);
[2-(2,6-dioxopiperidin-3-yl)-4-methoxy-3-oxo-2,3-dihydro-1H-isoindol-5-yl]methyl N-[4-(4-fluoro-3-methylphenoxy)phenyl]carbamate (96);
[2-(2,6-dioxopiperidin-3-yl)-4-methoxy-3-oxo-2,3-dihydro-1H-isoindol-5-yl]methyl N-[4-(4-fluoro-3-methylphenoxy)-2-methoxyphenyl]carbamate (97);
[2-(2,6-dioxopiperidin-3-yl)-4-methoxy-3-oxo-2,3-dihydro-1H-isoindol-5-yl]methyl N-[4-(3-chloro-4-fluorophenoxy)-2-fluorophenyl]carbamate (98);
[2-(2,6-dioxopiperidin-3-yl)-4-methoxy-3-oxo-2,3-dihydro-1H-isoindol-5-yl]methyl N-[2-fluoro-4-(4-fluoro-3-methylphenoxy)phenyl]carbamate (99);
[2-(2,6-dioxopiperidin-3-yl)-4-methoxy-3-oxo-2,3-dihydro-1H-isoindol-5-yl]methyl N-[4-(3-chloro-4-fluorophenoxy)-2-methoxyphenyl]carbamate (100);
[2-(2,6-dioxopiperidin-3-yl)-4-methoxy-3-oxo-2,3-dihydro-1H-isoindol-5-yl]methyl N-[4-(2-methylphenoxy)phenyl]carbamate (101);
[2-(2,6-dioxopiperidin-3-yl)-4-methoxy-3-oxo-2,3-dihydro-1H-isoindol-5-yl]methyl N-[4-(2-chloro-4-fluorophenoxy)phenyl]carbamate (102);
[2-(2,6-dioxopiperidin-3-yl)-4-methoxy-3-oxo-2,3-dihydro-1H-isoindol-5-yl]methyl N-[4-(2-chloro-4-fluorophenoxy)-2-methylphenyl]carbamate (103);
[2-(2,6-dioxopiperidin-3-yl)-4-methoxy-3-oxo-2,3-dihydro-1H-isoindol-5-yl]methyl N-[4-(2-chloro-4-fluorophenoxy)-2-methoxyphenyl]carbamate (104);
[2-(2,6-dioxopiperidin-3-yl)-4-methoxy-3-oxo-2,3-dihydro-1H-isoindol-5-yl]methyl N-[4-(2-chloro-4-fluorophenoxy)-2-fluorophenyl]carbamate (105);
[2-(2,6-dioxopiperidin-3-yl)-4-methoxy-3-oxo-2,3-dihydro-1H-isoindol-5-yl]methyl N-[4-(2-chloro-5-fluorophenoxy)phenyl]carbamate (106);
[2-(2,6-dioxopiperidin-3-yl)-4-methoxy-3-oxo-2,3-dihydro-1H-isoindol-5-yl]methyl N-[4-(2-chlorophenoxy)-2-fluorophenyl]carbamate (107);
[2-(2,6-dioxopiperidin-3-yl)-4-methoxy-3-oxo-2,3-dihydro-1H-isoindol-5-yl]methyl N-[4-(2-chloro-4,5-difluorophenoxy)-2-methoxyphenyl]carbamate (108);
[2-(2,6-dioxopiperidin-3-yl)-4-methoxy-3-oxo-2,3-dihydro-1H-isoindol-5-yl]methyl N-[4-(2-chloro-4,5-difluorophenoxy)-2-fluorophenyl]carbamate (109);
[2-(2,6-dioxopiperidin-3-yl)-4-methoxy-3-oxo-2,3-dihydro-1H-isoindol-5-yl]methyl N-[4-(2,3-difluorophenoxy)phenyl]carbamate (110);
[2-(2,6-dioxopiperidin-3-yl)-4-methoxy-3-oxo-2,3-dihydro-1H-isoindol-5-yl]methyl N-[4-(2-chloro-3-fluorophenoxy)phenyl]carbamate (111);
[2-(2,6-dioxopiperidin-3-yl)-4-methoxy-3-oxo-2,3-dihydro-1H-isoindol-5-yl]methyl N-[4-(2,6-difluorophenoxy)phenyl]carbamate (112);
[2-(2,6-dioxopiperidin-3-yl)-4-methoxy-3-oxo-2,3-dihydro-1H-isoindol-5-yl]methyl N-[4-(naphthalen-1-yloxy)phenyl]carbamate (113);
[2-(2,6-dioxopiperidin-3-yl)-4-methoxy-3-oxo-2,3-dihydro-1H-isoindol-5-yl]methyl N-[4-(1-benzothiophen-5-yloxy)phenyl]carbamate (114);
[2-(2,6-dioxopiperidin-3-yl)-4-methoxy-3-oxo-2,3-dihydro-1H-isoindol-5-yl]methyl N-[4-(1-benzothiophen-6-yloxy)phenyl]carbamate (115);
[2-(2,6-dioxopiperidin-3-yl)-4-methoxy-3-oxo-2,3-dihydro-1H-isoindol-5-yl]methyl N-[4-(2,3-dichlorophenoxy)-2-fluorophenyl]carbamate (116);
[2-(2,6-dioxopiperidin-3-yl)-4-methoxy-3-oxo-2,3-dihydro-1H-isoindol-5-yl]methyl N-[4-(3,4-dichlorophenoxy)-2-fluorophenyl]carbamate (117);
[2-(2,6-dioxopiperidin-3-yl)-4-methoxy-3-oxo-2,3-dihydro-1H-isoindol-5-yl]methyl N-[4-(3,5-dichlorophenoxy)-2-fluorophenyl]carbamate (118);
[2-(2,6-dioxopiperidin-3-yl)-3-oxo-4-(propan-2-yloxy)-2,3-dihydro-1H-isoindol-5-yl] methyl N-[4-(3,4-difluorophenoxy)-2-fluorophenyl]carbamate (119);
[2-(2,6-dioxopiperidin-3-yl)-3-oxo-4-(propan-2-yloxy)-2,3-dihydro-1H-isoindol-5-yl] methyl N-[4-(2,3-difluorophenoxy)phenyl]carbamate (120);
[2-(2,6-dioxopiperidin-3-yl)-3-oxo-4-(propan-2-yloxy)-2,3-dihydro-1H-isoindol-5-yl] methyl N-[4-(2-fluorophenoxy)phenyl]carbamate (121);
[2-(2,6-dioxopiperidin-3-yl)-3-oxo-4-(propan-2-yloxy)-2,3-dihydro-1H-isoindol-5-yl] methyl N-[4-(2-chlorophenoxy)phenyl]carbamate (122);
[2-(2,6-dioxopiperidin-3-yl)-3-oxo-4-(propan-2-yloxy)-2,3-dihydro-1H-isoindol-5-yl] methyl N-[4-(3-fluorophenoxy)phenyl]carbamate (123);
[2-(2,6-dioxopiperidin-3-yl)-3-oxo-4-(propan-2-yloxy)-2,3-dihydro-1H-isoindol-5-yl] methyl N-[4-(2-chloro-4-fluorophenoxy)phenyl]carbamate (124);
{2-[(3S)-2,6-dioxopiperidin-3-yl]-3-oxo-4-(propan-2-yloxy)-2,3-dihydro-1H-isoindol-5-yl}methyl N-[4-(3,4-difluorophenoxy)phenyl]carbamate (125);
[4-cyclobutoxy-2-(2,6-dioxopiperidin-3-yl)-3-oxo-2,3-dihydro-1H-isoindol-5-yl]methyl N-[4-(3,4-difluorophenoxy)phenyl]carbamate (126);
[4-cyclobutoxy-2-(2,6-dioxopiperidin-3-yl)-3-oxo-2,3-dihydro-1H-isoindol-5-yl]methyl N-[4-(2-chlorophenoxy)phenyl]carbamate (127);
[4-cyclobutoxy-2-(2,6-dioxopiperidin-3-yl)-3-oxo-2,3-dihydro-1H-isoindol-5-yl]methyl N-[4-(2-chloro-4-fluorophenoxy)phenyl]carbamate (128);
[2-(2,6-dioxopiperidin-3-yl)-4-(oxetan-3-yloxy)-3-oxo-2,3-dihydro-1H-isoindol-5-yl] methyl N-[4-(3,4-difluorophenoxy)phenyl]carbamate (129);
[2-(2,6-dioxopiperidin-3-yl)-4-(oxan-4-yloxy)-3-oxo-2,3-dihydro-1H-isoindol-5-yl] methyl N-[4-(3,4-difluorophenoxy)phenyl]carbamate (130);
[2-(2,6-dioxopiperidin-3-yl)-4-(oxan-4-yloxy)-3-oxo-2,3-dihydro-1H-isoindol-5-yl] methyl N-[4-(2-chlorophenoxy)phenyl]carbamate (131);
[2-(2,6-dioxopiperidin-3-yl)-4-(oxan-4-yloxy)-3-oxo-2,3-dihydro-1H-isoindol-5-yl] methyl N-[4-(3,4-difluorophenoxy)-2-fluorophenyl]carbamate (132);
[2-(2,6-dioxopiperidin-3-yl)-4-(oxan-4-yloxy)-3-oxo-2,3-dihydro-1H-isoindol-5-yl] methyl N-[4-(4-fluorophenoxy)phenyl]carbamate (133);
[2-(2,6-dioxopiperidin-3-yl)-4-(oxan-4-yloxy)-3-oxo-2,3-dihydro-1H-isoindol-5-yl] methyl N-(4-phenoxyphenyl)carbamate (134);
[2-(2,6-dioxopiperidin-3-yl)-4-(oxan-4-yloxy)-3-oxo-2,3-dihydro-1H-isoindol-5-yl] methyl N-[2-fluoro-4-(4-fluorophenoxy)phenyl]carbamate (135);
[2-(2,6-dioxopiperidin-3-yl)-4-(oxan-4-yloxy)-3-oxo-2,3-dihydro-1H-isoindol-5-yl] methyl N-[4-(2,4-difluorophenoxy)-2-fluorophenyl]carbamate (136);
[4-(cyclohexyloxy)-2-(2,6-dioxopiperidin-3-yl)-3-oxo-2,3-dihydro-1H-isoindol-5-yl] methyl N-[4-(3,4-difluorophenoxy)phenyl]carbamate (137);
[2-(2,6-dioxopiperidin-3-yl)-4-(2-methylpropoxy)-3-oxo-2,3-dihydro-1H-isoindol-5-yl] methyl N-[4-(3,4-difluorophenoxy)phenyl]carbamate (138);
[2-(2,6-dioxopiperidin-3-yl)-4-(oxan-4-yloxy)-3-oxo-2,3-dihydro-1H-isoindol-5-yl] methyl N-[4-(2,4-difluorophenoxy)phenyl]carbamate (139);
[2-(2,6-dioxopiperidin-3-yl)-4-(oxan-4-yloxy)-3-oxo-2,3-dihydro-1H-isoindol-5-yl] methyl N-[4-(3,5-difluorophenoxy)-2-fluorophenyl]carbamate (140);
[2-(2,6-dioxopiperidin-3-yl)-4-[(3R)-oxan-3-yloxy]-3-oxo-2,3-dihydro-1H-isoindol-5-yl] methyl N-[4-(3,4-difluorophenoxy)phenyl]carbamate (141);
[2-(2,6-dioxopiperidin-3-yl)-4-[(3R)-oxan-3-yloxy]-3-oxo-2,3-dihydro-1H-isoindol-5-yl] methyl N-(4-phenoxyphenyl)carbamate (142);
[2-(2,6-dioxopiperidin-3-yl)-4-[(3R)-oxan-3-yloxy]-3-oxo-2,3-dihydro-1H-isoindol-5-yl] methyl N-[4-(3-fluorophenoxy)phenyl]carbamate (143);
[2-(2,6-dioxopiperidin-3-yl)-4-[(3R)-oxan-3-yloxy]-3-oxo-2,3-dihydro-1H-isoindol-5-yl] methyl N-[4-(4-fluorophenoxy)phenyl]carbamate (144);
[2-(2,6-dioxopiperidin-3-yl)-4-[(3R)-oxan-3-yloxy]-3-oxo-2,3-dihydro-1H-isoindol-5-yl] methyl N-[4-(2,4-difluorophenoxy)phenyl]carbamate (145);
[2-(2,6-dioxopiperidin-3-yl)-4-[(3R)-oxan-3-yloxy]-3-oxo-2,3-dihydro-1H-isoindol-5-yl] methyl N-[4-(3,4-difluorophenoxy)-2-fluorophenyl]carbamate (146);
[2-(2,6-dioxopiperidin-3-yl)-4-[(3R)-oxan-3-yloxy]-3-oxo-2,3-dihydro-1H-isoindol-5-yl] methyl N-[2-fluoro-4-(4-fluorophenoxy)phenyl]carbamate (147);
[2-(2,6-dioxopiperidin-3-yl)-4-[(3R)-oxan-3-yloxy]-3-oxo-2,3-dihydro-1H-isoindol-5-yl] methyl N-[4-(2,4-difluorophenoxy)-2-fluorophenyl]carbamate (148);
[2-(2,6-dioxopiperidin-3-yl)-4-[(3R)-oxan-3-yloxy]-3-oxo-2,3-dihydro-1H-isoindol-5-yl] methyl N-[4-(3,5-difluorophenoxy)-2-fluorophenyl]carbamate (149);
[2-(2,6-dioxopiperidin-3-yl)-4-[(3R)-oxan-3-yloxy]-3-oxo-2,3-dihydro-1H-isoindol-5-yl] methyl N-[2-fluoro-4-(3,4,5-trifluorophenoxy)phenyl]carbamate (150);
[4-(cyclopentyloxy)-2-(2,6-dioxopiperidin-3-yl)-3-oxo-2,3-dihydro-1H-isoindol-5-yl] methyl N-[4-(3,4-difluorophenoxy)phenyl]carbamate (151);
[2-(2,6-dioxopiperidin-3-yl)-3-oxo-4-[(3 S)-oxolan-3-yloxy]-2,3-dihydro-1H-isoindol-5-yl]methyl N-[4-(3,4-difluorophenoxy)phenyl]carbamate (152);
[2-(2,6-dioxopiperidin-3-yl)-3-oxo-4-[(3R)-oxolan-3-yloxy]-2,3-dihydro-1H-isoindol-5-yl]methyl N-[4-(3,4-difluorophenoxy)phenyl]carbamate (153);
[2-(2,6-dioxopiperidin-3-yl)-3-oxo-4-[(3R)-oxolan-3-yloxy]-2,3-dihydro-1H-isoindol-5-yl]methyl N-[4-(3,4-difluorophenoxy)-2-fluorophenyl]carbamate (154);
[2-(2,6-dioxopiperidin-3-yl)-3-oxo-4-[(3R)-oxolan-3-yloxy]-2,3-dihydro-1H-isoindol-5-yl]methyl N-[4-(2-chloro-4-fluorophenoxy)phenyl]carbamate (155);
[2-(2,6-dioxopiperidin-3-yl)-3-oxo-4-[(3R)-oxolan-3-yloxy]-2,3-dihydro-1H-isoindol-5-yl]methyl N-[4-(2,3-difluorophenoxy)phenyl]carbamate (156);
[2-(2,6-dioxopiperidin-3-yl)-3-oxo-4-[(3R)-oxolan-3-yloxy]-2,3-dihydro-1H-isoindol-5-yl]methyl N-[4-(2,4-difluorophenoxy)phenyl]carbamate (157);
[2-(2,6-dioxopiperidin-3-yl)-3-oxo-4-[(3R)-oxolan-3-yloxy]-2,3-dihydro-1H-isoindol-5-yl]methyl N-[4-(3-chlorophenoxy)phenyl]carbamate (158);
[2-(2,6-dioxopiperidin-3-yl)-4-(2-hydroxy-2-methylpropoxy)-3-oxo-2,3-dihydro-1H-isoindol-5-yl]methyl N-[4-(3,4-difluorophenoxy)phenyl]carbamate (159);
[2-(2,6-dioxopiperidin-3-yl)-4-(2-hydroxy-2-methylpropoxy)-3-oxo-2,3-dihydro-1H-isoindol-5-yl]methyl N-(4-phenoxyphenyl)carbamate (160);
[2-(2,6-dioxopiperidin-3-yl)-4-(2-hydroxy-2-methylpropoxy)-3-oxo-2,3-dihydro-1H-isoindol-5-yl]methyl N-[4-(3,4-difluorophenoxy)-2-fluorophenyl]carbamate (161);
[2-(2,6-dioxopiperidin-3-yl)-4-(2-hydroxy-2-methylpropoxy)-3-oxo-2,3-dihydro-1H-isoindol-5-yl]methyl N-[2-fluoro-4-(4-fluorophenoxy)phenyl]carbamate (162);
[2-(2,6-dioxopiperidin-3-yl)-4-(2-hydroxy-2-methylpropoxy)-3-oxo-2,3-dihydro-1H-isoindol-5-yl]methyl N-[4-(2,4-difluorophenoxy)-2-fluorophenyl]carbamate (163);
[2-(2,6-dioxopiperidin-3-yl)-4-(2-hydroxy-2-methylpropoxy)-3-oxo-2,3-dihydro-1H-isoindol-5-yl]methyl N-[4-(3,5-difluorophenoxy)-2-fluorophenyl]carbamate (164);
[2-(2,6-dioxopiperidin-3-yl)-4-(2-hydroxy-2-methylpropoxy)-3-oxo-2,3-dihydro-1H-isoindol-5-yl]methyl N-[2-fluoro-4-(3,4,5-trifluorophenoxy)phenyl]carbamate (165);
[2-(2,6-dioxopiperidin-3-yl)-3-oxo-4-{[(1r,4r)-4-hydroxycyclohexyl]oxy}-2,3-dihydro-1H-isoindol-5-yl]methyl N-[4-(3,4-difluorophenoxy)phenyl]carbamate (166);
[2-(2,6-dioxopiperidin-3-yl)-3-oxo-4-{[(1s,4s)-4-hydroxycyclohexyl]oxy}-2,3-dihydro-1H-isoindol-5-yl]methyl N-[4-(3,4-difluorophenoxy)phenyl]carbamate (167);
[2-(2,6-dioxopiperidin-3-yl)-4-(2-methoxyethoxy)-3-oxo-2,3-dihydro-1H-isoindol-5-yl] methyl N-[4-(3,4-difluorophenoxy)phenyl]carbamate (168);
[2-(2,6-dioxopiperidin-3-yl)-4-(2-methoxyethoxy)-3-oxo-2,3-dihydro-1H-isoindol-5-yl] methyl N-[4-(3,4-difluorophenoxy)-2-fluorophenyl]carbamate (169);
[2-(2,6-dioxopiperidin-3-yl)-4-(oxepan-4-yloxy)-3-oxo-2,3-dihydro-1H-isoindol-5-yl] methyl N-[4-(3,4-difluorophenoxy)phenyl]carbamate (170);
[2-(2,6-dioxopiperidin-3-yl)-3-oxo-4-(pyridin-3-yloxy)-2,3-dihydro-1H-isoindol-5-yl] methyl N-(4-phenoxyphenyl)carbamate (171);
[2-(2,6-dioxopiperidin-3-yl)-3-oxo-4-(pyridin-3-yloxy)-2,3-dihydro-1H-isoindol-5-yl] methyl N-[4-(3,4-difluorophenoxy)phenyl]carbamate (172);
{2-[(3S)-2,6-dioxopiperidin-3-yl]-3-oxo-4-(3-phenylpropoxy)-2,3-dihydro-1H-isoindol-5-yl}methyl N-[4-(3,4-difluorophenoxy)phenyl]carbamate (173);
methyl 2-({5-[({[4-(3,4-difluorophenoxy)phenyl]carbamoyl}oxy)methyl]-2-(2,6-dioxopiperidin-3-yl)-3-oxo-2,3-dihydro-1H-isoindol-4-yl}oxy)acetate (174);
{4-[(dimethylcarbamoyl)methoxy]-2-(2,6-dioxopiperidin-3-yl)-3-oxo-2,3-dihydro-1H-isoindol-5-yl}methyl N-[4-(3,4-difluorophenoxy)phenyl]carbamate (175);
[4-(carbamoylmethoxy)-2-(2,6-dioxopiperidin-3-yl)-3-oxo-2,3-dihydro-1H-isoindol-5-yl] methyl N-[4-(3,4-difluorophenoxy)phenyl]carbamate (176);
[2-(2,6-dioxopiperidin-3-yl)-4-{[ethyl(methyl)carbamoyl]methoxy}-3-oxo-2,3-dihydro-1H-isoindol-5-yl]methyl N-[4-(3,4-difluorophenoxy)phenyl]carbamate (177);
[2-(2,6-dioxopiperidin-3-yl)-4-[2-(morpholin-4-yl)-2-oxoethoxy]-3-oxo-2,3-dihydro-1H-isoindol-5-yl]methyl N-[4-(3,4-difluorophenoxy)phenyl]carbamate (178);
{4-[(1-acetylpiperidin-4-yl)oxy]-2-(2,6-dioxopiperidin-3-yl)-3-oxo-2,3-dihydro-1H-isoindol-5-yl}methyl N-[4-(3,4-difluorophenoxy)phenyl]carbamate (179); or
{2-[(3S)-2,6-dioxo(3,4,4,5,5-²H₅)piperidin-3-yl]-4-methoxy-3-oxo-2,3-dihydro-1H-isoindol-5-yl}methyl N-[4-(3,4-difluorophenoxy)phenyl]carbamate (180).

10. A pharmaceutical composition comprising a compound according to any one of the preceding claims or tautomers or a pharmaceutically acceptable salt thereof; and a pharmaceutically acceptable carrier.

11. A compound, a tautomer or a pharmaceutically acceptable salt thereof according to any one of the preceding claims for use in a method for the treatment of cancer, in a patient comprising administering to said patient a therapeutically effective amount of a compound.

12. The compound, tautomer or pharmaceutically acceptable salt thereof for use according to claim 11 wherein said cancer is selected from cancer of the colon, gastric, pancreatic cancer, breast cancer, prostate cancer, lung cancer, ovarian cancer, cervical cancer, renal cancer, cancer of the head and neck, lymphoma, leukemia, and melanoma.

13. The compound, tautomer or pharmaceutically acceptable salt thereof for use according to claim 11 further comprising administering to said patient a therapeutically effective amount of a second agent, wherein said second agent is selected from an antagonist of PD1/PD-L1 axis, an antagonist of CTLA4, a chemotherapeutic agent, radiation, or an anti-tumor vaccine, prior to, simultaneously with or after administration of said compound.

14. The compound, tautomer or pharmaceutically acceptable salt thereof for use according to claim 11, wherein said compound decreases the level of Cyclin E1 protein and wherein said Cyclin E1 is the amino acid sequence encoded by SEQ ID NOs: 1, 2, 3, 4, 5, 6, 7, 8, or 9.

15. The compound, tautomer or pharmaceutically acceptable salt thereof for use according to claim 14, wherein said Cyclin E1 protein level is decreased by at least 50%.

## Patentansprüche

1. Verbindung der Formel (I): 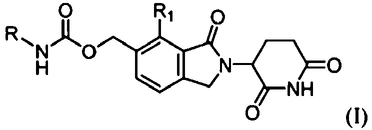 oder Stereoisomere, Tautomere oder Salze davon, wobei:
R 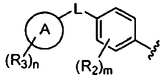 ist;
L-O-, -CH₂-, -C(CH₃)₂-, -CF₂-, -NH -, -N(CH₃)-, oder 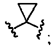 ist;
Ring A Phenyl, Naphthalenyl oder Benzo[b]thiophenyl ist;
R₁ -OCH₃, -OCH₂CH₃, -OCH(CH₃)₂, -OCH₂CH(CH₃)₂, -OCHF₂, -OCH₂C(CH₃)₂OH, - OCH₂CH₂OCH₃, -OCH₂C(O)OCH₃, -OCH₂C(O)NH₂, -OCH₂C(O)N(CH₃)₂, - OCH₂C(O)N(CH₃)(CH₂CH₃), -OCH₂CH₂CH₂(Phenyl), -OCH₂C(O)(Morpholinyl), -O(C₄₋₆ Cycloalkyl), -O(Hydroxycyclohexyl), -O(Oxetanyl), -O(Tetrahydrofuranyl), - O(Tetrahydropyranyl), -O(Oxepanyl), -O(Phenyl), -O(Pyridinyl) oder -O(Acetylpiperidinyl) ist;
R₂ F, Cl, -CH₃oder -OCH₃ist;
jedes R₃ unabhängig F, Cl, Br, -CH₃, -CD₃, -CHF₂, -CF₃, -OCH₃, oder Cyclopropyl ist;
m null oder 1 ist; und
n null, 1, 2 oder 3 ist.

2. Verbindung nach Anspruch 1 oder Stereoisomere, Tautomere oder Salze davon, wobei
R 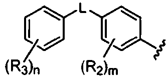 ist;
R₁ -OCH₃, -OCH₂CH₃, -OCH(CH₃)₂, -OCHF₂oder -O(Phenyl) ist; und
jedes R₃ unabhängig F, Cl, Br, -CH₃oder -OCH₃ist.

3. Verbindung nach Anspruch 1 oder Stereoisomere, Tautomere oder Salze davon, wobei Ring A Phenyl ist; und/oder L-O- ist.

4. Verbindung nach Anspruch 1 oder Stereoisomere, Tautomere oder Salze davon, wobei R₁ -OCH₃, -OCH₂CH₃oder -OCH(CH₃)₂ist.

5. Verbindung nach Anspruch 1 oder Stereoisomere, Tautomere oder Salze davon, wobei Ring A Phenyl ist;
L-O- ist; und
R₁ -OCH₃ist.

6. Verbindung nach Anspruch 1 oder Stereoisomere oder Tautomere davon, die die folgende Struktur aufweisen: 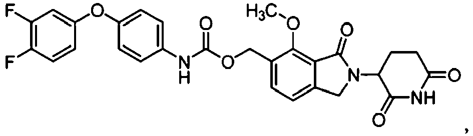 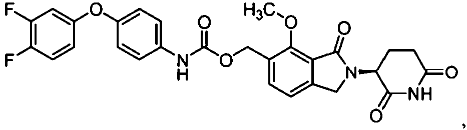 ode

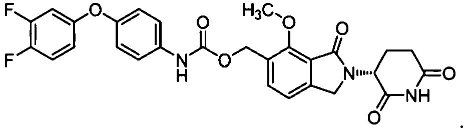

7. Verbindung nach Anspruch 1 oder Stereoisomere oder Tautomere davon, die die folgende Struktur aufweisen: 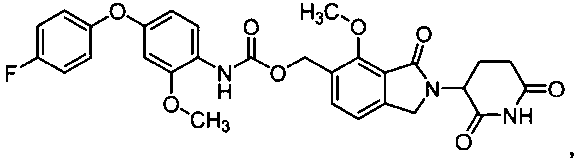 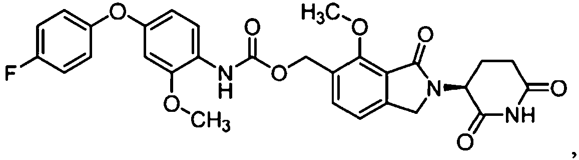 ode

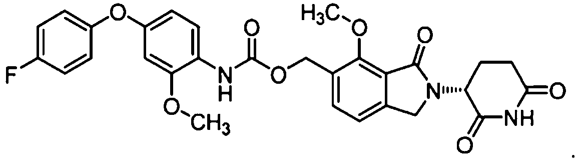

8. Verbindung nach Anspruch 1 oder Stereoisomere oder Tautomere davon, die die folgende Struktur aufweisen: 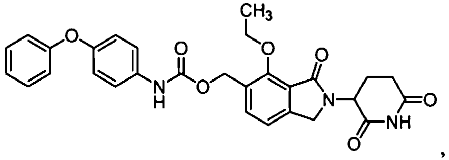 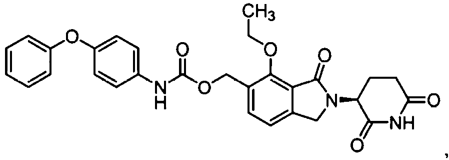 ode

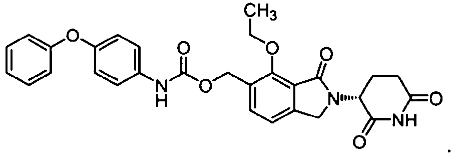

9. Verbindung nach Anspruch 1 oder Tautomere oder pharmazeutisch akzeptable Salze davon, wobei die Verbindung ist:
[2-(2,6-Dioxopiperidin-3-yl)-4-methoxy-3-oxo-2,3-dihydro-1H-isoindol-5-yl]methyl N-[4-(3,4-difluorphenoxy)phenyl]carbamat (1);
[2-(2,6-Dioxopiperidin-3-yl)-4-methoxy-3-oxo-2,3-dihydro-1H-isoindol-5-yl]methyl N-(4-phenoxyphenyl)carbamat (2);
[2-(2,6-Dioxopiperidin-3-yl)-4-methoxy-3-oxo-2,3-dihydro-1H-isoindol-5-yl]methyl N-[4-(3-chlorphenoxy)phenyl]carbamat (3);
[2-(2,6-Dioxopiperidin-3-yl)-4-methoxy-3-oxo-2,3-dihydro-1H-isoindol-5-yl]methyl N-[4-(3-fluorphenoxy)phenyl]carbamat (4);
[2-(2,6-Dioxopiperidin-3-yl)-4-methoxy-3-oxo-2,3-dihydro-1H-isoindol-5-yl]methyl N-[4-(2,4-difluorphenoxy)phenyl]carbamat (5);
[2-(2,6-Dioxopiperidin-3-yl)-4-methoxy-3-oxo-2,3-dihydro-1H-isoindol-5-yl]methyl N-{4-[(3 -fluorphenyl)methyl]phenyl}carbamat (6);
[2-(2,6-Dioxopiperidin-3-yl)-4-methoxy-3-oxo-2,3-dihydro-1H-isoindol-5-yl]methyl N-[4-(1-phenylcyclopropyl)phenyl]carbamat (7);
[2-(2,6-Dioxopiperidin-3-yl)-4-methoxy-3-oxo-2,3-dihydro-1H-isoindol-5-yl]methyl N-{4-[(3-fluorphenyl)(methyl)amino]phenyl}carbamat (8);
[2-(2,6-Dioxopiperidin-3-yl)-4-methoxy-3-oxo-2,3-dihydro-1H-isoindol-5-yl]methyl N-{4-[(3-fluorphenyl)amino]phenyl}carbamat (9);
[2-(2,6-Dioxopiperidin-3-yl)-4-methoxy-3-oxo-2,3-dihydro-1H-isoindol-5-yl]methyl N-[4-(3-bromphenoxy)phenyl]carbamat (10);
[2-(2,6-Dioxopiperidin-3-yl)-4-methoxy-3-oxo-2,3-dihydro-1H-isoindol-5-yl]methyl N-[4-(3-methylphenoxy)phenyl]carbamat (11);
[2-(2,6-Dioxopiperidin-3-yl)-4-methoxy-3-oxo-2,3-dihydro-1H-isoindol-5-yl]methyl N-[4-(3,5-dimethylphenoxy)phenyl]carbamat (12);
[2-(2,6-Dioxopiperidin-3-yl)-4-methoxy-3-oxo-2,3-dihydro-1H-isoindol-5-yl]methyl N-[4-(3,5-difluorphenoxy)phenyl]carbamat (13);
[2-(2,6-Dioxopiperidin-3-yl)-4-methoxy-3-oxo-2,3-dihydro-1H-isoindol-5-yl]methyl N-[4-(3-chlor-4-fluorphenoxy)phenyl]carbamat (14);
[2-(2,6-Dioxopiperidin-3-yl)-4-methoxy-3-oxo-2,3-dihydro-1H-isoindol-5-yl]methyl N-[2-fluor-4-(3,4,5-trifluorphenoxy)phenyl]carbamat (15);
[2-(2,6-Dioxopiperidin-3-yl)-4-methoxy-3-oxo-2,3-dihydro-1H-isoindol-5-yl]methyl N-[2-fluor-4-(3-fluorphenoxy)phenyl]carbamat (16);
[2-(2,6-Dioxopiperidin-3-yl)-4-methoxy-3-oxo-2,3-dihydro-1H-isoindol-5-yl]methyl N-{4-[(3,4-difluorphenyl)methyl]phenyl}carbamat (17);
[2-(2,6-Dioxopiperidin-3-yl)-4-methoxy-3-oxo-2,3-dihydro-1H-isoindol-5-yl]methyl N-{4-[(3,4-difluorphenyl)methyl]-2-methylphenyl}carbamat (18);
[2-(2,6-Dioxopiperidin-3-yl)-4-methoxy-3-oxo-2,3-dihydro-1H-isoindol-5-yl]methyl N-{4-[(3,4,5-trifluorphenyl)methyl]phenyl}carbamat (19);
[2-(2,6-Dioxopiperidin-3-yl)-4-methoxy-3-oxo-2,3-dihydro-1H-isoindol-5-yl]methyl N-{2-fluor-4-[(3-fluorphenyl)methyl]phenyl}carbamat (20);
[2-(2,6-Dioxopiperidin-3-yl)-4-methoxy-3-oxo-2,3-dihydro-1H-isoindol-5-yl]methyl N-{4-[(3-chlor-4-fluorphenyl)methyl]phenyl}carbamat (21);
[2-(2,6-Dioxopiperidin-3-yl)-4-methoxy-3-oxo-2,3-dihydro-1H-isoindol-5-yl]methyl N-[4-(2,3-difluorphenoxy)-2-fluorphenyl]carbamat (22);
[2-(2,6-Dioxopiperidin-3-yl)-4-methoxy-3-oxo-2,3-dihydro-1H-isoindol-5-yl]methyl N-[4-(3,4-difluorphenoxy)-2-fluorphenyl]carbamat (23);
[2-(2,6-Dioxopiperidin-3-yl)-4-methoxy-3-oxo-2,3-dihydro-1H-isoindol-5-yl]methyl N-[4-(3,5-difluorphenoxy)-2-fluorphenyl]carbamat (24);
[2-(2,6-Dioxopiperidin-3-yl)-4-methoxy-3-oxo-2,3-dihydro-1H-isoindol-5-yl]methyl N-[2-chlor-4-(3,4,5-trifluorphenoxy)phenyl]carbamat (25);
[2-(2,6-Dioxopiperidin-3-yl)-4-methoxy-3-oxo-2,3-dihydro-1H-isoindol-5-yl]methyl N-[4-(3-chlorphenoxy)-2-fluorphenyl]carbamat (26);
[2-(2,6-Dioxopiperidin-3-yl)-4-methoxy-3-oxo-2,3-dihydro-1H-isoindol-5-yl]methyl N-[2-chlor-4-(3-fluorphenoxy)phenyl]carbamat (27);
[2-(2,6-Dioxopiperidin-3-yl)-4-methoxy-3-oxo-2,3-dihydro-1H-isoindol-5-yl]methyl N-[2-fluor-4-(4-fluorphenoxy)phenyl]carbamat (28);
[2-(2,6-Dioxopiperidin-3-yl)-4-methoxy-3-oxo-2,3-dihydro-1H-isoindol-5-yl]methyl N-{4-[difluor(phenyl)methyl]phenyllcarbamat (29);
[2-(2,6-Dioxopiperidin-3-yl)-4-methoxy-3-oxo-2,3-dihydro-1H-isoindol-5-yl]methyl N-[4-(2,4-difluorphenoxy)-2-fluorphenyl]carbamat (30);
[2-(2,6-Dioxopiperidin-3-yl)-4-methoxy-3-oxo-2,3-dihydro-1H-isoindol-5-yl]methyl N-[4-(2-methoxyphenoxy)phenyl]carbamat (31);
[2-(2,6-Dioxopiperidin-3-yl)-4-methoxy-3-oxo-2,3-dihydro-1H-isoindol-5-yl]methyl N-[4-(3-methoxyphenoxy)phenyl]carbamat (32);
[2-(2,6-Dioxopiperidin-3-yl)-4-methoxy-3-oxo-2,3-dihydro-1H-isoindol-5-yl]methyl N-[4-(4,5-difluor-2-methoxyphenoxy)-2-methylphenyl]carbamat (33);
[2-(2,6-Dioxopiperidin-3-yl)-4-methoxy-3-oxo-2,3-dihydro-1H-isoindol-5-yl]methyl N-[2-methoxy-4-(2,4,5-trifluorphenoxy)phenyl]carbamat (34);
{2-[(3S)-2,6-dioxopiperidin-3-yl]-4-methoxy-3-oxo-2,3-dihydro-1H-isoindol-5-yl} methyl N-[4-(3,4-difluorphenoxy)phenyl]carbamat (35);
{2-[(3R)-2,6-dioxopiperidin-3-yl]-4-methoxy-3-oxo-2,3-dihydro-1H-isoindol-5-yl} methyl N-[4-(3,4-difluorphenoxy)phenyl]carbamat (36);
[2-(2,6-Dioxopiperidin-3-yl)-4-methoxy-3-oxo-2,3-dihydro-1H-isoindol-5-yl]methyl N-[4-(4-fluorphenoxy)-2-methoxyphenyl]carbamat (37);
[2-(2,6-Dioxopiperidin-3-yl)-4-methoxy-3-oxo-2,3-dihydro-1H-isoindol-5-yl]methyl N-[4-(3,4-difluorphenoxy)-2-methylphenyl]carbamat (38);
[2-(2,6-Dioxopiperidin-3-yl)-4-methoxy-3-oxo-2,3-dihydro-1H-isoindol-5-yl]methyl N-[4-(3-fluorphenoxy)-2-methylphenyl]carbamat (39);
[2-(2,6-Dioxopiperidin-3-yl)-4-methoxy-3-oxo-2,3-dihydro-1H-isoindol-5-yl]methyl N-[2-methyl-4-(3,4,5-trifluorphenoxy)phenyl]carbamat (40);
[2-(2,6-Dioxopiperidin-3-yl)-4-methoxy-3-oxo-2,3-dihydro-1H-isoindol-5-yl]methyl N-[4-(2-phenylpropan-2-yl)phenyl]carbamat (41);
[2-(2,6-Dioxopiperidin-3-yl)-4-methoxy-3-oxo-2,3-dihydro-1H-isoindol-5-yl]methyl N-[4-(2,4-difluorphenoxy)-2-methylphenyl]carbamat (42);
[2-(2,6-Dioxopiperidin-3-yl)-4-methoxy-3-oxo-2,3-dihydro-1H-isoindol-5-yl]methyl N-[2-fluor-4-(2,4,5-trifluorphenoxy)phenyl]carbamat (43);
[2-(2,6-Dioxopiperidin-3-yl)-4-methoxy-3-oxo-2,3-dihydro-1H-isoindol-5-yl]methyl N-[4-(2,4,5-trifluorphenoxy)phenyl]carbamat (44);
[2-(2,6-Dioxopiperidin-3-yl)-4-methoxy-3-oxo-2,3-dihydro-1H-isoindol-5-yl]methyl N-[2-methyl-4-(2,4,5-trifluorphenoxy)phenyl]carbamat (45);
[2-(2,6-Dioxopiperidin-3-yl)-4-methoxy-3-oxo-2,3-dihydro-1H-isoindol-5-yl]methyl N-[2-chlor-4-(3,4-difluorphenoxy)phenyl]carbamat (46);
[2-(2,6-Dioxopiperidin-3-yl)-4-methoxy-3-oxo-2,3-dihydro-1H-isoindol-5-yl]methyl N-[4-(3,4-difluorphenoxy)-2-methoxyphenyl]carbamat (47);
[2-(2,6-Dioxopiperidin-3-yl)-4-methoxy-3-oxo-2,3-dihydro-1H-isoindol-5-yl]methyl N-[4-(3-fluorphenoxy)-2-methoxyphenyl]carbamat (48);
[2-(2,6-Dioxopiperidin-3-yl)-4-methoxy-3-oxo-2,3-dihydro-1H-isoindol-5-yl]methyl N-[4-(2,4-difluorphenoxy)-2-methoxyphenyl]carbamat (49);
[2-(2,6-Dioxopiperidin-3-yl)-4-methoxy-3-oxo-2,3-dihydro-1H-isoindol-5-yl]methyl N-[4-(4-fluor-2-methoxyphenoxy)-2-methoxyphenyl]carbamat (50);
[2-(2,6-Dioxopiperidin-3-yl)-4-methoxy-3-oxo-2,3-dihydro-1H-isoindol-5-yl]methyl N-[4-(4-fluorphenoxy)-2-methylphenyl]carbamat (51);
[2-(2,6-Dioxopiperidin-3-yl)-4-ethoxy-3-oxo-2,3-dihydro-1H-isoindol-5-yl]methyl N- (4-phenoxyphenyl)carbamat (52);
[2-(2,6-Dioxopiperidin-3-yl)-4-ethoxy-3-oxo-2,3-dihydro-1H-isoindol-5-yl]methyl N- [4-(3,4-difluorphenoxy)phenyl]carbamat (53);
[2-(2,6-Dioxopiperidin-3-yl)-4-ethoxy-3-oxo-2,3-dihydro-1H-isoindol-5-yl]methyl N- [4-(3-chlorphenoxy)phenyl]carbamat (54);
[2-(2,6-Dioxopiperidin-3-yl)-4-ethoxy-3-oxo-2,3-dihydro-1H-isoindol-5-yl]methyl N- [4-(3-fluorphenoxy)phenyl] carbamat (55);
[2-(2,6-Dioxopiperidin-3-yl)-3-oxo-4-(propan-2-yloxy)-2,3-dihydro-1H-isoindol-5-yl] methyl N-[4-(3,4-difluorphenoxy)phenyl]carbamat (56);
[2-(2,6-Dioxopiperidin-3-yl)-3-oxo-4-(propan-2-yloxy)-2,3-dihydro-1H-isoindol-5-yl] methyl-N-(4-phenoxyphenyl)carbamat (57);
[2-(2,6-Dioxopiperidin-3-yl)-3-oxo-4-(propan-2-yloxy)-2,3-dihydro-1H-isoindol-5-yl] methyl N-[4-(3-chlorphenoxy)phenyl]carbamat (58);
[2-(2,6-Dioxopiperidin-3-yl)-3-oxo-4-(propan-2-yloxy)-2,3-dihydro-1H-isoindol-5-yl] methyl N-[4-(4-fluorphenoxy)phenyl]carbamat (59);
[4-(Difluormethoxy)-2-(2,6-dioxopiperidin-3-yl)-3-oxo-2,3-dihydro-1H-isoindol-5-yl] methyl-N-(4-phenoxyphenyl)carbamat (60);
[4-(Difluormethoxy)-2-(2,6-dioxopiperidin-3-yl)-3-oxo-2,3-dihydro-1H-isoindol-5-yl] methyl N-[4-(3,4-difluorphenoxy)phenyl]carbamat (61);
[2-(2,6-Dioxopiperidin-3-yl)-3-oxo-4-phenoxy-2,3-dihydro-1H-isoindol-5-yl]methyl N-(4-phenoxyphenyl)carbamat (62);
[2-(2,6-Dioxopiperidin-3-yl)-3-oxo-4-phenoxy-2,3-dihydro-1H-isoindol-5-yl]methyl N-[4-(3,4-difluorphenoxy)phenyl]carbamat (63);
[2-(2,6-Dioxopiperidin-3-yl)-3-oxo-4-phenoxy-2,3-dihydro-1H-isoindol-5-yl]methyl N-[4-(3-fluorphenoxy)phenyl]carbamat (64);
[2-(2,6-Dioxopiperidin-3-yl)-3-oxo-4-phenoxy-2,3-dihydro-1H-isoindol-5-yl]methyl N-[4-(2-fluorphenoxy)phenyl]carbamat (65);
[2-(2,6-Dioxopiperidin-3-yl)-3-oxo-4-phenoxy-2,3-dihydro-1H-isoindol-5-yl]methyl N-[4-(2-chlorphenoxy)phenyl]carbamat (66);
[2-(2,6-Dioxopiperidin-3-yl)-3-oxo-4-phenoxy-2,3-dihydro-1H-isoindol-5-yl]methyl N-[4-(2,4-difluorphenoxy)phenyl]carbamat (67);
[2-(2,6-Dioxopiperidin-3-yl)-3-oxo-4-phenoxy-2,3-dihydro-1H-isoindol-5-yl]methyl N-[4-(2,3-difluorphenoxy)phenyl]carbamat (68);
[2-(2,6-Dioxopiperidin-3-yl)-3-oxo-4-phenoxy-2,3-dihydro-1H-isoindol-5-yl]methyl N-[4-(3,4-difluorphenoxy)-2-fluorphenyl]carbamat (69);
[2-(2,6-Dioxopiperidin-3-yl)-3-oxo-4-phenoxy-2,3-dihydro-1H-isoindol-5-yl]methyl N-[4-(2-chlor-4-fluorphenoxy)phenyl]carbamat (70);
[2-(2,6-Dioxopiperidin-3-yl)-4-methoxy-3-oxo-2,3-dihydro-1H-isoindol-5-yl]methyl N-[4-(4-chlor-3-fluorphenoxy)phenyl]carbamat (71);
[2-(2,6-Dioxopiperidin-3-yl)-4-methoxy-3-oxo-2,3-dihydro-1H-isoindol-5-yl]methyl N-[2-methoxy-4-(3,4,5-trifluorphenoxy)phenyl]carbamat (72);
[2-(2,6-Dioxopiperidin-3-yl)-4-methoxy-3-oxo-2,3-dihydro-1H-isoindol-5-yl]methyl N-[4-(4,5-difluor-2-methylphenoxy)-2-methoxyphenyl]carbamat (73);
[2-(2,6-Dioxopiperidin-3-yl)-4-methoxy-3-oxo-2,3-dihydro-1H-isoindol-5-yl]methyl N-[4-(4,5-difluor-2-methylphenoxy)-2-methylphenyl]carbamat (74);
[2-(2,6-Dioxopiperidin-3-yl)-4-methoxy-3-oxo-2,3-dihydro-1H-isoindol-5-yl]methyl N-[4-(4-fluor-2-methylphenoxy)-2-methoxyphenyl]carbamat (75);
[2-(2,6-Dioxopiperidin-3-yl)-4-methoxy-3-oxo-2,3-dihydro-1H-isoindol-5-yl]methyl N-[4-(4-fluor-2-methylphenoxy)-2-methylphenyl]carbamat (76);
[2-(2,6-Dioxopiperidin-3-yl)-4-methoxy-3-oxo-2,3-dihydro-1H-isoindol-5-yl]methyl N-[4-(3,4,5-trifluorphenoxy)phenyl]carbamat (77);
[2-(2,6-Dioxopiperidin-3-yl)-4-methoxy-3-oxo-2,3-dihydro-1H-isoindol-5-yl]methyl N-[4-(4,5 -difluor-2-methylphenoxy)phenyl]carbamat (78);
[2-(2,6-Dioxopiperidin-3-yl)-4-methoxy-3-oxo-2,3-dihydro-1H-isoindol-5-yl]methyl N-[4-(2-fluorphenoxy)phenyl]carbamat (79);
[2-(2,6-Dioxopiperidin-3-yl)-4-methoxy-3-oxo-2,3-dihydro-1H-isoindol-5-yl]methyl N-[4-(2-chlorphenoxy)phenyl]carbamat (80);
[2-(2,6-Dioxopiperidin-3-yl)-4-methoxy-3-oxo-2,3-dihydro-1H-isoindol-5-yl]methyl N-[4-(4-fluor-2-methylphenoxy)phenyl]carbamat (81);
[2-(2,6-Dioxopiperidin-3-yl)-4-methoxy-3-oxo-2,3-dihydro-1H-isoindol-5-yl]methyl N-[4-(4-fluor-3-methylphenoxy)-2-methylphenyl]carbamat (82);
[2-(2,6-Dioxopiperidin-3-yl)-4-methoxy-3-oxo-2,3-dihydro-1H-isoindol-5-yl]methyl N-[4-(3-chlor-4-fluorphenoxy)-2-methylphenyl]carbamat (83);
[2-(2,6-Dioxopiperidin-3-yl)-4-methoxy-3-oxo-2,3-dihydro-1H-isoindol-5-yl]methyl N-[4-(4-fluorphenoxy)phenyl]carbamat (84);
[2-(2,6-Dioxopiperidin-3-yl)-4-methoxy-3-oxo-2,3-dihydro-1H-isoindol-5-yl]methyl N-{4-[2-(difluormethyl)phenoxy]phenyl}carbamat (85);
[2-(2,6-Dioxopiperidin-3-yl)-4-methoxy-3-oxo-2,3-dihydro-1H-isoindol-5-yl]methyl N-{4-[2-(difluormethyl)phenoxy]-2-fluorphenyl}carbamat (86);
[2-(2,6-Dioxopiperidin-3-yl)-4-methoxy-3-oxo-2,3-dihydro-1H-isoindol-5-yl]methyl N-[4-(2-cyclopropylphenoxy)phenyl]carbamat (87);
[2-(2,6-Dioxopiperidin-3-yl)-4-methoxy-3-oxo-2,3-dihydro-1H-isoindol-5-yl]methyl N-{4-[2-(difluormethyl)phenoxy]-2-methoxyphenyl}carbamat (88);
[2-(2,6-Dioxopiperidin-3-yl)-4-methoxy-3-oxo-2,3-dihydro-1H-isoindol-5-yl]methyl N-{4-[2-(difluormethyl)-4-fluorphenoxy]phenyl}carbamat (89);
[2-(2,6-Dioxopiperidin-3-yl)-4-methoxy-3-oxo-2,3-dihydro-1H-isoindol-5-yl]methyl N-{2-chlor-4-[2-(trifluormethyl)phenoxy]phenyl}carbamat (90);
[2-(2,6-Dioxopiperidin-3-yl)-4-methoxy-3-oxo-2,3-dihydro-1H-isoindol-5-yl]methyl N-[4-(4-chlor-3-fluorphenoxy)-2-fluorphenyl]carbamat (91);
[2-(2,6-Dioxopiperidin-3-yl)-4-methoxy-3-oxo-2,3-dihydro-1H-isoindol-5-yl]methyl N-{4-[2-(2H3)methylphenoxy]phenyl}carbamat (92);
[2-(2,6-Dioxopiperidin-3-yl)-4-methoxy-3-oxo-2,3-dihydro-1H-isoindol-5-yl]methyl N-(2-Methoxy-4-phenoxyphenyl)carbamat (93);
[2-(2,6-Dioxopiperidin-3-yl)-4-methoxy-3-oxo-2,3-dihydro-1H-isoindol-5-yl]methyl N-[4-(4,5-difluor-2-methylphenoxy)-2-fluorphenyl]carbamat (94);
[2-(2,6-Dioxopiperidin-3-yl)-4-methoxy-3-oxo-2,3-dihydro-1H-isoindol-5-yl]methyl N-[2-fluor-4-(4-fluor-2-methylphenoxy)phenyl]carbamat (95);
[2-(2,6-Dioxopiperidin-3-yl)-4-methoxy-3-oxo-2,3-dihydro-1H-isoindol-5-yl]methyl N-[4-(4-fluor-3-methylphenoxy)phenyl]carbamat (96);
[2-(2,6-Dioxopiperidin-3-yl)-4-methoxy-3-oxo-2,3-dihydro-1H-isoindol-5-yl]methyl N-[4-(4-fluor-3-methylphenoxy)-2-methoxyphenyl]carbamat (97);
[2-(2,6-Dioxopiperidin-3-yl)-4-methoxy-3-oxo-2,3-dihydro-1H-isoindol-5-yl]methyl N-[4-(3-chlor-4-fluorphenoxy)-2-fluorphenyl]carbamat (98);
[2-(2,6-Dioxopiperidin-3-yl)-4-methoxy-3-oxo-2,3-dihydro-1H-isoindol-5-yl]methyl N-[2-fluor-4-(4-fluor-3-methylphenoxy)phenyl]carbamat (99);
[2-(2,6-Dioxopiperidin-3-yl)-4-methoxy-3-oxo-2,3-dihydro-1H-isoindol-5-yl]methyl N-[4-(3-chlor-4-fluorphenoxy)-2-methoxyphenyl]carbamat (100);
[2-(2,6-Dioxopiperidin-3-yl)-4-methoxy-3-oxo-2,3-dihydro-1H-isoindol-5-yl]methyl N-[4-(2-methylphenoxy)phenyl]carbamat (101);
[2-(2,6-Dioxopiperidin-3-yl)-4-methoxy-3-oxo-2,3-dihydro-1H-isoindol-5-yl]methyl N-[4-(2-chlor-4-fluorphenoxy)phenyl]carbamat (102);
[2-(2,6-Dioxopiperidin-3-yl)-4-methoxy-3-oxo-2,3-dihydro-1H-isoindol-5-yl]methyl N-[4-(2-chlor-4-fluorphenoxy)-2-methylphenyl]carbamat (103);
[2-(2,6-Dioxopiperidin-3-yl)-4-methoxy-3-oxo-2,3-dihydro-1H-isoindol-5-yl]methyl N-[4-(2-chlor-4-fluorphenoxy)-2-methoxyphenyl]carbamat (104);
[2-(2,6-Dioxopiperidin-3-yl)-4-methoxy-3-oxo-2,3-dihydro-1H-isoindol-5-yl]methyl N-[4-(2-chlor-4-fluorphenoxy)-2-fluorphenyl]carbamat (105);
[2-(2,6-Dioxopiperidin-3-yl)-4-methoxy-3-oxo-2,3-dihydro-1H-isoindol-5-yl]methyl N-[4-(2-chlor-5-fluorphenoxy)phenyl]carbamat (106);
[2-(2,6-Dioxopiperidin-3-yl)-4-methoxy-3-oxo-2,3-dihydro-1H-isoindol-5-yl]methyl N-[4-(2-chlorphenoxy)-2-fluorphenyl]carbamat (107);
[2-(2,6-Dioxopiperidin-3-yl)-4-methoxy-3-oxo-2,3-dihydro-1H-isoindol-5-yl]methyl N-[4-(2-chlor-4,5-difluorphenoxy)-2-methoxyphenyl]carbamat (108);
[2-(2,6-Dioxopiperidin-3-yl)-4-methoxy-3-oxo-2,3-dihydro-1H-isoindol-5-yl]methyl N-[4-(2-chlor-4,5-difluorphenoxy)-2-fluorphenyl]carbamat (109);
[2-(2,6-Dioxopiperidin-3-yl)-4-methoxy-3-oxo-2,3-dihydro-1H-isoindol-5-yl]methyl N-[4-(2,3-difluorphenoxy)phenyl]carbamat (110);
[2-(2,6-Dioxopiperidin-3-yl)-4-methoxy-3-oxo-2,3-dihydro-1H-isoindol-5-yl]methyl N-[4-(2-chlor-3-fluorphenoxy)phenyl]carbamat (111);
[2-(2,6-Dioxopiperidin-3-yl)-4-methoxy-3-oxo-2,3-dihydro-1H-isoindol-5-yl]methyl N-[4-(2,6-difluorphenoxy)phenyl]carbamat (112);
[2-(2,6-Dioxopiperidin-3-yl)-4-methoxy-3-oxo-2,3-dihydro-1H-isoindol-5-yl]methyl N-[4-(naphthalen-1-yloxy)phenyl]carbamat (113);
[2-(2,6-Dioxopiperidin-3-yl)-4-methoxy-3-oxo-2,3-dihydro-1H-isoindol-5-yl]methyl N-[4-(1-benzothiophen-5-yloxy)phenyl]carbamat (114);
[2-(2,6-Dioxopiperidin-3-yl)-4-methoxy-3-oxo-2,3-dihydro-1H-isoindol-5-yl]methyl N-[4-(1-benzothiophen-6-yloxy)phenyl]carbamat (115);
[2-(2,6-Dioxopiperidin-3-yl)-4-methoxy-3-oxo-2,3-dihydro-1H-isoindol-5-yl]methyl N-[4-(2,3-dichlorphenoxy)-2-fluorphenyl]carbamat (116);
[2-(2,6-Dioxopiperidin-3-yl)-4-methoxy-3-oxo-2,3-dihydro-1H-isoindol-5-yl]methyl N-[4-(3,4-dichlorphenoxy)-2-fluorphenyl]carbamat (117);
[2-(2,6-Dioxopiperidin-3-yl)-4-methoxy-3-oxo-2,3-dihydro-1H-isoindol-5-yl]methyl N-[4-(3,5-dichlorphenoxy)-2-fluorphenyl]carbamat (118);
[2-(2,6-Dioxopiperidin-3-yl)-3-oxo-4-(propan-2-yloxy)-2,3-dihydro-1H-isoindol-5-yl] methyl N-[4-(3,4-difluorphenoxy)-2-fluorphenyl]carbamat (119);
[2-(2,6-Dioxopiperidin-3-yl)-3-oxo-4-(propan-2-yloxy)-2,3-dihydro-1H-isoindol-5-yl] methyl N-[4-(2,3-difluorphenoxy)phenyl]carbamat (120);
[2-(2,6-Dioxopiperidin-3-yl)-3-oxo-4-(propan-2-yloxy)-2,3-dihydro-1H-isoindol-5-yl] methyl N-[4-(2-fluorphenoxy)phenyl]carbamat (121);
[2-(2,6-Dioxopiperidin-3-yl)-3-oxo-4-(propan-2-yloxy)-2,3-dihydro-1H-isoindol-5-yl] methyl N-[4-(2-chlorphenoxy)phenyl]carbamat (122);
[2-(2,6-Dioxopiperidin-3-yl)-3-oxo-4-(propan-2-yloxy)-2,3-dihydro-1H-isoindol-5-yl] methyl N-[4-(3-fluorphenoxy)phenyl]carbamat (123);
[2-(2,6-Dioxopiperidin-3-yl)-3-oxo-4-(propan-2-yloxy)-2,3-dihydro-1H-isoindol-5-yl] methyl N-[4-(2-chlor-4-fluorphenoxy)phenyl]carbamat (124);
{2-[(3S)-2,6-dioxopiperidin-3-yl]-3-oxo-4-(propan-2-yloxy)-2,3-dihydro-1H-isoindol-5-yl}methyl N-[4-(3,4-difluorphenoxy)phenyl]carbamat (125);
[4-Cyclobutoxy-2-(2,6-dioxopiperidin-3-yl)-3-oxo-2,3-dihydro-1H-isoindol-5-yl]methyl N-[4-(3,4-difluorphenoxy)phenyl]carbamat (126);
[4-Cyclobutoxy-2-(2,6-dioxopiperidin-3-yl)-3-oxo-2,3-dihydro-1H-isoindol-5-yl]methyl N-[4-(2-chlorphenoxy)phenyl]carbamat (127);
[4-Cyclobutoxy-2-(2,6-dioxopiperidin-3-yl)-3-oxo-2,3-dihydro-1H-isoindol-5-yl]methyl N-[4-(2-chlor-4-fluorphenoxy)phenyl]carbamat (128);
[2-(2,6-Dioxopiperidin-3-yl)-4-(oxetan-3-yloxy)-3-oxo-2,3-dihydro-1H-isoindol-5-yl] methyl N-[4-(3,4-difluorphenoxy)phenyl]carbamat (129);
[2-(2,6-Dioxopiperidin-3-yl)-4-(oxan-4-yloxy)-3 -oxo-2,3-dihydro-1H-isoindol-5-yl] methyl N-[4-(3,4-difluorphenoxy)phenyl]carbamat (130);
[2-(2,6-Dioxopiperidin-3-yl)-4-(oxan-4-yloxy)-3 -oxo-2,3-dihydro-1H-isoindol-5-yl] methyl N-[4-(2-chlorphenoxy)phenyl]carbamat (131);
[2-(2,6-Dioxopiperidin-3-yl)-4-(oxan-4-yloxy)-3 -oxo-2,3-dihydro-1H-isoindol-5-yl] methyl N-[4-(3,4-difluorphenoxy)-2-fluorphenyl]carbamat (132);
[2-(2,6-Dioxopiperidin-3-yl)-4-(oxan-4-yloxy)-3-oxo-2,3-dihydro-1H-isoindol-5-yl] methyl N-[4-(4-fluorphenoxy)phenyl]carbamat (133);
[2-(2,6-Dioxopiperidin-3-yl)-4-(oxan-4-yloxy)-3-oxo-2,3-dihydro-1H-isoindol-5-yl] methyl N-(4-phenoxyphenyl)carbamat (134);
[2-(2,6-Dioxopiperidin-3-yl)-4-(oxan-4-yloxy)-3-oxo-2,3-dihydro-1H-isoindol-5-yl] methyl N-[2-fluor-4-(4-fluorphenoxy)phenyl]carbamat (135);
[2-(2,6-Dioxopiperidin-3-yl)-4-(oxan-4-yloxy)-3-oxo-2,3-dihydro-1H-isoindol-5-yl] methyl N-[4-(2,4-difluorphenoxy)-2-fluorphenyl]carbamat (136);
[4-(Cyclohexyloxy)-2-(2,6-dioxopiperidin-3-yl)-3-oxo-2,3-dihydro-1H-isoindol-5-yl] methyl N-[4-(3,4-difluorphenoxy)phenyl]carbamat (137);
[2-(2,6-Dioxopiperidin-3-yl)-4-(2-methylpropoxy)-3-oxo-2,3-dihydro-1H-isoindol-5-yl] methyl N-[4-(3,4-difluorphenoxy)phenyl]carbamat (138);
[2-(2,6-Dioxopiperidin-3-yl)-4-(oxan-4-yloxy)-3-oxo-2,3-dihydro-1H-isoindol-5-yl] methyl N-[4-(2,4-difluorphenoxy)phenyl]carbamat (139);
[2-(2,6-Dioxopiperidin-3-yl)-4-(oxan-4-yloxy)-3-oxo-2,3-dihydro-1H-isoindol-5-yl] methyl N-[4-(3,5-difluorphenoxy)-2-fluorphenyl]carbamat (140);
[2-(2,6-Dioxopiperidin-3-yl)-4-[(3R)-oxan-3-yloxy]-3-oxo-2,3-dihydro-1H-isoindol-5-yl] methyl N-[4-(3,4-difluorphenoxy)phenyl]carbamat (141);
[2-(2,6-Dioxopiperidin-3-yl)-4-[(3R)-oxan-3-yloxy]-3-oxo-2,3-dihydro-1H-isoindol-5-yl] methyl-N-(4-phenoxyphenyl)carbamat (142);
[2-(2,6-Dioxopiperidin-3-yl)-4-[(3R)-oxan-3-yloxy]-3-oxo-2,3-dihydro-1H-isoindol-5-yl] methyl N-[4-(3-fluorphenoxy)phenyl]carbamat (143);
[2-(2,6-Dioxopiperidin-3-yl)-4-[(3R)-oxan-3-yloxy]-3-oxo-2,3-dihydro-1H-isoindol-5-yl] methyl N-[4-(4-fluorphenoxy)phenyl]carbamat (144);
[2-(2,6-Dioxopiperidin-3-yl)-4-[(3R)-oxan-3-yloxy]-3-oxo-2,3-dihydro-1H-isoindol-5-yl] methyl N-[4-(2,4-difluorphenoxy)phenyl]carbamat (145);
[2-(2,6-Dioxopiperidin-3-yl)-4-[(3R)-oxan-3-yloxy]-3-oxo-2,3-dihydro-1H-isoindol-5-yl] methyl N-[4-(3,4-difluorphenoxy)-2-fluorphenyl]carbamat (146);
[2-(2,6-Dioxopiperidin-3-yl)-4-[(3R)-oxan-3-yloxy]-3-oxo-2,3-dihydro-1H-isoindol-5-yl] methyl N-[2-fluor-4-(4-fluorphenoxy)phenyl]carbamat (147);
[2-(2,6-Dioxopiperidin-3-yl)-4-[(3R)-oxan-3-yloxy]-3-oxo-2,3-dihydro-1H-isoindol-5-yl] methyl N-[4-(2,4-difluorphenoxy)-2-fluorphenyl]carbamat (148);
[2-(2,6-Dioxopiperidin-3-yl)-4-[(3R)-oxan-3-yloxy]-3-oxo-2,3-dihydro-1H-isoindol-5-yl] methyl N-[4-(3,5-difluorphenoxy)-2-fluorphenyl]carbamat (149);
[2-(2,6-Dioxopiperidin-3-yl)-4-[(3R)-oxan-3-yloxy]-3-oxo-2,3-dihydro-1H-isoindol-5-yl] methyl N-[2-fluor-4-(3,4,5-trifluorphenoxy)phenyl]carbamat (150);
[4-(Cyclopentyloxy)-2-(2,6-dioxopiperidin-3-yl)-3-oxo-2,3-dihydro-1H-isoindol-5-yl] Methyl N-[4-(3,4-difluorphenoxy)phenyl]carbamat (151);
[2-(2,6-Dioxopiperidin-3-yl)-3-oxo-4-[(3S)-oxolan-3-yloxy]-2,3-dihydro-1H-isoindol-5-yl]methyl N-[4-(3,4-difluorphenoxy)phenyl]carbamat (152);
[2-(2,6-Dioxopiperidin-3-yl)-3-oxo-4-[(3R)-oxolan-3-yloxy]-2,3-dihydro-1H-isoindol- 5-yl]methyl N-[4-(3,4-difluorphenoxy)phenyl]carbamat (153);
[2-(2,6-Dioxopiperidin-3-yl)-3-oxo-4-[(3R)-oxolan-3-yloxy]-2,3-dihydro-1H-isoindol- 5-yl]methyl N-[4-(3,4-difluorphenoxy)-2-fluorphenyl]carbamat (154);
[2-(2,6-Dioxopiperidin-3-yl)-3-oxo-4-[(3R)-oxolan-3-yloxy]-2,3-dihydro-1H-isoindol- 5-yl]methyl N-[4-(2-chlor-4-fluorphenoxy)phenyl]carbamat (155);
[2-(2,6-Dioxopiperidin-3-yl)-3-oxo-4-[(3R)-oxolan-3-yloxy]-2,3-dihydro-1H-isoindol- 5-yl]methyl N-[4-(2,3-difluorphenoxy)phenyl]carbamat (156);
[2-(2,6-Dioxopiperidin-3-yl)-3-oxo-4-[(3R)-oxolan-3-yloxy]-2,3-dihydro-1H-isoindol- 5-yl]methyl N-[4-(2,4-difluorphenoxy)phenyl]carbamat (157);
[2-(2,6-Dioxopiperidin-3-yl)-3-oxo-4-[(3R)-oxolan-3-yloxy]-2,3-dihydro-1H-isoindol- 5-yl]methyl N-[4-(3-chlorphenoxy)phenyl]carbamat (158);
[2-(2,6-Dioxopiperidin-3-yl)-4-(2-hydroxy-2-methylpropoxy)-3-oxo-2,3-dihydro-1H- isoindol-5-yl]methyl N-[4-(3,4-difluorphenoxy)phenyl]carbamat (159);
[2-(2,6-Dioxopiperidin-3-yl)-4-(2-hydroxy-2-methylpropoxy)-3-oxo-2,3-dihydro-1H- isoindol-5-yl]methyl N-(4-phenoxyphenyl)carbamat (160);
[2-(2,6-Dioxopiperidin-3-yl)-4-(2-hydroxy-2-methylpropoxy)-3-oxo-2,3-dihydro-1H- isoindol-5-yl]methyl N-[4-(3,4-difluorphenoxy)-2-fluorphenyl]carbamat (161);
[2-(2,6-Dioxopiperidin-3-yl)-4-(2-hydroxy-2-methylpropoxy)-3-oxo-2,3-dihydro-1H-isoindol-5-yl]methyl N-[2-fluor-4-(4-fluorphenoxy)phenyl]carbamat (162);
[2-(2,6-Dioxopiperidin-3-yl)-4-(2-hydroxy-2-methylpropoxy)-3-oxo-2,3-dihydro-1H-isoindol-5-yl]methyl N-[4-(2,4-difluorphenoxy)-2-fluorphenyl]carbamat (163);
[2-(2,6-Dioxopiperidin-3-yl)-4-(2-hydroxy-2-methylpropoxy)-3-oxo-2,3-dihydro-1H-isoindol-5-yl]methyl N-[4-(3,5-difluorphenoxy)-2-fluorphenyl]carbamat (164);
[2-(2,6-Dioxopiperidin-3-yl)-4-(2-hydroxy-2-methylpropoxy)-3-oxo-2,3-dihydro-1H- isoindol-5-yl]methyl N-[2-fluor-4-(3,4,5-trifluorphenoxy)phenyl]carbamat (165);
[2-(2,6-Dioxopiperidin-3-yl)-3-oxo-4-{[(1r,4r)-4-hydroxycyclohexyl]oxy}-2,3-dihydro-1H-isoindol-5-yl]methyl N-[4-(3,4-difluorphenoxy)phenyl]carbamat (166);
[2-(2,6-Dioxopiperidin-3-yl)-3-oxo-4-{[(1s,4s)-4-hydroxycyclohexyl]oxy}-2,3-dihydro-1H-isoindol-5-yl]methyl N-[4-(3,4-difluorphenoxy)phenyl]carbamat (167);
[2-(2,6-Dioxopiperidin-3-yl)-4-(2-methoxyethoxy)-3-oxo-2,3-dihydro-1H-isoindol-5-yl]methyl N-[4-(3,4-difluorphenoxy)phenyl]carbamat (168);
[2-(2,6-Dioxopiperidin-3-yl)-4-(2-methoxyethoxy)-3-oxo-2,3-dihydro-1H-isoindol-5-yl] methyl N-[4-(3,4-difluorphenoxy)-2-fluorphenyl]carbamat (169);
[2-(2,6-Dioxopiperidin-3-yl)-4-(oxepan-4-yloxy)-3-oxo-2,3-dihydro-1H-isoindol-5-yl] methyl N-[4-(3,4-difluorphenoxy)phenyl]carbamat (170);
[2-(2,6-Dioxopiperidin-3-yl)-3-oxo-4-(pyridin-3-yloxy)-2,3-dihydro-1H-isoindol-5-yl] methyl-N-(4-phenoxyphenyl)carbamat (171);
[2-(2,6-Dioxopiperidin-3-yl)-3-oxo-4-(pyridin-3-yloxy)-2,3-dihydro-1H-isoindol-5-yl] methyl N-[4-(3,4-difluorphenoxy)phenyl]carbamat (172);
{2-[(3S)-2,6-dioxopiperidin-3-yl]-3-oxo-4-(3-phenylpropoxy)-2,3-dihydro-1H-isoindol-5-yl}methyl N-[4-(3,4-difluorphenoxy)phenyl]carbamat (173);
Methyl 2-({5-[({[4-(3,4-difluorphenoxy)phenyl]carbamoyl}oxy)methyl]-2-(2,6-dioxopiperidin-3-yl)-3 -oxo-2,3 -dihydro-1 H-isoindol-4-yl} oxy)acetat (174);
{4-[(Dimethylcarbamoyl)methoxy]-2-(2,6-dioxopiperidin-3-yl)-3-oxo-2,3-dihydro-1H-isoindol-5-yl}methyl N-[4-(3,4-difluorphenoxy)phenyl]carbamat (175);
[4-(Carbamoylmethoxy)-2-(2,6-dioxopiperidin-3-yl)-3-oxo-2,3-dihydro-1H-isoindol-5-yl]methyl N-[4-(3,4-difluorphenoxy)phenyl]carbamat (176);
[2-(2,6-Dioxopiperidin-3-yl)-4-{[ethyl(methyl)carbamoyl]methoxy}-3-oxo-2,3-dihydro-1H-isoindol-5-yl]methyl N-[4-(3,4-difluorphenoxy)phenyl] carbamat (177);
[2-(2,6-Dioxopiperidin-3-yl)-4-[2-(morpholin-4-yl)-2-oxoethoxy]-3-oxo-2,3-dihydro- 1H-isoindol-5-yl]methyl N-[4-(3,4-difluorphenoxy)phenyl]carbamat (178);
{4-[(L-acetylpiperidin-4-yl)oxy]-2-(2,6-dioxopiperidin-3-yl)-3-oxo-2,3-dihydro-1H-isoindol-5-yl}methyl N-[4-(3,4-difluorphenoxy)phenyl]carbamat (179) oder
{2-[(3S)-2,6-dioxo(3,4,4,5,5-2H5)piperidin-3-yl]-4-methoxy-3-oxo-2,3-dihydro-1H-isoindol-5-yl}methyl N-[4-(3,4-difluorphenoxy)phenyl]carbamat (180).

10. Pharmazeutische Zusammensetzung, die eine Verbindung nach einem der vorstehenden Ansprüche oder Tautomere oder ein pharmazeutisch akzeptables Salz davon umfasst; und einen pharmazeutisch akzeptablen Träger.

11. Verbindung, ein Tautomer oder ein pharmazeutisch akzeptables Salz davon nach einem der vorstehenden Ansprüche zur Verwendung in einem Verfahren zur Behandlung von Krebs bei einem Patienten, umfassend Verabreichen einer therapeutisch wirksamen Menge einer Verbindung an den Patienten.

12. Verbindung, Tautomer oder pharmazeutisch akzeptables Salz davon zur Verwendung nach Anspruch 11, wobei der Krebs aus Dickdarmkrebs, Magenkrebs, Bauchspeicheldrüsenkrebs, Brustkrebs, Prostatakrebs, Lungenkrebs, Eierstockkrebs, Gebärmutterhalskrebs, Nierenkrebs, Krebs von Kopf und Hals, Lymphom, Leukämie und Melanom ausgewählt ist.

13. Verbindung, Tautomer oder pharmazeutisch akzeptables Salz davon zur Verwendung nach Anspruch 11, weiter umfassend Verabreichen einer therapeutisch wirksamen Menge eines zweiten Wirkstoffs an den Patienten, wobei der zweite Wirkstoff aus einem Antagonisten der PD1/PD-L1-Achse, einem Antagonisten von CTLA4, einem Chemotherapeutikum, Bestrahlung oder einem Antitumorimpfstoff ausgewählt ist, vor, gleichzeitig mit oder nach der Verabreichung der Verbindung.

14. Verbindung, Tautomer oder pharmazeutisch akzeptables Salz davon zur Verwendung nach Anspruch 11, wobei die Verbindung den Spiegel von Cyclin-E1-Protein vermindert und wobei das Cyclin E1 die Aminosäuresequenz ist, codiert durch die SEQ ID NOs: 1, 2, 3, 4, 5, 6, 7, 8 oder 9.

15. Verbindung, Tautomer oder pharmazeutisch akzeptables Salz davon zur Verwendung nach Anspruch 14, wobei der Cyclin-E1-Proteinspiegel um mindestens 50 % vermindert wird.

## Revendications

1. Composé de formule (I) : 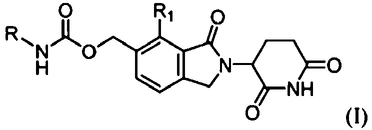 ou ses stéréoisomères, tautomères ou sels, dans lesquels :
R est 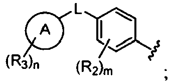
L est -O-, -CH₂-, -C(CH₃)₂-, -CF₂-, -NH-, -N(CH₃)-, ou 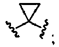
Le cycle A est un phényle, un naphtalényle ou un benzo[b]thiophényle ;
R₁ représente -OCH₃, -OCH₂CH₃, -OCH(CH₃)₂, -OCH₂CH(CH₃)₂, -OCHF₂, - OCH₂C(CH₃)₂OH, -OCH₂CH₂OCH₃, -OCH₂C(O)OCH₃, -OCH₂C(O)NH₂, -OCH₂C(O)N(CH₃)₂, - OCH₂C(O)N(CH₃)(CH₂CH₃), -OCH₂CH₂CH₂(phényle), -OCH₂C(O) (morpholinyle), - O(cycloalkyle en C₄₋₆), -O(hydroxycyclohexyle), -O(oxétanyl), -O(tétrahydrofuranyle), - O(tétrahydropyranyle), -O(oxépanyl), -O(phényle), -O(pyridinyle) ou - O(acétylpipéridinyle) ;
R₂ est F, CI, -CH₃, ou -OCH₃ ;
chaque R₃ est indépendamment un F, Cl, Br, -CH₃, -CD₃, -CHF₂, -CF₃, -OCH₃, ou cyclopropyle ;
m est égal à zéro ou 1 ; et
n est égal à zéro, 1, 2 ou 3.

2. Composé selon la revendication 1, ou ses stéréoisomères, tautomères ou sels, dans lequel
R est 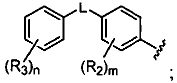 R₁ représente -OCH₃, -OCH₂CH₃, -OCH(CH₃)₂, -OCHF₂ou -O(phényle) ; et chaque R₃ est indépendamment un F, Cl, Br, -CH₃, ou -OCH₃.

3. Composé selon la revendication 1, ou ses stéréoisomères, tautomères ou sels, dans lequel le cycle A est un phényle ; et/ou L est -O-.

4. Composé selon la revendication 1, ou ses stéréoisomères, tautomères ou sels, dans lequel R₁ est -OCH₃, -OCH₂CH₃, ou -OCH(CH₃)₂.

5. Composé selon la revendication 1, ou ses stéréoisomères, tautomères ou sels, dans lequel le cycle A est un phényle ;
L est -O- ; et
R₁ est -OCH₃.

6. Composé selon la revendication 1, ou ses stéréoisomères ou tautomères, présentant la structure suivante : 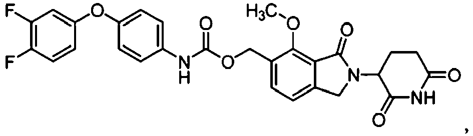 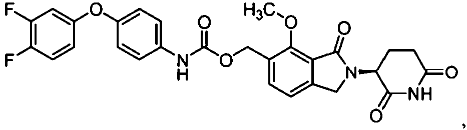 ou

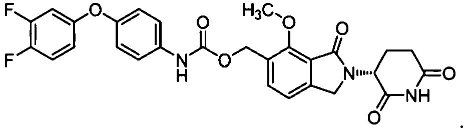

7. Composé selon la revendication 1, ou ses stéréoisomères ou tautomères, présentant la structure suivante : 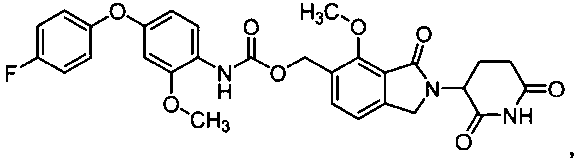 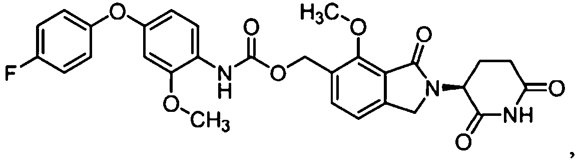 ou

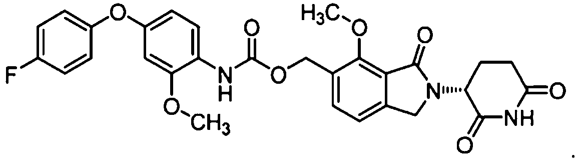

8. Composé selon la revendication 1, ou ses stéréoisomères ou tautomères, présentant la structure suivante : 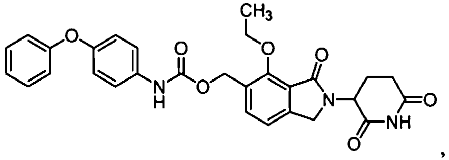 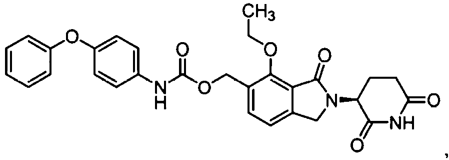 ou

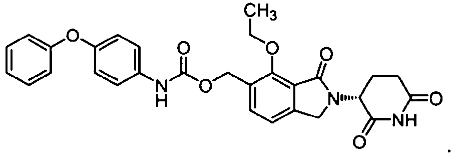

9. Composé selon la revendication 1, ou ses tautomères ou sels pharmaceutiquement acceptables, dans lequel ledit composé est un :
2-(2,6-dioxopipéridin-3-yl)-4-méthoxy-3-oxo-2,3-dihydro-1H-isoindol-5-yl]méthyle N-[4-(3,4-difluorophénoxy)phényl]carbamate (1) ;
[2-(2,6-dioxopipéridin-3-yl)-4-méthoxy-3-oxo-2,3-dihydro-1H-isoindol-5-yl]méthyle N-(4-phénoxyphényl)carbamate (2) ;
[2-(2,6-dioxopipéridin-3-yl)-4-méthoxy-3-oxo-2,3-dihydro-1H-isoindol-5-yl]méthyle N-[4-(3-chlorophénoxy)phényl]carbamate (3) ;
[2-(2,6-dioxopipéridin-3-yl)-4-méthoxy-3-oxo-2,3-dihydro-1H-isoindol-5-yl]méthyle N-[4-(3-Fluorophénoxy)phényl]carbamate (4) ;
[2-(2,6-dioxopipéridin-3-yl)-4-méthoxy-3-oxo-2,3-dihydro-1H-isoindol-5-yl]méthyle N-[4-(2,4-difluorophénoxy)phényl]carbamate (5) ;
[2-(2,6-dioxopipéridin-3-yl)-4-méthoxy-3-oxo-2,3-dihydro-1H-isoindol-5-yl]méthyle N-{4-[(3 -fluorophényl)méthyl]phényl} carbamate (6) ;
[2-(2,6-dioxopipéridin-3-yl)-4-méthoxy-3-oxo-2,3-dihydro-1H-isoindol-5-yl]méthyle N-[4-(1-phenylcyclopropyl)phenyl]carbamate (7);
[2-(2,6-dioxopipéridin-3-yl)-4-méthoxy-3-oxo-2,3-dihydro-1H-isoindol-5-yl]méthyle N-{4-[(3-fluorophényl)(méthyl)amino]phényl}carbamate (8) ;
[2-(2,6-dioxopipéridin-3-yl)-4-méthoxy-3-oxo-2,3-dihydro-1H-isoindol-5-yl]méthyle N-{4-[(3-fluorophényl)amino]phényl}carbamate (9) ;
[2-(2,6-dioxopipéridin-3-yl)-4-méthoxy-3-oxo-2,3-dihydro-1H-isoindol-5-yl]méthyle N-[4-(3-bromophénoxy)phényl]carbamate (10) ;
[2-(2,6-dioxopipéridin-3-yl)-4-méthoxy-3-oxo-2,3-dihydro-1H-isoindol-5-yl]méthyle N-[4-(3-méthylphénoxy)phényl]carbamate (11) ;
[2-(2,6-dioxopipéridin-3-yl)-4-méthoxy-3-oxo-2,3-dihydro-1H-isoindol-5-yl]méthyle N-[4-(3,5-diméthylphénoxy)phényl]carbamate (12) ;
[2-(2,6-dioxopipéridin-3-yl)-4-méthoxy-3-oxo-2,3-dihydro-1H-isoindol-5-yl]méthyle N-[4-(3,5-difluorophénoxy)phényl]carbamate (13) ;
[2-(2,6-dioxopipéridin-3-yl)-4-méthoxy-3-oxo-2,3-dihydro-1H-isoindol-5-yl]méthyle N-[4-(3-chloro-4-fluorophénoxy)phényl]carbamate (14) ;
[2-(2,6-dioxopipéridin-3-yl)-4-méthoxy-3-oxo-2,3-dihydro-1H-isoindol-5-yl]méthyle N-[2-fluoro-4-(3,4,5-trifluorophénoxy)phényl]carbamate (15) ;
[2-(2,6-dioxopipéridin-3-yl)-4-méthoxy-3-oxo-2,3-dihydro-1H-isoindol-5-yl]méthoxyN-[2-fluoro-4-(3-fluorophénoxy)phényl]carbamate (16) ;
[2-(2,6-dioxopipéridin-3-yl)-4-méthoxy-3-oxo-2,3-dihydro-1H-isoindol-5-yl]méthyle N-{4-[(3,4-difluorophényl)méthyl]phényl}carbamate (17) ;
[2-(2,6-dioxopipéridin-3-yl)-4-méthoxy-3-oxo-2,3-dihydro-1H-isoindol-5-yl]méthyle N-{4-[(3,4-difluorophényl)méthyl]-2-méthylphényl}carbamate (18) ;
[2-(2,6-dioxopipéridin-3-yl)-4-méthoxy-3-oxo-2,3-dihydro-1H-isoindol-5-yl]méthyle N-{4-[(3,4,5-trifluorophényl)méthyl]phényl}carbamate (19) ;
[2-(2,6-dioxopipéridin-3-yl)-4-méthoxy-3-oxo-2,3-dihydro-1H-isoindol-5-yl]méthyle N-{2-fluoro-4-[(3-fluorophényl)méthyl]phényl}carbamate (20) ;
[2-(2,6-dioxopipéridin-3-yl)-4-méthoxy-3-oxo-2,3-dihydro-1H-isoindol-5-yl]méthyle N-{4-[(3 -chloro-4-fluorophényl)méthyl]phényl}carbamate (21) ;
[2-(2,6-dioxopipéridin-3-yl)-4-méthoxy-3-oxo-2,3-dihydro-1H-isoindol-5-yl]méthyle N-[4-(2,3-difluorophénoxy)-2-fluorophényl]carbamate (22) ;
[2-(2,6-dioxopipéridin-3-yl)-4-méthoxy-3-oxo-2,3-dihydro-1H-isoindol-5-yl]méthyle N-[4-(3,4-difluorophénoxy)-2-fluorophényl]carbamate (23) ;
[2-(2,6-dioxopiperidin-3-yl)-4-méthoxy-3-oxo-2,3-dihydro-1H-isoindol-5-yl]méthyle N-[4-(3,5-difluorophénoxy)-2-fluorophényl]carbamate (24) ;
[2-(2,6-dioxopipéridin-3-yl)-4-méthoxy-3-oxo-2,3-dihydro-1H-isoindol-5-yl]méthyle N-[2-chloro-4-(3,4,5-trifluorophénoxy)phényl]carbamate (25) ;
[2-(2,6-dioxopipéridin-3-yl)-4-méthoxy-3-oxo-2,3-dihydro-1H-isoindol-5-yl]méthyle N-[4-(3-chlorophénoxy)-2-fluorophényl]carbamate (26) ;
[2-(2,6-dioxopipéridin-3-yl)-4-méthoxy-3-oxo-2,3-dihydro-1H-isoindol-5-yl]méthyle N-[2-chloro-4-(3-fluorophénoxy)phényl]carbamate (27) ;
[2-(2,6-dioxopipéridin-3-yl)-4-méthoxy-3-oxo-2,3-dihydro-1H-isoindol-5-yl]méthyle N-[2-fluoro-4-(4-fluorophénoxy)phényl]carbamate (28) ;
[2-(2,6-dioxopipéridin-3-yl)-4-méthoxy-3-oxo-2,3-dihydro-1H-isoindol-5-yl]méthyle N-{4-[difluoro(phényl)méthyl]phényl}carbamate (29) ;
[2-(2,6-dioxopipéridin-3-yl)-4-méthoxy-3-oxo-2,3-dihydro-1H-isoindol-5-yl]méthyle N-[4-(2,4-difluorophénoxy)-2-fluorophényl]carbamate (30) ;
[2-(2,6-dioxopipéridin-3-yl)-4-méthoxy-3-oxo-2,3-dihydro-1H-isoindol-5-yl]méthyle N-[4-(2-méthoxyphénoxy)phényl]carbamate (31) ;
[2-(2,6-dioxopipéridin-3-yl)-4-méthoxy-3-oxo-2,3-dihydro-1H-isoindol-5-yl]méthyle N-[4-(3-méthoxyphénoxy)phényl]carbamate (32) ;
[2-(2,6-dioxopipéridin-3-yl)-4-méthoxy-3-oxo-2,3-dihydro-1H-isoindol-5-yl]méthyle N-[4-(4,5-diAuoro-2-méthoxyphénoxy)-2-méthylphényl]carbamate (33) ;
[2-(2,6-dioxopipéridin-3-yl)-4-méthoxy-3-oxo-2,3-dihydro-1H-isoindol-5-yl]méthyle N-[2-méthoxy-4-(2,4,5-trifluorophénoxy)phényl]carbamate (34) ;
{2-[(3S)-2,6-dioxopipéridin-3-yl]-4-méthoxy-3-oxo-2,3-dihydro-1H-isoindol-5-yl}méthyle N-[4-(3,4-difluorophénoxy)phényl]carbamate (35) ;
{2-[(3R)-2,6-dioxopipéridin-3-yl]-4-méthoxy-3-oxo-2,3-dihydro-1H-isoindol-5-yl}méthyle N-[4-(3,4-difluorophénoxy)phényl]carbamate (36) ;
[2-(2,6-dioxopipéridin-3-yl)-4-méthoxy-3-oxo-2,3-dihydro-1H-isoindol-5-yl]méthyle N-[4-(4-fluorophénoxy)-2-méthoxyphényl]carbamate (37) ;
[2-(2,6-dioxopipéridin-3-yl)-4-méthoxy-3-oxo-2,3-dihydro-1H-isoindol-5-yl]méthyle N-[4-(3,4-difluorophénoxy)-2-méthylphényl]carbamate (38) ;
[2-(2,6-dioxopipéridin-3-yl)-4-méthoxy-3-oxo-2,3-dihydro-1H-isoindol-5-yl]méthyle N-[4-(3-fluorophénoxy)-2-méthylphényl]carbamate (39) ;
[2-(2,6-dioxopipéridin-3-yl)-4-méthoxy-3-oxo-2,3-dihydro-1H-isoindol-5-yl]méthyle N-[2-méthyl-4-(3,4,5-trifluorophénoxy)phényl]carbamate (40) ;
[2-(2,6-dioxopipéridin-3-yl)-4-méthoxy-3-oxo-2,3-dihydro-1H-isoindol-5-yl]méthyle N-[4-(2-phénylpropan-2-yl)phényl]carbamate (41) ;
[2-(2,6-dioxopipéridin-3-yl)-4-méthoxy-3-oxo-2,3-dihydro-1H-isoindol-5-yl]méthyle N-[4-(2,4-difluorophénoxy)-2-méthylphényl]carbamate (42) ;
[2-(2,6-dioxopipéridin-3-yl)-4-méthoxy-3-oxo-2,3-dihydro-1H-isoindol-5-yl]méthyle N-[2-fluoro-4-(2,4,5-trifluorophénoxy)phényl]carbamate (43) ;
[2-(2,6-dioxopipéridin-3-yl)-4-méthoxy-3-oxo-2,3-dihydro-1H-isoindol-5-yl]méthyle N-[4-(2,4,5-trifluorophénoxy)phényl]carbamate (44) ;
[2-(2,6-dioxopipéridin-3-yl)-4-méthoxy-3-oxo-2,3-dihydro-1H-isoindol-5-yl]méthyle N-[2-méthyl-4-(2,4,5-trifluorophénoxy)phényl]carbamate (45) ;
[2-(2,6-dioxopipéridin-3-yl)-4-méthoxy-3-oxo-2,3-dihydro-1H-isoindol-5-yl]méthyle N-[2-chloro-4-(3,4-difluorophénoxy)phényl]carbamate (46) ;
[2-(2,6-dioxopipéridin-3-yl)-4-méthoxy-3-oxo-2,3-dihydro-1H-isoindol-5-yl]méthyle N-[4-(3,4-difluorophénoxy)-2-méthoxyphényl]carbamate (47) ;
[2-(2,6-dioxopipéridin-3-yl)-4-méthoxy-3-oxo-2,3-dihydro-1H-isoindol-5-yl]méthyle N-[4-(3-fluorophénoxy)-2-méthoxyphényl]carbamate (48) ;
[2-(2,6-dioxopipéridin-3-yl)-4-méthoxy-3-oxo-2,3-dihydro-1H-isoindol-5-yl]méthyle N-[4-(2,4-difluorophénoxy)-2-méthoxyphényl]carbamate (49) ;
[2-(2,6-dioxopipéridin-3-yl)-4-méthoxy-3-oxo-2,3-dihydro-1H-isoindol-5-yl]méthyle N-[4-(4-fluoro-2-méthoxyphénoxy)-2-méthoxyphényl]carbamate (50) ;
[2-(2,6-dioxopipéridin-3-yl)-4-méthoxy-3-oxo-2,3-dihydro-1H-isoindol-5-yl]méthyle N-[4-(4-fluorophénoxy)-2-méthylphényl]carbamate (51) ;
[2-(2,6-dioxopipéridin-3-yl)-4-éthoxy-3-oxo-2,3-dihydro-1H-isoindol-5-yl]méthyle N-(4-phénoxyphényl)carbamate (52) ;
[2-(2,6-dioxopipéridin-3-yl)-4-éthoxy-3-oxo-2,3-dihydro-1H-isoindol-5-yl]méthyle N-[4-(3,4-difluorophénoxy)phényl]carbamate (53) ;
[2-(2,6-dioxopipéridin-3-yl)-4-éthoxy-3-oxo-2,3-dihydro-1H-isoindol-5-yl]méthyle N- [4-(3-chlorophénoxy)phényl]carbamate (54) ;
[2-(2,6-dioxopipéridin-3-yl)-4-éthoxy-3-oxo-2,3-dihydro-1H-isoindol-5-yl]méthyle N- [4-(3-fluorophénoxy)phényl] carbamate (55) ;
[2-(2,6-dioxopipéridin-3-yl)-3-oxo-4-(propan-2-yloxy)-2,3-dihydro-1H-isoindol-5-yl]méthyle N-[4-(3,4-difluorophénoxy)phényl]carbamate (56) ;
[2-(2,6-dioxopipéridin-3-yl)-3-oxo-4-(propan-2-yloxy)-2,3-dihydro-1H-isoindol-5-yl] méthyle N-(4-phénoxyphényl)carbamate (57) ;
[2-(2,6-dioxopipéridin-3-yl)-3-oxo-4-(propan-2-yloxy)-2,3-dihydro-1H-isoindol-5-yl] méthyle N-[4-(3-chlorophénoxy)phényl]carbamate (58) ;
[2-(2,6-dioxopipéridin-3-yl)-3-oxo-4-(propan-2-yloxy)-2,3-dihydro-1H-isoindol-5-yl] méthyle N-[4-(4-fluorophénoxy)phényl]carbamate (59) ;
[4-(difluorométhoxy)-2-(2,6-dioxopipéridin-3-yl)-3-oxo-2,3-dihydro-1H-isoindol-5-yl] méthyle N-(4-phénoxyphényl)carbamate (60) ;
[4-(difluorométhoxy)-2-(2,6-dioxopipéridin-3-yl)-3-oxo-2,3-dihydro-1H-isoindol-5-yl] méthyle N-[4-(3,4-difluorophénoxy)phényl]carbamate (61) ;
[2-(2,6-dioxopipéridin-3-yl)-3-oxo-4-phénoxy-2,3-dihydro-1H-isoindol-5-yl]méthyle N-(4-phénoxyphényl)carbamate (62) ;
[2-(2,6-dioxopipéridin-3-yl)-3-oxo-4-phénoxy-2,3-dihydro-1H-isoindol-5-yl]méthyle N-[4-(3,4-difluorophénoxy)phényl]carbamate (63) ;
[2-(2,6-dioxopipéridin-3-yl)-3-oxo-4-phénoxy-2,3-dihydro-1H-isoindol-5-yl]méthyle N-[4-(3-fluorophénoxy)phényl]carbamate (64) ;
[2-(2,6-dioxopipéridin-3-yl)-3-oxo-4-phénoxy-2,3-dihydro-1H-isoindol-5-yl]méthyle N-[4-(2-fluorophénoxy)phényl]carbamate (65) ;
[2-(2,6-dioxopipéridin-3-yl)-3-oxo-4-phénoxy-2,3-dihydro-1H-isoindol-5-yl]méthyle N-[4-(2-chlorophénoxy)phényl]carbamate (66) ;
[2-(2,6-dioxopipéridin-3-yl)-3-oxo-4-phénoxy-2,3-dihydro-1H-isoindol-5-yl]méthyle N-[4-(2,4-difluorophénoxy)phényl]carbamate (67) ;
[2-(2,6-dioxopipéridin-3-yl)-3-oxo-4-phénoxy-2,3-dihydro-1H-isoindol-5-yl]méthyle N-[4-(2,3-difluorophénoxy)phényl]carbamate (68) ;
[2-(2,6-dioxopipéridin-3-yl)-3-oxo-4-phénoxy-2,3-dihydro-1H-isoindol-5-yl]méthyle N-[4-(3,4-difluorophénoxy)-2-fluorophényl]carbamate (69) ;
[2-(2,6-dioxopipéridin-3-yl)-3-oxo-4-phénoxy-2,3-dihydro-1H-isoindol-5-yl]méthyle N-[4-(2-chloro-4-fluorophénoxy)phényl]carbamate (70) ;
[2-(2,6-dioxopipéridin-3-yl)-4-méthoxy-3-oxo-2,3-dihydro-1H-isoindol-5-yl]méthyle N-[4-(4-chloro-3-fluorophénoxy)phényl]carbamate (71) ;
[2-(2,6-dioxopipéridin-3-yl)-4-méthoxy-3-oxo-2,3-dihydro-1H-isoindol-5-yl]méthyle N-[2-méthoxy-4-(3,4,5-trifluorophénoxy)phényl]carbamate (72) ;
[2-(2,6-dioxopipéridin-3-yl)-4-méthoxy-3-oxo-2,3-dihydro-1H-isoindol-5-yl]méthyle N-[4-(4,5-difluoro-2-méthylphénoxy)-2-méthoxyphényl]carbamate (73) ;
[2-(2,6-dioxopipéridin-3-yl)-4-méthoxy-3-oxo-2,3-dihydro-1H-isoindol-5-yl]méthyle N-[4-(4,5-difluoro-2-méthylphénoxy)-2-méthylphényl]carbamate (74) ;
[2-(2,6-dioxopipéridin-3-yl)-4-méthoxy-3-oxo-2,3-dihydro-1H-isoindol-5-yl]méthyle N-[4-(4-fluoro-2-méthylphénoxy)-2-méthoxyphényl]carbamate (75) ;
[2-(2,6-dioxopipéridin-3-yl)-4-méthoxy-3-oxo-2,3-dihydro-1H-isoindol-5-yl]méthyle N-[4-(4-fluoro-2-méthylphénoxy)-2-méthylphényl]carbamate (76) ;
[2-(2,6-dioxopipéridin-3-yl)-4-méthoxy-3-oxo-2,3-dihydro-1H-isoindol-5-yl]méthyle N-[4-(3,4,5-trifluorophénoxy)phényl]carbamate (77) ;
[2-(2,6-dioxopipéridin-3-yl)-4-méthoxy-3-oxo-2,3-dihydro-1H-isoindol-5-yl]méthyle N- [4-(4,5 -difluoro-2-méthylphénoxy)phényl] carbamate (78) ;
[2-(2,6-dioxopipéridin-3-yl)-4-méthoxy-3-oxo-2,3-dihydro-1H-isoindol-5-yl]méthyle N-[4-(2-fluorophénoxy)phényl]carbamate (79) ;
[2-(2,6-dioxopipéridin-3-yl)-4-méthoxy-3-oxo-2,3-dihydro-1H-isoindol-5-yl]méthyle N-[4-(2-chlorophénoxy)phényl]carbamate (80) ;
[2-(2,6-dioxopipéridin-3-yl)-4-méthoxy-3-oxo-2,3-dihydro-1H-isoindol-5-yl]méthyle N-[4-(4-fluoro-2-méthylphénoxy)phényl]carbamate (81) ;
[2-(2,6-dioxopipéridin-3-yl)-4-méthoxy-3-oxo-2,3-dihydro-1H-isoindol-5-yl]méthyle N-[4-(4-fluoro-3-méthylphénoxy)-2-méthylphényl]carbamate (82) ;
[2-(2,6-dioxopipéridin-3-yl)-4-méthoxy-3-oxo-2,3-dihydro-1H-isoindol-5-yl]méthyle N-[4-(3-chloro-4-fluorophénoxy)-2-méthylphényl]carbamate (83) ;
[2-(2,6-dioxopipéridin-3-yl)-4-méthoxy-3-oxo-2,3-dihydro-1H-isoindol-5-yl]méthyle N-[4-(4-fluorophénoxy)phényl]carbamate (84) ;
[2-(2,6-dioxopipéridin-3-yl)-4-méthoxy-3-oxo-2,3-dihydro-1H-isoindol-5-yl]méthyle N-{4-[2-(difluorométhyl)phénoxy]phényl}carbamate (85) ;
[2-(2,6-dioxopiperidin-3-yl)-4-methoxy-3-oxo-2,3-dihydro-1H-isoindol-5-yl]méthyle N-{4-[2-(difluoromethyl)phenoxy]-2-fluorophenyl}carbamate (86);
[2-(2,6-dioxopipéridin-3-yl)-4-méthoxy-3-oxo-2,3-dihydro-1H-isoindol-5-yl]méthyle N-[4-(2-cyclopropylphénoxy)phényl]carbamate (87) ;
[2-(2,6-dioxopipéridin-3-yl)-4-méthoxy-3-oxo-2,3-dihydro-1H-isoindol-5-yl]méthyle N-{4-[2-(difluorométhyl)phénoxy]-2-méthoxyphényl}carbamate (88) ;
[2-(2,6-dioxopipéridin-3-yl)-4-méthoxy-3-oxo-2,3-dihydro-1H-isoindol-5-yl]méthyle N-{4-[2-(difluorométhyl)-4-fluorophénoxy]phényl}carbamate (89) ;
[2-(2,6-dioxopipéridin-3-yl)-4-méthoxy-3-oxo-2,3-dihydro-1H-isoindol-5-yl]méthyle N-{2-chloro-4-[2-(trifluorométhyl)phénoxy]phényl}carbamate (90) ;
[2-(2,6-dioxopipéridin-3-yl)-4-méthoxy-3-oxo-2,3-dihydro-1H-isoindol-5-yl]méthyle N-[4-(4-chloro-3-fluorophénoxy)-2-fluorophényl]carbamate (91) ;
[2-(2,6-dioxopipéridin-3-yl)-4-méthoxy-3-oxo-2,3-dihydro-1H-isoindol-5-yl]méthyle N-{4-[2-(2H3)méthylphénoxy]phényl}carbamate (92) ;
[2-(2,6-dioxopipéridin-3-yl)-4-méthoxy-3-oxo-2,3-dihydro-1H-isoindol-5-yl]méthyle N-(2-méthoxy-4-phénoxyphényl)carbamate (93) ;
[2-(2,6-dioxopipéridin-3-yl)-4-méthoxy-3-oxo-2,3-dihydro-1H-isoindol-5-yl]méthyle N-[4-(4,5-difluoro-2-méthylphénoxy)-2-fluorophényl]carbamate (94) ;
[2-(2,6-dioxopipéridin-3-yl)-4-méthoxy-3-oxo-2,3-dihydro-1H-isoindol-5-yl]méthyle N-[2-fluoro-4-(4-fluoro-2-méthylphénoxy)phényl]carbamate (95) ;
[2-(2,6-dioxopipéridin-3-yl)-4-méthoxy-3-oxo-2,3-dihydro-1H-isoindol-5-yl]méthyle N-[4-(4-fluoro-3-méthylphénoxy)phényl]carbamate (96) ;
[2-(2,6-dioxopipéridin-3-yl)-4-méthoxy-3-oxo-2,3-dihydro-1H-isoindol-5-yl]méthyle N-[4-(4-fluoro-3-méthylphénoxy)-2-méthoxyphényl]carbamate (97) ;
[2-(2,6-dioxopipéridin-3-yl)-4-méthoxy-3-oxo-2,3-dihydro-1H-isoindol-5-yl]méthyle N-[4-(3-chloro-4-fluorophénoxy)-2-fluorophényl]carbamate (98) ;
[2-(2,6-dioxopipéridin-3-yl)-4-méthoxy-3-oxo-2,3-dihydro-1H-isoindol-5-yl]méthyle N-[2-fluoro-4-(4-fluoro-3-méthylphénoxy)phényl]carbamate (99) ;
[2-(2,6-dioxopipéridin-3-yl)-4-méthoxy-3-oxo-2,3-dihydro-1H-isoindol-5-yl]méthyle N-[4-(3-chloro-4-fluorophénoxy)-2-methoxyphenyl]carbamate (100) ;
[2-(2,6-dioxopipéridin-3-yl)-4-méthoxy-3-oxo-2,3-dihydro-1H-isoindol-5-yl]méthyle N-[4-(2-méthylphénoxy)phényl]carbamate (101) ;
[2-(2,6-dioxopipéridin-3-yl)-4-méthoxy-3-oxo-2,3-dihydro-1H-isoindol-5-yl]méthyle N-[4-(2-chloro-4-fluorophénoxy)phenyl]carbamate (102);
[2-(2,6-dioxopipéridin-3-yl)-4-méthoxy-3-oxo-2,3-dihydro-1H-isoindol-5-yl]méthyle N-[4-(2-chloro-4-fluorophénoxy)-2-méthylphényl]carbamate (103) ;
[2-(2,6-dioxopipéridin-3-yl)-4-méthoxy-3-oxo-2,3-dihydro-1H-isoindol-5-yl]méthyle N-[4-(2-chloro-4-fluorophénoxy)-2-méthoxyphényl]carbamate (104) ;
[2-(2,6-dioxopipéridin-3-yl)-4-méthoxy-3-oxo-2,3-dihydro-1H-isoindol-5-yl]méthyle N-[4-(2-chloro-4-fluorophénoxy)-2-fluorophényl]carbamate (105) ;
[2-(2,6-dioxopipéridin-3-yl)-4-méthoxy-3-oxo-2,3-dihydro-1H-isoindol-5-yl]méthyle N-[4-(2-chloro-5-fluorophénoxy)phényl]carbamate (106) ;
[2-(2,6-dioxopipéridin-3-yl)-4-méthoxy-3-oxo-2,3-dihydro-1H-isoindol-5-yl]méthyle N-[4-(2-chlorophénoxy)-2-fluorophényl]carbamate (107) ;
[2-(2,6-dioxopipéridin-3-yl)-4-méthoxy-3-oxo-2,3-dihydro-1H-isoindol-5-yl]méthyle N-[4-(2-chloro-4,5-difluorophénoxy)-2-méthoxyphényl]carbamate (108) ;
[2-(2,6-dioxopipéridin-3-yl)-4-méthoxy-3-oxo-2,3-dihydro-1H-isoindol-5-yl]méthyle N-[4-(2-chloro-4,5-difluorophénoxy)-2-fluorophényl]carbamate (109) ;
[2-(2,6-dioxopipéridin-3-yl)-4-méthoxy-3-oxo-2,3-dihydro-1H-isoindol-5-yl]méthyle N-[4-(2,3-difluorophénoxy)phényl]carbamate (110) ;
[2-(2,6-dioxopipéridin-3-yl)-4-méthoxy-3-oxo-2,3-dihydro-1H-isoindol-5-yl]méthyle N-[4-(2-chloro-3-fluorophénoxy)phényl]carbamate (111) ;
[2-(2,6-dioxopipéridin-3-yl)-4-méthoxy-3-oxo-2,3-dihydro-1H-isoindol-5-yl]méthyle N-[4-(2,6-difluorophénoxy)phényl]carbamate (112) ;
[2-(2,6-dioxopipéridin-3-yl)-4-méthoxy-3-oxo-2,3-dihydro-1H-isoindol-5-yl]méthyle N-[4-(naphthalèn-1-yloxy)phényl]carbamate (113) ;
[2-(2,6-dioxopipéridin-3-yl)-4-méthoxy-3-oxo-2,3-dihydro-1H-isoindol-5-yl]méthyle N-[4-(1-benzothiophèn-5-yloxy)phényl]carbamate (114) ;
[2-(2,6-dioxopipéridin-3-yl)-4-méthoxy-3-oxo-2,3-dihydro-1H-isoindol-5-yl]méthyle N-[4-(1-benzothiophèn-6-yloxy)phényl]carbamate (115) ;
[2-(2,6-dioxopipéridin-3-yl)-4-méthoxy-3-oxo-2,3-dihydro-1H-isoindol-5-yl]méthyle N-[4-(2,3-dichlorophénoxy)-2-fluorophényl]carbamate (116) ;
[2-(2,6-dioxopipéridin-3-yl)-4-méthoxy-3-oxo-2,3-dihydro-1H-isoindol-5-yl]méthyle N-[4-(3,4-dichlorophénoxy)-2-fluorophényl]carbamate (117);
[2-(2,6-dioxopipéridin-3-yl)-4-méthoxy-3-oxo-2,3-dihydro-1H-isoindol-5-yl]méthyle N-[4-(3,5-dichlorophénoxy)-2-fluorophényl]carbamate (118) ;
[2-(2,6-dioxopipéridin-3-yl)-3-oxo-4-(propan-2-yloxy)-2,3-dihydro-1H-isoindol-5-yl]méthyle N-[4-(3,4-difluorophénoxy)-2-fluorophényl]carbamate (119) ;
[2-(2,6-dioxopipéridin-3-yl)-3-oxo-4-(propan-2-yloxy)-2,3-dihydro-1H-isoindol-5-yl] méthyle N-[4-(2,3-difluorophénoxy)phényl]carbamate (120) ;
[2-(2,6-dioxopipéridin-3-yl)-3-oxo-4-(propan-2-yloxy)-2,3-dihydro-1H-isoindol-5-yl] méthyle N-[4-(2-fluorophénoxy)phényl]carbamate (121) ;
[2-(2,6-dioxopipéridin-3-yl)-3-oxo-4-(propan-2-yloxy)-2,3-dihydro-1H-isoindol-5-yl] méthyle N-[4-(2-chlorophénoxy)phényl]carbamate (122) ;
[2-(2,6-dioxopipéridin-3-yl)-3-oxo-4-(propan-2-yloxy)-2,3-dihydro-1H-isoindol-5-yl] méthyle N-[4-(3-fluorophénoxy)phényl]carbamate (123) ;
[2-(2,6-dioxopipéridin-3-yl)-3-oxo-4-(propan-2-yloxy)-2,3-dihydro-1H-isoindol-5-yl] méthyle N-[4-(2-chloro-4-fluorophénoxy)phényl]carbamate (124) ;
{2-[(3S)-2,6-dioxopipéridin-3-yl]-3-oxo-4-(propan-2-yloxy)-2,3-dihydro-1H-isoindol-5-yl}méthyle N-[4-(3,4-difluorophénoxy)phényl]carbamate (125) ;
[4-cyclobutoxy-2-(2,6-dioxopipéridin-3-yl)-3-oxo-2,3-dihydro-1H-isoindol-5-yl]méthyle N-[4-(3,4-difluorophénoxy)phényl]carbamate (126) ;
[4-cyclobutoxy-2-(2,6-dioxopipéridin-3-yl)-3-oxo-2,3-dihydro-1H-isoindol-5-yl]méthyle N-[4-(2-chlorophénoxy)phényl]carbamate (127) ;
[4-cyclobutoxy-2-(2,6-dioxopipéridin-3-yl)-3-oxo-2,3-dihydro-1H-isoindol-5-yl]méthyle N-[4-(2-chloro-4-fluorophénoxy)phényl]carbamate (128) ;
[2-(2,6-dioxopipéridin-3-yl)-4-(oxétan-3-yloxy)-3-oxo-2,3-dihydro-1H-isoindol-5-yl] méthyle N-[4-(3,4-difluorophénoxy)phényl]carbamate (129) ;
[2-(2,6-dioxopipéridin-3-yl)-4-(oxan-4-yl oxy )-3-oxo-2,3-dihydro-1H-isoindol-5-yl] méthyle N-[4-(3,4-difluorophénoxy)phényl]carbamate (130) ;
[2-(2,6-di oxopipéridin-3-yl)-4-(oxan-4-yl oxy )-3 -oxo-2,3-dihydro-1H-isoindol-5-yl] méthyle N-[4-(2-chlorophénoxy)phényl]carbamate (131) ;
[2-(2,6-di oxopipéridin-3-yl)-4-(oxan-4-yl oxy )-3 -oxo-2,3-dihydro-1H-isoindol-5-yl] méthyle N-[4-(3,4-difluorophénoxy)-2-fluorophényl]carbamate (132) ;
[2-(2,6-dioxopipéridin-3-yl)-4-(oxan-4-yl oxy )-3-oxo-2,3-dihydro-1H-isoindol-5-yl] méthyle N-[4-(4-fluorophénoxy)phényl]carbamate (133) ;
[2-(2,6-dioxopipéridin-3-yl)-4-(oxan-4-yl oxy )-3-oxo-2,3-dihydro-1H-isoindol-5-yl] méthyle N-(4-phénoxyphényl)carbamate (134) ;
[2-(2,6-dioxopipéridin-3-yl)-4-(oxan-4-yl oxy )-3-oxo-2,3-dihydro-1H-isoindol-5-yl] méthyle N-[2-fluoro-4-(4-fluorophénoxy)phényl]carbamate (135) ;
[2-(2,6-dioxopipéridin-3-yl)-4-(oxan-4-yl oxy )-3-oxo-2,3-dihydro-1H-isoindol-5-yl] méthyle N-[4-(2,4-difluorophénoxy)-2-fluorophényl]carbamate (136) ;
[4-(cyclohexyloxy)-2-(2,6-dioxopipéridin-3-yl)-3-oxo-2,3-dihydro-1H-isoindol-5-yl] méthyle N-[4-(3,4-difluorophénoxy)phényl]carbamate (137) ;
[2-(2,6-dioxopipéridin-3-yl)-4-(2-méthylpropoxy)-3-oxo-2,3-dihydro-1H-isoindol-5-yl] méthyle N-[4-(3,4-difluorophénoxy)phenyl]carbamate (138) ;
[2-(2,6-dioxopipéridin-3-yl)-4-(oxan-4-yl oxy)-3-oxo-2,3-dihydro-1H-isoindol-5-yl] méthyle N-[4-(2,4-difluorophénoxy)phényl]carbamate (139) ;
[2-(2,6-dioxopipéridin-3-yl)-4-(oxan-4-yl oxy)-3-oxo-2,3-dihydro-1H-isoindol-5-yl] méthyle N-[4-(3,5-difluorophénoxy)-2-fluorophényl]carbamate (140) ;
[2-(2,6-dioxopipéridin-3-yl)-4-[(3R)-oxan-3-yloxy]-3-oxo-2,3-dihydro-1H-isoindol-5-yl] méthyle N-[4-(3,4-difluorophénoxy)phényl]carbamate (141) ;
[2-(2,6-dioxopipéridin-3-yl)-4-[(3R)-oxan-3-yloxy]-3-oxo-2,3-dihydro-1H-isoindol-5-yl] méthyle N-(4-phénoxyphényl)carbamate (142) ;
[2-(2,6-dioxopipéridin-3-yl)-4-[(3R)-oxan-3-yloxy]-3-oxo-2,3-dihydro-1H-isoindol-5-yl] méthyle N-[4-(3-fluorophénoxy)phényl]carbamate (143) ;
[2-(2,6-dioxopipéridin-3-yl)-4-[(3R)-oxan-3-yloxy]-3-oxo-2,3-dihydro-1H-isoindol-5-yl] méthyle N-[4-(4-fluorophénoxy)phényl]carbamate (144) ;
[2-(2,6-dioxopipéridin-3-yl)-4-[(3R)-oxan-3-yloxy]-3-oxo-2,3-dihydro-1H-isoindol-5-yl] méthyle N-[4-(2,4-difluorophénoxy)phényl]carbamate (145) ;
[2-(2,6-dioxopipéridin-3-yl)-4-[(3R)-oxan-3-yloxy]-3-oxo-2,3-dihydro-1H-isoindol-5-yl] méthyle N-[4-(3,4-difluorophénoxy)-2-fluorophényl]carbamate (146) ;
[2-(2,6-dioxopipéridin-3-yl)-4-[(3R)-oxan-3-yloxy]-3-oxo-2,3-dihydro-1H-isoindol-5-yl] méthyle N-[2-fluoro-4-(4-fluorophénoxy)phényl]carbamate (147) ;
[2-(2,6-dioxopipéridin-3-yl)-4-[(3R)-oxan-3-yloxy]-3-oxo-2,3-dihydro-1H-isoindol-5-yl] méthyle N-[4-(2,4-difluorophénoxy)-2-fluorophényl]carbamate (148) ;
[2-(2,6-dioxopipéridin-3-yl)-4-[(3R)-oxan-3-yloxy]-3-oxo-2,3-dihydro-1H-isoindol-5-yl] méthyle N-[4-(3,5-difluorophénoxy)-2-fluorophényl]carbamate (149) ;
[2-(2,6-dioxopipéridin-3-yl)-4-[(3R)-oxan-3-yloxy]-3-oxo-2,3-dihydro-1H-isoindol-5-yl] méthyle N-[2-fluoro-4-(3,4,5-trifluorophénoxy)phényl]carbamate (150) ;
[4-(cyclopentyloxy)-2-(2,6-dioxopipéridin-3-yl)-3-oxo-2,3-dihydro-1H-isoindol-5-yl] méthyle N-[4-(3,4-difluorophénoxy)phényl]carbamate (151) ;
[2-(2,6-dioxopipéridin-3-yl)-3-oxo-4-[(3S)-oxolan-3-yloxy]-2,3-dihydro-1H-isoindol-5-yl]méthyle N-[4-(3,4-difluorophénoxy)phényl]carbamate (152) ;
[2-(2,6-dioxopipéridin-3-yl)-3-oxo-4-[(3R)-oxolan-3-yloxy]-2,3-dihydro-1H-isoindol-5-yl]méthyle N-[4-(3,4-difluorophénoxy)phényl]carbamate (153) ;
[2-(2,6-dioxopipéridin-3-yl)-3-oxo-4-[(3R)-oxolan-3-yloxy]-2,3-dihydro-1H-isoindol-5-yl]méthyle N-[4-(3,4-difluorophénoxy)-2-fluorophényl]carbamate (154) ;
[2-(2,6-dioxopipéridin-3-yl)-3-oxo-4-[(3R)-oxolan-3-yloxy]-2,3-dihydro-1H-isoindol-5-yl]méthyle N-[4-(2-chloro-4-fluorophénoxy)phényl]carbamate (155) ;
[2-(2,6-dioxopipéridin-3-yl)-3-oxo-4-[(3R)-oxolan-3-yloxy]-2,3-dihydro-1H-isoindol-5-yl]méthyle N-[4-(2,3-difluorophénoxy)phényl]carbamate (156) ;
[2-(2,6-dioxopipéridin-3-yl)-3-oxo-4-[(3R)-oxolan-3-yloxy]-2,3-dihydro-1H-isoindol-5-yl]méthyle N-[4-(2,4-difluorophénoxy)phényl]carbamate (157) ;
[2-(2,6-dioxopipéridin-3-yl)-3-oxo-4-[(3R)-oxolan-3-yloxy]-2,3-dihydro-1H-isoindol-5-yl]méthyle N-[4-(3-chlorophénoxy)phényl]carbamate (158) ;
[2-(2,6-dioxopipéridin-3-yl)-4-(2-hydroxy-2-méthylpropoxy)-3-oxo-2,3-dihydro-1H-isoindol-5-yl]méthyle N-[4-(3,4-difluorophénoxy)phényl]carbamate (159) ;
[2-(2,6-dioxopipéridin-3-yl)-4-(2-hydroxy-2-méthylpropoxy)-3-oxo-2,3-dihydro-1H-isoindol-5-yl]méthyle N-(4-phénoxyphényl)carbamate (160) ;
[2-(2,6-dioxopipéridin-3-yl)-4-(2-hydroxy-2-méthylpropoxy)-3-oxo-2,3-dihydro-1H-isoindol-5-yl]méthyle N-[4-(3,4-difluorophénoxy)-2-fluorophényl]carbamate (161) ;
[2-(2,6-dioxopipéridin-3-yl)-4-(2-hydroxy-2-méthylpropoxy)-3-oxo-2,3-dihydro-1H-isoindol-5-yl]méthyle N-[2-fluoro-4-(4-fluorophénoxy)phényl]carbamate (162) ;
[2-(2,6-dioxopipéridin-3-yl)-4-(2-hydroxy-2-méthylpropoxy)-3-oxo-2,3-dihydro-1H-isoindol-5-yl]méthyle N-[4-(2,4-difluorophénoxy)-2-fluorophényl]carbamate (163) ;
[2-(2,6-dioxopipéridin-3-yl)-4-(2-hydroxy-2-méthylpropoxy)-3-oxo-2,3-dihydro-1H-isoindol-5-yl]méthyle N-[4-(3,5-difluorophénoxy)-2-fluorophényl]carbamate (164) ;
[2-(2,6-dioxopipéridin-3-yl)-4-(2-hydroxy-2-méthylpropoxy)-3-oxo-2,3-dihydro-1H-isoindol-5-yl]méthyle N-[2-fluoro-4-(3,4,5-trifluorophénoxy)phényl]carbamate (165) ;
[2-(2,6-dioxopipéridin-3-yl)-3-oxo-4-{[(1r,4r)-4-hydroxycyclohexyl]oxy}-2,3-dihydro-1H-isoindol-5-yl]méthyle N-[4-(3,4-difluorophénoxy)phényl]carbamate (166) ;
[2-(2,6-dioxopipéridin-3-yl)-3-oxo-4-{[(1s,4s)-4-hydroxycyclohexyl]oxy}-2,3-dihydro-1H-isoindol-5-yl]méthyle N-[4-(3,4-difluorophénoxy)phényl]carbamate (167) ;
[2-(2,6-dioxopipéridin-3-yl)-4-(2-méthoxyéthoxy )-3-oxo-2,3-dihydro-1H-isoindol-5-yl] méthyle N-[4-(3,4-difluorophénoxy)phényl]carbamate (168) ;
[2-(2,6-dioxopipéridin-3-yl)-4-(2-méthoxyéthoxy)-3-oxo-2,3-dihydro-1H-isoindol-5-yl] méthyle N-[4-(3,4-difluorophénoxy)-2-fluorophényl]carbamate (169) ;
[2-(2,6-dioxopipéridin-3-yl)-4-(oxépan-4-yloxy)-3-oxo-2,3-dihydro-1H-isoindol-5-yl] méthyle N-[4-(3,4-difluorophénoxy)phényl]carbamate (170) ;
[2-(2,6-dioxopipéridin-3-yl)-3-oxo-4-(pyridin-3-yloxy)-2,3-dihydro-1H-isoindol-5-yl] méthyle N-(4-phénoxyphényl)carbamate (171) ;
[2-(2,6-dioxopipéridin-3-yl)-3-oxo-4-(pyridin-3-yloxy)-2,3-dihydro-1H-isoindol-5-yl] méthyle N-[4-(3,4-difluorophénoxy)phényl]carbamate (172) ;
{2-[(3S)-2,6-dioxopipéridin-3-yl]-3-oxo-4-(3-phénylpropoxy)-2,3-dihydro-1H-isoindol-5-yl}méthyle N-[4-(3,4-difluorophénoxy)phényl]carbamate (173) ;
2-({5-[({[4-(3,4-difluorophénoxy)phényl]carbamoyl}oxy)méthyl]-2-(2,6-dioxopipéridin-3-yl)-3 -oxo-2,3 -dihydro-1 H-isoindol-4-yl} oxy )acétate de méthyle (174) ;
{4-[(diméthylcarbamoyl)méthoxy]-2-(2,6-dioxopipéridin-3-yl)-3-oxo-2,3-dihydro-1H-isoindol-5-yl}méthyle N-[4-(3,4-difluorophénoxy)phényl]carbamate (175) ;
[4-(carbamoylméthoxy)-2-(2,6-dioxopipéridin-3-yl)-3-oxo-2,3-dihydro-1H-isoindol-5-yl] méthyle N-[4-(3,4-difluorophénoxy)phényl]carbamate (176) ;
[2-(2,6-dioxopipéridin-3-yl)-4-{[éthyl(méthyl)carbamoyl]méthoxy }-3-oxo-2,3-dihydro-1H-isoindol-5-yl]méthyl N-[4-(3,4-difluorophénoxy)phényl] carbamate (177) ;
[2-(2,6-dioxopipéridin-3-yl)-4-[2-(morpholin-4-yl)-2-oxoéthoxy]-3-oxo-2,3-dihydro-1H-isoindol-5-yl]méthyle N-[4-(3,4-difluorophénoxy)phényl]carbamate (178) ;
{4-[(I-acetylpipéridin-4-yl)oxy]-2-(2,6-dioxopipéridin-3-yl)-3-oxo-2,3-dihydro-1H-isoindol-5-yl}méthyle N-[4-(3,4-difluorophénoxy)phényl]carbamate (179) ; ou
{2-[(3S)-2,6-dioxo(3,4,4,5,5-2H5)pipéridin-3-yl]-4-méthoxy-3-oxo-2,3-dihydro-1H-isoindol-5-yl}méthyle N-[4-(3,4-difluorophénoxy)phényl]carbamate (180).

10. Composition pharmaceutique comprenant un composé selon l'une quelconque des revendications précédentes ou des tautomères ou un sel pharmaceutiquement acceptable de celui-ci ; et un support pharmaceutiquement acceptable.

11. Composé, un tautomère ou un sel pharmaceutiquement acceptable de celui-ci selon l'une quelconque des revendications précédentes, destiné à être utilisé dans une méthode de traitement du cancer, chez un patient, consistant à administrer audit patient une quantité thérapeutiquement efficace d'un composé.

12. Composé, son tautomère ou son sel pharmaceutiquement acceptable pour utilisation selon la revendication 11, dans lequel ledit cancer est choisi parmi le cancer du côlon, de l'estomac, du pancréas, du sein, de la prostate, du poumon, de l'ovaire, du col de l'utérus, du rein, de la tête et du cou, le lymphome, la leucémie et le mélanome.

13. Composé, son tautomère ou son sel pharmaceutiquement acceptable, destiné à être utilisé selon la revendication 11, comprenant en outre l'administration audit patient d'une quantité thérapeutiquement efficace d'un deuxième agent, ledit deuxième agent étant choisi parmi un antagoniste de l'axe PD1/PD-L1, un antagoniste de CTLA4, un agent chimiothérapeutique, une radiothérapie ou un vaccin antitumoral, avant, simultanément ou après l'administration dudit composé.

14. Composé, son tautomère ou son sel pharmaceutiquement acceptable pour une utilisation selon la revendication 11, dans lequel ledit composé diminue le niveau de la protéine Cycline E1 et dans lequel ladite Cycline E1 est la séquence d'acides aminés codée par les numéros d'identification SEQ: 1, 2, 3, 4, 5, 6, 7, 8 ou 9.

15. Composé, son tautomère ou son sel pharmaceutiquement acceptable, destiné à être utilisé selon la revendication 14, dans lequel le taux de protéine cycline E1 est diminué d'au moins 50 %.
